# EUROPEAN PATENT APPLICATION

(11) **EP 4 105 218 A1**
(43) Date of publication of application: **21.12.2022**
(21) Application number: 21315095.6
(22) Date of filing: 15.06.2021
(51) Int. Cl.: C07D 487/04, C07B 59/00, A61K 31/519, A61P 25/16, A61P 25/28

(54) **SUBSTITUTED PYRROLO[2,3-D] PYRIMIDINES, THEIR PREPARATION AND THEIR THERAPEUTIC APPLICATION**

(71) Applicant: SANOFI, 75008 Paris (FR)
(72) Inventor: The designation of the inventor has not yet been filed
(74) Representative: Sanofi

(57) **Abstract**

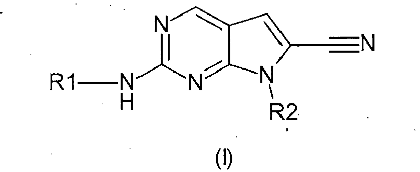

Disclosed are compounds of formula (I), or a pharmaceutically acceptable salt thereof, wherein R1 and R2 are defined herein. Also disclosed are methods of using such compounds as inhibitors of LRRK2, and pharmaceutical compositions comprising such compounds. These compounds are useful in treating neurodegenerative diseases such as Parkinson's disease,

## Description

Disclosed herein are substituted pyrrolo[2,3-d]pyrimidine compounds, processes for their preparation, pharmaceutical compositions containing the compounds, as well as therapeutic uses thereof.

### BACKGROUND

Parkinson's disease (PD) is an age-dependent neurodegenerative disorder with high unmet medical need, in the context of an aging population. Mutations in several genes segregate PD in families. Among them, seven leucine rich repeat kinase 2 (LRRK2) mutations are linked to autosomal dominant forms of PD. LRRK2 polymorphs were identified as risk factors for sporadic PD in Genome Wide Association Studies (J.H. Kluss Biochemical Society Transactions 2019). LRRK2 carriers share similar clinical symptoms, disease onset and progression with sporadic patients (H. Tomiyama, Hum Mov Disord. 2006) suggesting that LRRK2 signaling pathways may be central to the processes underlying both LRRK2 familial and sporadic late onset PD. All pathogenic LRRK2 mutations, as well as VPS35 D620N mutation which is another target genetically linked to PD, induce increased LRRK2 kinase activity (M. Steger et al., eLife 2016, R, Mir et al., Biochem J. 2018). Beyond familial PD, increased LRRK2 activity or level was reported in human brains from idiopathic PD patients (R. Di Maio et al., Sci. Transl. Med. 2018).These results support the hypothesis that dysregulated LRRK2 kinase activity may contribute to pathogenesis, suggesting the therapeutic potential of LRRK2 kinase inhibitors to block aberrant LRRK2-dependent signaling in both LRRK2 and idiopathic form of PD (A.B. West Exp. Neurol. 2017). Accumulation of synuclein aggregates and loss of dopaminergic neurons are the main hallmarks of PD. Blockade of these phenotypes after LRRK2 kinase inhibitor treatment was demonstrated in numerous reports (E.M. Rocha et al Neurobiol. Of Disease 2019). These results support the hypothesis that potent, brain penetrant LRRK2 kinase inhibitors have therapeutic potential for the treatment of PD.

A growing body of evidence suggests a role of LRRK2 in the regulation of lysosomal activity (J. Schapansky et al, Neurobiol. of Disease 2018). Increased bis(monoacylglycerol)phosphate levels, a marker of lysosomal storage diseases such as Pick's disease, was observed in fluids from LRRK2 gain of function mutations carriers (R.N. Alcalay, Movement Disorders, 2020). Lysosomal glucocerebrosidase (GBA) mutations are the largest risk factor for development of PD (GBA-PD). Decreased glucocerebrosidase activity was reported in neurons from GBA and LRRK2 mutation carriers (D. Ysselstein, Nature com. 2019). Conversely, normalization of glucocerebrosidase activity and level were achieved in vitro and in vivo after treatment with LRRK2 kinase inhibitors, suggesting a potential benefit in patients from lysosomal storage diseases such a GBA-PD (A. Sanyal et al, Mov. Disorders 2020).

Immunofluorescence experiments in human brain showed colocalization of LRRK2 with neurofibrillary tangles (J. Miklossy, J Neuropathol Exp Neurol. 2006). Moreover, LRRK2 has been reported to phosphorylate tubulin-associated Tau (F. Kawakami et al. PloS One 2012), and Tau hyperphosphorylation was observed in LRRK2 kinase activating mutant transgenic mice (Y. Li et al. Nat. Neurosci. 2009). These data indicate that LRRK2 kinase inhibitor treatment might be useful in treating tauopathy disorders such as Pick's disease, progressive supranuclear palsy and frontotemporal dementia.

LRRK2 is expressed in brain glial cells, and attenuation of neuroinflammation was achieved after LRRK2 kinase inhibitor treatment in various in vivo models (M.S. Moehle et al. J. Neurosci. 2012). Neuroinflammation is often observed in neurodegenerative diseases such as Parkinson's disease, Alzheimer disease, multiple sclerosis, HIV-induced dementia and Amyotrophic lateral sclerosis; LRRK2 kinase inhibitors may therefore have utility in the treatment of these pathologies.

### SUMMARY OF THE INVENTION

In accordance with one aspect, disclosed herein are compounds of Formula (I): 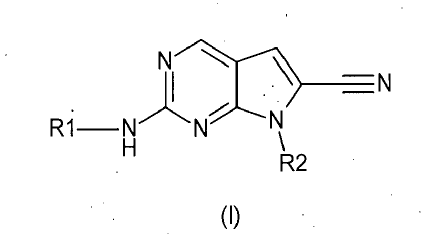 wherein:
R1 is selected from the group consisting of an aryl group, an ortho-fused bicyclic heteroaryl group and a heteroaryl group, wherein said ortho-fused bicyclic heteroaryl group is unsubstituted or substituted with one or more -(C₁-C₃)-alkyl groups; and wherein said aryl and heteroaryl groups are unsubstituted or substituted with one or more substituents independently selected from the group consisting of
   a) a fluorine atom,
   b) a deuterium atom,
   c) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
   d) a cycloalkyl group which is unsubstituted or substituted with one or more fluorine atom, or lower alkyl group,
   e) a heterocycloalkyl group which is unsubstituted or substituted with one or more substituents independently selected from an alkyl group, an alkyloxyl group, and an alkylcarbonyl group,
   f) an alkyloxyl group which is unsubstituted or substituted with one or more substituents independently selected from the group consisting of a hydroxy group and a fluorine atom,
   g) an -O-cycloalkyl group which is unsubstituted or substituted with one or more fluorine atom or lower alkyl group,
   h) an -O-heterocycloalkyl group which is unsubstituted or substituted with one or more substituents independently selected from the group consisting of an alkyl group, an alkyloxyl group, and an alkylcarbonyl group,
   i) an -O-spirocycle group,
   j) an alkylsulfonylalkyl group, and
   k) an alkylsulfonyl group; and
R2 is selected from the group consisting an alkyloxylalkyl group and a heterocycloalkyl group, wherein said heterocycloalkyl group represented by R2 is attached via a carbon atom and is unsubstituted or substituted with an alkyl group, an alkyloxyl group or one or more fluorine atom;
or a pharmaceutically acceptable salt thereof.

Another aspect of the present disclosure is a pharmaceutical composition comprising a pharmaceutically effective amount of a compound of Formula (I), or a pharmaceutically acceptable salt thereof, and a pharmaceutically acceptable excipient.

The compounds of Formula (I) above and pharmaceutically acceptable salts thereof exhibit inhibitory activity against wild type and mutant LRRK2 and are useful in the treatment of neurodegenerative diseases.

### DESCRIPTION

As used herein, the following abbreviations, unless otherwise indicated, shall be understood to have the following meanings:
As used herein, the term "alkyl" means a straight or branched aliphatic hydrocarbon group having 1 to 6 carbon atoms in the chain. In another aspect, an alkyl has 1 to 4 carbon atoms in the chain. "Lower alkyl" means an alkyl group having 1 to about 3 carbon atoms in an alkyl chain that may be straight or branched. Branched means that one or more lower alkyl groups, such as methyl, ethyl or propyl, are attached to a linear alkyl chain. Additionally, the term "(C₁-C₄)-alkyl" denotes a straight or branched alkyl group having one to four carbon atoms. The term "(C₁-C₃)-alkyl" denotes a straight or branched alkyl group having one to three carbon atoms. Exemplary alkyl includes methyl, ethyl, *i*-propyl, *t*-butyl, and the like.

As used herein, the term "alkylamino" means an alkyl-N(H)- wherein the alkyl is as herein defined.

As used herein, the term "dialkylamino" means an amino group having two linear or branched alkyl groups as defined herein, which are independent from each other. The term "dialkylamino" comprises for example: dimethylamino, diethylamino, N-ethyl-N-methylamino, N-methyl-N-n-propylamino,N-isopropyl-N-n-propylamino, N-t-butyl-N-methylamino.

As used herein, the term "alkylcarbonyl" means an alkyl-C(=O)- wherein the alkyl is as herein defined. Exemplary alkylcarbonyl include methylcarbonyl, ethylcarbonyl, propylcarbonyl, isopropylcarbonyl, butylcarbonyl and isobutylcarbonyl.

As used herein, the term "alkyloxyl" means an alkyl-O- wherein alkyl is as herein described. Examples of alkyl-O- are methoxyl, ethoxyl, n-propoxyl, isopropoxyl, n-butoxyl, isobutoxyl, sec-butoxyl, tert-butoxyl and the like.

As used herein, the term "alkyloxylalkyl" means an alkyl-O-alkyl- group having two linear or branched alkyl groups as defined herein, which are independent from each other.

As used herein, the term "alkylsulfonyl" means an alkyl-S(=O)2- wherein the alkyl is as herein defined. Exemplary alkylsulfonyl include methylsulfonyl, ethylsulfonyl, propylsulfonyl, isopropylsulfonyl, butylsulfonyl and isobutylsulfonyl.

As used herein, the term "alkylsulfonylalkyl" means an alkyl-S(=O)2-alkyl- wherein the alkyl is as herein defined.

As used herein, the term "aryl" means an aromatic monocyclic or bicyclic ring system of about 5 to about 10 carbon atoms. Exemplary aryl include phenyl and naphthyl.

As used herein, the term "cycloalkyl" means a non-aromatic monocyclic ring system of 3 to 6 carbon atoms. A (C3-C6)-cycloalkyl is a cycloalkyl with 3, 4, 5 or 6 ring carbon atoms. Examples of cycloalkyl are cyclopropyl, cyclobutyl, cyclopentyl and cyclohexyl.

As used herein, the term "heteroaryl" whether used alone or with other terms, such as "heteroaryl group", means a cyclic aromatic group containing 2 to 10 carbon atoms and containing between 1 and 4 heteroatoms, such as nitrogen, oxygen or sulfur. Said heteroaryl group may be monocyclic or bicyclic. As used herein, the term "monocyclic heteroaryl" means a cyclic aromatic group containing 2 to 5 carbon atoms and containing between 1 and 3 heteroatoms, such as nitrogen, oxygen or sulfur. By way of examples of monocyclic heteroaryl groups, mention may be made of, but not limited to: benzimidazole, benzothiazole, benzothiadiazole, benzofuran, benzotriazole, benzoxazole, furan, furazan, indole, imidazole, isoxazole, isothiazole, oxadiazole, oxazole, pyridine, pyrimidine, pyrrolo[2,3-b]pyridine, pyrazine, pyrazole, pyridazine, pyrrole, 1,2,4-thiadiazole, 1,2,4-triazine, 1,3,4-thiadiazole, thiazole, triazole, thiophene and the like.

As used herein, the term "heterocycloalkyl" means a 4-, 5-, 6-, or 7-membered non-aromatic monocyclic ring system having at least one carbon atom and at least one heteroatom other than carbon, for example nitrogen, oxygen or sulfur. Examples of a heterocycloalkyl include azetidinyl, oxetanyl, thietanyl, diazetidinyl, dioxetanyl, dithietanyl, pyrrolidinyl, tetrahydrofuranyl, tetrahydrothiophenyl, imidazolidinyl, pyrazolidinyl, oxazolidinyl, isoxazolidinyl, thiazolidinyl, isothiazolidinyl, dioxolanyl, dioxepanyl, dithiolanyl, piperidinyl, tetrahydropyranyl, thianyl, piperazinyl, morpholinyl, thiomorpholinyl, dioxanyl, dithianyl, hexahydro-1,3,5-triazinyl, trioxanyl, trithianyl, azepanyl, oxepanyl, thiepanyl, and diazepanyl.

As used herein, the term "ortho-fused" means a ring system where the two adjacent rings have two adjacent atoms in common and where the second ring system is alpha to the branching carbon atom. The term "ortho-fused heteroaryl" means a bicyclic ring system comprising 7 to 10 carbon atoms and comprising from 1 to 4 heteroatoms independently selected from oxygen, nitrogen and sulfur. Included within the scope of the definition of ortho-fused heteroaryl group is a bicyclic ring system wherein one of the rings is monocyclic heteroaryl and the other ring is aryl ring or heterocycloalkyl ring or both rings are monocyclic heteroaryl. Examples include indolyl and benzimidazolyl.

As used herein, the term "Spirocycle" or "spirocyclic" means carbogenic bicyclic ring systems with both rings connected through a single atom. The ring can be different in size and nature, or identical in size and nature. Examples include spiropentane, spirohexane, spiroheptane, spirooctane, spirononane, or spirodecane. One or both of the rings in a spirocycle can be fused to another carbocyclic, heterocyclic, aromatic, or heteroaromatic ring. One or more of the carbon atoms in the spirocycle can be substituted with a heteroatom (e.g., O, N, S, or P). A C₅-C₁₂ spirocycle is a spirocycle containing between 5 and 12 carbon atoms. One or more of the carbon atoms can be substituted with a heteroatom.

As used herein, the term "substituted" means that a hydrogen radical of the designated moiety is replaced with the radical of a specified substituent, provided that the substitution results in a stable or chemically feasible compound. Unless otherwise noted, a substituent can be in any position, provided that the respective compound is sufficiently stable and is suitable as a pharmaceutical active compound. The prerequisite that a specific group and a compound of the formula I are sufficiently stable and suitable as a pharmaceutical active compound applies in general with respect to the definitions of all groups in the compounds of the formula I.

As used herein, the term "one or more substituents" refers to a number of substituents that equals from one to the maximum number of substituents possible based on the number of available bonding sites, provided that the above conditions of stability and chemical feasibility are met. Unless otherwise indicated, an optionally substituted group may have a substituent at each substitutable position of the group, and the substituents may be either the same or different.

As used herein, the terms "independently" or "independently selected" means that the same or different values may be selected for multiple instances of a given variable in a single compound.

As used herein, the term "unsubstituted" indicates that the respective group does not carry any of the specified substituents.

As used herein, the term "pharmaceutically acceptable salt" refers to the relatively non-toxic, inorganic and organic acid addition salts, and base addition salts, of a compound of Formula (I). These salts can be prepared *in situ* during the final isolation and purification of the compounds.

If the compounds of the formula (I) comprise one or more acidic or basic groups, for example basic heterocyclic groups, the corresponding physiologically or toxicologically acceptable salts are also included in the disclosure, especially the pharmaceutically acceptable salts. The compounds of the formula (I) may thus be deprotonated on an acidic group and be used for example as alkali metal salts or as ammonium salts. Compounds of the formula (I) comprising at least one basic group may also be prepared and used in the form of their acid addition salts, for example in the form of pharmaceutically acceptable salts with inorganic acids and organic acids. Salts can in general be prepared from acidic and basic compounds of the formula (I) by reaction with an acid or base in a solvent or diluent according to customary procedures. If the compounds of the formula (I) simultaneously contain an acidic and a basic group in the molecule, the disclosure also includes internal salts (betaines, zwitterions) in addition to the salt forms mentioned. The present disclosure also comprises all salts of the compounds of the formula (I) which, because of low physiological tolerability, are not directly suitable for use as a pharmaceutical, but are suitable as intermediates for chemical reactions or for the preparation of physiologically acceptable salts, for example by means of anion exchange or cation exchange.

As used herein, the term "Pharmaceutically acceptable excipient" refers to a non-toxic solvent, dispersant, excipient, adjuvant, or other material which is mixed with the compound of the present disclosure in order to permit the formation of a pharmaceutical composition, i.e., a dosage form capable of administration to the patient. The said excipients are selected, in accordance with the pharmaceutical form and method of administration desired, from the customary excipients, which are known to a person skilled in the art.

As used herein, the term "Pharmaceutically effective amount" or "therapeutically effective amount" means an amount of a compound/composition according to the present disclosure effective in producing the desired therapeutic effect.

As used herein, the term "treating" or "treatment" means to arrest, slow down or reduce the progression of the disease; to cause regression of its biological manifestations and/or clinical symptom; to inhibit further progression or worsening of at least one symptom, i.e. by reducing the.severity or frequency of at least one symptom.

As used herein, the term "patient" refers to a human suffering from disease.

As used herein, the term "Compounds of Formula (I)", and equivalent expressions, are meant to include racemic compounds of Formula (I), and their enantiomers, epimers, diastereoisomers, geometric isomers, tautomers and mixtures thereof, where the context so permits.

As used herein, the term "isomers" refer compounds that have the same molecular formula but differ in the nature or sequence of bonding of their atoms or the arrangement of their atoms in space. The terms "isomer 1" and "isomer 2" can be assigned to isomers of known absolute configuration or can be used to describe stereoisomers of unknown absolute configuration. Thus, the use of the terms "isomer 1" and "isomer 2" is not to be interpreted as indicating that the absolute configuration of both isomers is known. The term "isomeric mixture" refer to a mixture of isomers.

As used herein, the term "stereoisomers" is a general term used for all isomers of the individual molecules that differ only in the orientation of their atoms in space. The term "diastereomers" refer to stereoisomers that are not mirror images of one another and the term "enantiomers" refer to stereoisomers that are non-superimposable mirror images of each other. An enantiomer can be characterized by the absolute configurations of its asymmetric centers and is described by the R- and S-sequencing rules of Cahn, Ingold and Prelog, or by the manner in which the molecule rotates the plane of polarized light and designated as dextrorotatory or levorotatory (i.e., as (+) or (-)-isomers respectively). A chiral compound can exist as either individual enantiomer, individual diastereomer or as a mixture thereof. A mixture containing equal proportions of the enantiomers is called a "racemic mixture." A mixture containing any proportions of the diastereomers is called a "diastereomeric mixture". For a compound with two chiral centers in a cyclic system, "trans" refers to the substituents (other than hydrogen) of the chiral centers are on opposite sides of the ring; "cis" refers to the substituents of the chiral centers are on the same sides of the ring. "Racemic trans" refers to equal proportions of two trans enantiomers and "Racemic cis" refers to equal proportions of two cis enantiomers. Diastereomeric mixtures can be separated into their individual diastereomers on the basis of their physical chemical differences by methods well-known to those skilled in the art, such as, for example, by chromatography and/or fractional crystallization. Enantiomers can also be directly separated using chiral chromatographic techniques or indirectly using enzymatic methods. It is to be understood that all such isomers and mixtures thereof in any proportion are encompassed within the scope of the present disclosure.

Provided herein is a compound of the formula (I): 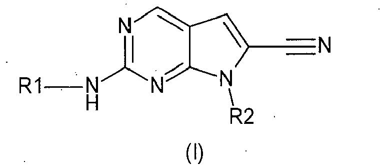 wherein:
R1 is selected from the group consisting of an aryl group, an ortho-fused bicyclic heteroaryl group and a heteroaryl group, wherein said ortho-fused bicyclic heteroaryl group is unsubstituted or substituted with one or more -(C₁-C₃)-alkyl group; and wherein said aryl and heteroaryl groups are unsubstituted or substituted with one or more substituents independently selected from the group consisting of
   a) a fluorine atom,
   b) a deuterium atom,
   c) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
   d) a cycloalkyl group which is unsubstituted or substituted with one or more fluorine atom or lower alkyl group,
   e) a heterocycloalkyl group which is unsubstituted or substituted with one or more substituents independently selected from an alkyl group, an alkyloxyl group, and an alkylcarbonyl group,
   f) an alkyloxyl group which is unsubstituted or substituted with one or more substituents independently selected from the group consisting of a hydroxy group and a fluorine atom,
   g) an -O-cycloalkyl group which is unsubstituted or substituted with one or more fluorine atom or lower alkyl group,
   h) an -O-heterocycloalkyl group which is unsubstituted or substituted with one or more substituents independently selected from the group consisting of an alkyl group, an alkyloxyl group, and an alkylcarbonyl group,
   i) an -O-spirocycle group,
   j) an alkylsulfonylalkyl group, and
   k) an alkylsulfonyl group; and
R2 is selected from the group consisting an alkyloxylalkyl group and a heterocycloalkyl group, wherein said heterocycloalkyl group represented by R2 is attached via a carbon atom and is unsubstituted or substituted with an alkyl group, an alkyloxyl group or one or more fluorine atom;
or a pharmaceutically acceptable salt thereof.

One embodiment is a compound of formula (I), wherein
R1 is selected from the group consisting of a phenyl group and a heteroaryl group, wherein said aryl and heteroaryl groups are unsubstituted or substituted with one or more substituents independently selected from the group consisting of
   a) a fluorine atom,
   b) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
   c) a cycloalkyl group which is unsubstituted or substituted with one or more fluorine atom or lower alkyl group,
   d) a heterocycloalkyl group which is unsubstituted or substituted with one or more substituents independently selected from an alkyl group, an alkyloxyl group, and an alkylcarbonyl group,
   e) an alkyloxyl group which is unsubstituted or substituted with one or more substituents independently selected from the group consisting of a hydroxy group and a fluorine atom,
   f) an -O-cycloalkyl group which is unsubstituted or substituted with one or more fluorine atom or lower alkyl group,
   g) an -O-heterocycloalkyl group which is unsubstituted or substituted with one or more substituents independently selected from the group consisting of an alkyl group, an alkyloxyl group, and an alkylcarbonyl group,
   h) an -O-spirocycle group,
   i) an alkylsulfonylalkyl group, and
   j) an alkylsulfonyl group; and
R2 is selected from the group consisting an alkyloxylalkyl group and a heterocycloalkyl group, wherein said heterocycloalkyl group represented by R2 is attached via a carbon atom and is unsubstituted or substituted with an alkyl group, an alkyloxyl group or one or more fluorine atom; or a pharmaceutically acceptable salt thereof.

One embodiment is a compound of formula (I), wherein
R1 is selected from the group consisting of a substituted or unsubstituted phenyl group, an ortho-fused bicyclic heteroaryl group, and 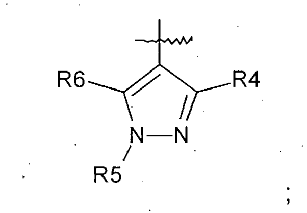
wherein said ortho-fused bicyclic heteroaryl group is unsubstituted or substituted with one or more -(C₁-C₃)-alkyl group;
R2 is selected from the group consisting an alkyloxylalkyl group and a heterocycloalkyl group, wherein said heterocycloalkyl group is attached via a carbon atom and is unsubstituted or substituted with an alkyl group, an alkyloxyl group or one or more fluorine atom;
R4 is selected from the group consisting of
   a) an alkyloxyl group which is unsubstituted or substituted with one or more substituents independently selected from the group consisting of a hydroxy group and a fluorine atom,
   b) an -O-cycloalkyl group which is unsubstituted or substituted with one or more fluorine atom or lower alkyl group,
   c) an -O-heterocycloalkyl group which is unsubstituted or substituted with one or more substituents independently selected from the group consisting of an alkyl group, an alkyloxyl group, and an alkylcarbonyl group, and
   d) an -O-spirocycle group;
R5 is selected from the group consisting of
   a) a hydrogen atom,
   b) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
   c) a cycloalkyl group which is unsubstituted or substituted with one or more fluorine atom or lower alkyl group,
   d). a heterocycloalkyl group which is unsubstituted or substituted with one or more substituents independently selected from an alkyl group, an alkyloxyl group, and an alkylcarbonyl group,
   e) an alkylsulfonylalkyl group, and
   f) an alkylsulfonyl group; and
R6 is selected from the group consisting a hydrogen atom and a deuterium atom;
or a pharmaceutically acceptable salt thereof.

One embodiment is a compound of formula (I), wherein
R1 is 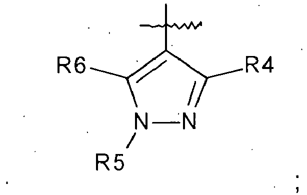
R2 is selected from the group consisting an alkyloxylalkyl group and 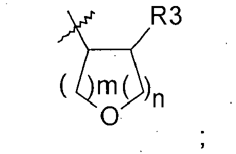
   Wherein R3 is selected from the group consisting a hydrogen atom, a -(C₁-C₃)-alkyl group and a -(C₁-C₃)-alkyloxyl group;
   m represents 1, 2 or 3;
   n represents 0 or 1;
R4 is selected from the group consisting of
   a) an alkyloxyl group which is unsubstituted or substituted with one or more substituents independently selected from the group consisting of a hydroxy group and a fluorine atom,
   b) an -O-cycloalkyl group which is unsubstituted or substituted with one or more fluorine atom or lower alkyl group,
   c) an -O-heterocycloalkyl group which is unsubstituted or substituted with one or more substituents independently selected from the group consisting of an alkyl group, an alkyloxyl group, and an alkylcarbonyl group, and
   d) an -O-spirocycle group;
R5 is selected from the group consisting of
   a) a hydrogen atom,
   b) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
   c) a cycloalkyl group which is unsubstituted or substituted with one or more fluorine atom or lower alkyl group,
   d) a heterocycloalkyl group which is unsubstituted or substituted with one or more substituents independently selected from an alkyl group, an alkyloxyl group, and an alkylcarbonyl group,
   e) an alkylsulfonylalkyl group, and
   f) an alkylsulfonyl group; and
R6 is selected from the group consisting a hydrogen atom and a deuterium atom;
or a pharmaceutically acceptable salt thereof.

One embodiment is a compound of formula (I), wherein
R1 is selected from the group consisting of a phenyl group and a heteroaryl group, wherein said phenyl and heteroaryl groups are unsubstituted or substituted with one or more substituents independently selected from the group consisting of
   a) a fluorine atom,
   b) a deuterium atom,
   c) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
   d) a cycloalkyl group which is unsubstituted or substituted with one or more fluorine atom or lower alkyl group,
   e) a heterocycloalkyl group which is unsubstituted or substituted with one or more substituents independently selected from an alkyl group, an alkyloxyl group, and an alkylcarbonyl group,
   f) an alkyloxyl group which is unsubstituted or substituted with one or more substituents independently selected from the group consisting of a hydroxy group and a fluorine atom,
   g) an -O-cycloalkyl group which is unsubstituted or substituted with one or more fluorine atom or lower alkyl group,
   h) an -O-heterocycloalkyl group which is unsubstituted or substituted with one or more substituents independently selected from the group consisting of an alkyl group, an alkyloxyl group, and an alkylcarbonyl group,
   i) an -O-spirocycle group,
   j) an alkylsulfonylalkyl group, and
   k) an alkylsulfonyl group; and
R2 is a heterocycloalkyl group, which is attached via a carbon atom and is unsubstituted or substituted with an alkyl group, an alkyloxyl group or one or more fluorine atom;
or a pharmaceutically acceptable salt thereof.

One embodiment is a compound of formula (I), wherein
R1 is 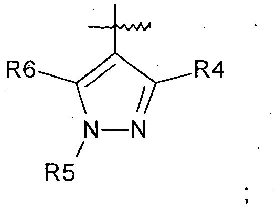
R2 is a heterocycloalkyl group, which is attached via a carbon atom and is unsubstituted or substituted with an alkyl group, an alkyloxyl group or one or more fluorine atom;
R4 is selected from the group consisting of
   a) an alkyloxyl group which is unsubstituted or substituted with one or more substituents independently selected from the group consisting of a hydroxy group and a fluorine atom,
   b) an -O-cycloalkyl group which is unsubstituted or substituted with one or more fluorine atom, lower alkyl group,
   c) an -O-heterocycloalkyl group which is unsubstituted or substituted with one or more substituents independently selected from the group consisting of an alkyl group, an alkyloxyl group, and an alkylcarbonyl group, and
   d) an -O-spirocycle group;
R5 is selected from the group consisting of
   a) a hydrogen atom,
   b) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
   c) a cycloalkyl group which is unsubstituted or substituted with one or more fluorine atom or lower alkyl group,
   d) a heterocycloalkyl group which is unsubstituted or substituted with one or more substituents independently selected from an alkyl group, an alkyloxyl group, and an alkylcarbonyl group,
   e) an alkylsulfonylalkyl group, and
   f) an alkylsulfonyl group; and
R6 is selected from the group consisting a hydrogen atom and a deuterium atom;
or a pharmaceutically acceptable salt thereof.

One embodiment is a compound of formula (la) 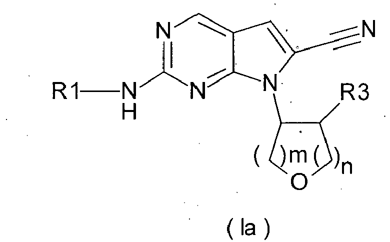 wherein:
R1 is selected from the group consisting of an aryl group, an ortho-fused bicyclic heteroaryl group and a heteroaryl group, wherein said ortho-fused bicyclic heteroaryl group is unsubstituted or substituted with one or more -(C₁-C₃)-alkyl group; said aryl and heteroaryl groups are unsubstituted or substituted with one or more substituents independently selected from the group consisting of
   a) a deuterium atom,
   b) a fluorine atom,
   c) an alkyl group which is unsubstituted or substituted with one or more substituents independently selected from the group consisting of a hydroxy group, a fluorine atom, a deuterium atom, a cyano group, an alkyloxyl group, an alkylamino group,
   d) a cycloalkyl group which is unsubstituted or substituted with one or more fluorine atom, lower alkyl group,
   e) a heterocycloalkyl group which is unsubstituted or substituted with one or more substituents independently selected from an alkyl group, an alkyloxyl group, or an alkylcarbonyl group,
   f) an alkyloxyl group which is unsubstituted or substituted with one or more substituents independently selected from the group consisting of a hydroxy group and a fluorine atom,
   g) an -O-cycloalkyl group which is unsubstituted or substituted with one or more fluorine atom, lower alkyl group
   h) an -O-heterocycloalkyl group which is unsubstituted or substituted with one or more substituents independently selected from the group consisting of an alkyl group, an alkyloxyl group, and an alkylcarbonyl group,
   i) an -O-spirocycle group,
   j) an alkylsulfonylalkyl group, and
   k) an alkylsulfonyl group;
R3 is selected from the group consisting a hydrogen atom, a -(C₁-C₃)-alkyl group and a -(C₁-C₃)-alkyloxyl group;
m represents 1, 2 or 3; and
n represents 0 or 1;
or a pharmaceutically acceptable salt thereof.

One embodiment is a compound of formula (Ia), wherein
R1 is selected from the group consisting of a phenyl group and a heteroaryl group, wherein said phenyl and heteroaryl groups are unsubstituted or substituted with one or more substituents independently selected from the group consisting of
   a) a fluorine atom,
   b) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
   c) a cycloalkyl group which is unsubstituted or substituted with one or more fluorine atom or lower alkyl group,
   d) a heterocycloalkyl group which is unsubstituted or substituted with one or more substituents independently selected from an alkyl group, an alkyloxyl group, and an alkylcarbonyl group,
   e) an alkyloxyl group which is unsubstituted or substituted with one or more substituents independently selected from the group consisting of a hydroxy group and a fluorine atom,
   f) an -O-cycloalkyl group which is unsubstituted or substituted with one or more fluorine atom or lower alkyl group,
   g) an -O-heterocycloalkyl group which is unsubstituted or substituted with one or more substituents independently selected from the group consisting of an alkyl group, an alkyloxyl group, and an alkylcarbonyl group,
   h) an -O-spirocycle group,
   i) an alkylsulfonylalkyl group, and
   j) an alkylsulfonyl group;
m represents 1 or 2; and
n represents 0 or 1;
or a pharmaceutically acceptable salt thereof.

One embodiment is a compound of formula (Ib) 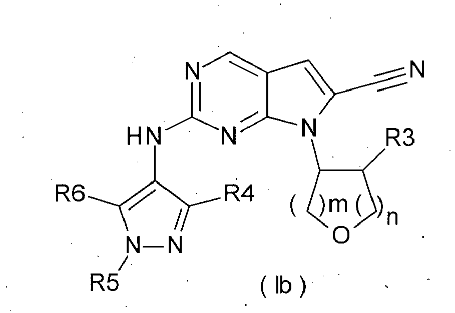 wherein:
R3 is selected from the group consisting a hydrogen atom, a -(C₁-C₃)-alkyl group and a -(C₁-C₃)-alkyloxyl group;
R4 is selected from the group consisting of
   a) an alkyloxyl group which is unsubstituted or substituted with one or more substituents independently selected from the group consisting of a hydroxy group and a fluorine atom,
   b) an -O-cycloalkyl group which is unsubstituted or substituted with one or more fluorine atom, lower alkyl group,
   c) an -O-heterocycloalkyl group which is unsubstituted or substituted with one or more substituents independently selected from the group consisting of an alkyl group, an alkyloxyl group, and an alkylcarbonyl group, and
   d) an -O-spirocycle group;
m represents 1, 2 or 3;
n represents 0 or 1;
R5 is selected from the group consisting of
   a) a hydrogen atom,
   b) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
   c) a cycloalkyl group which is unsubstituted or substituted with one or more fluorine atom or lower alkyl group,
   d) a heterocycloalkyl group which is unsubstituted or substituted with one or more substituents independently selected from an alkyl group, an alkyloxyl group, and an alkylcarbonyl group,
   e) an alkylsulfonylalkyl group, and
   f) an alkylsulfonyl group; and
R6 is selected from the group consisting a hydrogen atom and a deuterium atom;
or a pharmaceutically acceptable salt thereof.

One embodiment is a compound of formula (lb) 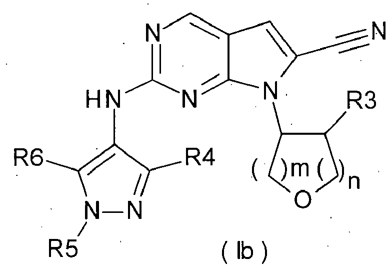 wherein:
R3 is selected from the group consisting a -(C₁-C₃)-alkyl group and a -(C₁-C₃)-alkyloxyl group;
R4 is selected from the group consisting of
   a) an alkyloxyl group which is unsubstituted or substituted with one or more substituents independently selected from the group consisting of a hydroxy group and a fluorine atom,
   b) an -O-cycloalkyl group which is unsubstituted or substituted with one or more fluorine atom, lower alkyl group,
   c) an -O-heterocycloalkyl group which is unsubstituted or substituted with one or more substituents independently selected from the group consisting of an alkyl group, an alkyloxyl group, and an alkylcarbonyl group, and
   d) an -O-spirocycle group;
m represents 1 or 2;
n represents 0 or 1;
R5 is selected from the group consisting of
   a) a hydrogen atom,
   b) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
   c) a cycloalkyl group which is unsubstituted or substituted with one or more fluorine atom or lower alkyl group,
   d) a heterocycloalkyl group which is unsubstituted or substituted with one or more substituents independently selected from an alkyl group, an alkyloxyl group, and an alkylcarbonyl group,
   e) an alkylsulfonylalkyl group, and
   f) an alkylsulfonyl group; and
R6 is a hydrogen atom;
or a pharmaceutically acceptable salt thereof.

One embodiment is a compound of formula (Ib) 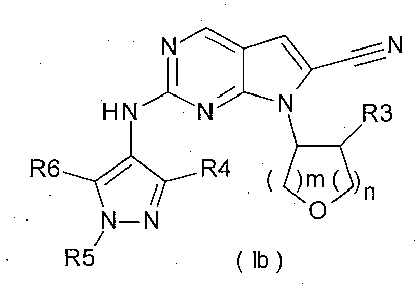 wherein:
R3 is selected from the group consisting a -(C₁-C₃)-alkyl group and a -(C₁-C₃)-alkyloxyl group;
R4 is selected from the group consisting of
   a) an alkyloxyl group which is unsubstituted or substituted with one or more substituents independently selected from the group consisting of a hydroxy group and a fluorine atom,
   b) an -O-cycloalkyl group which is unsubstituted or substituted with one or more fluorine atom, lower alkyl group, and
   c) an -O-heterocycloalkyl group which is unsubstituted or substituted with one or more substituents independently selected from the group consisting of an alkyl group, an alkyloxyl group, and an alkylcarbonyl group,
m represents 1;
n represents 1;
R5 is selected from the group consisting of
   a) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
   b) a cycloalkyl group which is unsubstituted or substituted with one or more fluorine atom or lower alkyl group, and
   c) a heterocycloalkyl group which is unsubstituted or substituted with one or more substituents independently selected from an alkyl group, an alkyloxyl group, and an alkylcarbonyl group ; and
R6 is a hydrogen atom;
or a pharmaceutically acceptable salt thereof.

One embodiment is a compound of formula (Ib) 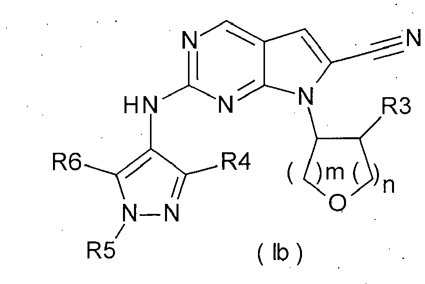 wherein:
R3 is a -(C₁-C₃)-alkyl group;
R4 is selected from the group consisting of
   a) an -O-cycloalkyl group which is unsubstituted or substituted with one or more fluorine atom, lower alkyl group,
   b) an -O-heterocycloalkyl group which is unsubstituted or substituted with one or more substituents independently selected from the group consisting of an alkyl group, an alkyloxyl group, and an alkylcarbonyl group, and
m represents 1;
n represents 1;
R5 is an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group, and
R6 is a hydrogen atom;
or a pharmaceutically acceptable salt thereof.

One embodiment is a compound of formula (Ic) 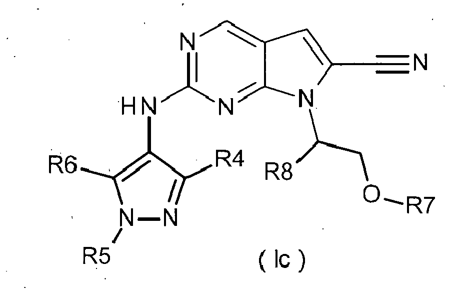 wherein:
R4 is selected from the group consisting of
   a) an alkyloxyl group which is unsubstituted or substituted with one or more substituents independently selected from the group consisting of a hydroxy group and a fluorine atom,
   b) an -O-cycloalkyl group which is unsubstituted or substituted with one or more fluorine atom or lower alkyl group,
   c) an -O-heterocycloalkyl group which is unsubstituted or substituted with one or more substituents independently selected from the group consisting of an alkyl group, an alkyloxyl group, and an alkylcarbonyl group, and
   d) an -O-spirocycle group;
R5 is selected from the group consisting of
   a) a hydrogen atom,
   b) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
   c) a cycloalkyl group which is unsubstituted or substituted with one or more fluorine atom or lower alkyl group,
   d) a heterocycloalkyl group which is unsubstituted or substituted with one or more substituents independently selected from an alkyl group, an alkyloxyl group, and an alkylcarbonyl group,
   e) an alkylsulfonylalkyl group, and
   f) an alkylsulfonyl group;
R6 is selected from the group consisting a hydrogen atom and a deuterium atom;
R7 is a -(C₁-C₃)-alkyl group; and
R8 is a -(C₁-C₃)-alkyl group;
or a pharmaceutically acceptable salt thereof.

One embodiment is a compound of formula (I), wherein R1 represents a substituted or unsubstituted aryl group.

One embodiment is a compound of formula (I), wherein R1 represents a substituted or unsubstituted phenyl group.

One embodiment is a compound of formula (I), wherein R1 represents an ortho-fused bicyclic heteroaryl group, which is unsubstituted or substituted with one or more - (C₁-C₃)-alkyl group.

One embodiment is a compound of formula (I), wherein R1 represents an ortho-fused bicyclic heteroaryl group selected from the following list:

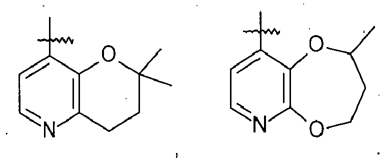

One embodiment is a compound of formula (I), wherein R1 represents a substituted or unsubstituted heteroaryl group.

One embodiment is a compound of formula (I), wherein R1 represents a 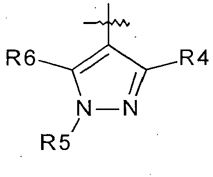 group. wherein:
R4 is selected from the group consisting of
   a) an alkyloxyl group which is unsubstituted or substituted with one or more substituents independently selected from the group consisting of a hydroxy group and a fluorine atom,
   b) an -O-cycloalkyl group which is unsubstituted or substituted with one or more fluorine atom or lower alkyl,
   c) an -O-heterocycloalkyl group which is unsubstituted or substituted with one or more substituents independently selected from the group consisting of an alkyl group, an alkyloxyl group, and an alkylcarbonyl group, and
   d) an -O-spirocycle group;
R5 is selected from the group consisting of
   a) a hydrogen atom,
   b) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
   c) a cycloalkyl group,
   d) a heterocycloalkyl group,
   e) an alkylsulfonylalkyl group, and
   f) an alkylsulfonyl group; and
R6 is selected from the group consisting a hydrogen atom and a deuterium atom;
or a pharmaceutically acceptable salt thereof.

One embodiment is a compound of formula (I), wherein R2 represents an alkyloxylalkyl group.

One embodiment is a compound of formula (I), wherein R2 represents a heterocycloalkyl group.

One embodiment is a compound of formula (I), wherein R2 represents a 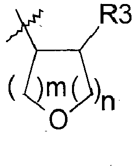 group.

One embodiment is a compound of formula (I), wherein R2 represents a heterocycloalkyl group selected from the following list:

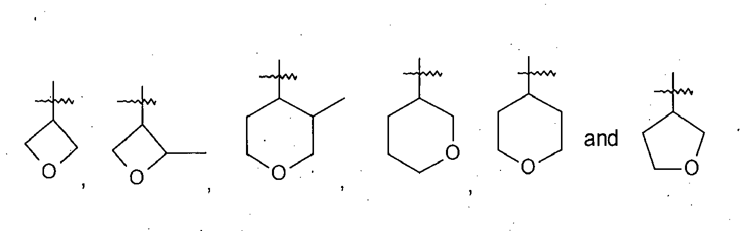

One embodiment is a compound of formula (Ia), wherein R3 represents a hydrogen atom.

One embodiment is a compound of formula (Ia), wherein R3 represents a -(C₁-C₃)-alkyl group.

One embodiment is a compound of formula (Ia), wherein R3 represents a -(C₁-C₃)-alkyloxyl group.

One embodiment is a compound of formula (Ia), wherein m represents 1.

One embodiment is a compound of formula (Ia), wherein m represents 2.

One embodiment is a compound of formula (Ia), wherein m represents 3.

One embodiment is a compound of formula (Ia), wherein n represents 0.

One embodiment is a compound of formula (Ib), wherein n represents 1.

One embodiment is a compound of formula (Ib), wherein R4 represents an alkyloxyl group which is unsubstituted or substituted with one or more substituents independently selected from the group consisting of a hydroxy group and a fluorine atom.

One embodiment is a compound of formula (Ib), wherein R4 represents an -O-cycloalkyl group which is unsubstituted or substituted with one or more fluorine atom, or lower alkyl group.

One embodiment is a compound of formula (Ib), wherein R4 represents an -O-heterocycloalkyl group which is unsubstituted or substituted with one or more substituents independently selected from the group consisting of an alkyl group, an alkyloxyl group, and an alkylcarbonyl group.

One embodiment is a compound of formula (Ib), wherein R4 represents an -O-spirocycle group.

One embodiment is a compound of formula (Ib), wherein R4 represents a group selected from the following list:

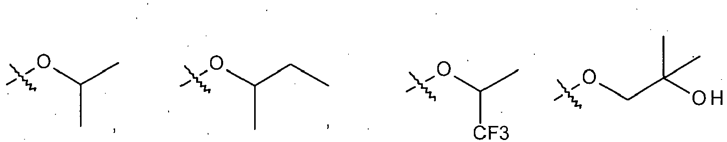

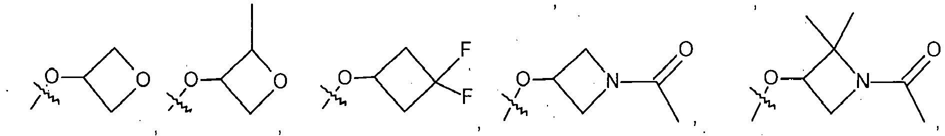

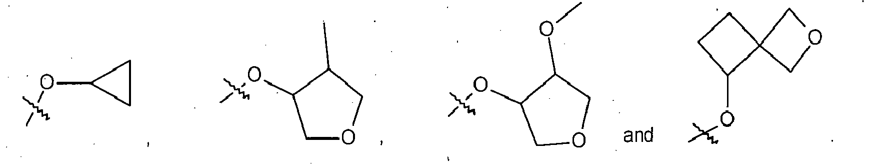

One embodiment is a compound of formula (Ib), wherein R5 represents a hydrogen atom.

One embodiment is a compound of formula (Ib), wherein R5 represents an alkyl group which is unsubstituted or substituted with one or more substituents independently selected from the group consisting of a hydroxy group, a fluorine atom, a deuterium atom, a cyano group, an alkyloxyl group, an alkylamino group and a dialkylamino group.

One embodiment is a compound of formula (Ib), wherein R5 represents a cycloalkyl group.

One embodiment is a compound of formula (Ib), wherein R5 represents a heterocycloalkyl group.

One embodiment is a compound of formula (Ib), wherein R5 represents an alkylsulfonylalkyl group.

One embodiment is a compound of formula (Ib), wherein R5 represents an alkylsulfonyl group.

One embodiment is a compound of formula (Ib), wherein R5 represents a group selected from the following list:

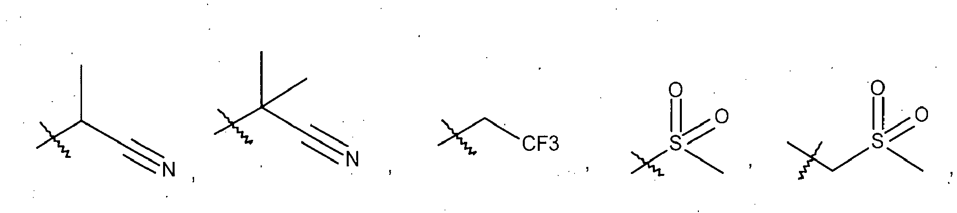

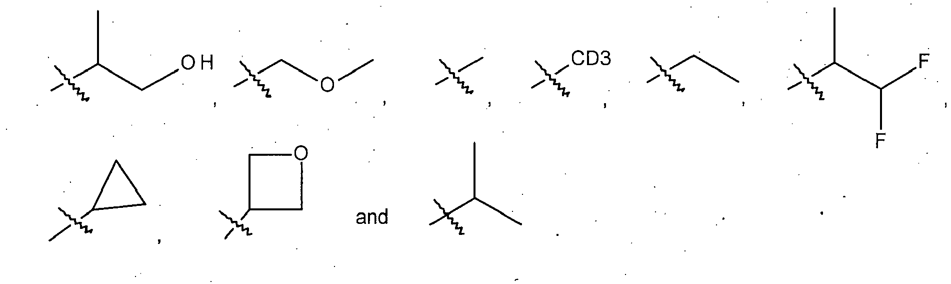

In one embodiment, the compound of formula (I) is selected from the group consisting of:
2-[[3-(1-acetylazetidin-3-yl)oxy-1-methyl-pyrazol-4-yl]amino]-7-[(3R,4R)-4-methyltetrahydrofuran-3-yl]pyrrolo[2,3-d]pyrimidine-6-carbonitrile;
2-[[1-methyl-3-[(3S,4R)-4-methyltetrahydrofuran-3-yl]oxy-1H-pyrazol-4-yl]amino]-7-[(3R,4R)-4-methyltetrahydrofuran-3-yl]pyrrolo[2,3-d]pyrimidine-6-carbonitrile;
2-[[1-methyl-3-[(3R,4S)-4-methyltetrahydrofuran-3-yl]oxy-1H-pyrazol-4-yl]amino]-7-[(3R,4R)-4-methyltetrahydrofuran-3-yl]pyrrolo[2,3-d]pyrimidine-6-carbonitrile;
2-[[1-methyl-3-[(trans)-4-methyltetrahydrofuran-3-yl]oxy-1H-pyrazol-4-yl]amino]-7-[(3R,4R)-4-methyltetrahydrofuran-3-yl]pyrrolo[2,3-d]pyrimidine-6-carbonitrile;
2-[[1-[(1R)-2,2-difluoro-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile;
2-[[1-[(1S)-2,2-difluoro-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile;
2-[[1-(2,2-difluoro-1-methyl-ethyl)-3-(oxetan-3-yloxy)pyrazol-4-yl]amino]-7-[(3R,4R)-4-methyltetrahydrofuran-3-yl]pyrrolo[2,3-d]pyrimidine-6-carbonitrile;
2-((1-methyl-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-methyl-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-methyl-3-(((trans)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-[[1-methyl-3-(oxetan-3-yloxy)pyrazol-4-yl]amino]-7-[(3R,4R)-4-methyltetrahydrofuran-3-yl]pyrrolo[2,3-d]pyrimidine-6-carbonitrile;
2-[[1-[2-hydroxy-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile,
7-[(3R,4R)-4-methyltetrahydrofuran-3-yl]-2-[[1-(oxetan-3-yl)-3-[(1R)-2,2,2-trifluoro-1-methyl-ethoxy]pyrazol-4-yl]amino]pyrrolo[2,3-d]pyrimidine-6-carbonitrile;
7-[(3R,4R)-4-methyltetrahydrofuran-3-yl]-2-[[1-(oxetan-3-yl)-3-[(1S)-2,2,2-trifluoro-1-methyl-ethoxy]pyrazol-4-yl]amino]pyrrolo[2,3-d]pyrimidine-6-carbonitrile;
7-[(3R,4R)-4-methyltetrahydrofuran-3-yl]-2-[[1-(oxetan-3-yl)-3-(2,2,2-trifluoro-1-methyl-ethoxy)pyrazol-4-yl]amino]pyrrolo[2,3-d]pyrimidine-6-carbonitrile;
2-[[3-[(3S,4S)-4-methoxytetrahydrofuran-3-yl]oxy-1-methyl-pyrazol-4-yl]amino]-7-[(3R,4R)-4-methyltetrahydrofuran-3-yl]pyrrolo[2,3-d]pyrimidine-6-carbonitrile;
2-[[3-[(3R,4R)-4-methoxytetrahydrofuran-3-yl]oxy-1-methyl-pyrazol-4-yl]amino]-7-[(3R,4R)-4-methyltetrahydrofuran-3-yl]pyrrolo[2,3-d]pyrimidine-6-carbonitrile;
2-[[3-[(trans)-4-methoxytetrahydrofuran-3-yl]oxy-1-methyl-pyrazol-4-yl]amino]-7-[(3R,4R)-4-methyltetrahydrofuran-3-yl]pyrrolo[2,3-d]pyrimidine-6-carbonitrile;
2-[[1-(methylsulfonylmethyl)-3-(oxetan-3-yloxy)pyrazol-4-yl]amino]-7-[(3R,4R)-4-methyltetrahydrofuran-3-yl]pyrrolo[2,3-d]pyrimidine-6-carbonitrile;
2-((1-(methyl-d3)-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-(methyl-d3)-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-(methyl-d3)-3-(((trans)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((3-(((R)-1-acetyl-2,2-dimethylazetidin-3-yl)oxy)-1-methyl-1H-pyrazol-4-yl)amino)-7-((3R,4R)-4-methyltetrahydrofuran-3-yl)-7H-pyrrolo[2,3-d]pyrimidine-6-carbonitrile;
2-((3-(((S)-1-acetyl-2,2-dimethylazetidin-3-yl)oxy)-1-methyl-1H-pyrazol-4-yl)amino)-7-((3R,4R)-4-methyltetrahydrofuran-3-yl)-7H-pyrrolo[2,3-d]pyrimidine-6-carbonitrile;
2-((3-((1-acetyl-2,2-dimethylazetidin-3-yl)oxy)-1-methyl-1H-pyrazol-4-yl)amino)-7-((3R,4R)-4-methyltetrahydrofuran-3-yl)-7H-pyrrolo[2,3-d]pyrimidine-6-carbonitrile;
2-((1-(methyl-d3)-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile;
2-((4-(2-hydroxypropan-2-yl)-2-(oxetan-3-yloxy)phenyl)amino)-7-((3R,4R)-4-methyltetrahydrofuran-3-yl)-7H-pyrrolo[2,3-d]pyrimidine-6-carbonitrile;
2-((1-methyl-3-(((R)-1,1,1-trifluoropropan-2-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-methyl-3-(((S)-1,1,1-trifluoropropan-2-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-methyl-3-((1,1,1-trifluoropropan-2-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-(methyl-d3)-3-(((2R,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-(methyl-d3)-3-(((2S,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-(methyl-d3)-3-(((cis)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((3-isopropoxy-1-(2,2,2-trifluoroethyl)-1H-pyrazol-4-yl)amino)-7-((3R,4R)-4-methyltetrahydrofuran-3-yl)-7H-pyrrolo[2,3-d]pyrimidine-6-carbonitrile;
2-((3-isopropoxy-1-methyl-1H-pyrazol-4-yl)amino)-7-((3R,4R)-4-methyltetrahydrofuran-3-yl)-7H-pyrrolo[2,3-d]pyrimidine-6-carbonitrile;
2-((1-(2-cyanopropan-2-yl)-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile;
2-((3-cyclopropoxy-1-(methyl-d3)-1H-pyrazol-4-yl)amino)-7-((3R,4R)-4-methyltetrahydrofuran-3-yl)-7H-pyrrolo[2,3-d]pyrimidine-6-carbonitrile;
2-((1-(2-cyanopropan-2-yl)-3-isopropoxy-1H-pyrazol-4-yl)amino)-7-((3R,4R)-4-methyltetrahydrofuran-3-yl)-7H-pyrrolo[2,3-d]pyrimidine-6-carbonitrile;
2-((3-cyclopropoxy-1-(oxetan-3-yl)-1H-pyrazol-4-yl)amino)-7-((3R,4R)-4-methyltetrahydrofuran-3-yl)-7H-pyrrolo[2,3-d]pyrimidine-6-carbonitrile;
2-((3-isopropoxy-1-(oxetan-3-yl)-1H-pyrazol-4-yl)amino)-7-((3R,4R)-4-methyltetrahydrofuran-3-yl)-7H-pyrrolo[2,3-d]pyrimidine-6-carbonitrile;
2-((3-(2-hydroxy-2-methylpropoxy)-1-methyl-1H-pyrazol-4-yl)amino)-7-((3R,4R)-4-methyltetrahydrofuran-3-yl)-7H-pyrrolo[2,3-d]pyrimidine-6-carbonitrile;
2-((1-(methoxymethyl)-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-(methylsulfonyl)-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile;
2-((3-cyclopropoxy-1-(methoxymethyl)-1H-pyrazol-4-yl)amino)-7-((3R,4R)-4-methyltetrahydrofuran-3-yl)-7H-pyrrolo[2,3-d]pyrimidine-6-carbonitrile;
2-((3-cyclopropoxy-1-((methylsulfonyl)methyl)-1H-pyrazol-4-yl)amino)-7-((3R,4R)-4-methyltetrahydrofuran-3-yl)-7H-pyrrolo[2,3-d]pyrimidine-6-carbonitrile;
2-((1-cyclopropyl-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-ethyl-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile;
2-[[1-(methoxymethyl)-3-[(trans)-2-methyloxetan-3-yl]oxy-pyrazol-4-yl]amino]-7-[(3R,4R)-4-methyltetrahydrofuran-3-yl]pyrrolo[2,3-d]pyrimidine-6-carbonitrile;
2-[[1-methyl-3-[(2-oxaspiro[3.3]heptan-7-yl)oxy]pyrazol-4-yl]amino]-7-[(3R,4R)-4-methyltetrahydrofuran-3-yl]pyrrolo[2,3-d]pyrimidine-6-carbonitrile;
2-((2-cyclopropoxy-4-((dimethylamino)methyl)phenyl)amino)-7-((3R,4R)-4-methyltetrahydrofuran-3-yl)-7H-pyrrolo[2,3-d]pyrimidine-6-carbonitrile;
2-((3-isopropoxy-1-(methyl-d3)-1H-pyrazol-4-yl)amino)-7-((3R,4R)-4-methyltetrahydrofuran-3-yl)-7H-pyrrolo[2,3-d]pyrimidine-6-carbonitrile;
2-((3-cyclopropoxy-1-(1,1-difluoropropan-2-yl)-1H-pyrazol-4-yl)amino)-7-((3R,4R)-4-methyltetrahydrofuran-3-yl)-7H-pyrrolo[2,3-d]pyrimidine-6-carbonitrile;
2-((1-methyl-3-(oxetan-3-yloxy)-1H-pyrazol-4-yl)amino)-7-((3S,4S)-4-methyltetrahydrofuran-3-yl)-7H-pyrrolo[2,3-d]pyrimidine-6-carbonitrile;
2-((1-methyl-3-(oxetan-3-yloxy)-1H-pyrazol-4-yl)amino)-7-((3S,4R)-4-methyltetrahydrofuran-3-yl)-7H-pyrrolo[2,3-d]pyrimidine-6-carbonitrile;
2-((1-methyl-3-(oxetan-3-yloxy)-1H-pyrazol-4-yl)amino)-7-((3R,4S)-4-methyltetrahydrofuran-3-yl)-7H-pyrrolo[2,3-d]pyrimidine-6-carbonitrile;
2-((1-methyl-3-(oxetan-3-yloxy)-1H-pyrazol-4-yl)amino)-7-((cis)-4-methyltetrahydrofuran-3-yl)-7H-pyrrolo[2,3-d]pyrimidine-6-carbonitrile;
2-((3-isopropoxy-1-methyl-1H-pyrazol-4-yl)amino)-7-((cis)-4-methyltetrahydrofuran-3-yl)-7H-pyrrolo[2,3-d]pyrimidine-6-carbonitrile;
2-((1-((R)-1-cyanoethyl)-3-isopropoxy-1H-pyrazol-4-yl)amino)-7-((3S,4R)-4-methyltetrahydrofuran-3-yl)-7H-pyrrolo[2,3-d]pyrimidine-6-carbonitrile;
2-((1-(1-cyanoethy))-3-isopropoxy-1H-pyrazol-4-yl)amino)-7-((cis)-4-methyltetrahydrofuran-3-yl)-7H-pyrrolo[2,3-d]pyrimidine-6-carbonitrile;
2-((1-((S)-1-cyanoethyl)-3-isopropoxy-1H-pyrazol-4-yl)amino)-7-((3S,4R)-4-methyltetrahydrofuran-3-yl)-7H-pyrrolo[2,3-d]pyrimidine-6-carbonitrile;
2-((1-((R)-1-cyanoethyl)-3-isopropoxy-1H-pyrazol-4-yl)amino)-7-((3R,4S)-4-methyltetrahydrofuran-3-yl)-7H-pyrrolo[2,3-d]pyrimidine-6-carbonitrile;
2-((1-((S)-1-cyanoethyl)-3-isopropoxy-1H-pyrazol-4-yl)amino)-7-((3R,4S)-4-methyltetrahydrofuran-3-yl)-7H-pyrrolo[2,3-d]pyrimidine-6-carbonitrile;
(R)-2-((1-methyl-3-(oxetan-3-yloxy)-1H-pyrazol-4-yl)amino)-7-(tetrahydrofuran-3-yl)-7H-pyrrolo[2,3-d]pyrimidine-6-carbonitrile;
(S)-2-((1-methyl-3-(oxetan-3-yloxy)-1H-pyrazol-4-yl)amino)-7-(tetrahydrofuran-3-yl)-7H-pyrrolo[2,3-d]pyrimidine-6-carbonitrile;
2-((1-methyl-3-(oxetan-3-yloxy)-1H-pyrazol-4-yl)amino)-7-(tetrahydrofuran-3-yl)-7H-pyrrolo[2,3-d]pyrimidine-6-carbonitrile;
7-((3R,4R)-4-Methoxytetrahydrofuran-3-yl)-2-((1-(oxetan-3-yl)-3-((1,1,1-trifluoropropan-2-yl)oxy)-1H-pyrazol-4-yl)amino)-7H-pyrrolo[2,3-d]pyrimidine-6-carbonitrile;
2-((4-(2-hydroxypropan-2-yl)-2-(oxetan-3-yloxy)phenyl)amino)-7-((3R,4R)-4-methoxytetrahydrofuran-3-yl)-7H-pyrrolo[2,3-d]pyrimidine-6-carbonitrile;
2-((1-((S)-1-cyanoethyl)-3-isopropoxy-1H-pyrazol-4-yl)amino)-7-((3R,4R)-4-methoxytetrahydrofuran-3-yl)-7H-pyrrolo[2,3-d]pyrimidine-6-carbonitrile;
2-((1-((R)-1-cyanoethyl)-3-isopropoxy-1H-pyrazol-4-yl)amino)-7-((3R,4R)-4-methoxytetrahydrofuran-3-yl)-7H-pyrrolo[2,3-d]pyrimidine-6-carbonitrile;
2-((1-(1-cyanoethyl)-3-isopropoxy-1H-pyrazol-4-yl)amino)-7-((3R,4R)-4-methoxytetrahydrofuran-3-yl)-7H-pyrrolo[2,3-d]pyrimidine-6-carbonitrile;
2-((1-(1-cyanoethyl)-3-cyclopropoxy-1H-pyrazol-4-yl)amino)-7-((3R,4R)-4-methoxytetrahydrofuran-3-yl)-7H-pyrrolo[2,3-d]pyrimidine-6-carbonitrile;
2-((1-(2-cyanopropan-2-yl)-3-cyclopropoxy-1H-pyrazol-4-yl)amino)-7-((3R,4R)-4-methoxytetrahydrofuran-3-yl)-7H-pyrrolo[2,3-d]pyrimidine-6-carbonitrile;
2-((1-(2-cyanopropan-2-yl)-3-isopropoxy-1H-pyrazol-4-yl)amino)-7-((3R,4R)-4-methoxytetrahydrofuran-3-yl)-7H-pyrrolo[2,3-d]pyrimidine-6-carbonitrile;
2-((3-cyclopropoxy-1-isopropyl-1H-pyrazol-4-yl)amino)-7-((3R,4R)-4-methoxytetrahydrofuran-3-yl)-7H-pyrrolo[2,3-d]pyrimidine-6-carbonitrile;
2-((3-isopropoxy-1-methyl-1H-pyrazol-4-yl)amino)-7-((3R,4R)-4-methoxytetrahydrofuran-3-yl)-7H-pyrrolo[2,3-d]pyrimidine-6-carbonitrile;
2-((3-isopropoxy-1-(oxetan-3-yl)-1H-pyrazol-4-yl)amino)-7-((3R,4R)-4-methoxytetrahydrofuran-3-yl)-7H-pyrrolo[2,3-d]pyrimidine-6-carbonitrile;
2-((3-cyclopropoxy-1-(oxetan-3-yl)-1H-pyrazol-4-yl)amino)-7-((3R,4R)-4-methoxytetrahydrofuran-3-yl)-7H-pyrrolo[2,3-d]pyrimidine-6-carbonitrile;
2-((3-(3,3-difluorocyclobutoxy)-1-methyl-1H-pyrazol-4-yl)amino)-7-((3R,4R)-4-methoxytetrahydrofuran-3-yl)-7H-pyrrolo[2,3-d]pyrimidine-6-carbonitrile;
2-((3-((R)-sec-butoxy)-1-methyl-1H-pyrazol-4-yl)amino)-7-((3R,4R)-4-methoxytetrahydrofuran-3-yl)-7H-pyrrolo[2,3-d]pyrimidine-6-carbonitrile;
2-((3-((S)-sec-butoxy)-1-methyl-1H-pyrazol-4-yl)amino)-7-((3R,4R)-4-methoxytetrahydrofuran-3-yl)-7H-pyrrolo[2,3-d]pyrimidine-6-carbonitrile;
2-((2,2-dimethyl-3,4-dihydro-2H-pyrano[3,2-b]pyridin-8-yl)amino)-7-((3R,4R)-4-methoxytetrahydrofuran-3-yl)-7H-pyrrolo[2,3-d]pyrimidine-6-carbonitrile;
7-((3R,4R)-4-methoxytetrahydrofuran-3-yl)-2-(((R)-2-methyl-3,4-dihydro-2H-[1,4]dioxepino[2,3-b]pyridin-9-yl)amino)-7H-pyrrolo[2,3-d]pyrimidine-6-carbonitrile;
7-((trans)-4-methoxytetrahydrofuran-3-yl)-2-((1-methyl-3-(oxetan-3-yloxy)-1H-pyrazol-4-yl)amino)-7H-pyrrolo[2,3-d]pyrimidine-6-carbonitrile;
2-((4-(2-hydroxypropan-2-yl)-2-(oxetan-3-yloxy)phenyl)amino)-7-((trans)-4-methoxytetrahydrofuran-3-yl)-7H-pyrrolo[2,3-d]pyrimidine-6-carbonitrile;
7-((cis)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile;
7-((trans)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile;
2-((1-(2-cyanopropan-2-yl)-3-isopropoxy-1H-pyrazol-4-yl)amino)-7-(oxetan-3-yl)-7H-pyrrolo[2,3-d]pyrimidine-6-carbonitrile;
2-((3-isopropoxy-1-methyl-1H-pyrazol-4-yl)amino)-7-(oxetan-3-yl)-7H-pyrrolo[2,3-d]pyrimidine-6-carbonitrile;
7-[(2S,3R)-2-methyloxetan-3-yl]-2-[[1-methyl-3-(oxetan-3-yloxy)pyrazol-4-yl]amino]pyrrolo[2,3-d]pyrimidine-6-carbonitrile;
7-[(2R,3S)-2-methyloxetan-3-yl]-2-[[1-methyl-3-(oxetan-3-yloxy)pyrazol-4-yl]amino]pyrrolo[2,3-d]pyrimidine-6-carbonitrile;
7-[(trans)-2-methyloxetan-3-yl]-2-[[1-methyl-3-(oxetan-3-yloxy)pyrazol-4-yl]amino]pyrrolo[2,3-d]pyrimidine-6-carbonitrile;
7-[(2R,3S)-2-methyloxetan-3-yl]-2-[(1-methyl-3-propan-2-yloxypyrazol-4-yl)amino]pyrrolo[2,3-d]pyrimidine-6-carbonitrile;
7-[(2S,3R)-2-methyloxetan-3-yl]-2-[(1-methyl-3-propan-2-yloxypyrazol-4-yl)amino]pyrrolo[2,3-d]pyrimidine-6-carbonitrile;
7-[(trans)-2-methyloxetan-3-yl]-2-[(1-methyl-3-propan-2-yloxypyrazol-4-yl)amino]pyrrolo[2,3-d]pyrimidine-6-carbonitrile;
2-((1-methyl-3-(oxetan-3-yloxy)-1H-pyrazol-4-yl)amino)-7-(tetrahydro-2H-pyran-4-yl)-7H-pyrrolo[2,3-d]pyrimidine-6-carbonitrile;
(R)-2-((1-methyl-3-(oxetan-3-yloxy)-1H-pyrazol-4-yl)amino)-7-(tetrahydro-2H-pyran-3-yl)-7H-pyrrolo[2,3-d]pyrimidine-6-carbonitrile;
(S)-2-((1-methyl-3-(oxetan-3-yloxy)-1H-pyrazol-4-yl)amino)-7-(tetrahydro-2H-pyran-3-yl)-7H-pyrrolo[2,3-d]pyrimidine-6-carbonitrile;
2-((1-methyl-3-(oxetan-3-yloxy)-1H-pyrazol-4-yl)amino)-7-(tetrahydro-2H-pyran-3-yl)-7H-pyrrolo[2,3-d]pyrimidine-6-carbonitrile;
2-((1-methyl-3-(oxetan-3-yloxy)-1H-pyrazol-4-yl)amino)-7-((3R,4S)-3-methyltetrahydro-2H-pyran-4-yl)-7H-pyrrolo[2,3-d]pyrimidine-6-carbonitrile;
2-((1-methyl-3-(oxetan-3-yloxy)-1H-pyrazol-4-yl)amino)-7-((3S,4R)-3-methyltetrahydro-2H-pyran-4-yl)-7H-pyrrolo[2,3-d]pyrimidine-6-carbonitrile;
2-((1-methyl-3-(oxetan-3-yloxy)-1H-pyrazol-4-yl)amino)-7-((trans)-3-methyltetrahydro-2H-pyran-4-yl)-7H-pyrrolo[2,3-d]pyrimidine-6-carbonitrile;
2-((1-methyl-3-(oxetan-3-yloxy)-1H-pyrazol-4-yl)amino)-7-((3R,4R)-3-methyltetrahydro-2H-pyran-4-yl)-7H-pyrrolo[2,3-d]pyrimidine-6-carbonitrile;
2-((1-methyl-3-(oxetan-3-yloxy)-1H-pyrazol-4-yl)amino)-7-((3S,4S)-3-methyltetrahydro-2H-pyran-4-yl)-7H-pyrrolo[2,3-d]pyrimidine-6-carbonitrile;
((1-methyl-3-(oxetan-3-yloxy)-1H-pyrazol-4-yl)amino)-7-((cis)-3-methyltetrahydro-2H-pyran-4-yl)-7H-pyrrolo[2,3-d]pyrimidine-6-carbonitrile;
7-[(1S)-2-methoxy-1-methyl-ethyl]-2-[[1-methyl-3-[(2S,3R)-2-methyloxetan-3-yl]oxy-pyrazol-4-yl]amino]pyrrolo[2,3-d]pyrimidine-6-carbonitrile;
7-[(1S)-2-methoxy-1-methyl-ethyl]-2-[[1-methyl-3-[(2R,3S)-2-methyloxetan-3-yl]oxy-pyrazol-4-yl]amino]pyrrolo[2,3-d]pyrimidine-6-carbonitrile;
7-[(1S)-2-methoxy-1-methyl-ethyl]-2-[[1-methyl-3-[(trans)-2-methyloxetan-3-yl]oxy-pyrazol-4-yl]amino]pyrrolo[2,3-d]pyrimidine-6-carbonitrile;
7-[(1S)-2-methoxy-1-methyl-ethyl]-2-[[3-[(2S,3R)-2-methyloxetan-3-yl]oxy-1-(methyl-d3)pyrazol-4-yl]amino]pyrrolo[2,3-d]pyrimidine-6-carbonitrile;
7-[(1S)-2-methoxy-1-methyl-ethyl]-2-[[3-[(2R,3S)-2-methyloxetan-3-yl]oxy-1-(methyl-d3)pyrazol-4-yl]amino]pyrrolo[2,3-d]pyrimidine-6-carbonitrile;
7-[(1S)-2-methoxy-1-methyl-ethyl]-2-[[3-[(trans)-2-methyloxetan-3-yl]oxy-1-(methyl-d3)pyrazol-4-yl]amino]pyrrolo[2,3-d]pyrimidine-6-carbonitrile;
7-[(2S)-1-methoxypropan-2-yl]-2-[[3-(oxetan-3-yloxy)-1-(methyl-d3)pyrazol-4-yl]amino]pyrrolo[2,3-d]pyrimidine-6-carbonitrile;
2-[[3-(cyclopropoxy)-1-(methoxymethyl)pyrazol-4-yl]amino]-7-[(1S)-2-methoxy-1-methyl-ethyl]pyrrolo[2,3-d]pyrimidine-6-carbonitrile;
7-[(1S)-2-methoxy-1-methyl-ethyl]-2-[[1-(methylsulfonylmethyl)-3-(oxetan-3-yloxy)pyrazol-4-yl]amino]pyrrolo[2,3-d]pyrimidine-6-carbonitrile; and
2-((1-methyl-3-(oxetan-3-yloxy)-1H-pyrazol-4-yl-5-d)amino)-7-((3R,4R)-4-methyltetrahydrofuran-3-yl)-7H-pyrrolo[2,3-d]pyrimidine-6-carbonitrile;
or a pharmaceutically acceptable salt thereof.

In one embodiment, the compound of formula (I) is selected from the group consisting of:
2-((1-methyl-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-methyl-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-[[1-methyl-3-(oxetan-3-yloxy)pyrazol-4-yl]amino]-7-[(3R,4R)-4-methyltetrahydrofuran-3-yl]pyrrolo[2,3-d]pyrimidine-6-carbonitrile;
2-((1-(methyl-d3)-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-(methyl-d3)-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-(methyl-d3)-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile;
2-((3-isopropoxy-1-methyl-1H-pyrazol-4-yl)amino)-7-((3R,4R)-4-methyltetrahydrofuran-3-yl)-7H-pyrrolo[2,3-d]pyrimidine-6-carbonitrile;
2-((1-methyl-3-(oxetan-3-yloxy)-1H-pyrazol-4-yl)amino)-7-(tetrahydro-2H-pyran-4-yl)-7H-pyrrolo[2,3-d]pyrimidine-6-carbonitrile;
2-((1-methyl-3-(oxetan-3-yloxy)-1H-pyrazol-4-yl)amino)-7-((3R,4S)-3-methyltetrahydro-2H-pyran-4-yl)-7H-pyrrolo[2,3-d]pyrimidine-6-carbonitrile;
2-((1-methyl-3-(oxetan-3-yloxy)-1H-pyrazol-4-yl)amino)-7-((3S,4R)-3-methyltetrahydro-2H-pyran-4-yl)-7H-pyrrolo[2,3-d]pyrimidine-6-carbonitrile;
7-[(1S)-2-methoxy-1-methyl-ethyl]-2-[[1-methyl-3-[(2S,3R)-2-methyloxetan-3-yl]oxy-pyrazol-4-yl]amino]pyrrolo[2,3-d]pyrimidine-6-carbonitrile;
7-[(1S)-2-methoxy-1-methyl-ethyl]-2-[[1-methyl-3-[(2R,3S)-2-methyloxetan-3-yl]oxy-pyrazol-4-yl]amino]pyrrolo[2,3-d]pyrimidine-6-carbonitrile;
7-[(1S)-2-methoxy-1-methyl-ethyl]-2-[[3-[(2S,3R)-2-methyloxetan-3-yl]oxy-1-(methyl-d3)pyrazol-4-yl]amino]pyrrolo[2,3-d]pyrimidine-6-carbonitrile;
7-[(1S)-2-methoxy-1-methyl-ethyl]-2-[[3-[(2R,3S)-2-methyloxetan-3-yl]oxy-1-(methyl-d3)pyrazol-4-yl]amino]pyrrolo[2,3-d]pyrimidine-6-carbonitrile; and
2-[[3-(cyclopropoxy)-1-(methoxymethyl)pyrazol-4-yl]amino]-7-[(1S)-2-methoxy-1-methyl-ethyl]pyrrolo[2,3-d]pyrimidine-6-carbonitrile;
or a pharmaceutically acceptable salt thereof.

In one embodiment, the compound of formula (I) is 2-((1-methyl-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile; or a pharmaceutically acceptable salt thereof.

In one embodiment, the compound of formula (I) is 2-((1-methyl-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile; or a pharmaceutically acceptable salt thereof.

In one embodiment, the compound of formula (I) is 2-[[1-methyl-3-(oxetan-3-yloxy)pyrazol-4-yl]amino]-7-[(3R,4R)-4-methyltetrahydrofuran-3-yl]pyrrolo[2,3-d]pyrimidine-6-carbonitrile; or a pharmaceutically acceptable salt thereof.

In one embodiment, the compound of formula (I) is 2-((1-(methyl-d3)-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile; or a pharmaceutically acceptable salt thereof.

In one embodiment, the compound of formula (I) is 2-((1-(methyl-d3)-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile; or a pharmaceutically acceptable salt thereof.

In one embodiment, the compound of formula (I) is 2-((1-(methyl-d3)-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile; or a pharmaceutically acceptable salt thereof.

In one embodiment, the compound of formula (I) is 2-((3-isopropoxy-1-methyl-1H-pyrazol-4-yl)amino)-7-((3R,4R)-4-methyltetrahydrofuran-3-yl)-7H-pyrrolo[2,3-d]pyrimidine-6-carbonitrile; or a pharmaceutically acceptable salt thereof.

In one embodiment, the compound of formula (I) is 2-((1-methyl-3-(oxetan-3-yloxy)-1H-pyrazol-4-yl)amino)-7-(tetrahydro-2H-pyran-4-yl)-7H-pyrrolo[2,3-d]pyrimidine-6-carbonitrile; or a pharmaceutically acceptable salt thereof.

In one embodiment, the compound of formula (I) is 2-((1-methyl-3-(oxetan-3-yloxy)-1H-pyrazol-4-yl)amino)-7-((3R,4S)-3-methyltetrahydro-2H-pyran-4-yl)-7H-pyrrolo[2,3-d]pyrimidine-6-carbonitrile; or a pharmaceutically acceptable salt thereof.

In one embodiment, the compound of formula (I) is 2-((1-methyl-3-(oxetan-3-yloxy)-1H-pyrazol-4-yl)amino)-7-((3S,4R)-3-methyltetrahydro-2H-pyran-4-yl)-7H-pyrrolo[2,3-d]pyrimidine-6-carbonitrile; or a pharmaceutically acceptable salt thereof.

In one embodiment, the compound of formula (I) is 7-[(1S)-2-methoxy-1-methylethyl]-2-[[1-methyl-3-[(2S,3R)-2-methyloxetan-3-yl]oxy-pyrazol-4-yl]amino]pyrrolo[2,3-d]pyrimidine-6-carbonitrile; or a pharmaceutically acceptable salt thereof.

In one embodiment, the compound of formula (I) is 7-[(1S)-2-methoxy-1-methylethyl]-2-[[1-methyl-3-[(2R,3S)-2-methyloxetan-3-yl]oxy-pyrazol-4-yl]amino]pyrrolo[2,3-d]pyrimidine-6-carbonitrile; or a pharmaceutically acceptable salt thereof.

In one embodiment, the compound of formula (I) is 7-[(1S)-2-methoxy-1-methylethyl]-2-[[3-[(2S,3R)-2-methyloxetan-3-yl]oxy-1-(methyl-d3)pyrazol-4-yl]amino]pyrrolo[2,3-d]pyrimidine-6-carbonitrile; or a pharmaceutically acceptable salt thereof.

In one embodiment, the compound of formula (I) is 7-[(1S)-2-methoxy-1-methylethyl]-2-[[3-[(2R,3S)-2-methyloxetan-3-yl]oxy-1-(methyl-d3)pyrazol-4-yl]amino]pyrrolo[2,3-d]pyrimidine-6-carbonitrile; or a pharmaceutically acceptable salt thereof.

In one embodiment, the compound of formula (I) is 2-[[3-(cyclopropoxy)-1-(methoxymethyl)pyrazol-4-yl]amino]-7-[(1S)-2-methoxy-1-methyl-ethyl]pyrrolo[2,3-d]pyrimidine-6-carbonitrile; or a pharmaceutically acceptable salt thereof.

The compounds of formula (I) may comprise one or more asymmetric carbon atoms. They may thus exist in the form of enantiomers or diastereoisomers. These enantiomers and diastereoisomers, and also mixtures thereof, including racemic mixtures are provided herein.

Some of the compounds of formula (I) may exist in the form of bases or of acid-addition salts. Such addition salts form part of the disclosure. These salts are advantageously prepared with pharmaceutically acceptable acids, but the salts of other acids that are useful, for example, for purification or isolation of the compounds of formula (I) also form part of the disclosure.

Another embodiment is a process for preparing a compound of formula (I), comprising reacting a compound of formula (11X) with a compound of formula (15X): 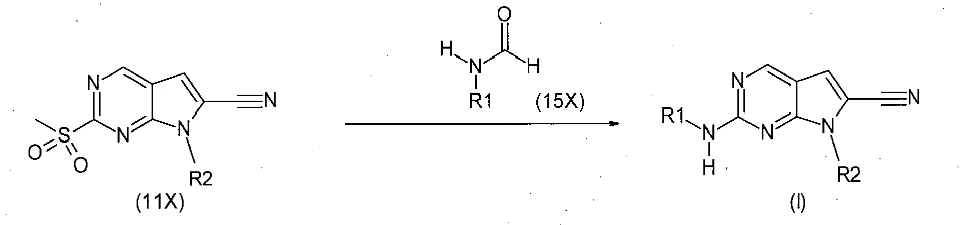 wherein R1 and R2 are as defined herein for a compound of formula (I).

Another embodiment is a pharmaceutical composition comprising a compound of formula (I) or a pharmaceutically acceptable salt thereof, an enantiomer, or a diastereomer and a pharmaceutically acceptable excipient.

Another embodiment is a pharmaceutical composition comprising as active principle an effective dose of a compound of formula (I), or a pharmaceutically acceptable salt thereof, or an enantiomer, or a diastereomer of said compound or pharmaceutically acceptable salt thereof, and at least one pharmaceutically acceptable excipient.

One embodiment is a pharmaceutical composition comprising a compound of formula (I) or a pharmaceutically acceptable salt thereof and at least one pharmaceutically acceptable excipient, wherein the compound of formula (I) is 2-((1-methyl-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile; or a pharmaceutically acceptable salt thereof.

One embodiment is a pharmaceutical composition comprising a compound of formula (I) or a pharmaceutically acceptable salt thereof and at least one pharmaceutically acceptable excipient, wherein the compound of formula (I) is 2-((1-methyl-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile; or a pharmaceutically acceptable salt thereof.

One embodiment is a pharmaceutical composition comprising a compound of formula (I) or a pharmaceutically acceptable salt thereof and at least one pharmaceutically acceptable excipient, wherein the compound of formula (I) is 2-[[1-methyl-3-(oxetan-3-yloxy)pyrazol-4-yl]amino]-7-[(3R,4R)-4-methyltetrahydrofuran-3-yl]pyrrolo[2,3-d]pyrimidine-6-carbonitrile; or a pharmaceutically acceptable salt thereof.

One embodiment is a pharmaceutical composition comprising a compound of formula (I) or a pharmaceutically acceptable salt thereof and at least one pharmaceutically acceptable excipient, wherein the compound of formula (I) is 2-((1-(methyl-d3)-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile; or a pharmaceutically acceptable salt thereof.

One embodiment is a pharmaceutical composition comprising a compound of formula (I) or a pharmaceutically acceptable salt thereof and at least one pharmaceutically acceptable excipient, wherein the compound of formula (I) is 2-((1-(methyl-d3)-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile; or a pharmaceutically acceptable salt thereof.

One embodiment is a pharmaceutical composition comprising a compound of formula (I) or a pharmaceutically acceptable salt thereof and at least one pharmaceutically acceptable excipient, wherein the compound of formula (I) is 2-((1-(methyl-d3)-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile; or a pharmaceutically acceptable salt thereof.

One embodiment is a pharmaceutical composition comprising a compound of formula (I) or a pharmaceutically acceptable salt thereof and at least one pharmaceutically acceptable excipient, wherein the compound of formula (I) is 2-((3-isopropoxy-1-methyl-1H-pyrazol-4-yl)amino)-7-((3R,4R)-4-methyltetrahydrofuran-3-yl)-7H-pyrrolo[2,3-d]pyrimidine-6-carbonitrile; or a pharmaceutically acceptable salt thereof.

One embodiment is a pharmaceutical composition comprising a compound of formula (I) or a pharmaceutically acceptable salt thereof and at least one pharmaceutically acceptable excipient, wherein the compound of formula (I) is 2-((1-methyl-3-(oxetan-3-yloxy)-1H-pyrazol-4-yl)amino)-7-(tetrahydro-2H-pyran-4-yl)-7H-pyrrolo[2,3-d]pyrimidine-6-carbonitrile; or a pharmaceutically acceptable salt thereof.

One embodiment is a pharmaceutical composition comprising a compound of formula (I) or a pharmaceutically acceptable salt thereof and at least one pharmaceutically acceptable excipient, wherein the compound of formula (I) is 2-((1-methyl-3-(oxetan-3-yloxy)-1H-pyrazol-4-yl)amino)-7-((3R,4S)-3-methyltetrahydro-2H-pyran-4-yl)-7H-pyrrolo[2,3-d]pyrimidine-6-carbonitrile; or a pharmaceutically acceptable salt thereof.

One embodiment is a pharmaceutical composition comprising a compound of formula (I) or a pharmaceutically acceptable salt thereof and at least one pharmaceutically acceptable excipient, wherein the compound of formula (I) is 2-((1-methyl-3-(oxetan-3-yloxy)-1H-pyrazol-4-yl)amino)-7-((3S,4R)-3-methyltetrahydro-2H-pyran-4-yl)-7H-pyrrolo[2,3-d]pyrimidine-6-carbonitrile; or a pharmaceutically acceptable salt thereof.

One embodiment is a pharmaceutical composition comprising a compound of formula (I) or a pharmaceutically acceptable salt thereof and at least one pharmaceutically acceptable excipient, wherein the compound of formula (I) is 7-[(1S)-2-methoxy-1-methyl-ethyl]-2-[[1-methyl-3-[(2S,3R)-2-methyloxetan-3-yl]oxy-pyrazol-4-yl]amino]pyrrolo[2,3-d]pyrimidine-6-carbonitrile; or a pharmaceutically acceptable salt thereof.

One embodiment is a pharmaceutical composition comprising a compound of formula (I) or a pharmaceutically acceptable salt thereof and at least one pharmaceutically acceptable excipient, wherein the compound of formula (I) is 7-[(1S)-2-methoxy-1-methyl-ethyl]-2-[[1-methyl-3-[(2R,3S)-2-methyloxetan-3-yl]oxy-pyrazol-4-yl]amino]pyrrolo[2,3-d]pyrimidine-6-carbonitrile; or a pharmaceutically acceptable salt thereof.

One embodiment is a pharmaceutical composition comprising a compound of formula (I) or a pharmaceutically acceptable salt thereof and at least one pharmaceutically acceptable excipient, wherein the compound of formula (I) is 7-[(1S)-2-methoxy-1-methyl-ethyl]-2-[[3-[(2S,3R)-2-methyloxetan-3-yl]oxy-1-(methyl-d3)pyrazol-4-yl]amino]pyrrolo[2,3-d]pyrimidine-6-carbonitrile; or a pharmaceutically acceptable salt thereof.

One embodiment is a pharmaceutical composition comprising a compound of formula (I) or a pharmaceutically acceptable salt thereof and at least one pharmaceutically acceptable excipient, wherein the compound of formula (I) is 7-[(1S)-2-methoxy-1-methyl-ethyl]-2-[[3-[(2R,3S)-2-methyloxetan-3-yl]oxy-1-(methyl-d3)pyrazol-4-yl]amino]pyrrolo[2,3-d]pyrimidine-6-carbonitrile; or a pharmaceutically acceptable salt thereof.

One embodiment is a pharmaceutical composition comprising a compound of formula (I) or a pharmaceutically acceptable salt thereof and at least one pharmaceutically acceptable excipient, wherein the compound of formula (I) is 2-[[3-(cyclopropoxy)-1-(methoxymethyl)pyrazol-4-yl]amino]-7-[(1S)-2-methoxy-1-methylethyl]pyrrolo[2,3-d]pyrimidine-6-carbonitrile; or a pharmaceutically acceptable salt thereof.

One embodiment is a method for treating a disease or disorder selected from the group consisting of neurodegenerative diseases, said method comprising administering to a patient in need thereof a therapeutically effective amount of the compound of formula (I), or a pharmaceutically acceptable salt thereof.

One embodiment is a method for treating a neurodegenerative disease is selected from the group consisting of Parkinson's disease, multiple sclerosis, HIV-induced dementia, Amyotrophic lateral sclerosis, Lewy body dementia, Pick disease, progressive supranuclear palsy, and frontotemporal dementia, said method comprising administering to a patient in need thereof a therapeutically effective amount of the compound of formula (I), or a pharmaceutically acceptable salt thereof.

One embodiment is a method for treating a neurodegenerative disease is selected from the group consisting of Parkinson's disease, multiple sclerosis, HIV-induced dementia, Amyotrophic lateral sclerosis and Lewy body dementia, said method comprising administering to a patient in need thereof a therapeutically effective amount of the compound of formula (I), or a pharmaceutically acceptable salt thereof.

One embodiment is a method for treating a tauopathy disorder is selected from the group consisting of Pick disease, progressive supranuclear palsy, and frontotemporal dementia, said method comprising administering to a patient in need thereof a therapeutically effective amount of the compound of formula (I), or a pharmaceutically acceptable salt thereof.

One embodiment is a method of treating Parkinson's disease, comprising administering to a patient in need thereof a therapeutically effective amount of a compound of formula (I), or a pharmaceutically acceptable salt thereof.

One embodiment is a medicament, characterized in that it comprises a compound of formula (I), or a pharmaceutically acceptable salt thereof.

One embodiment is a compound of formula (I) or a pharmaceutically acceptable salt thereof, for use in the treatment of a disease or disorder selected from the group consisting of neurodegenerative diseases.

One embodiment is a compound of formula (I) or a pharmaceutically acceptable salt thereof, for use in the treatment of a neurodegenerative disease is selected from the group consisting of Parkinson's disease, multiple sclerosis, HIV-induced dementia, Amyotrophic lateral sclerosis, Lewy body dementia, Pick disease, progressive supranuclear palsy and frontotemporal dementia.

One embodiment is a compound of formula (I) or a pharmaceutically acceptable salt thereof, for use in the treatment of a neurodegenerative disease is selected from the group consisting of Parkinson's disease, multiple sclerosis, HIV-induced dementia, Amyotrophic lateral sclerosis, and Lewy body dementia.

One embodiment is a compound of formula (I) or a pharmaceutically acceptable salt thereof, for use in the treatment of a tauopathy disorder is selected from the group consisting of Pick disease, progressive supranuclear palsy, and frontotemporal dementia.

One embodiment is a compound of formula (I) or a pharmaceutically acceptable salt thereof, for use in the treatment of Parkinson' s disease.

### GENERAL PROCEDURES:

Starting materials and solvents used in the synthesis were obtained from chemical vendors such as ABCR, Aldrich, Acros, Apollo, Fluka, Netchem, Lancaster and others.

Generally, crude products were purified by column chromatography or flash chromatography.

The compounds of formula (I) herein can be prepared by the methods outlined in the following reaction schemes and examples.

The preparation of the compounds of formula (11X) can be carried out according to Scheme 1.

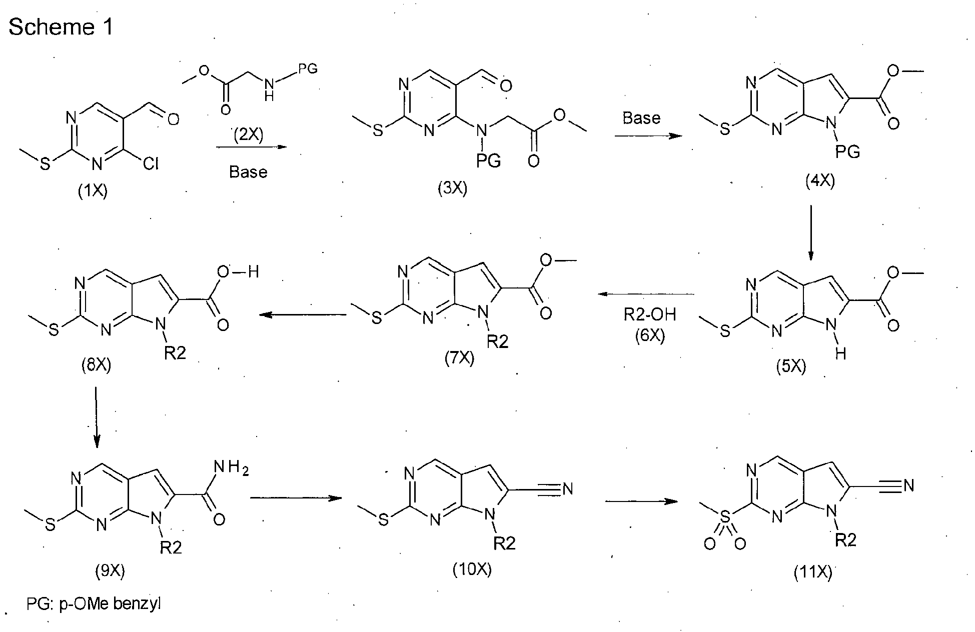

Commercially available 4-chloro-2-(methylthio)pyrimidine-5-carbaldehyde (1X) can be converted to derivative (3X) by reacting with protected methyl glycinate (2X) in the presence of a base such as triethylamine in a polar aprotic solvent such as DCM or THF. Derivative (3X) is subjected to cyclization reaction in the presence of an organic base such as DBU, or an inorganic base such as sodium hydride in a polar aprotic solvent such as acetonitrile, DMF or THF to give compound (4X). The protecting group can be removed according to the methods known to those skilled in the art to give derivative (5X).

The Mitsunobu reaction of compound (5X) with alcohol R2-OH of formula (6X) in the presence of diazodicarboxylate (DIAD or DEAD) and triphenylphosphine in a polar aprotic solvent such as THF, at a temperature between ambient to 60°C, followed by an hydrolysis of the ester (7X) with a source of hydroxide such as NaOH or LiOH gives the carboxylic acid (8X) in which R2 is defined as above. Reacting the carboxylic acid (8X) with NH₄OH, in the presence of a coupling agent such as CDI, HATU, HBTU, in a solvent such as DMF, gives amide (9X). Nitrile derivative (10X) can be obtained by dehydration of carboxamide (9X) using phosphorus oxychloride or ethyl phosphorodichloridate in the presence of DBU in a solvent such as DCM, or using trifluoroacetic anhydride in the presence of triethylamine in a solvent such as THF or using a propanephosphonic anhydride such as T3P. Finally, oxidation of the sulfur with an oxidizing agent such as 3-chloroperbenzoic acid, aqueous hydrogen peroxide, sodium perborate tetrahydrate or sodium bromate or oxone gives the derivative (11X).

The preparation of the compounds of formula (7X) can also be carried out according to Scheme 2 in which R2 is defined as above.

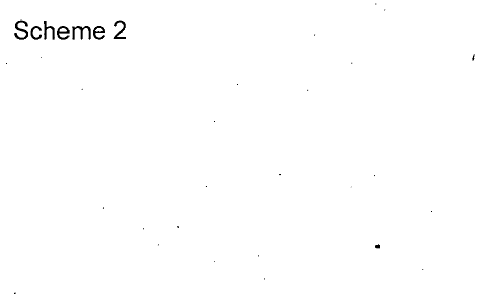

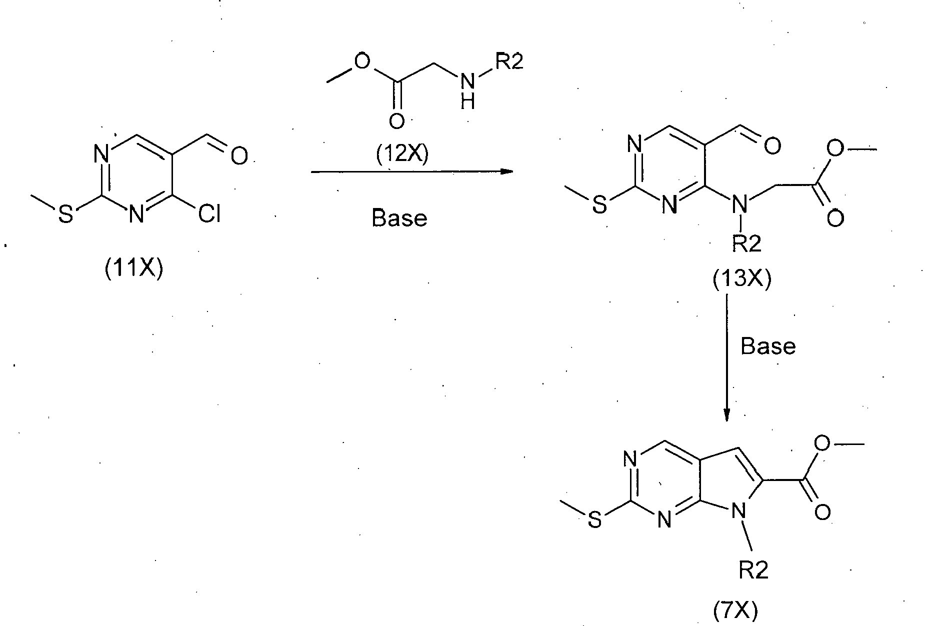

Commercially available 4-chloro-2-(methylthio)pyrimidine-5-carbaldehyde (11X) can be converted to compound (13X) in which R2 is defined as above by reacting with substituted methyl glycinate of formula (12X) in the presence of a base such as triethylamine in a polar aprotic solvent such as DCM or THF. Cyclization of compound (13X) in the presence of an organic base such as DBU, or an inorganic base such as sodium hydride in a polar aprotic solvent such as acetonitrile, DMF or THF, gives the compound (7X).

The preparation of the compounds of formula (15X) can be carried out according to Scheme 3.

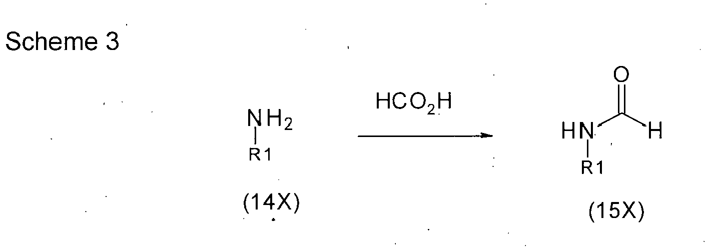

Compounds of formula (15X) in which R1 is defined as above can be prepared from the compound (14X) by reaction with formic acid, optionally in the presence of acetic anhydride, at a temperature between 0°C and ambient temperature. Most of the compound of formula (14X) can be prepared according to the methods known to those skilled in the art.

The following scheme (Scheme 4) provides a process to prepare compound of formula (14X) where R1 is a substituted pyrazole.

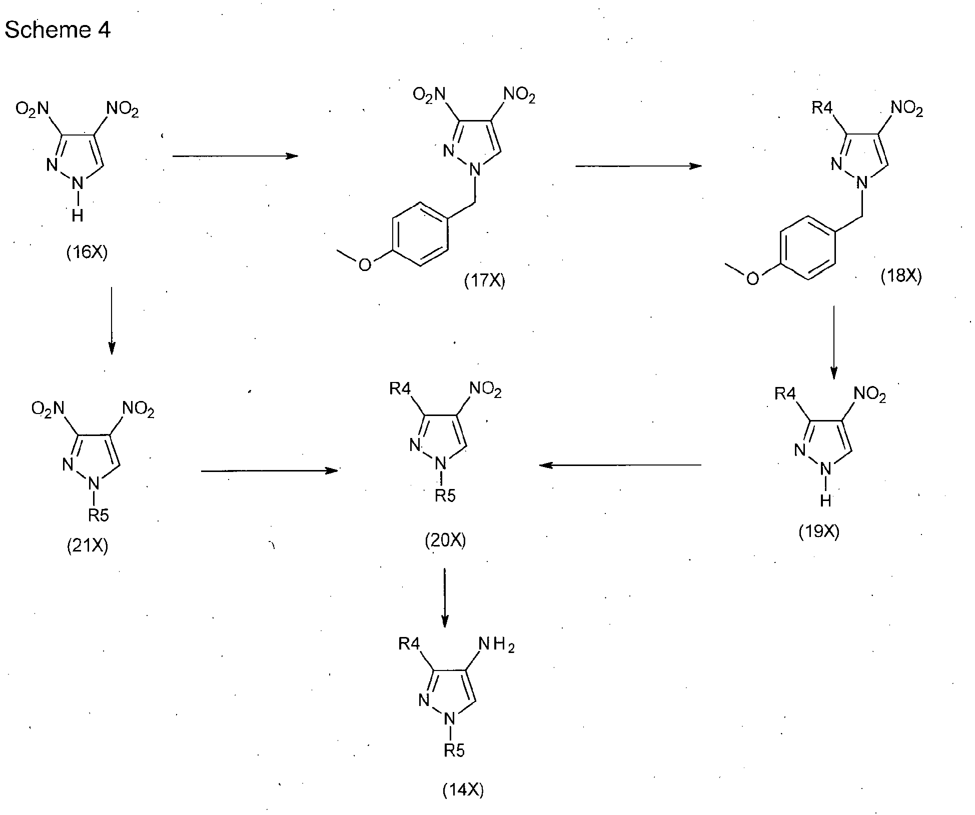

A pyrazole of general formula (14X) in which R4 is an alkyloxyl group and R5 is defined in compounds of formula (Ib) herein above can be synthesized from commercially available 3,4-dinitro-1H-pyrazole (16X). Pyrazole (16X) can be alkylated into compound (21X) with an halide R5X wherein X is Cl, Br or I or with a sulfonate R5OSO₂R' such as a mesylate (R'= CH₃), a tosylate (R'= PhMe), a triflate (R'= CF₃) or a nonaflate (R'= CF₂CF₂CF₂CF₃) in presence of a base such as potassium carbonate or cesium carbonate or sodium hydride in a polar aprotic solvent such as DMF, NMP or DMSO, at a temperature between ambient and 80°C. Alternatively, 3,4-dinitro-1H-pyrazole (16X) can be converted to pyrazole (21X) via a Chan Lam coupling by reacting with a boronic acid R5B(OH)₂ in presence of Cu(OAc)₂ and a base such as pyridine or 4'-di-tert-butyl-2,2'-bipyridine in an aprotic solvent such as dichloromethane or 1,2-dichloroethane at reflux. When R5 is an alkylsulfonyl group, pyrazole (16X) can be sulfonylated with a sulfonyl chloride in presence of a base such as triethylamine in an aprotic solvent such as dichloromethane at a temperature between 0°C and ambient temperature.

Dinitro-pyrazole (21X) can be treated with an alcohol R4H in presence of a base such as potassium carbonate, cesium carbonate, or sodium hydride in a polar aprotic solvent such as DMF, NMP, or DMSO, at a temperature between ambient and 80°C to provide compound (20X).

Alternatively, 4-dinitro-1H-pyrazole (16X) can be protected with a para-methoxybenzyl group under the alkylation conditions leading to compound (17X). The resulting protected pyrazole (17X) can be treated with an alcohol of formula R4H to give compound (18X) similarly to the transformation of (21X) to (20X). Deprotection of compound (18X) to pyrazole (19X) can be achieved in conditions known by the person skilled in the art, for instance by reacting with ceric ammonium nitrate. The resulting deprotected pyrazole (19X) can be alkylated into compound (20X) with similar conditions than in the step going from compound (16X) to (21X).

Nitro-substituted pyrazole (20X) can be reduced into the corresponding amino-pyrazole (14X) for example under hydrogen pressure in presence of palladium on carbon in an aprotic or protic solvent.

The preparation of the compounds of general formula (I) can be carried out according to Scheme 5.

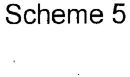

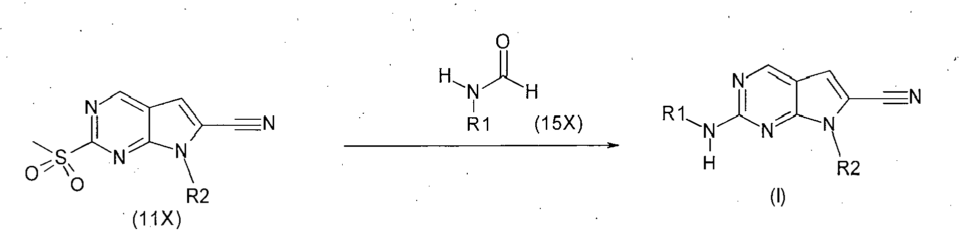

Reacting compound (15X) with pyrrolopyrimidine (11X) in the presence of an organic base such as DBU or BTTP, or an inorganic base such as cesium carbonate, potassium tertiobutylate, or sodium hydride, in a polar aprotic solvent such as DMF, or DMSO, at a temperature between ambient and 60°C, gives the compound of general formula (I).

The embodiments provided herein will be explained more specifically with reference to the following examples, however, the scope of the embodiments provided herein is not limited to these examples.

### ABBREVIATION

Unless otherwise stated, the following abbreviations have the stated meanings in the examples below:
- AcOH: acetic acid
- BTTP: *tert*-butylimino-tri(pyrrolidino)phosphorane
- HCI: hydrogen chloride
- CDI: 1,1'-carbonyldiimidazole
- DBU: 1,8-diazabicyclo[5.4.0]undec-7-ene
- DCM: dichloromethane
- DIAD: diisopropyl azodiformate
- DEAD: diethoxycarbonyldiazene
- DMF: *N*,*N*-dimethylformamide
- DMSO: dimethyl sulfoxide
- eq.: equivalent
- Et: ethyl
- EtOH: ethanol
- Et₂O: diethyl ether
- EtOAc: ethyl acetate
- HAUT: 1-[bis(dimethylamino)methylene]-1*H*-1,2,3-triazolo[4,5-*b*]pyridinium 3-oxide hexafluorophosphate
- HBTU: *N*,*N*,*N*',*N*'-tetramethyl-O-(1*H*-benzotriazol-1-yl)uronium hexafluorophosphate
- HPLC: high performance liquid chromatography
- LC/MS: liquid chromatography-mass spectrometry
- LiOH: lithum hydroxide
- NaOH: sodium hydroxide
- NH₄OH: ammonium hydroxide
- NMP: 1-Methyl-2-pyrrolidone
- Me: methyl
- MeOH: methanol
- nM: nanomolar
- NMR: nuclear magnetic spectroscopy
- r.t.: room temperature
- THF: tetrahydrofuran
- T₃P: 1-Propanephosphonic anhydride

LC/MS analysis is performed using the following methods
Method A:
UPLC waters and mass spectrometer SQD2 Waters
Purity is measured by UV diode array detector (192 - 400 nm)
Eluent A: H₂O (+ 0.1% of HCO₂H)
Eluent B: CH₃CN (+ 0.1% of HCO₂H)
Gradient:

| Time (min) | A% | B% |
|---|---|---|
| 0.00 | 95 | 5 |
| 0.20 | 95 | 5 |
| 3.60 | 2 | 98 |
| 4.10 | 2 | 98 |
| 5.00 | 95 | 5 |

Flow rate: 0.8 mL/min
Column: Acquity UPLC CSH Waters C18, 2.1x50mm, 1.7µm
Method B:
Waters UPLC-SQD2
Eluent A: H₂O (+ 0.1% of HCO₂H)
Eluent B: CH₃CN (+ 0.1% of HCO₂H)
Gradient (2.5 minutes): from 3 to 100% of B in 2.1 minutes; 2.45 minutes: 100% of B; 2.5 minutes: 3% of B
Flow rate: 1 mL/min
Column: ACQUITY CSH C18, 1.7 µm, 2.1 × 50 mm
Method C:
HPLC Waters XeVo - QTof
Eluent A: H₂O (+ 0.1% of HCO₂H)
Eluent B: CH₃CN (+ 0.1% of HCO₂H)
Gradient (5.3 minutes): 5% of B from 0 to 0.3 min; from 5 to 100% of B in 4 minutes; 100% of B from 4 to 4.6 min; 5.3 minutes: 5% of B
Flow rate: 0.5 mL/min
Column: ACQUITY CSH C18, 1.7 µm, 2.1 × 100 mm
Method D:
UPLC waters and mass spectrometer SQD Waters
Eluent A: H₂O (+ 0.1% of HCO₂H)
Eluent B: CH₃CN (+ 0.1% of HCO₂H)
Gradient:

| Time (min) | A% | B% |
|---|---|---|
| 0 | 98 | 2 |
| 2 | 0 | 100 |
| 2.6 | 0 | 100 |
| 2.7 | 98 | 2 |
| 3 | 98 | 2 |

Flow rate: 1 mL/min
Column: Acquity CORTECS C18, 2.1×50mm, 1.6µm
Method E:
UPLC HCLASS and mass spectrometer SQD2 Waters
Eluent A: H₂O (+ 0.1% of HCO₂H)
Eluent B: CH₃CN (+ 0.1% of HCO₂H)
Gradient:

| Time (min) | A% | B% |
|---|---|---|
| 0 | 95 | 5 |
| 1.00 | 50 | 50 |
| 1.30 | 0 | 100 |
| 1.45 | 0 | 100 |
| 1.75 | 98 | 2 |
| 2.00 | 98 | 2 |

Flow rate: 0.8 mL/min
Column: Cortecs UPLC C18, 2.1×50mm,1.6pm
Method F:
UPLC HCLASS and mass spectrometer SQD2 Waters
Eluent A: H₂O (+ 0.1% of HCO₂H)
Eluent B: CH₃CN (+ 0.1% of HCO₂H)
Gradient:

| Time (min) | A% | B% |
|---|---|---|
| 0 | 98 | 2 |
| 2.50 | 0 | 100 |
| 2.90 | 0 | 100 |
| 2.95 | 98 | 2 |
| 3.00 | 98 | 2 |

Flow rate: 0.8 mL/min
Column: Cortecs UPLC C18, 2.1x50mm, 1.6µm
Method G:
UPLC HCLASS and mass spectrometer SQD2 Waters
Eluent A: H₂O (+ 0.1% of HCO₂H)
Eluent B: CH₃CN (+ 0.1% of HCO₂H)
Gradient:

| Time (min) | %A | %B |
|---|---|---|
| 0 | 98 | 2 |
| 0.5 | 98 | 2 |
| 3.0 | 0 | 100 |
| 3.30 | 0 | 100 |
| 3.40 | 98 | 2 |
| 4.00 | 98 | 2 |

Flow rate: 0.8 mL/min
Column: Cortecs UPLC C18, 2.1×50mm, 1.6µm
Method H:
UPLC waters and mass spectrometer SQD Waters
Purity is measured by UV diode array detector (192 - 400 nm)
Eluent A: H₂O (+ 0.1% of HCO₂H)
Eluent B: CH₃CN (+0.1% of HCO₂H)
Gradient:

| Time (min) | A% | B% |
|---|---|---|
| 0.00 | 95 | 5 |
| 0.20 | 95 | 5 |
| 3.60 | 2 | 98 |
| 4.10 | 2 | 98 |
| 5.00 | 95 | 5 |

Flow rate: 0.8 mL/min
Column: Acquity UPLC CSH Waters C18, 2.1x50mm, 1.7µm
Method I:
LCMS waters and mass spectrometer SQD Waters
Eluent A: H₂O (+ 0.1% of HCO₂H)
Eluent B: CH₃CN (+ 0.1% of HCO₂H)
Gradient: t=0min: 5% of B, t=1.5min, 99% of B, t=1.9min: 99% of B
Flow rate: 1 mL/min
Column: Cortecs UPLC C18, 2.1×50mm, 1.6µm

### INTERMEDIATES

### Intermediate 1: Methyl 2-(methylthio)-7H-pyrrolo[2,3-d]pyrimidine-6-carboxylate

### Step 1: Preparation of methyl (4-methoxybenzyl)glycinate

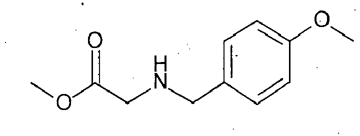

53 mL (1 eq.) of methyl 2-bromoacetate in 225 mL of tetrahydrofuran were slowly added (35 minutes) to a solution of 71 mL (1 eq.) of (4-methoxyphenyl)methanamine and 116 mL of triethylamine (1.5 eq.) in 750 mL of tetrahydrofuran at 0°C (ice water/methanol bath) under argon. After 5 hours at room temperature and completion of the reaction, the mixture was filtered. The filtrate was taken into 975 mL of ethyl acetate and 375 mL of water was added. After drying of the organic layer on magnesium sulfate and concentration under vacuum, the residue was purified on silica gel eluting with dichloromethane 100% then dichloromethane/ethyl acetate (90/10) then dichloromethane/ethyl acetate (70/30) to give 60.9 g of methyl(4-methoxybenzyl)glycinate.

### Step 2: Preparation of methyl N-(5-formyl-2-(methylthio)pyrimidin-4-yl)-N-(4-methoxybenzyl)glycinate

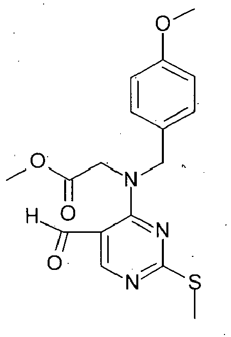

52.5 mL of triethylamine (1.5 eq.) was added to a solution of 49 g of 4-chloro-2-(methylthio)pyrimidine-5-carbaldehyde (1 eq.) in 500 mL of tetrahydrofuran and 50 mL of dichloromethane at 0°C. 63 g of methyl (4-methoxybenzyl)glycinate (1.1 eq) was added dropwise. The mixture was stirred at room temperature for 17 hours. The reaction mixture was diluted with 500 mL of ethyl acetate and water. The organic layer was separated, washed twice with 1000 mL of water and then 1000 mL of a 0.5N HCl aqueous solution. The combined organic layers were dried over magnesium sulfate and concentrated under reduced pressure. The residue was taken into 500 mL of heptane and stirred for 12 hours. The resulting precipitate was filtered and dried under vacuum to afford 90 g of methyl N-(5-formyl-2-(methylthio)pyrimidin-4-yl)-N-(4-methoxybenzyl)glycinate. MS (method B) m/z 362 [M+1]+; t=1.53 min.

### Step 3: Preparation of methyl 7-(4-methoxybenzyl)-2-(methylthio)-7H-pyrrolo[2,3-d]pyrimidine-6-carboxylate

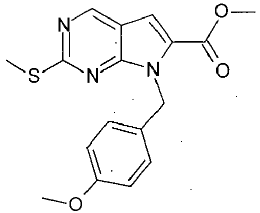

167 mL (4 eq.) of 1,8-diazabicyclo[5.4.0]undec7-ene was added dropwise to a solution of 99 g of methyl N-(5-formyl-2-(methylthio)pyrimidin-4-yl)-N-(4-methoxybenzyl)glycinate (1 eq.) in 1000 mL of acetonitrile. The reaction mixture was heated at 85°C for 40 minutes. After cooling down to room temperature, the reaction mixture was concentrated under reduced pressure. The residue was taken into 800 mL of ethyl acetate and 500 mL of water. The organic layer was washed with 300 mL of a 1N HCI aqueous solution, 300 mL of a saturated aqueous sodium hydrogenocarbonate solution, 500 mL of water, and then 300 mL of brine and concentrated under reduced pressure to afford 82.4 g of methyl 7-(4-methoxybenzyl)-2-(methylthio)-7H-pyrrolo[2,3-d]pyrimidine-6-carboxylate. MS (method B) m/z 344 [M+1]+; t = 1.74 min

### Step 4: Preparation of methyl 2-(methylthio)-7H-pyrrolo[2,3-d]pyrimidine-6-carboxylate

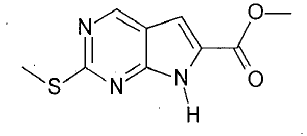

169 mL of trifluoromethanesulfonic acid (10 eq.) was added dropwise to a solution of 62.8 g of methyl 7-(4-methoxybenzyl)-2-(methylthio)-7H-pyrrolo[2,3-d]pyrimidine-6-carboxylate (1 eq.) in 604 mL of trifluoroacetic acid (44 eq.). The reaction mixture was heated at 75°C for 90 minutes. After cooling down to room temperature, the trifluoroacetic acid was concentrated under reduced pressure. The reaction mixture was diluted with 500 mL of dichloromethane and cooled to -15°C. 360 mL of 5M sodium hydroxide solution was added dropwise while maintaining the temperature below 5 °C. When pH 6 was reached a precipitate was formed. The precipitate was filtered then washed with water (twice with 250 mL) and 250 mL of heptane then dried under vacuum to afford 37.72 g of methyl 2-(methylthio)-7H-pyrrolo[2,3-d]pyrimidine-6-carboxylate. MS (method B) m/z 224 [M+1]+; t = 1.18 min

### Intermediate 2 and 3: (3S,4S)-4-methyltetrahydrofuran-3-ol (intermediate 2) and (3R,4R)-4-methyltetrahydrofuran-3-ol (intermediate 3)

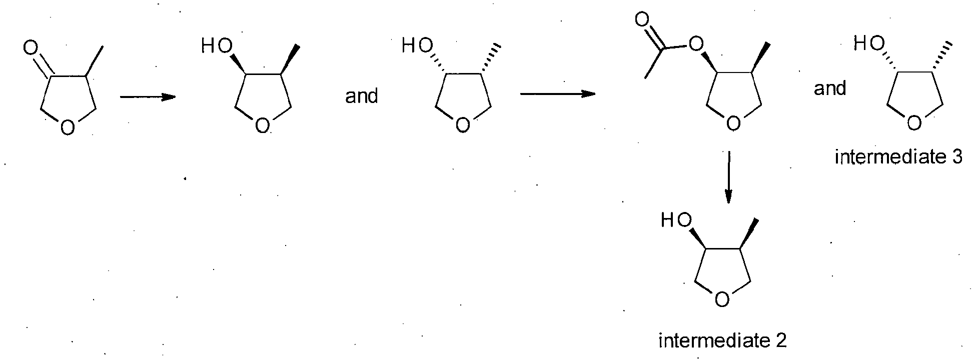

### Step 1: Preparation of cis-4-methyltetrahydrofuran-3-ol (racemic mixture of (3S,4S)-4-methyltetrahydrofuran-3-ol and (3R,4R)-4-methyltetrahydrofuran-3-ol)

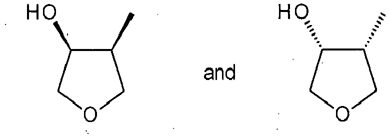

A solution of 20 g (1 eq.) of 4-methyldihydrofuran-3(2H)-one (1 eq) (commercially available) in 100 mL of anhydrous tetrahydrofuran was added in 30 min to a solution of 68 g (1.06 eq.) of (+)-B-Chlorodiisopinocampheylborane [(+)-DIP chloride] in 270 mL of anhydrous tetrahydrofuran cooled at -25°C under argon. The mixture was stirred for 2 hours at -25°C. Then, 46 g (2.16 eq.) of diethanolamine in suspension in tetrahydrofuran was added by portions. The mixture was stirred at room temperature for 18 hours then diluted with 200 mL of pentane and filtered. The filter cake was washed twice with 50 mL of diethyl ether and the filtrate was carefully concentrated at 40°C under reduced pressure of 120 mbars. The residue was dissolved in 50 mL of dichloromethane and 50 mL of cylohexane, filtered and purified on silica gel eluting with 10%, 30%, 50%, and 100% of diethyl ether in cyclohexane. Concentration of the pure fractions at 40°C under reduced pressure of 120 mbars gave 17 g of a racemic mixture of (3S,4S)-4-methyltetrahydrofuran-3-ol and (3R,4R)-4-methyltetrahydrofuran-3-ol.
¹H NMR (400 MHz, CDCl₃) δ in ppm: 1.05 (d, J=7 Hz, 3 H); 2.21 (m, 1 H); 2.24 (s, 1 H); 3.44 (dd, J=8 Hz and J=11 Hz, 1 H); 3.74 (dd, J=1.4 Hz and J=10 Hz, 1 H); 3.90 (t, J=8 Hz, 1 H); 3.93 (dd, J=4 Hz and J=10 Hz, 1 H); 4.19 (m, 1 H).

### Step 2: Preparation of (3S,4S)-4-methyltetrahydrofuran-3-yl acetate (precursor of intermediate 2) and enantiopure (3R,4R)-4-methyltetrahydrofuran-3-ol ― intermediate 3

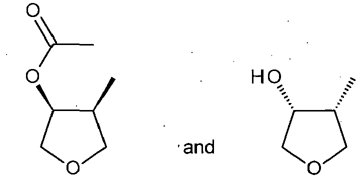

To a solution of 14.7 g (1 eq.) of a racemic mixture of (3S,4S)-4-methyltetrahydrofuran-3-ol and (3R,4R)-4-methyltetrahydrofuran-3-ol in 130 mL of vinyl acetate and 130 mL of pentane were added 1.5 g of lipase AMANO AK (Ref. ALDRICH: Amano lipase from *Pseudomonas fluorescens* 20.000 U/g; catalog Number: 534730-50G), and the suspension was stirred for 16 hours at 22°C then filtered over a pad of decalite. The filter cake was rinsed twice with 50 mL of diethyl ether, and the filtrate was carefully concentrated at 40°C under reduced pressure of 180 mbars. The residue was purified on silica, eluting with 0-50% of ether in cyclohexane to afford successively 9.15 g of (3S,4S)-4-methyltetrahydrofuran-3-yl acetate (precursor of intermediate 2) and 5.64 g of enantiopure (3R,4R)-4-methyltetrahydrofuran-3-ol (intermediate 3).
(3S,4S)-4-methyltetrahydrofuran-3-yl acetate:
   ¹H NMR (400 MHz, CDCl₃) δ in ppm: 1.01 (d, J=7 Hz, 3 H); 2.09 (s, 3 H); 2.39 (m, 1 H); 3.45 (dd, J=8 Hz and J=10 Hz, 1 H); 3.79 (dd, J=2 Hz and J=10 Hz, 1 H); 3.98 (t, J=8 Hz, 1 H); 4.05 (dd, J=4 Hz and J=10 Hz, 1 H); 5.25 (m, 1 H).
(3R,4R)-4-methyltetrahydrofuran-3-ol-intermediate 3:
   ¹H NMR (400 MHz, CDCl₃) δ in ppm: 1.05 (d, J=7 Hz, 3 H); 2.21 (m, 1 H); 2.24 (s, 1 H); 3.44 (dd, J=8 Hz and J=11 Hz, 1 H); 3.74 (dd, J=1.4 Hz and 10 Hz, 1 H); 3.90 (t, J=8 Hz, 1 H); 3.93 (dd, J=4 Hz and 10 Hz, 1 H); 4.19 (m, 1 H).

### Step 3: Preparation of (3S,4S)-4-methyltetrahydrofuran-3-ol-intermediate 2

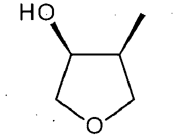

94 mL (1.09 eq.) of a pre-cooled (0°C) 1N solution of sodium methoxide in methanol was added to 12.4 g (86 mmoles; 1 eq.) of (3S,4S)-4-methyltetrahydrofuran-3-yl acetate (precursor of intermediate 2) in 20 mL of methanol at 0°C. The mixture was stirred at 0°C for 1 hour 30 minutes then quenched with 47 mL (1.09 eq.) of a 2N hydrogen chloride solution in diethyl ether. After addition of another 100 mL of diethyl ether, the suspension was filtered over a pad of decalite and the filter cake rinsed twice with 50 mL of diethyl ether. The filtrate was concentrated at 40°C under reduced pressure of 120 mbars, and the residue was dissolved in 50 mL of dichloromethane and 50 mL of cylohexane, filtered and purified on silica gel eluting with 10%, 30%, 50% and 100% of diethyl ether in cyclohexane to afford (after concentration at 40°C under reduced pressure of 120 mbars) 7.2g of enantiopure (3S,4S)-4-methyltetrahydrofuran-3-ol (intermediate 2).
¹H NMR (400 MHz, CDCl₃) δ in ppm: 1.05 (d, J=7 Hz, 3 H); 2.21 (m, 1 H); 2.24 (s, 1 H); 3.44 (dd, J=8 Hz and J=11 Hz, 1 H); 3.74 (dd, J=1.4 Hz and 10 Hz, 1 H); 3.90 (t, J=8 Hz, 1 H); 3.93 (dd, J=4 Hz and 10 Hz, 1 H); 4.19 (m, 1 H).

### Intermediate 4: N-(1-methyl-3-(oxetan-3-yloxy)-1H-pyrazol-4-yl)formamide

### Step 1: Preparation of 1-methyl-4-nitro-3-(oxetan-3-yloxy)-1H-pyrazole

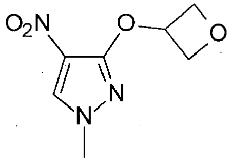

2.03 g (1.3 eq.) of oxetan-3-ol and 13.74 g (2 eq.) of cesium carbonate were added to a solution of 3.63 g (1 eq.) of 1-methyl-3,4-dinitro-1H-pyrazole (commercially available) in 12 mL of acetonitrile. The mixture was heated at 50°C for 2 hours then allowed to cool to room temperature and poured onto ethyl acetate and water. The aqueous layer was separated and extracted three times with ethyl acetate. The combined organic layers were dried over magnesium sulfate and concentrated under vacuum. The residue was suspended into 15 mL of diethyl ether and filtered, concentrated under vacuum and purified on silica gel, eluting with a gradient of 0 to 50% of ethyl acetate in heptane, to give 3.7 g of 1-methyl-4-nitro-3-(oxetan-3-yloxy)-1H-pyrazole. MS (method B) m/z 200 [M+1]+; t=0.98 min.

### Step 2: Preparation of N-(1-methyl-3-(oxetan-3-yloxy)-1H-pyrazol-4-yl)formamide

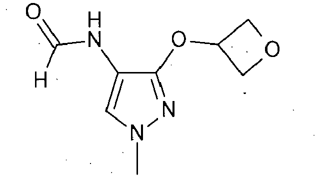

A solution of 3.5 g (1 eq.) of 1-methyl-4-nitro-3-(oxetan-3-yloxy)-1H-pyrazole in 170 mL of methanol was treated with 0.1 g of palladium on carbon (10%) under 2.5 bars of hydrogen for 2 hours. The mixture was filtered with dichloromethane washes and concentrated under reduced pressure. The residue was taken into dichloromethane and concentrated under reduced pressure twice. A solution of 6.63 mL of acetic anhydride in 12.13 mL of formic acid that had been premixed for 30 minutes was added dropwise to a solution of the residue in 12 mL of tetrahydrofuran at 0°C. The reaction mixture was stirred for 2 hours allowing the temperature to warm up to room temperature. It was then poured onto a 10% aqueous solution of sodium bicarbonate, stirred for 15 minutes, and extracted twice with ethyl acetate. The combined organic layers were dried over magnesium sulfate and concentrated under vacuum. The residue was purified on silica gel, eluting with a gradient of 0 to 100% of ethyl acetate in heptane, to afford 2.02 g of N-(1-methyl-3-(oxetan-3-yloxy)-1H-pyrazol-4-yl)formamide. MS (method B) m/z 198 [M+1]+; t=0.62 min.

### Intermediate 5: N-(1-methyl-3-(((3S,4R)-4-methyltetrahydrofuran-3-yl)oxy)-1H-pyrazol-4-yl)formamide and N-(1-methyl-3-(((3R,4S)-4-methyltetrahydrofuran-3-yl)oxy)-1H-pyrazol-4-yl)formamide (racemic mixture)

### Step 1: Preparation of racemic mixture of 1-methyl-3-(((3S,4R)-4-methyltetrahydrofuran-3-yl)oxy)-4-nitro-1H-pyrazole and 1-methyl-3-(((3R,4S)-4-methyltetrahydrofuran-3-yl)oxy)-4-nitro-1H-pyrazole

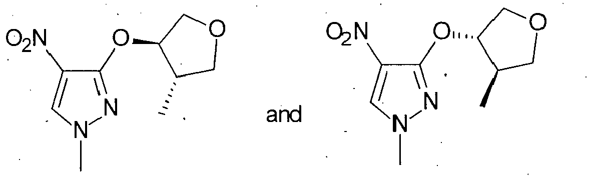

1.54 g (1.3 eq.) of trans-4-methyltetrahydrofuran-3-ol (commercially available) and 7.34 g (2 eq.) of cesium carbonate were added to a solution of 2 g (1 eq.) of 1-methyl-3,4-dinitro-1H-pyrazole (commercially available) in 40 mL of acetonitrile. The mixture was heated at 80°C for 4 hours then allowed to cool to room temperature and poured onto ethyl acetate and water. The aqueous layer was separated and extracted three times with ethyl acetate. The combined organic layers were dried over magnesium sulfate and concentrated under vacuum. The residue was purified on silica gel, eluting with a gradient of 10 to 50% of ethyl acetate in heptane, to give 1.1 g of a racemic mixture of 1-methyl-3-(((3S,4R)-4-methyltetrahydrofuran-3-yl)oxy)-4-nitro-1H-pyrazole and 1-methyl-3-(((3R,4S)-4-methyltetrahydrofuran-3-yl)oxy)-4-nitro-1H-pyrazole. MS (method A) m/z 228 [M+1]+; t=1.68 min.

### Step 2: Preparation of a racemic mixture of 1-methyl-3-(((3S,4R)-4-methyltetrahydrofuran-3-yl)oxy)-1H-pyrazol-4-amine and 1-methyl-3-(((3R,4S)-4-methyltetrahydrofuran-3-yl)oxy)-1H-pyrazol-4-amine

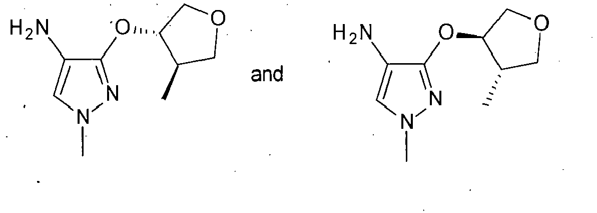

In a microwave vial, a solution of 1.09 g (1 eq.) of a racemic mixture of 1-methyl-3-(((3S,4R)-4-methyltetrahydrofuran-3-yl)oxy)-4-nitro-1H-pyrazole and 1-methyl-3-(((3R,4S)-4-methyltetrahydrofuran-3-yl)oxy)-4-nitro-1H-pyrazole in 32 mL of methanol was treated with 1.15 g of ammonium formate (7 eq.) and 0.326 g of palladium on carbon (10%). The reaction mixture was heated at 70°C for 10 minutes. The mixture was filtered on celite with methanol washes and concentrated under reduced pressure. The residue was taken into dichloromethane and filtered, and the filtrate was concentrated under reduced pressure to afford 904 mg of a racemic mixture of 1-methyl-3-(((3S,4R)-4-methyltetrahydrofuran-3-yl)oxy)-1H-pyrazol-4-amine and 1-methyl-3-(((3R,4S)-4-methyltetrahydrofuran-3-yl)oxy)-1H-pyrazol-4-amine. MS (method A) m/z 198 [M+1]+; t=0.26 min.

### Step 3: Preparation of a racemic mixture of N-(1-methyl-3-(((3S,4R)-4-methyltetrahydrofuran-3-yl)oxy)-1H-pyrazol-4-yl)formamide and N-(1-methyl-3-(((3R,4S)-4-methyltetrahydrofuran-3-yl)oxy)-1H-pyrazol-4-yl)formamide

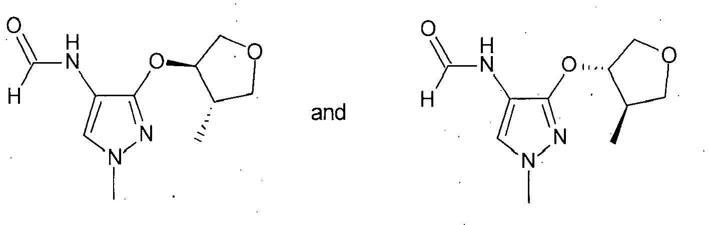

A solution of 904 mg (1 eq.) of a racemic mixture of 1-methyl-3-(((3S,4R)-4-methyltetrahydrofuran-3-yl)oxy)-1H-pyrazol-4-amine and 1-methyl-3-(((3R,4S)-4-methyltetrahydrofuran-3-yl)oxy)-1H-pyrazol-4-amine in 34 mL of tetrahydrofuran was dropwise added to a cooled (0°C) solution of 1.29 mL of acetic anhydride (3 eq.) in 1.07 mL of formic acid (6 eq.) that had been premixed for 30 minutes at room temperature. The reaction mixture was stirred at 5°C for 5 minutes, at room temperature for 1.5 hours, and then concentrated under vacuum. The residue was purified on silica gel, eluting with a gradient of 0 to 40% of acetone in dichloromethane, to afford 810 mg of a racemic mixture of N-(1-methyl-3-(((3S,4R)-4-methyltetrahydrofuran-3-yl)oxy)-1H-pyrazol-4-yl)formamide and N-(1-methyl-3-(((3R,4S)-4-methyltetrahydrofuran-3-yl)oxy)-1H-pyrazol-4-yl)formamide. MS (method B) m/z 226 [M+1]+; t=0.94 min.

### Intermediate 6: N-(3-(2-hydroxy-2-methylpropoxy)-1-methyl-1H-pyrazol-4-yl)formamide

### Step 1: Preparation of 2-methyl-1-((1-methyl-4-nitro-1H-pyrazol-3-yl)oxy)propan-2-ol

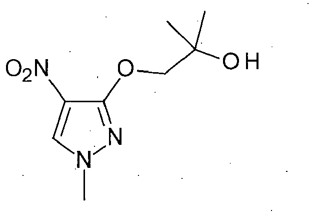

409 mg (1.3 eq.) of 2-methylpropane-1,2-diol and 3.44 g (3 eq.) of cesium carbonate were added to a solution of 600 mg (1 eq.) of 1-methyl-3,4-dinitro-1H-pyrazole (commercially available) in 30 mL of acetonitrile. The mixture was heated at 80°C for 2 hours then allowed to cool to room temperature and poured onto ethyl acetate and water. The aqueous layer was separated and extracted three times with ethyl acetate. The combined organic layers were dried over magnesium sulfate and concentrated under vacuum. The residue was purified on silica gel, eluting with 50% ethyl acetate in heptane, to give 358 mg of 2-methyl-1-((1-methyl-4-nitro-1H-pyrazol-3-yl)oxy)propan-2-ol. MS (method A) m/z 216 [M+ 1]+; t=1.45 min.

### Step 2: Preparation of N-(3-(2-hydroxy-2-methylpropoxy)-1-methyl-1H-pyrazol-4-yl)formamide

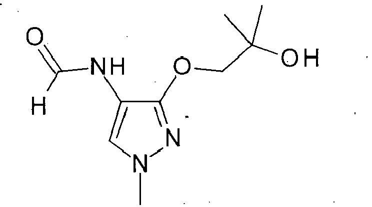

In a microwave vial, a solution of 115 mg (1 eq.) of 2-methyl-1-((1-methyl-4-nitro-1H-pyrazol-3-yl)oxy)propan-2-ol in 4 mL of methanol was treated with 98 mg of ammonium formate (2.8 eq.) and 57 mg (0.1 eq.) of palladium on carbon (10%). The reaction mixture was heated at 70°C for 10 minutes. The mixture was filtered on celite with methanol washes and concentrated under reduced pressure. The crude material dissolved in 2 mL of tetrahydrofuran was dropwise added to a cooled (0°C) solution of 204 µL of acetic anhydride (4 eq.) in 373 µL of formic acid (18 eq.) that had been premixed for 30 minutes at room temperature. The reaction mixture was stirred at 5°C for 1 hour then concentrated under vacuum. The residue was used in the next step without further purification. MS (method A) m/z 214 [M+1]+; t=1.1 min.

### Intermediate 7: N-(1.,methyl-3-((1,1,1-trifluoropropan-2-yl)oxy)-1H-pyrazol-4-yl)formamide

### Step 1: Preparation of 1-methyl-4-nitro-3-((1,1,1-trifluoropropan-2-yl)oxy)-1H-pyrazole (racemic)

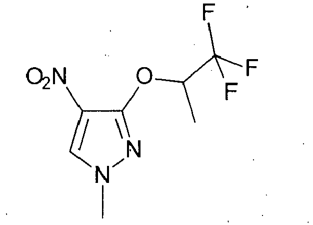

819 mg (1.3 eq.) of racemic 1,1,1-trifluoropropan-2-ol and 3.67 g (2 eq.) of cesium carbonate were added to a solution of 1 g (1 eq.) of 1-methyl-3,4-dinitro-1H-pyrazole (commercially available) in 20 mL of acetonitrile. The mixture was heated at 65°C for 2 hours then allowed to cool to room temperature and poured onto ethyl acetate and water. The aqueous layer was separated and extracted three times with ethyl acetate. The combined organic layers were dried over magnesium sulfate and concentrated under vacuum. The residue was purified on silica gel, eluting with a gradient of 20 to 50% of ethyl acetate in heptane, to afford 1.25 g of racemic 1-methyl-4-nitro-3-((1,1,1-trifluoropropan-2-yl)oxy)-1H-pyrazole. MS (method A) m/z 240 [M+1]+; t=1.48 min.

### Step 2: Preparation of 1-methyl-3-((1,1,1-trifluoropropan-2-yl)oxy)-1H-pyrazol-4-amine (racemic)

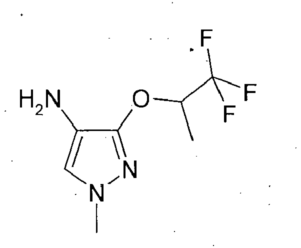

In a microwave vial, a solution of 1.2 g (1 eq.) of a 1-methyl-4-nitro-3-((1,1,1-trifluoropropan-2-yl)oxy)-1H-pyrazole in 15 mL of methanol was treated with 904 mg of ammonium formate (2.8 eq.) and 0.267 g (0.05 eq.) of palladium on carbon (10%). The reaction mixture was heated at 70°C for 10 minutes. The mixture was filtered on celite with methanol washes and concentrated under reduced pressure. The crude material was taken into the next step without further purification.

### Step 3: Preparation of N-(1-methyl-3-((1,1,1-trifluoropropan-2-yl)oxy)-1H-pyrazol-4-yl)formamide (racemic)

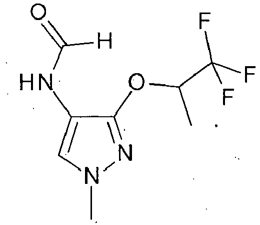

A solution of 1050 mg (1 eq.) of racemic 1-methyl-3-((1,1,1-trifluoropropan-2-yl)oxy)-1H-pyrazol-4-amine in 7 mL of tetrahydrofuran was dropwise added to a cooled (0°C) solution of 1.9 mL of acetic anhydride (4 eq.) in 3.87 mL of formic acid (18 eq.) that had been premixed for 30 minutes at room temperature. The reaction mixture was stirred at 5°C for 5 minutes, at room temperature for 1.5 hours then concentrated under vacuum to afford 895 mg of racemic N-(1-methyl-3-((1,1,1-trifluoropropan-2-yl)oxy)-1H-pyrazol-4-yl)formamide. MS (method A) m/z 238 [M+1]+; t=1.61 min.

### Intermediate 8: N-(3-isopropoxy-1-(2,2,2-trifluoroethyl)-1H-pyrazol-4-yl)formamide

### Step 1: Preparation of 3-isopropoxy-4-nitro-1-(2,2,2-trifluoroethyl)-1H-pyrazole

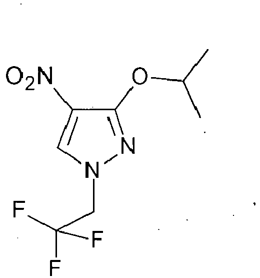

3.35 g (1.5 eq.) of 2,2,2-trifluoroethyl 1,1,2,2,3,3,4,4,4-nonafluorobutane-1-sulfonate and 2.45 g (3 eq.) of potassium carbonate were added to a solution of 1 g (1 eq.) of 3-isopropoxy-4-nitro-1H-pyrazole (commercially available) in 16 mL of dimethylformamide. The mixture was heated at 80°C for 1.5 hours then allowed to cool to room temperature and poured onto ethyl acetate and water. The aqueous layer was separated and extracted three times with ethyl acetate. The combined organic layers were dried over magnesium sulfate and concentrated under vacuum. The residue was purified on silica gel, eluting with a gradient of 20 to 50% of ethyl acetate in heptane, to afford 1.22 g of 3-isopropoxy-4-nitro-1-(2,2,2-trifluoroethyl)-1H-pyrazole. MS (method B) m/z 254 [M+1]+; t=1.59 min.

### Step 2: Preparation of 3-isopropoxy-1-(2,2,2-trifluoroethyl)-1H-pyrazol-4-amine

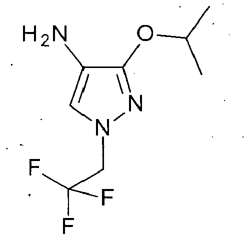

In a microwave vial, a solution of 885 mg (1 eq.) of 3-isopropoxy-4-nitro-1-(2,2,2-trifluoroethyl)-1H-pyrazole in 17 mL of methanol was treated with 630 mg of ammonium formate (2.8 eq.) and 186 mg (0.05 eq.) of palladium on carbon (10%). The reaction mixture was heated at 70°C for 10 minutes. the mixture was filtered on celite with methanol washes and concentrated under reduced pressure. The crude material was taken into the next step without further purification.

### Step 3: Preparation ofN-(3-isopropoxy-1-(2,2,2-trifluoroethyl)-1H-pyrazol-4-yl)formamide

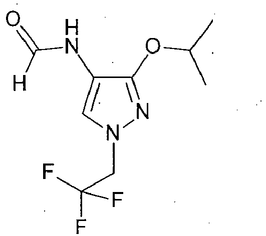

A solution of 780 mg (1 eq.) of 3-isopropoxy-1-(2,2,2-trifluoroethyl)-1H-pyrazol-4-amine in 5 mL of tetrahydrofuran was dropwise added to a cooled (0°C) solution of 1.33 mL of acetic anhydride (4 eq.) in 2.7 mL of formic acid (18 eq.) that had been premixed for 30 minutes at room temperature. The reaction mixture was stirred at 5°C for 5 minutes, at room temperature for 1 hour and then concentrated under vacuum. The residue was purified on silica gel, eluting by 18% ethyl acetate/2% of 7N NH₃ in MeOH/80% of heptane to 45% ethyl acetate/5% of 7N NH₃ in MeOH/50% heptane, to afford 730 mg N-(3-isopropoxy-1-(2,2,2-trifluoroethyl)-1H-pyrazol-4-yl)formamide. MS (method A) m/z 252 [M+1]+; t=1.94 min.

### Intermediate 9: N-(3-((1-acetylazetidin-3-yl)oxy)-1-methyl-1H-pyrazol-4-yl)formamide

### Step 1: Preparation of tert-butyl 3-((1-methyl-4-nitro-1H-pyrazol-3-yl)oxy)azetidine-1-carboxylate

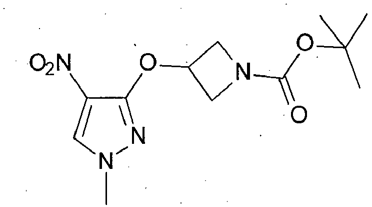

13.71 g (1.1 eq.) of tert-butyl 3-iodoazetidine-1-carboxylate and 12.17 g (2 eq.) of potassium carbonate were added to a solution of 6.3 g (1 eq.) of 1-methyl-4-nitro-1H-pyrazol-3-ol (commercially available) in 125 mL of dimethylformamide. The mixture was heated at 90°C for 30 hours, then at room temperature for 70 hours, and then poured onto ethyl acetate (100 mL) and water (10 mL). The aqueous layer was separated and extracted three times with ethyl acetate. The combined organic layers were dried over magnesium sulfate and concentrated under vacuum. The residue was purified on silica gel, eluting with a gradient of 10 to 60% of ethyl acetate in heptane, to afford 8 g of tert-butyl 3-((1-methyl-4-nitro-1H-pyrazol-3-yl)oxy)azetidine-1-carboxylate. MS (method A) m/z 243 [M-tBu]+; t=1.87 min.

### Step 2: Preparation of 3-(azetidin-3-yloxy)-1-methyl-4-nitro-1H-pyrazole (TFA salt)

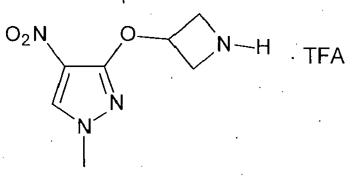

32 mL of trifluoroacetic acid was added to a solution of 8 g (1 eq.) of tert-butyl 3-((1-methyl-4-nitro-1H-pyrazol-3-yl)oxy)azetidine-1-carboxylate in 160 mL of dichloromethane at 0°C. The mixture was stirred at room temperature for 1.5 hours and cooled to 0°C. 10 mL of MeOH then 150 mL of 7N NH₃ in MeOH were added slowly to the reaction mixture. The mixture was concentrated under vacuum. The residue was purified on silica gel, eluting with a mixture of dichloromethane/ MeOH/7N methanolic ammonia solution from the ratio (95/4.5/0.5) to (80/18/2) to afford 9.9 g of 3-(azetidin-3-yloxy)-1-methyl-4-nitro-1H-pyrazole (TFA salt). MS (method A) m/z 199 [M+1]+; t=0.26 min.

### Step 3: Preparation of 1-(3-((1-methyl-4-nitro-1H-pyrazol-3-yl)oxy)azetidin-1-yl)ethan-1-one

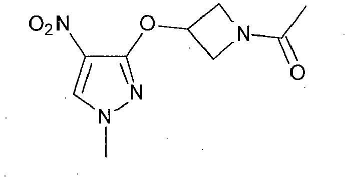

13.4 mL (10 eq.) of triethylamine was added to a solution of 3 g (1 eq.) of 3-(azetidin-3-yloxy)-1-methyl-4-nitro-1H-pyrazole (TFA salt) in 100 mL of tetrahydrofuran. The mixture was cooled to 0°C and 1.4 mL (2 eq.) of acetyl chloride was added dropwise. The reaction mixture was stirred at 0°C for 5 minutes, then 1 hour at room temperature, and then poured onto ethyl acetate (100 mL) and water (10 mL). The aqueous layer was separated and extracted three times with ethyl acetate. The combined organic layers were dried over magnesium sulfate and concentrated under vacuum. The residue was purified on silica gel, eluting with a gradient of 10 to 50% of acetone in dichloromethane, to afford 1.34 g of 1-(3-((1-methyl-4-nitro-1H-pyrazol-3-yl)oxy)azetidin-1-yl)ethan-1-one. MS (method A) m/z 241 [M+1]+; t=1.22 min.

### Step 4: Preparation of 1-(3-((4-amino-1-methyl-1H-pyrazol-3-yl)oxy)azetidin-1-yl)ethan-1-one

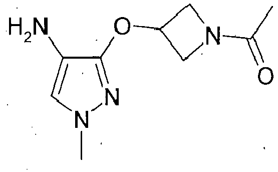

In a microwave vial, a solution of 1.34 g (1 eq.) of 1-(3-((1-methyl-4-nitro-1H-pyrazol-3-yl)oxy)azetidin-1-yl)ethan-1-one in 32 mL of methanol was treated with 2.18 g of ammonium formate (6 eq.) and 403 mg (0.07 eq.) of palladium on carbon (10%). The reaction mixture was heated at 70°C for 10 minutes. The mixture was filtered on celite with methanol washes and concentrated under reduced pressure. The crude material was taken into the next step without further purification.

### Step 5: Preparation of N-(3-((1-acetylazetidin-3-yl)oxy)-1-methyl-1H-pyrazol-4-yl)formamide

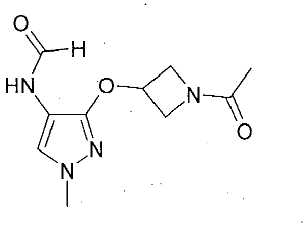

A solution of 1.29 g (1 eq.) of 1-(3-((4-amino-1-methyl-1H-pyrazol-3-yl)oxy)azetidin-1-yl)ethan-1-onein 20 mL of tetrahydrofuran was dropwise added to a cooled (0°C) solution of 2.32 mL of acetic anhydride (4 eq.) in 1.88 mL of formic acid (8 eq.) that had been premixed for 30 minutes at room temperature. 15 mL of tetrahydrofuran was added and the reaction mixture was stirred at room temperature for 35 minutes. The residue was concentrated under vacuum and the residue triturated in a mixture of dichloromethane and diisopropyl ether. The solid was filtered and the filtrate evaporated under reduced pressure to afford 1.08 g of N-(3-((1-acetylazetidin-3-yl)oxy)-1-methyl-1H-pyrazol-4-yl)formamide. MS (method A) m/z 239 [M+1]+; t= 0.87 min.

### Intermediate 10: N-(2-cyclopropoxy-4-((dimethylamino)methyl)phenyl)formamide

### Step 1: Preparation of 1-(3-cyclopropoxy-4-nitrophenyl)-N,N-dimethylmethanamine

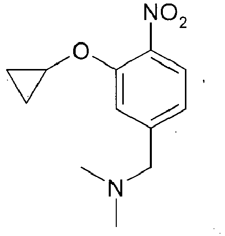

A solution of 1g (1 eq.) of 3-cyclopropoxy-4-nitrobenzaldehyde, 4.6 mL of acetic acid and 2.41 mL of a 2N solution of dimethylamine in tetrahydrofuran in 23 mL of dichloromethane was stirred at room temperature for 15 minutes. 2.05 g (2 eq.) of sodium triacetoxyborohydrate was added, and the reaction mixture stirred at room temperature for 70 hours. 40 mL of dichloromethane and 40 mL of statured aqueous solution of sodium bicarbonate were added. The organic layer was dried over magnesium sulfate and concentrated under vacuum. The residue was purified on silica gel, eluting with a mixture of dichloromethane/ethyle acetate/7N NH₃ in MeOH (80/18/2) to afford 471 mg of 1-(3-cyclopropoxy-4-nitrophenyl)-N,N-dimethylmethanamine. MS (method A) m/z 237 [M+1]+; t=0.72 min.

### Step 2: Preparation of 2-cyclopropoxy-4-((dimethylamino)methyl)aniline

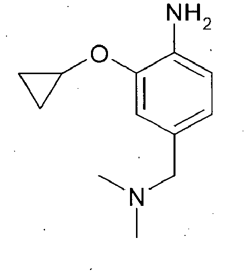

In a microwave vial, a solution of 471 mg (1 eq.) of 1-(3-cyclopropoxy-4-nitrophenyl)-N,N-dimethylmethanamine in 12 mL of methanol was treated with 363 mg of ammonium formate (2.8 eq.) and 212 mg (0.1 eq.) of palladium on carbon (10%). The reaction mixture was heated at 80°C for 15 minutes. The mixture was filtered on celite with methanol washes and concentrated under reduced pressure. The crude material was triturated in diisopropyl ether and the filtrate evaporated to afford 337 mg of 2-cyclopropoxy-4-((dimethylamino)methyl)aniline. The crude material was taken into the next step without further purification.

### Step3: Preparation of N-(2-cyclopropoxy-4-((dimethylamino)methyl)phenyl)formamide

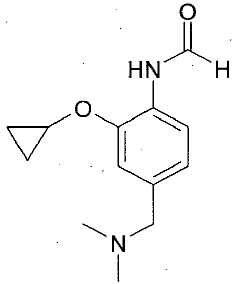

A solution of 337 mg (1 eq.) of 2-cyclopropoxy-4-((dimethylamino)methyl)aniline in 10 mL of tetrahydrofuran was added dropwise to a cooled (0°C) solution of 468 µL of acetic anhydride (3 eq.) in 779 µL of formic acid (12 eq.) that had been premixed for 30 minutes at room temperature. The reaction mixture was stirred at room temperature for 1 hour. The residue was concentrated under vacuum to afford 337 mg of N-(2-cyclopropoxy-4-((dimethylamino)methyl)phenyl)formamide. MS (method A) m/z 235 [M+1]+; t= 0.36 min.

### Intermediate 11: N-(3-cyclopropoxy-1-(oxetan-3-yl)-1H-pyrazol-4-yl)formamide

### Step 1: Preparation of 3-cyclpropoxy-4-nitro-1-(oxetan-3-yl)-1H-pyrazole

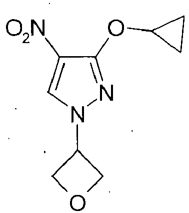

653 mg (1.2 eq.) of 3-iodooxetane and 817 mg (2 eq.) of potassium carbonate were added to a solution of 0.5 g (1 eq.) of 3-cyclopropoxy-4-nitro-1H-pyrazole (Intermediate 15, step 2) in 20 mL of 1-methyl-2-pyrrolidinone. The mixture was heated at 80°C for 24 hours then allowed to cool to room temperature and poured onto ethyl acetate and water. The aqueous layer was separated and extracted three times with ethyl acetate. The combined organic layers were dried over magnesium sulfate and concentrated under vacuum. The residue was purified on silica gel, eluting with a gradient of 20 to 50% of ethyl acetate in heptane, to afford 296 mg.of 3-cyclopropoxy-4-nitro-1-(oxetan-3-yl)-1H-pyrazole. MS (method A) m/z 226 [M+1]+; t=1.68 min.

### Step 2: Preparation of 3-cyclopropoxy-1-(oxetan-3-yl)-1H-pyrazol-4-amine

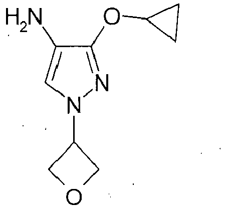

In a microwave vial, a solution of 296 mg (1 eq.) of 3-cyclopropoxy-4-nitro -1-(oxetan-3-yl)-1H-pyrazole in 9 mL of methanol was treated with 240 mg of ammonium formate (2.8 eq.) and 140 mg (0.1 eq.) of palladium on carbon (10%). The reaction mixture was heated at 70°C for 10. minutes. The mixture was filtered on celite with methanol washes and concentrated under reduced pressure. The crude material was taken into the next step without further purification.

### Step 3: Preparation of N-(3-cyclopropoxy-1-(oxetan-3-yl)-1H-pyrazol-4-yl)formamide

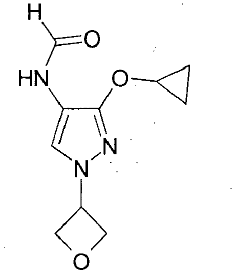

A solution of 250 mg (1 eq.) of 3-cyclopropoxy-1-(oxetan-3-yl)-1H-pyrazol-4-amine in 5 mL of tetrahydrofuran was dropwise added to a cooled (0°C) solution of 484 µL of acetic anhydride (4 eq.) in 884 µL of formic acid (18 eq.) that had been premixed for 30 minutes at room temperature. The reaction mixture was stirred at 0°C for 1 hour. The residue was concentrated under vacuum to afford 200 mg of N-(3-cyclopropoxy-1-(oxetan-3-yl)-1H-pyrazol-4-yl)formamide. MS (method A) m/z 224 [M+1]+; t=1.17 min.

### Intermediate 12: N-(3-isopropoxy-1-(oxetan-3-yl)-1H-pyrazol-4-yl)formamide

### Step 1: Preparation of 3-isopropoxy-4-nitro-1-(oxetan-3-yl)-1H-pyrazole

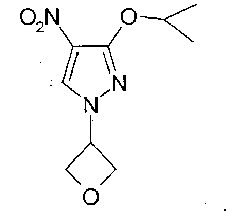

653 mg (1.2 eq.) of 3-iodooxetane and 817 mg (2 eq.) of potassium carbonate were added to a solution of 0.5 g (1 eq.) of 3-isopropoxy-4-nitro-1H-pyrazole (commercially available) in 20 mL of 1-methyl-2-pyrrolidinone. The mixture was heated at 80°C for 24 hours then allowed to cool to room temperature and poured onto ethyl acetate and water. The aqueous layer was separated and extracted three times with ethyl acetate. The combined organic layers were dried over magnesium sulfate and concentrated under vacuum. The residue was purified on silica gel, eluting with a gradient of 20 to 50% of ethyl acetate in heptane, to afford 564 mg of 3-isopropoxy-4-nitro-1-(oxetan-3-yl)-1H-pyrazole. MS (method A) m/z 228 [M+1]+; t=1.86 min.

### Step 2: Preparation of 3-isopropoxy-1-(oxetan-3-yl)-1H-pyrazol-4-amine

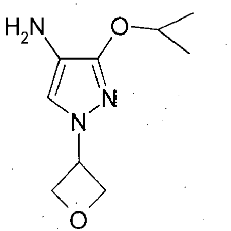

In a microwave vial, a solution of 150 mg (1 eq.) of 3-isopropoxy-4-nitro -1-(oxetan-3-yl)-1H-pyrazole in 4mL of methanol was treated with 120 mg of ammonium formate (2.8 eq.) and 71 mg (0.1 eq.) of palladium on carbon (10%). The reaction mixture was heated at 70°C for 10 minutes. The mixture was filtered on celite with methanol washes and concentrated under reduced pressure. The crude material was taken into the next step without further purification.

### Step 3: Preparation of N-(3-isopropoxy-1-(oxetan-3-yl)-1H-pyrazol-4-yl)formamide

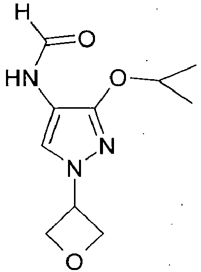

A solution of 130 mg (1 eq.) of 3-isopropoxy-1-(oxetan-3-yl)-1H-pyrazol-4-amine in 3 mL of tetrahydrofuran was dropwise added to a cooled (0°C) solution of 249 µL of acetic anhydride (4 eq.) in 455 µL of formic acid (18 eq.) that had been premixed for 30 minutes at room temperature. The reaction mixture was stirred at 0°C for 1 hour. The residue was concentrated under vacuum to afford 200 mg of N-(3-isopropoxy-1-(oxetan-3-yl)-1H-pyrazol-4-yl)formamide. MS (method A) m/z 226 [M+1]+; t=1.36 min.

### Intermediate 13: N-(1-((methylsulfonyl)methyl)-3-(oxetan-3-yloxy)-1H-pyrazol-4-yl)formamide

### Step 1: Preparation of 1-(4-methoxybenzyl)-3,4-dinitro-1H-pyrazole

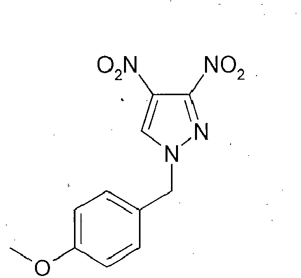

11.77 g (1.2 eq.) of 4-methoxybenzyl chloride, 27.15 g of tetrabutylammonium iodide and 36.02 g (1.8 eq.) of cesium carbonate were added to a solution of 10 g (1 eq.) of 3,4-dinitro-1H-pyrazole in 50 mL of dimethylformamide. The mixture was stirred at room temperature for 50 minutes and poured onto ethyl acetate and water. The aqueous layer was separated and extracted three times with ethyl acetate. The combined organic layers were dried over magnesium sulfate and concentrated under vacuum. The residue was purified on silica gel, eluting with a gradient of 10 to 50% of ethyl acetate in heptane, to afford 15.25 g of 1-(4-methoxybenzyl)-3,4-dinitro-1H-pyrazole. MS (method A) m/z 277 [M-1]; t=1.61 min.

### Step 2: Preparation of 1-(4-methoxybenzyl)-4-nitro-3-(oxetan-3-yloxy)-1H-pyrazole

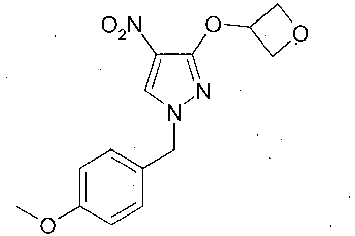

2.16 g (1.25 eq.) of oxetan-3-ol and 15.24 g (2 eq.) of cesium carbonate were added to a solution of 6.5 g (1 eq.) of 1-(4-methoxybenzyl)-3,4-dinitro-1H-pyrazole in 80 mL of acetonitrile. The mixture was heated at 80°C for 3 hours, then allowed to cool to room temperature, and then poured onto ethyl acetate and water. The aqueous layer was separated and extracted three times with ethyl acetate. The combined organic layers were dried over magnesium sulfate and concentrated under vacuum. The residue was triturated in diisopropyl ether to afford 7.1 g of 1-(4-methoxybenzyl)-4-nitro-3-(oxetan-3-yloxy)-1H-pyrazole. MS (method A) m/z 306 [M+1]+; t=2.16min.

### Step 3: Preparation of 4-nitro-3-(oxetan-3-yloxy)-1H-pyrazole

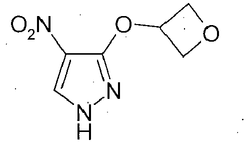

32.33 g (2.5eq.) of ceric ammonium nitrate was added to a solution of 7.1 g (1 eq.) of 1-(4-methoxybenzyl)-4-nitro-3-(oxetan-3-yloxy)-1H-pyrazole in 170 mL of acetonitrile and 170 mL of water. The mixture was stirred at room temperature for 16 hours. Another 4.5 g of ceric ammonium nitrate were added and the reaction mixture stirred for 30 minutes at room temperature and poured onto 85 mL of ethyl acetate and 85 mL of an aqueous saturated solution of sodium thiosulfate. The aqueous layer was separated and extracted three times with ethyl acetate. The combined organic layers were dried over magnesium sulfate and concentrated under vacuum. The residue was triturated in diethyl ether and the precipitate filtered to afford 2.38 g of 4-nitro-3-(oxetan-3-yloxy)1H-pyrazole. MS (method A) m/z 186 [M+1]+; t=0.90 min.

### Step 4: Preparation of 1-((methylthio)methyl)-4-nitro-3-(oxetan-3-yloxy)-1H-pyrazole

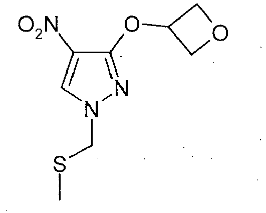

937 µL (1.4 eq.) of (chloromethyl)(methyl)sulfane and 2.24 g (2 eq.) of potassium carbonate were added to a solution of 1.5 g (1 eq.) of 4-nitro-3-(oxetan-3-yloxy)-1H-pyrazole in 50 mL of dimethylformamide. The mixture was heated at 80°C for 2.5 hours then allowed to cool to room temperature and poured onto ethyl acetate and water. The aqueous layer was separated and extracted three times with ethyl acetate. The combined. organic layers were dried over magnesium sulfate and concentrated under vacuum. The residue was purified on silica gel, eluting with a gradient of 20 to 50% of ethyl acetate in heptane, to afford 935 mg of 1-((methylthio)methyl)-4-nitro-3-(oxetan-3-yloxy)-1H-pyrazole. MS (method A) m/z 246 [M+1]+; t=1.15 min.

### Step 5: Preparation of 1-((methylsulfonyl)methyl)-4-nitro-3-(oxetan-3-yloxy)-1H-pyrazole

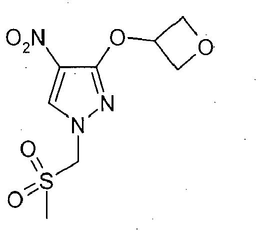

2.55 g (purity 77% - 3 eq.) of 3-chloroperoxybenzoic acid was added to a solution of 931 mg (1 eq.) of 1-((methylthio)methyl)-4-nitro-3-(oxetan-3-yloxy)-1H-pyrazole in 40 mL of dichloromethane at 0°C,. The mixture was allowed to warm to room temperature and then quenched with 50 mL of DCM and 20 mL of a 10% aqueous sodium thiosulfate solution. The mixture was stirred for 15 minutes. The organic layer was washed successively with 50 mL of saturated aqueous sodium carbonate solution, 50 mL of water, and 50 mL of brine then dried over magnesium sulfate and concentrated under reduced pressure. The precipitate formed in the interface of the aqueous and the organic layer was filtered and dried under vacuum to afford 255 mg of 1-((methylsulfonyl)methyl)-4-nitro-3-(oxetan-3-yloxy)-1H-pyrazole. The residue coming from the evaporation of the organic layer was purified on silica, eluting with heptane ethyl acetate/7N NH₃ in MeOH (80/18/2) to afford 291 mg of 1-((methylsulfonyl)methyl)-4-nitro-3-(oxetan-3-yloxy)-1H-pyrazole. MS (method B) m/z 278 [M+1]+; t=0.85 min.

### Step 6: Preparation of 1-((methylsulfonyl)methyl)-3-(oxetan-3-yloxy)-1H-pyrazol-4-amine

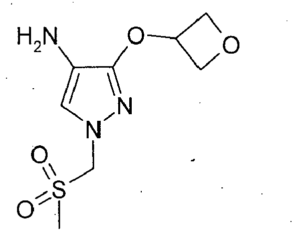

A solution of 546 mg (1 eq.) of 1-((methylsulfonyl)methyl)-4-nitro-3-(oxetan-3-yloxy)-1H-pyrazole in 50 mL of methanol was treated with 0.42 g of palladium on carbon (10%) under 4 bars of hydrogen for 1 hour. The mixture was filtered with methanol washes and concentrated under reduced pressure to afford 450 mg of 1-((methylsulfonyl)methyl)-3-(oxetan-3-yloxy)-1H-pyrazol-4-amine. MS (method A) m/z 248 [M+1]+; t=0.24 min.

### Step 7: Preparation of N-(1-((methylsulfonyl)methyl)-3-(oxetan-3-yloxy)-1H-pyrazol-4-yl)formamide

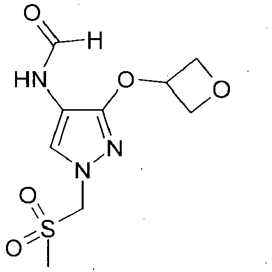

A solution of 486 mg (1 eq.) of 1-((methylsulfonyl)methyl)-3-(oxetan-3-yloxy)-1H-pyrazol-4-amine in 3 mL of tetrahydrofuran was dropwise added to a cooled (0°C) solution of 742 µL of acetic anhydride (4 eq.) in 1.36 mL of formic acid (18 eq.) that had been premixed for 30 minutes at room temperature. The reaction mixture was stirred at room temperature for 1 hour. The residue was concentrated under vacuum and purified on silica, eluting with heptane/ethyl acetate/7N NH₃ in MeOH (50/45/5) to afford 450 mg of N-(1-((methylsulfonyl)methyl)-3-(oxetan-3-yloxy)-1H-pyrazol-4-yl)formamide. MS (method A) m/z 276 [M+1]+; t=0.52 min.

### Intermediate 14: N-(1-(methoxymethyl)-3-(oxetan-3-yloxy)-1H-pyrazol-4-yl)formamide Step 1: Preparation of 1-(methoxymethyl)-4-nitro-3-(oxetan-3-yloxy)-1H-pyrazole

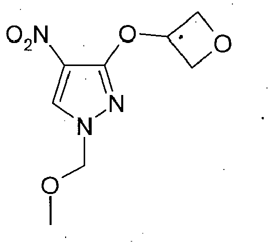

121 mg (1.4 eq.) of sodium hydride (60% of purity) was added to a solution of 400 mg (1 eq.) of 4-nitro-3-(oxetan-3-yloxy)-1H-pyrazole (Intermediate 13, step 3) in 12 mL of tetrahydrofuran at 0°C. The reaction mixture was stirred at 0°C for 30 minutes and 15 minutes at room temperature. The mixture was cooled to 0°C and 244 mg (1.4 eq.) of chloro(methoxy)methane was added. The mixture was stirred at 0°C for 30 minutes and slowly poured onto ethyl acetate and water. The aqueous layer was separated and extracted three times with ethyl acetate. The combined organic layers were dried over magnesium sulfate and concentrated under vacuum. The residue was purified on silica gel, eluting with a gradient of 20 to 50% of ethyl acetate in heptane, to afford 373 mg of 1-(methoxymethyl)-4-nitro-3-(oxetan-3-yloxy)-1H-pyrazole. MS (method A) m/z 230 [M+1]+; t=1.34 min.

### Step 2: Preparation of 1-(methoxymethyl)-3-(oxetan-3-yloxy)-1H-pyrazol-4-amine

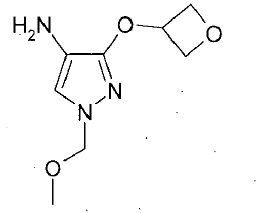

In a microwave vial, a solution of 453 mg (1 eq.) of 1-(methoxymethyl)-4-nitro-3-(oxetan-3-yloxy)-1H-pyrazole in 15 mL of methanol was treated with 356 mg of ammonium formate (2.8 eq.) and 210 mg (0.05 eq.) of palladium on carbon (10%). The reaction mixture was heated at 70°C for 10 minutes. The mixture was filtered on celite with methanol washes and concentrated under reduced pressure. The crude material was taken into the next step without further purification.

### Step 3: Preparation of N-(1-(methoxymethyl)-3-(oxetan-3-yloxy)-1H-pyrazol-4-yl)formamide

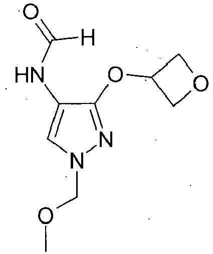

A solution of 393 mg (1 eq.) of 1-(methoxymethyl)-3-(oxetan-3-yloxy)-1H-pyrazol-4-amine in 3 mL of tetrahydrofuran was dropwise added to a cooled (0°C) solution of 745 µL of acetic anhydride (4 eq.) in 1.36 mL of formic acid (18 eq.) that had been premixed for 30 minutes at room temperature. The reaction mixture was stirred at 0°C for 1 hour. The residue was concentrated under vacuum and purified on silica, eluting with heptane/ethyl acetate/7N NH₃ in MeOH (50/45/5) to afford 312 mg of N-(1-(methoxymethyl)-3-(oxetan-3-yloxy)-1H-pyrazol-4-yl)formamide. MS (method A) m/z 228 [M+1]+; t=0.74 min.

### Intermediate 15: N-(3-cyclopropoxy-1-(methoxymethyl)-1H-pyrazol-4-yl)

### Step 1: Preparation of 4-cyclopropoxy-1-(4-methoxybenzyl)-3-nitro-1H-pyrazole

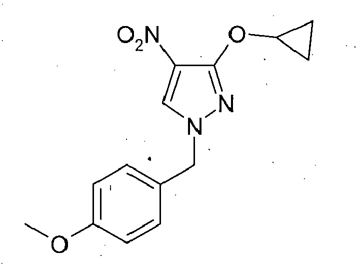

2.51 g (1.5 eq.) of cyclopropanol and 19.2 g (2 eq.) of cesium carbonate were added to a solution of 8 g (1 eq.) of 1-(4-methoxybenzyl)-3,4-dinitro-1H-pyrazole (Intermediate 13, step 1) in 100 mL of acetonitrile. The mixture was heated at 50°C for 3 hours then allowed to cool to room temperature and poured onto ethyl acetate and water. The aqueous layer was separated and extracted three times with ethyl acetate. The combined organic layers were dried over magnesium sulfate and concentrated under vacuum. The residue was purified on silica gel, eluting with a gradient of 20 to 50% of ethyl acetate in heptane, to afford 5.1 g of 4-cyclopropoxy-1-(4-methoxybenzyl)-3-nitro-1H-pyrazole as a yellow oil. MS (method A) m/z 290 [M+1]+; t=1.59 min.

### Step 2: Preparation of 3-cyclopropoxy-4-nitro-1H-pyrazole

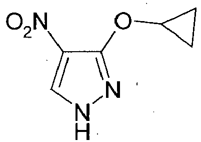

18.35 g (2.5 eq.) of ceric ammonium nitrate was added to a solution of 5.1 g (1 eq.) of 4-cyclopropoxy-1-(4-methoxybenzyl)-3-nitro-1H-pyrazole in 100mL of acetonitrile and 100 mL of water. The mixture was stirred at room temperature for 16 hours. Another 4 g of ceric ammonium nitrate was added, and the reaction mixture stirred for 30 minutes at room temperature and poured onto 85 mL of ethyl acetate and 85 mL of an aqueous saturated solution of sodium thiosulfate. The aqueous layer was separated and extracted three times with ethyl acetate. The combined organic layers were dried over magnesium sulfate and concentrated under vacuum. The residue was purified on silica gel, eluting with 5% of methanol in dichloromethane to afford 2.5 g of 3-cyclopropoxy-4-nitro-1H-pyrazole. MS (method A) m/z 170 [M+1]+; t=1.3 min.

### Step 3: Preparation of 3-cyclopropoxy-1-(methoxymethyl)-4-nitro-1H-pyrazole

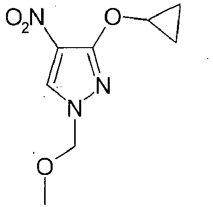

667 mg (1.4 eq.) of chloro(methoxy)methane was added to a solution of 1 g (1 eq.) of 3-cyclopropoxy-4-nitro-1H-pyrazole in 30 mL of acetonitrile and 1.65 g (2 eq.) of potassium carbonate at 0°C. The mixture was warmed to room temperature, stirred for 1.5 hour and poured onto ethyl acetate and water. The aqueous layer was separated and extracted three times with ethyl acetate. The combined organic layers were dried over magnesium sulfate and concentrated under vacuum. The residue was purified on silica gel, eluting with a gradient of 20 to 50% of ethyl acetate in heptane, to afford 815 mg of 3-cyclopropoxy-1-(methoxymethyl)-4-nitro-1H-pyrazole. MS (method B), m/z 170 [M+1]+; t=1.18 min.

### Step 4: Preparation of 3-cyclopropoxy-1-(methoxymethyl)-1H-pyrazol-4-amine

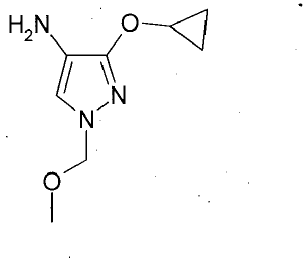

In a microwave vial, a solution of 815 mg (1 eq.) of 3-cyclopropoxy-1-(methoxymethyl)-4-nitro-1H-pyrazole in 10 mL of methanol was treated with 689 mg of ammonium formate (2.8 eq.) and 204 mg (0.05 eq.) of palladium on carbon (10%). The reaction mixture was heated at 100°C for 10 minutes. The mixture was filtered on celite with methanol washes and concentrated under reduced pressure. The crude material was taken into the next step without further purification.

### Step 5: Preparation of N-(3-cyclopropoxy-1-(methoxymethyl)-1H-pyrazol-4-yl)formamide

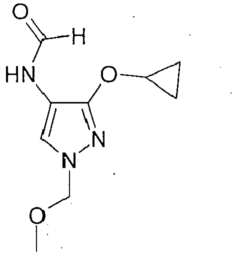

A solution of 700 mg (1 eq.) of 3-cyclopropoxy-1-(methoxymethyl)-1H-pyrazol-4-amine in 7 mL of tetrahydrofuran was dropwise added to a solution of 1.45 mL of acetic anhydride (4 eq.) in 1.31 mL of formic acid (18 eq.) that had been premixed for 30 minutes at room temperature. The reaction mixture was stirred for 1 hour. The residue was concentrated under vacuum and purified on silica gel eluting with dichloromethane then 2% of methanol in dichloromethane to afford 620 mg of N-(3-cyclopropoxy-1-(methoxymethyl)-1H-pyrazol-4-yl)formamide. MS(method B) m/z 212 [M+1]+; t=0.86 min.

### Intermediate 16: N-(3-cyclopropoxy-1-((methylsulfonyl)methyl)-1H-pyrazol-4-yl)formamide

### Step 1: Preparation of 3-cyclopropoxy-1-((methylthio)methyl)-4-nitro-1H-pyrazole

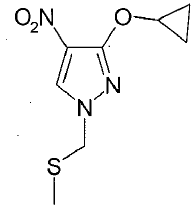

820 µL (1.4 eq.) of (chloromethyl)(methyl)sulfane and 1.96 g (2 eq.) of potassium carbonate were added to a solution of 1.2 g (1 eq.) of 3-cyclopropoxy-4-nitro-1H-pyrazole (Intermediate 15, step 2) in 45 mL of dimethylformamide. The mixture was heated at 50°C for 2 hours then allowed to cool to room temperature and poured onto ethyl acetate and water. The aqueous layer was separated and extracted three times with ethyl acetate. The combined organic layers were dried over magnesium sulfate and concentrated under vacuum. The residue was purified on silica gel, eluting with 20% of ethyl acetate in heptane, to afford 800 mg of 3-cyclopropoxy-1-((methylthio)methyl)-4-nitro-1H-pyrazole.

### Step 2: Preparation of 3-cyclopropoxy-1-((methylsulfonyl)methyl)-4-nitro-1H-pyrazole

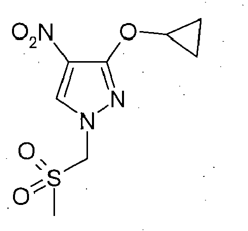

2.50 g (purity 70% - 3 eq.) of 3-chloroperoxybenzoic acid was added to a solution of 800 mg (1 eq.) of 3-cyclopropoxy-1-((methylthio)methyl)-4-nitro-1H-pyrazole in 10 mL of dichloromethane. The mixture was stirred for 3 hours and then quenched with 50 mL of dichloromethane and 20 mL of a 10% aqueous sodium thiosulfate sodium carbonate solution. The mixture was stirred for 15 minutes. The organic layer was washed successively with 50 mL of saturated aqueous sodium carbonate solution, 50 mL of water, and 50 mL of brine then dried over magnesium sulfate and concentrated under reduced pressure. The residue was triturated in diisopropyl ether and the solid filtered and dried under vacuum to afford 830 mg of 3-cyclopropoxy-1-((methylsulfonyl)methyl)-4-nitro-1H-pyrazole. MS (method A) m/z 263 [M+1]+; t=2.36 min

### Step 3: Preparation of 3-cyclopropoxy-1-((methylsulfonyl)methyl)-1H-pyrazol-4-amine

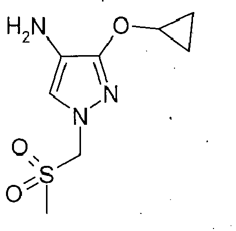

A solution of 730 mg (1 eq.) of 3-cyclopropoxy-1-((methylsulfonyl)methyl)-4-nitro-1H-pyrazole in 70 mL of methanol was treated with 595 mg of palladium on carbon (10%) under 4 bars of hydrogen for 1 hour. The mixture was filtered with methanol washes and concentrated under reduced pressure to afford 680 mg of 3-cyclopropoxy-1-((methylsulfonyl)methyl)-1H-pyrazol-4-amine. The crude material was taken into the next step without further purification.

### Step 4: Preparation of N-(3-cyclopropoxy-1-((methylsulfonyl)methyl)-1H-pyrazol-4-yl)formamide

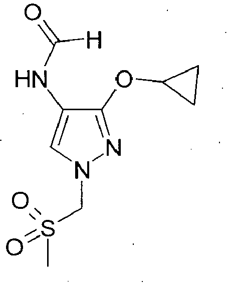

A solution of 680 mg (1 eq.) of 3-cyclopropoxy-1-((methylsulfonyl)methyl)-1H-pyrazol-4-amine in 7 mL of tetrahydrofuran was dropwise added to a solution of 1.12 mL of acetic anhydride (4 eq.) in 1.01 mL of formic acid (8 eq.) that had been premixed for 30 minutes at room temperature. The reaction mixture was stirred at room temperature for 1 hour. The residue was concentrated under vacuum and purified on silica, eluting with dichloromethane then 2% of methanol in dichloromethane to afford 490 mg of N-(3-cyclopropoxy-1-((methylsulfonyl)methyl)-1H-pyrazol-4-yl)formamide. MS (method B) m/z 260 [M+1]+; t=0.79 min.

### Intermediate 17: N-(3-(((3R,4R)-4-methoxytetrahydrofuran-3-yl)oxy)-1-methyl-1H-pyrazol-4-yl)formamide and N-(3-(((3S,4S)-4-methoxytetrahydrofuran-3-yl)oxy)-1-methyl-1H-pyrazol-4-yl)formamide

### Step 1: Preparation of (3S,4S)-4-methoxytetrahydrofuran-3-ol and (3R,4R)-4-methoxytetrahydrofuran-3-ol

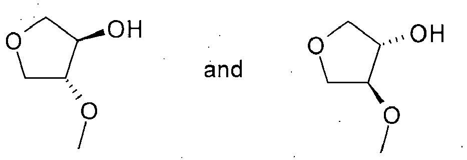

929 µL of sulfuric acid (0.05 eq.) was dropwise added to a solution of 30 g (1 eq.) of 3,4-epoxytetrahydrofuran in 450 mL of methanol. The reaction mixture was stirred at room temperature for 48 hours. 60 mL of an aqueous saturated solution of sodium hydrogenocarbonate was added and the reaction mixture stirred for 30 minutes. 150 mL of ethyl acetate and 60 mL of water were added. The aqueous layer was separated and extracted three times with ethyl acetate. The combined organic layers were dried over magnesium sulfate and concentrated under vacuum to afford 31.47 g of racemic mixture of (3S,4S)-4-methoxytetrahydrofuran-3-ol and (3R,4R)-4-methoxytetrahydrofuran-3-ol. The aqueous layer was further extracted with dichloromethane. The organic layer was dried over magnesium sulfate and concentrated under vacuum to afford another 4.88 g of a racemic mixture of (3S,4S)-4-methoxytetrahydrofuran-3-ol and (3R,4R)-4-methoxytetrahydrofuran-3-ol. The crude material was taken into the next step without further purification.

### Step 2: Preparation of 3-(((3R,4R)-4-methoxytetrahydrofuran-3-yl)oxy)-1-methyl-4-nitro-1H-pyrazole and 3-(((3S,4S)-4-methoxytetrahydrofuran-3-y))oxy)-1-methyl-4-nitro-1H-pyrazole

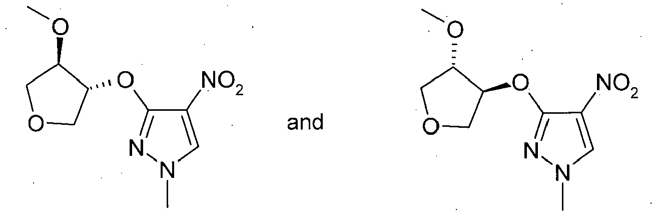

1.37 g (1.3 eq.) of a racemic mixture of (3S,4S)-4-methoxytetrahydrofuran-3-ol and (3R,4R)-4-methoxytetrahydrofuran-3-ol and 7.58 g (2 eq.) of cesium carbonate were added to a solution of 2 g (1 eq.) of 1-methyl-3,4-dinitro-1H-pyrazole (commercially available) in 40 mL of acetonitrile. The mixture was heated at 80°C for 6 h then allowed to cool to room temperature and poured onto ethyl acetate and water. The aqueous layer was separated and extracted three times with ethyl acetate. The combined organic layers were dried over magnesium sulfate and concentrated under vacuum. The residue was purified on silica gel, eluting with a gradient of 10 to 20% of ethyl acetate in dichloromethane, to afford 1.4 g of a racemic mixture of 3-(((3R,4R)-4-methoxytetrahydrofuran-3-yl)oxy)-1-methyl-4-nitro-1H-pyrazole and 3-(((3S,4S)-4-methoxytetrahydrofuran-3-yl)oxy)-1-methyl-4-nitro-1H-pyrazole. MS (method B) m/z 244 [M+1]+; t=1.08 min

### Step 3: Preparation of 3-(((3R,4R)-4-methoxytetrahydrofuran-3-yl)oxy)-1-methyl-1H-pyrazol-4-amine and 3-(((3S,4S)-4-methoxytetrahydrofuran-3-yl)oxy)-1-methyl-1H-pyrazol-4-amine

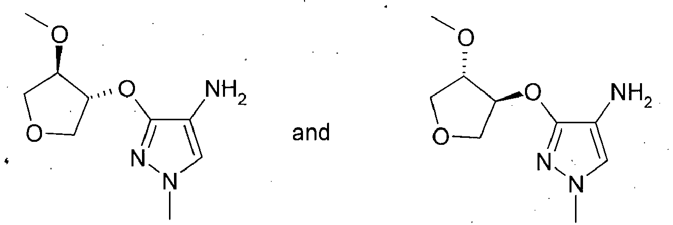

A solution of 1 g (1 eq.) of a racemic mixture of 3-(((3R,4R)-4-methoxytetrahydrofuran-3-yl)oxy)-1-methyl-4-nitro-1H-pyrazole and 3-(((3S,4S)-4-methoxytetrahydrofuran-3-yl)oxy)-1-methyl-4-nitro-1H-pyrazole in 32 mL of ethanol and 6.6 mL of water was treated with 1.11 g of ammonium chloride (5 eq.). The reaction mixture was heated at 80°C and 1.15 g (5 eq.) of iron was added. The reaction mixture was heated at 80°C for 2 hours, 10 mL of ethanol was added and the mixture was filtered on celite with ethanol washes. The filtrate was concentrated under reduced pressure. The crude material was diluted with 20 mL of ethyl acetate and 10 mL of an aqueous saturated solution of sodium hydrogenocarbonate. The aqueous layer was separated and extracted three times with ethyl acetate. The combined organic layers were dried over magnesium sulfate and concentrated under vacuum to afford 0.9 g of a racemic mixture of 3-(((3R,4R)-4-methoxytetrahydrofuran-3-yl)oxy)-1-methyl-1-H-pyrazol-4-amine and 3-(((3S,4S)-4-methoxytetrahydrofuran-3-yl)oxy)-1-methyl-1H-pyrazol-4-amine. MS (method A) m/z 214 [M+1]+

### Step 4: Preparation of N-(3-(((3R,4R)-4-methoxytetrahydrofuran-3-yl)oxy)-1-methyl-1H-pyrazol-4-yl)formamide and N-(3-(((3S,4S)-4-methoxytetrahydrofuran-3-yl)oxy)-1-methyl-1H-pyrazol-4-yl)formamide

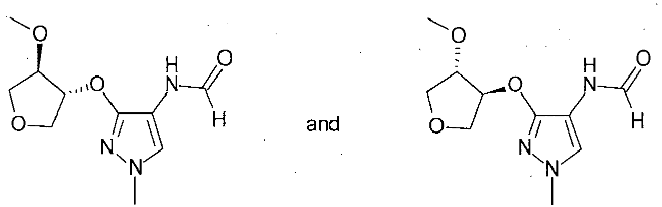

A solution of 1.3 g (1 eq.) of a racemic mixture of 3-(((3R,4R)-4-methoxytetrahydrofuran-3-yl)oxy)-1-methyl-1H-pyrazol-4-amine and 3-(((3S,4S)-4-methoxytetrahydrofuran-3-yl)oxy)-1-methyl-1H-pyrazol-4-amine in 8 mL of tetrahydrofuran was added dropwise to a cooled (0°C) solution of 2.33 mL of acetic anhydride (4 eq.) in 2.09 mL of formic acid (8 eq.) that had been premixed for 30 minutes at room temperature. The reaction mixture was stirred at 0°C for 15 minutes. The residue was concentrated under vacuum and purified on silica, eluting with 5% of methanol in dichloromethane to afford 300 mg of a racemic mixture of N-(3-(((3R,4R)-4-methoxytetrahydrofuran-3-yl)oxy)-1-methyl-1H-pyrazol-4-yl)formamide and N-(3-((-methyl-1H-pyrazol-4-yl)formamide. MS (method B) m/z 242 [M+1]+; t=0.87 min.

### Intermediate 18: N-(1-(methylsulfonyl)-3-(oxetan-3-yloxy)-1H-pyrazol-4-yl)formamide

### Step 1: Preparation of 1-(methylsulfonyl)-4-nitro-3-(oxetan-3-yloxy)-1H-pyrazole

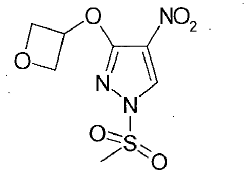

502 µL (1.5 eq.) of methanesulfonyl chloride was added to a solution of 0.8 g (1 eq.) of 4-nitro-3-(oxetan-3-yloxy)-1H-pyrazole (Intermediate 13, step 3) in 40 mL of dichoromethane and 1.21 mL of triethylamine (2 eq.) at 0°C. The mixture was stirred at room temperature for 2 hours and poured onto 20 mL of a saturated aqueous solution of ammonium chloride. The aqueous layer was separated and extracted twice with dichloromethane. The combined organic layers were dried over magnesium sulfate and concentrated under vacuum. The residue was purified on silica gel, eluting with 5% of ethyl acetate in dichloromethane, to afford 600 mg of 1-(methylsulfonyl)-4-nitro-3-(oxetan-3-yloxy)-1H-pyrazole. MS (method B) m/z 264 [M+1]+; t=2.21 min.

### Step 2: Preparation of N-(1-(methylsulfonyl)-3-(oxetan-3-yloxy)-1H-pyrazol-4-yl)formamide

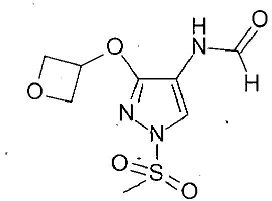

610 mg of ammonium chloride (5 eq.) and 600 mg of iron (5 eq.) was added to a solution of 0.6 g (1 eq.) of 1-(methylsulfonyl)-4-nitro-3-(oxetan-3-yloxy)-1H-pyrazole in 18 mL of ethanol and 2 mL of water. The reaction mixture was heated at 80°C under vigorous stirring for 10 minutes and the mixture was filtered on celite with ethanol washes and concentrated under reduced pressure. The crude material was diluted with 20 mL of ethyl acetate and washed with 20 mL of an aqueous saturated solution of sodium chloride. The organic layers were dried over magnesium sulfate and concentrated under vacuum.

A solution of the residue in 3 mL of tetrahydrofuran was dropwise added to a solution of 1.08 mL of acetic anhydride (5 eq.) in 1.55 mL of formic acid (18 eq.) that had been premixed for 30 minutes at room temperature. The reaction mixture was stirred at room temperature for 1 hour then concentrated under vacuum. The residue was purified on silica, eluting with 5% of methanol in dichloromethane to afford 250 mg of N-(1-(methylsulfonyl)-3-(oxetan-3-yloxy)-1H-pyrazol-4-yl)formamide. MS (method B) m/z 262 [M+1]+; t= 0.78 min.

### Intermediate 19: N-(1-methyl-3-(((2R,3S)-2-methyloxetan-3-yl)oxy)-1H-pyrazol-4-yl)formamide and N-(1-methyl-3-(((2S,3R)-2-methyloxetan-3-yl)oxy)-1H-pyrazol-4-yl)formamide (racemic trans)

### Step 1: Preparation of 1-methyl-3-(((2R,3S)-2-methyloxetan-3-yl)oxy)-4-nitro-1H-pyrazole and 1-methyl-3-(((2S,3R)-2-methyloxetan-3-yl)oxy)-4-nitro-1H-pyrazole (racemic trans) and 1-methyl-3-(((2S,3S)-2-methyloxetan-3-yl)oxy)-4-nitro-1H-pyrazole and 1-methyl-3-(((2R,3R)-2-methyloxetan-3-yl)oxy)-4-nitro-1H-pyrazole (racemic cis)

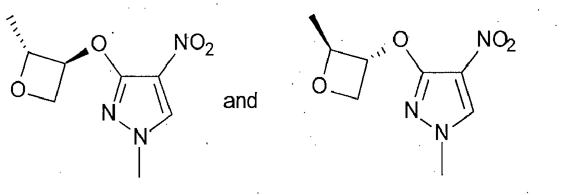

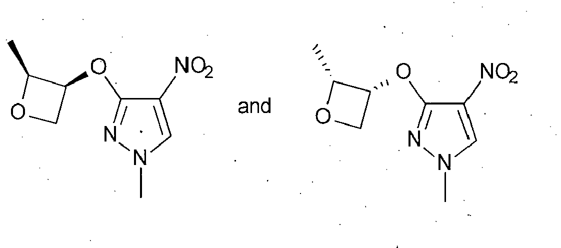

2.53 g (1.3 eq.) of 2-methyloxetan-3-ol and 14.68 g (2 eq.) of cesium carbonate were added to a solution of 4 g (1 eq.) of 1-methyl-3,4-dinitro-1H-pyrazole (commercially available) in 100 mL of acetonitrile. The mixture was heated at 65°C for 3 hours then allowed to cool to room temperature and poured onto ethyl acetate and water. The aqueous layer was separated and extracted three times with ethyl acetate. The combined organic layers were dried over magnesium sulfate and concentrated under vacuum. The residue was purified on silica gel, eluting with a gradient of 10 to 50% of ethyl acetate in heptane, to afford 2.18 g of a first mixture and 1.18 g of a second mixture.

The first mixture was characterized by NMR as the racemic *trans* mixture of 1-methyl-3-(((2R,3S)-2-methyloxetan-3-yl)oxy)-4-nitro-1H-pyrazole and 1-methyl-3-(((2S,3R)-2-methyloxetan-3-yl)oxy)-4-nitro-1H-pyrazole. MS (method B) m/z 214 [M+1]+; t=1.05 min (racemic *trans).*

The second mixture was characterized by NMR as the racemic *cis* mixture of 1-methyl-3-(((2S,3S)-2-methyloxetan⁻3-yl)oxy)-4-nitro-1H-pyrazole and 1-methyl-3-(((2R,3R)-2-methyloxetan-3-yl)oxy)-4-nitro-1H-pyrazole. MS (method B) m/z 214 [M+ 1]+; t=1.02 min (racemic *cis).*

### Step 2: Preparation of N-(1-methyl-3-(((2R,3S)-2-methyloxetan-3-yl)oxy)-1H-pyrazol-4-yl)formamide and N-(1-methyl-3-(((2S,3R)-2-methyloxetan-3-yl)oxy)-1H-pyrazol-4-yl)formamide (racemic trans)

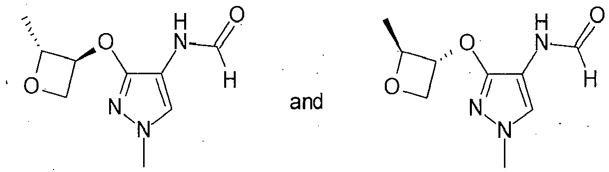

A solution of 535 mg (1 eq.) of a mixture of 1-methyl-3-(((2R,3S)-2-methyloxetan-3-yl)oxy)-4-nitro-1H-pyrazole and 1-methyl-3-(((2S,3R)-2-methyloxetan-3-yl)oxy)-4-nitro-1H-pyrazole (racemic *trans)* in 170 mL of methanol was treated with 0.26 g of palladium on carbon (10%) under 2.5 bars of hydrogen for 1.5 hours. The mixture was filtered with methanol washes and concentrated under reduced pressure. A solution of 943 µL of acetic anhydride (4 eq.) in 1.73 mL of formic acid (18 eq.) that had been premixed for 30 minutes was dropwise added to a solution of the residue in 12 mL of tetrahydrofuran at 0°C. The reaction mixture was stirred for 1 hour allowing the temperature to warm up to room temperature and then concentrated under vacuum. The residue was purified on silica gel, eluting with a gradient of 0 to 100% of ethyl acetate in heptane, to afford 410 mg of a mixture of N-(1-methyl-3-(((2R,3S)-2-methyloxetan-3-yl)oxy)-1H-pyrazol-4-yl)formamide and N-(1-methyl-3-(((2S,3R)-2-methyloxetan-3-y))oxy)-1H-pyrazol-4-yl)formamide (racemic *trans).* MS (method B) m/z 212 [M+ 1]+; t=0.81 min.

### Intermediate 20:

N-(1-methyl-3-(((2S,3S)-2-methyloxetan-3-yl)oxy)-1H-pyrazol-4-yl)formamide and N-(1-methyl-3-(((2R,3R)-2-methyloxetan-3-yl)oxy)-1H-pyrazol-4-yl)formamide (racemic *cis*)

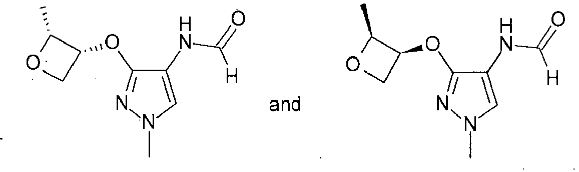

A solution of 350 mg (1 eq.) of a mixture of 1-methyl-3-(((2S,3S)-2-methyloxetan-3-yl)oxy)-4-nitro-1H-pyrazole and 1-methyl-3-(((2R,3R)-2-methyloxetan-3-yl)oxy)-4-nitro-1H-pyrazole (racemic *cis)* prepared in Intermediate 19 step 1 in 40 mL of methanol was treated with 0.1 g of palladium on carbon (10%) under 2.5 bars of hydrogen for 1 hour. The mixture was filtered with methanol washes and concentrated under reduced pressure. A solution of 619 µL of acetic anhydride (4 eq.) in 1.13 mL of formic acid (18 eq.) that had been premixed for 30 minutes was dropwise added to a solution of the residue in 10 mL of tetrahydrofuran at 0°C. The reaction mixture was stirred for 1 hour allowing the temperature to warm up to room temperature and concentrated under vacuum. The residue was purified on silica gel, eluting with a gradient of 0 to 100% of ethyl acetate in heptane, to afford 143 mg of a mixture of N-(1-methyl-3-(((2R,3R)-2-methyloxetan-3-yl)oxy)-1H-pyrazol-4-yl)formamide and N-(1-methyl-3-(((2S,3S)-2-methyloxetan-3-yl)oxy)-1H-pyrazol-4-yl)formamide (racemic *cis).* MS (method B) m/z 212 [M+ 1]+; t=0.75 min.

### Intermediate 21: N-(1-(methyl-d3)-3-(((2S,3R)-2-methyloxetan-3-yl)oxy)-1H-pyrazol-4-yl)formamide and N-(1-(methyl-d3)-3-(((2R,3S)-2-methyloxetan-3-yl)oxy)-1H-pyrazol-4-yl)formamide (racemic trans)

### Step 1: Preparation of 1-(methyl-d3)-3,4-dinitro-1H-pyrazole

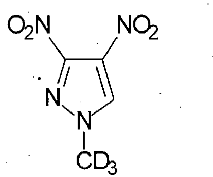

6.42 g (1.4 eq.) of iodomethane-d3 and 8.83 g (2 eq.) of potassium carbonate were added to a solution of 5 g (1 eq.) of 3,4-dinitro-1H-pyrazole (commercially available) in 150 mL of acetonitrile at 0°C. The mixture was stirred at room temperature for 6.5 hours then poured onto ethyl acetate and water. The aqueous layer was separated and extracted three times with ethyl acetate. The combined organic layers were dried over magnesium sulfate and concentrated under vacuum. The residue was purified on silica gel, eluting with a gradient of 20 to 50% of ethyl acetate in heptane, to afford 4.8 g of 1-(methyl-d3)-3,4-dinitro-1H-pyrazole. ¹H NMR (400 MHz, DMSO-d6) δ in ppm: 9.1 (s, 1H).

### Step 2: Preparation of 1-(methyl-d3)-3-(((2S,3R)-2-methyloxetan-3-yl)oxy)-4-nitro-1H-pyrazole and 1-(methyl-d3)-3-(((2R,3S)-2-methyloxetan-3-yl)oxy)-4-nitro-1H-pyrazole (racemic trans) and 1-(methyl-d3)-3-(((2S,3S)-2-methyloxetan-3-yl)oxy)-4-nitro-1H-pyrazole and 1-(methyl-d3)-3-(((2R,3R)-2-methyloxetan-3-yl)oxy)-4-nitro-1H-pyrazole (racemic cis)

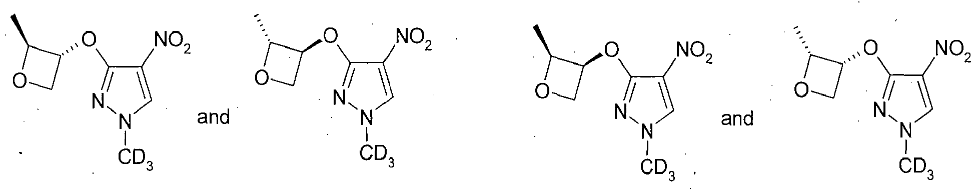

3.16 g (1.5 eq.) of 2-methyloxetan-3-ol and 15.63 g (2 eq.) of cesium carbonate were added to a solution of 4.2 g (1 eq.) of 1-(methyl-d3)-3,4-dinitro-1H-pyrazole in 115 mL of acetonitrile. The mixture was heated at 65°C for 7 hours then allowed to cool to room temperature and poured onto ethyl acetate and water. The aqueous layer was separated and extracted three times with ethyl acetate. The combined organic layers were dried over magnesium sulfate and concentrated under vacuum. The residue was purified on silica gel, eluting with 20% of ethyl acetate in heptane, to afford 2.2 g of a first mixture and 1.25 g of a second mixture.

The first mixture was characterized by NMR as the racemic *trans* mixture of 1-(methyl-d3)-3-(((2S,3R)-2-methyloxetan-3-yl)oxy)-4-nitro-1H-pyrazole and 1-(methyl-d3)-3-(((2R,3S)-2-methyloxetan-3-yl)oxy)-4-nitro-1H-pyrazole MS (method B) m/z 217 [M+1]+; t= 1.05 min.

The second mixture was characterized by NMR as the racemic *cis* mixture of 1-(methyl-d3)-3-(((2S,3S)-2-methyloxetan-3-yl)oxy)-4-nitro-1H-pyrazole and 1-(methyl-d3)-3-(((2R,3R)-2-methyloxetan-3-yl)oxy)-4-nitro-1H-pyrazole. MS (method B) m/z 217 [M+1]+; t= 1.02 min.

### Step 3: Preparation of 1-(methyl-d3)-3-(((2S,3R)-2-methyloxetan-3-yl)oxy)-1H-pyrazol-4-amine and 1-(methyl-d3)-3-(((2R,3S)-2-methyloxetan-3-yl)oxy)-1H-pyrazol-4-amine (racemic trans)

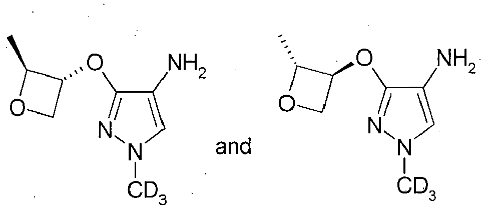

In a microwave vial, a solution of 2.2 g (1 eq.) of 1-(methyl-d3)-3-(((2S,3R)-2-methyloxetan-3-yl)oxy)-4-nitro-1H-pyrazole and 1-(methyl-d3)-3-(((2R,3S)-2-methyloxetan-3-yl)oxy)-4-nitro-1H-pyrazole (racemic *trans)* in 40 mL of methanol was treated with 1.83 g (2.8 eq) of ammonium formate and 541 mg of palladium on carbon (10%) (0.05 eq.). The reaction mixture was heated at 85°C for 30 minutes. The mixture was filtered on celite with methanol washes, concentrated under reduced pressure to afford 1.9 g of 1-(methyl-d3)-3-(((2S,3R)-2-methyloxetan-3-yl)oxy)-1H-pyrazol-4-amine and 1-(methyl-d3)-3-(((2R,3S)-2-methyloxetan-3-yl)oxy)-1H-pyrazol-4-amine (racemic *trans).* The crude material was taken into the next step without further purification.

### Step 4: Preparation of N-(1-(methyl-d3)-3-(((2S,3R)-2-methyloxetan-3-yl)oxy)-1H-pyrazol-4-yl)formamide and N-(1-(methyl-d3)-3-(((2R,3S)-2-methyloxetan-3-yl)oxy)-1H-pyrazol-4-yl)formamide (racemic trans)

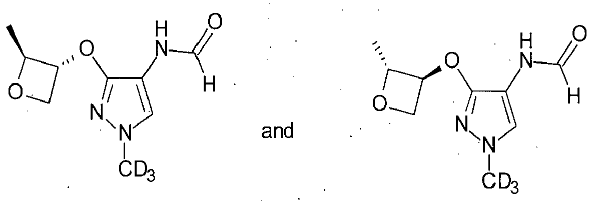

A solution of 1.9 g (1 eq.) of 1-(methyl-d3)-3-(((2S,3R)-2-methyloxetan-3-yl)oxy)-1H-pyrazol-4-amine and 1-(methyl-d3)-3-(((2R,3S)-2-methyloxetan-3-yl)oxy)-1H-pyrazol-4-amine (racemic *trans)* in 15 mL of tetrahydrofuran was dropwise added to a cooled (0°C) solution of 3.87 mL of acetic anhydride (4 eq.) in 3.5 mL of formic acid (8 eq.) that had been premixed for 30 minutes at room temperature. The reaction mixture was stirred at room temperature for 30 minutes then concentrated under vacuum. The residue was purified on silica gel, eluting with a 2% of methanol in dichloromethane, to afford 1.7 g of N-(1-(methyl-d3)-3-(((2S,3R)-2-methyloxetan-3-yl)oxy)-1H-pyrazol-4-yl)formamide and N-(1-(methyl-d3)-3-(((2R,3S)-2-methyloxetan-3-yl)oxy)-1H-pyrazol-4-yl)formamide (racemic *trans).* MS (method B) m/z 215 [M+1]+; t= 1.62 min.

### Intermediate 22: 1-(methyl-d3)-3-(((2S,3S)-2-methyloxetan-3-yl)oxy)-1H-pyrazol-4-amine and 1-(methyl-d3)-3-(((2R,3R)-2-methyloxetan-3-yl)oxy)-1H-pyrazol-4-amine (racemic cis)

### Step 1: Preparation of 1-(methyl-d3)-3-(((2S,3S)-2-methyloxetan-3-yl)oxy)-1H-pyrazol-4-amine and 1-(methyl-d3)-3-(((2R,3R)-2-methyloxetan-3-yl)oxy)-1H-pyrazol-4-amine (racemic cis)

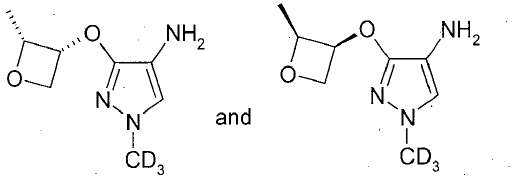

In a microwave vial, a solution of 1.25 g (1 eq.) of 1-(methyl-d3)-3-(((2S,3S)-2-methyloxetan-3-yl)oxy)-4-nitro-1H-pyrazole and 1-(methyl-d3)-3-(((2R,3R)-2-methyloxetan-3-yl)oxy)-4-nitro-1H-pyrazole (racemic *cis)* (Intermediate 21, step 2) in 20 mL of methanol was treated with 1.04 g (2.8 eq) of ammonium formate and 308 mg of palladium on carbon (0.05 eq.). The reaction mixture was heated at 85°C for 30 minutes. The mixture was filtered on celite with methanol washes, concentrated under reduced pressure to afford 1 g of 1-(methyl-d3)-3-(((2S,3S)-2-methyloxetan-3-yl)oxy)-1H-pyrazol-4-amine and 1-(methyl-d3)-3-(((2R,3R)-2-methyloxetan-3-yl)oxy)-1H-pyrazol-4-amine (racemic cis). The crude material was taken into the next step without further purification.

### Step 2: Preparation of N-(1-(methyl-d3)-3-(((2S,3S)-2-methyloxetan-3-yl)oxy)-1H-pyrazol-4-yl)formamide and N-(1-(methyl-d3)-3-(((2R,3R)-2-methyloxeian-3-yl)oxy)-1H-pyrazol-4-yl)formamide (racemic cis)

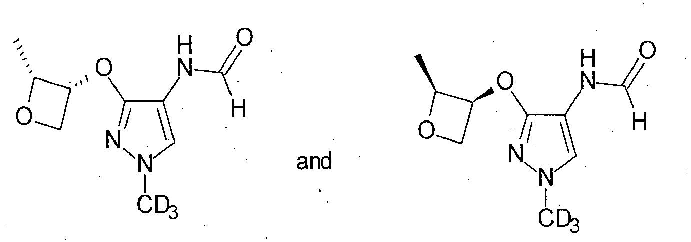

A solution of 1.08 g (1 eq.) of 1-(methyl-d3)-3-(((2S,3S)-2-methyloxetan-3-yl)oxy)-1H-pyrazol-4-amine and 1-(methyl-d3)-3-(((2R,3R)-2-methyloxetan-3-yl)oxy)-1H-pyrazol-4-amine (racemic *cis*)in 7 mL of tetrahydrofuran was dropwise added to a cooled (0°C) solution of 2.2 mL of acetic anhydride (4 eq.) in 2 mL of formic acid (8 eq.) that had been premixed for 30 minutes at room temperature. The reaction mixture was stirred at room temperature for 30 minutes then concentrated under vacuum. The residue was purified on silica gel, eluting with a 2% of methanol in dichloromethane, to afford 0.95 g of N-(1-(methyl-d3)-3-(((2S,3S)-2-methyloxetan-3-yl)oxy)-1H-pyrazol-4-yl)formamide and N-(1-(methyl-d3)-3-(((2R,3R)-2-methyloxetan-3-yl)oxy)-1H-pyrazol-4-yl)formamide (racemic *cis*) as a solid beige. MS (method B) m/z 215 [M+1]+; t=1.55 min.

### Intermediate 23: N-[1-(oxetan-3-yl)-3-[(2,2,2-trifluoro-1-methyl-ethoxy]pyrazol-4-yl]formamide (racemic)

### Step 1: Preparation of 1-[(4-methoxyphenyl)methyl]-4-nitro-3-[2,2,2-trifluoro-1-methyl-ethoxy]pyrazole (racemic)

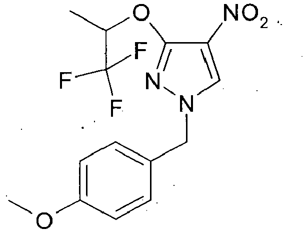

0.66 g (1.05 eq.) of racemic 1,1,1-trifluoro-2-propanol and 3.55 g (2 eq.) of cesium carbonate were added to a solution of 1.5 g (1 eq.) of 1-(4-methoxybenzyl)-3,4-dinitro-1H-pyrazole (Intermediate 13, step 1) in 15 mL of acetonitrile. The mixture was heated at 80°C for 4 hours then allowed to cool to room temperature and poured onto ethyl acetate and water. The aqueous layer was separated and extracted three times with ethyl acetate. The combined organic layers were dried over magnesium sulfate and concentrated under vacuum. The residue was triturated in 10 mL of isopropyl ether, the precipitate filtered and dried under vacuum to afford 2.0 g of racemic 1-[(4-methoxyphenyl)methyl]-4-nitro-3-[2,2,2-trifluoro-1-methyl-ethoxy]pyrazole. MS (method B) m/z 346 [M+1]+; t= 1.86 min.

### Step 2: Preparation of 4-nitro-3-[2,2,2-trifluoro-1-methyl-ethoxy]-1H-pyrazole (racemic)

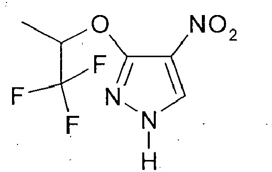

8.1 g (2.5 eq.) of ammonium cerium(IV) nitrate dissolved in 40 mL of water were added to a solution of 2. g (1 eq.) of racemic 1-[(4-methoxyphenyl)methyl]-4-nitro-3-[2,2,2-trifluoro-1-methyl-ethoxy]pyrazole in 40 mL of acetonitrile. The mixture was stirred at room temperature for 2 hours, poured onto 20 mL of a 1M sodium thiosulfate solution and 20 mL of dichloromethane. The aqueous layer was separated and extracted three times with dichloromethane. The combined organic layers were combined, dried over magnesium sulfate and concentrated under vacuum. The residue was triturated in 40 mL of diethyl ethyl ether, the solid filtered, dried under vacuum, to afford 1.1 g of racemic 4-nitro-3-[2,2,2-trifluoro-1-methyl-ethoxy]-1H-pyrazole. MS (method B) m/z 226 [M+1]+; t= 1.39 min.

### Step 3: Preparation of racemic 4-nitro-1-(oxetan-3-yl)-3-[2,2,2-trifluoro-1-methyl-ethoxy]pyrazole

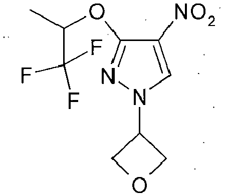

588 µL (1.3 eq.) of 3-iodo-oxetane and 1.3 g (2 eq.) of potassium carbonate were added to a solution of 1.1 g (1 eq.) of racemic 4-nitro-3-[2,2,2-trifluoro-1-methyl-ethoxy]-1H-pyrazole in 16 mL of N,N-dimethylformamide. The mixture was heated at 80°C for 24 hours and then allowed to cool to room temperature and poured onto ethyl acetate and water. The aqueous layer was separated and extracted three times with ethyl acetate. The combined organic layers were washed with an aqueous saturated sodium chloride solution, dried over magnesium sulfate, and concentrated under vacuum. The residue was purified on silica gel, eluting with a gradient of 0 to 5% of ethyl acetate in dichloromethane, to afford 700 mg of racemic 4-nitro-1-(oxetan-3-yl)-3-[2,2,2-trifluoro-1-methyl-ethoxy]pyrazole. MS (method B) m/z 282 [M+1]+; t= 1.57 min.

### Step 4: Preparation of racemic 1-(oxetan-3-yl)-3-[2,2,2-trifluoro-1-methyl-ethoxy]pyrazol-4-amine

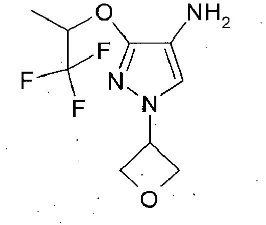

In a microwave vial, a solution of 450 mg (1 eq.) of racemic 4-nitro-1-(oxetan-3-yl)-3-[2,2,2-trifluoro-1-methyl-ethoxy]pyrazole in 15 mL of methanol was treated with 620 mg (6 eq.) of ammonium formate and 170 mg of palladium on carbon (10%). The reaction mixture was heated at 70°C for 20 minutes. The mixture was filtered on celite with methanol washes and concentrated under reduced pressure. The residue was taken into dichloromethane, the solid filtered and the filtrate concentrated under reduced pressure to afford 500 mg of racemic 1-(oxetan-3-yl)-3-[2,2,2-trifluoro-1-methyl-ethoxy]pyrazol-4-amine. The crude material was taken into the next step without further purification.

### Step 5: Preparation of racemic N-[1-(oxetan-3-yl)-3-[2,2,2-trifluoro-1-methyl-ethoxy]pyrazol-4-yl]formamide

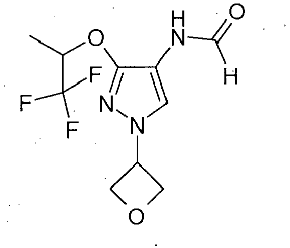

A solution of 500 mg (1 eq.) of racemic 1-(oxetan-3-yl)-3-[2,2,2-trifluoro-1-methyl-ethoxy]pyrazol-4-amine in 3 mL of tetrahydrofuran was dropwise added to a cooled (0°C) solution of 750 µL of acetic anhydride (3 eq.) in 700 µL of formic acid (6 eq.) that had been premixed for 30 minutes at room temperature. The reaction mixture was stirred at 5°C for 30 minutes, and at room temperature for 1 hour then concentrated under vacuum. The residue was diluted in ethyl acetate, poured onto a saturated aqueous solution of sodium hydrogen carbonate. The aqueous layer was separated and extracted three times with ethyl acetate. The combined organic layers were dried over magnesium sulfate and concentrated under vacuum. The residue was purified on silica gel, eluting with a gradient of 0 to 5% of methanol in dichloromethane, to afford 530 mg of racemic N-[1-(oxetan-3-yl)-3-[2,2,2-trifluoro-1-methyl-ethoxy]pyrazol-4-yl]formamide. MS (method B) m/z 280 [M+1]+; t=1.20 min.

### Intermediate 24: N-(1-cyclopropyl-3-(oxetan-3-yloxy)-1H-pyrazol-4-yl)formamide

### Step 1: Preparation of 1-cyclopropyl-4-nitro-3-(oxetan-3-yloxy)-1H-pyrazole

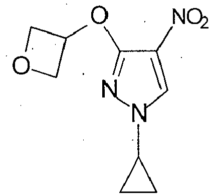

0.59 g (2 eq.) of cyclopropyl boronic acid, 0.87 g (1 eq.) of 4'-di-tert-butyl-2,2'-bipyridine, 0.7 g (2 eq.) of sodium carbonate, and 0.59 g (1 eq.) of copper(II) acetate were added to a solution of 0.6 g (1 eq.) of 4-nitro-3-(oxetan-3-yloxy)-1H-pyrazole) (Intermediate 13, step 3) in 22 mL of 1,2-dichloroethane. The mixture was refluxed for 3 hours then allowed to cool to room temperature, poured onto dichloromethane and water and filtered through a pad of Celite. The aqueous layer was separated and extracted three times with ethyl acetate. The combined organic layers were dried over magnesium sulfate and concentrated under vacuum. The residue was purified on silica gel, eluting with 10% to 50% of ethyl acetate in cyclohexane, to afford 0.6 g of 1-cyclopropyl-4-nitro-3-(oxetan-3-yloxy)-1H-pyrazole. MS (method A) m/z 226 [M+1]+; t= 1.69 min.

### Step 2: Preparation of 1-cyclopropyl-3-(oxetan-3-yloxy)-1H-pyrazol-4-amine

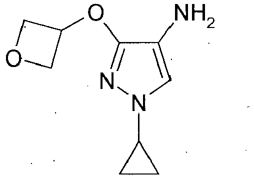

A solution of 0.6 g (1 eq.) of 1-cyclopropyl-4-nitro-3-(oxetan-3-yloxy)-1H-pyrazole in 15 mL of methanol was treated with 442 mg of ammonium formate (2.8 eq.) and 261 mg (0.05 eq.) of palladium on carbon (10%). The reaction mixture was heated at 70°C for 15 minutes. The mixture was filtered on celite with methanol washes and concentrated under reduced pressure to afford 1-cyclopropyl-3-(oxetan-3-yloxy)-1H-pyrazol-4-amine, which was used in the next step without further purification.

### Step 3: Preparation of N-(1-cyclopropyl-3-(oxetan-3-yloxy)-1H-pyrazol-4-yl)formamide

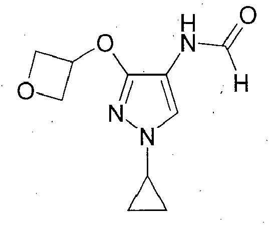

A solution of 478 mg (1 eq.) of 1-cyclopropyl-3-(oxetan-3-yloxy)-1H-pyrazol-4-amine in 2 mL of tetrahydrofuran was dropwise added at 0°C to a solution of 0.924 mL of acetic anhydride (4 eq.) in 1.69 mL of formic acid (18 eq.) that had been premixed for 30 minutes at room temperature. The reaction mixture was stirred at 0°C for 30 minutes, then concentrated under vacuum. The residue was purified on silica gel, eluting with heptane/ethyl acetate/7N NH₃ in MeOH (50/45/5) to afford 390 mg of N-(1-cyclopropyl-3-(oxetan-3-yloxy)-1H-pyrazol-4-yl)formamide. MS (method B) m/z 224 [M+1]+; t=1.26 min.

### Intermediate 25: N-[1-[2,2-difluoro-1-methyl-ethyl]-3-(oxetan-3-yloxy)pyrazol-4-yl]formamide

### Step 1: Preparation of 1,1-difluoropropan-2-yl methanesulfonate (racemic)

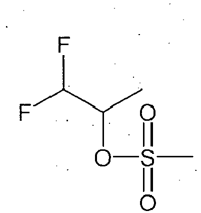

1.5 mL (1.05 eq.) of triethylamine was added to a solution of 1 g (1 eq.) of racemic 1,1-difluoropropan-2-ol in 20 mL of dichoromethane. The mixture was cooled at 0°C and 1.8 g (1 eq.) of methanesulfonic anhydride was added. The reaction mixture was warmed to room temperature and stirred for 2 hours then poured onto 50 mL of ethyl acetate and, 50 mL of diethyl ether. The organic layer was washed three times with 10 mL of water then dried over sodium sulfate and concentrated under vacuum to afford 1.5 g of racemic 1,1-difluoropropan-2-yl methanesulfonate.
¹H NMR (400 MHz, CDCl₃) δ in ppm: 1.45 (m, 3 H); 2.98 (s, 3 H); 4.80 (m, 1 H); 5.75 (dt, J=4.5 and 53 Hz, 1 H).

### Step 2: Preparation of 1-[2,2-difluoro-1-methyl-ethyl]-4-nitro-3-(oxetan-3-yloxy)-1H-pyrazole (racemic)

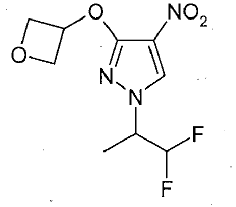

282 mg (1.5 eq.) of racemic 1,1-difluoropropan-2-yl methanesulfonate and 704 mg of cesium carbonate were added to a solution of 200 mg (1 eq.) of 4-nitro-3-(oxetan-3-yloxy)-1H-pyrazole (Intermediate 13, step 3) in 10 mL of acetonitrile. The mixture was heated at 80°C for 18 hours. The mixture was filtered on decalite, washed twice with 5 mL of acetonitrile, and concentrated under reduced pressure. The residue was purified on silica gel, eluting with a gradient of 0 to 40% of ethyl acetate in cyclohexane, to afford 100 mg of racemic 1-[2,2-difluoro-1-methyl-ethyl]-4-nitro-3-(oxetan-3-yloxy)-1H-pyrazole. MS (method E) m/z 264 [M+ 1]+; t=1.1 min.

### Step 3: Preparation of N-[1-[2,2-difluoro-1-methyl-ethyl]-3-(oxetan-3-yloxy)pyrazol-4-yl]formamide (racemic)

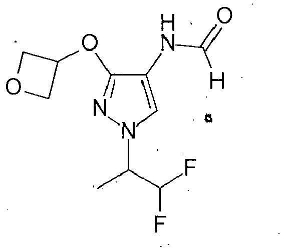

101 mg of ammonium chloride (5 eq.) and 106 mg of iron (5 eq.) were added to a solution of 100 mg of racemic 1-[2,2-difluoro-1-methyl-ethyl]-4-nitro-3-(oxetan-3-yloxy)-1H-pyrazole in 8 mL of ethanol and 1.2 mL of water. The reaction mixture was heated at 80°C under vigorous stirring for 10 minutes, filtered on celite with ethanol washes and concentrated under reduced pressure. The crude material was diluted with 20 mL of ethyl acetate and washed with 20 mL of an aqueous saturated solution of sodium chloride. The organic layer was dried over sodium sulfate and concentrated under vacuum. A solution of the residue in 3 mL of tetrahydrofuran was dropwise added to a solution of 179 µL of acetic anhydride (5 eq.) in 258 µL of formic acid (18 eq.) that had been premixed for 30 minutes at room temperature. The reaction mixture was stirred at room temperature for 1 hour then concentrated under vacuum. The residue was purified on silica, eluting with 5% of methanol in dichloromethane to afford 60 mg of racemic N-[1-[2,2-difluoro-1-methylethyl]-3-(oxetan-3-yloxy)pyrazol-4-yl]formamide as a brown oil. MS (method E) m/z 262 [M+ 1]+; t=0.92 min.

### Intermediate 26: N-(1-(methyl-d3)-3-(oxetan-3-yloxy)-1H-pyrazol-4-yl)formamide

### Step 1: Preparation of 1-(methyl-d3)-4-nitro-3-(oxetan-3-yloxy)-1H-pyrazole

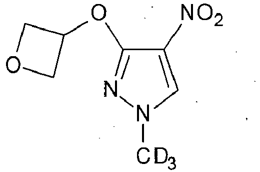

126 µL (1.5 eq.) of iodomethane-d3 (commercially available) and 880 mg of cesium carbonate were added to a solution of 250 mg (1 eq.) of 4-nitro-3-(oxetan-3-yloxy)-1H-pyrazole (Intermediate 13, step 3) in 10 mL of N, N-dimethylformamide. The mixture was stirred at room temperature for 1 hour and poured onto a mixture of 20 mL of ethyl acetate and 20 mL of diethyl ether. The organic layer was washed with 3 times with 10 mL of water then was dried over sodium sulfate and concentrated under vacuum. The residue was purified on silica gel, eluting with a gradient of 0 to 80% of ethyl acetate in cyclohexane, to afford 215 mg of 1-(methyl-d3)-4-nitro-3-(oxetan-3-yloxy)-1H-pyrazole. MS (method B) m/z 280 [M+1]+; t=1.20 min.

### Step 2: Preparation of N-(1-(methyl-d3)-3-(oxetan-3-yloxy)-1H-pyrazol-4-yl)formamide

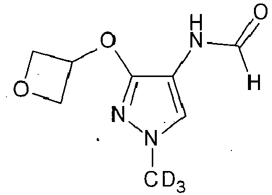

284 mg of ammonium chloride (5 eq.) and 297 mg of iron (5 eq.) were added to a solution of 215 mg of 1-(methyl-d3)-4-nitro-3-(oxetan-3-yloxy)-1H-pyrazole in 8 mL of ethanol and 1.2 mL of water. The reaction mixture was heated at 80°C under vigorous stirring for 10 minutes and the mixture was filtered on celite with ethanol washes and concentrated under reduced pressure. The crude material was diluted with 20 mL of ethyl acetate and washed with 20 mL of an aqueous saturated solution of sodium chloride. The organic layer was dried over sodium sulfate and concentrated under vacuum.

A solution of the residue in 5 mL of tetrahydrofuran was dropwise added to a solution of 503 µL of acetic anhydride (5 eq.) in 722 µL of formic acid (18 eq.) that had been premixed for 30 minutes at room temperature. The reaction mixture was stirred at room temperature for 1 hour then concentrated under vacuum. The residue was purified on silica, eluting with 5% of methanol in dichloromethane to afford 150 mg of N-(1-(methyl-d3)-3-(oxetan-3-yloxy)-1H-pyrazol-4-yl)formamide. MS (method B) m/z 201 [M+1]+; t=0.62 min.

### Intermediate 27: N-(1-(2-cyanopropan-2-yl)-3-(oxetan-3-yloxy)-1H-pyrazol-4-yl)formamide

### Step 1: Preparation of methyl 2-(3,4-dinitro-1H-pyrazol-1-yl)-2-methylpropanoate

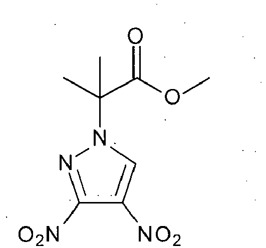

1.6 mL (1 eq.) of methyl 2-bromo-2-methylpropanoate and 8.2 g (2 eq.) of cesium carbonate were added to a solution of 2 g (1 eq.) of 3,4-dinitro-1H-pyrazole (commercially available) in 30 mL of N,N-dimethylformamide. The mixture was heated at 80°C for 4 h then allowed to cool to room temperature and poured onto 50 mL of ethyl acetate and 100 mL of diethyl ether. The organic layer was washed with 3 times with 50 mL of water, dried over sodium sulfate, and concentrated under vacuum. The residue was purified on silica gel, eluting with a gradient of 0 to 40% of ethyl acetate in cyclohexane, to afford 1 g of methyl 2-(3,4-dinitro-1H-pyrazol-1-yl)-2-methylpropanoate. MS (method E) m/z 257 [M+1]+; t=1.23 min.

### Step 2: Preparation of methyl 2-methyl-2-(4-nitro-3-(oxetan-3-yloxy)-1H-pyrazol-1-yl)propanoate

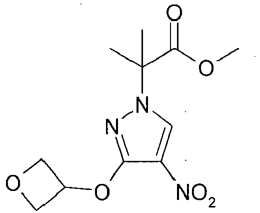

574 mg (2 eq.) of oxetan-3-ol and 3.1 g (2.5 eq.) of cesium carbonate were added to a solution of 1 g (1 eq.) of methyl 2-(3,4-dinitro-1H-pyrazol-1-yl)-2-methylpropanoate in 50 mL of acetonitrile. The mixture was heated at 130°C for 1 hour then allowed to cool to room temperature and poured onto 30 mL of ethyl acetate and 50 mL of diethyl ether. The organic layer was washed twice with 20 mL of water, dried over sodium sulfate and concentrated under vacuum. The residue was purified on silica gel, eluting with a gradient of 0 to 40% of ethyl acetate in cyclohexane, to afford 602 mg of methyl 2-methyl-2-(4-nitro-3-(oxetan-3-yloxy)-1H-pyrazol-1-yl)propanoate. MS (method E) m/z 286 [M+1]+; t=1.12 min.

### Step 3: Preparation of 2-methyl-2-(4-nitro-3-(oxetan-3-yloxy)-1H-pyrazol-1-yl)propanamide

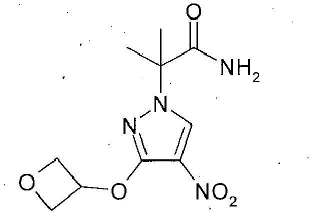

2.34 mL (4 eq.) of a 12N aqueous solution of sodium hydroxide was added to a solution of 2 g (1 eq.) of methyl 2-methyl-2-(4-nitro-3-(oxetan-3-yloxy)-1H-pyrazol-1-yl)propanoate in 8 mL of methanol. The mixture was heated at 40°C for 1 hour then -allowed to cool to room temperature and the pH was adjusted to ~ 1 with a 2N HCl aqueous solution. The mixture was concentrated under vacuum. The residue was purified on silica gel, eluting with a gradient of 0 to 10 % of methanol in dicloromethane to afford 1.8 g of corresponding acid. To the residue solubilized in 100 mL of dicloromethane was added 1.3 g (1.18 eq.) of 1,1'-carbonyldiimidazole. The mixture was stirred at room temperature for 18 hours, then 2.9 mL (5 eq) of ammonium hydroxide (12N) was added. The mixture was stirred for another 15 min and concentrated under vacuum. The residue was purified on silica gel, eluting with a gradient of 0 to 10 % of methanol in dicloromethane, to afford 1.5 g of 2-methyl-2-(4-nitro-3-(oxetan-3-yloxy)-1H-pyrazol-1-yl)propanamide. MS (method E) m/z 271 [M+1]+; t=0.91 min.

### Step 4: Preparation of 2-methyl-2-(4-nitro-3-(oxetan-3-yloxy)-1H-pyrazol-1-yl)propanenitrile

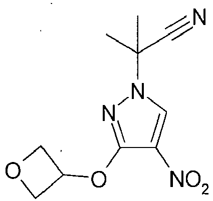

4.64 mL (6 eq.) of triethylamine was added to a solution of 1.5 g (1 eq.) of 2-methyl-2-(4-nitro-3-(oxetan-3-yloxy)-1H-pyrazol=1-yl)propanamide in 15 mL of tetrahydrofuran. The mixture was cooled at 0°C and 3.14 mL (4 eq.) of trifluoroacetic anhydride was added. The reaction mixture was allowed to warm up to room temperature and stirred for 1 hour then poured onto 50 mL of diethyl ether and 20 mL of water. The organic layer was dried over sodium sulfate and concentrated under vacuum to afford 1 g of 2-methyl-2-(4-nitro-3-(oxetan-3-yloxy)-1H-pyrazol-1-yl)propanenitrile. MS (method E) m/z 253 [M+1]+; t=1.11 min.

### Step 5: Preparation of N-(1-(2-cyanopropan-2-yl)-3-(oxetan-3-yloxy)-1H-pyrazol-4-yl)formamide

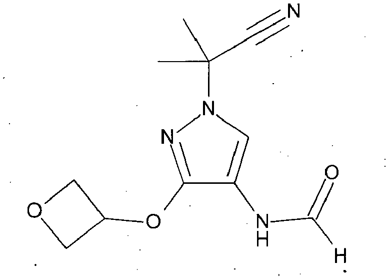

1.06 g of ammonium chloride (5 eq.) and 1.11 g of iron (5 eq.) were added to a solution of 1 g of 2-methyl-2-(4-nitro-3-(oxetan-3-yloxy)-1H-pyrazol-1-yl)propanenitrile in 16 mL of ethanol and 4 mL of water. The reaction mixture was heated at 80°C under vigorous stirring for 10 minutes and the mixture was filtered on celite with ethanol washes and concentrated under reduced pressure. The crude material was diluted with 20 mL of ethyl acetate and washed with 20 mL of an aqueous saturated solution of sodium chloride. The organic layer was dried over sodium sulfate and concentrated under vacuum.

A solution of the residue in 10 mL of tetrahydrofuran was dropwise added to a solution of 1.87 mL of acetic anhydride (5 eq.) in 2.69 mL of formic acid (18 eq.) that had been premixed for 30 minutes at room temperature. The reaction mixture was stirred at room temperature for 1 hour then concentrated under vacuum. The residue was purified on silica, eluting with 5% of methanol in dichloromethane to afford 750 mg of N-(1-(2-cyanopropan-2-yl)-3-(oxetan-3-yloxy)-1H-pyrazol-4-yl)formamide. MS (method E) m/z 251 [M+1]+; t= 0.94 min.

### Intermediate 28: (3-cyclopropoxy-1-(1,1-difluoropropan-2-yl)-1H-pyrazol-4-yl)formamide

### Step 1: Preparation of 3-cyclopropoxy-1-(1,1-difluoropropan-2-yl)-4-nitro-1H-pyrazole (racemic)

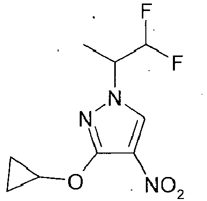

200 mg of racemic 3-cyclopropoxy-1-(1,1-difluoropropan-2-yl)-4-nitro-1H-pyrazole was prepared according to the basic procedure described in Intermediate 25, step 2 starting with 300 mg of 3-cyclopropoxy-4-nitro-1H-pyrazole. MS (method E) m/z 248 [M+1]+; t= 1.27 min.

### Step 2: Preparation of N-(3-cyclopropoxy-1-(1,1-difluoropropan-2-yl)-1H-pyrazol-4-yl)formamide (racemic)

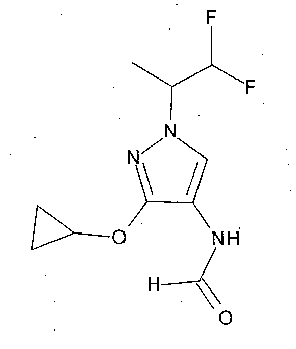

216 mg of ammonium chloride (5 eq.) and 226 mg of iron (5 eq.) were added to a solution of 200 mg of racemic 3-cyclopropoxy-1-(1,1-difluoropropan-2-yl)-4-nitro-1H-pyrazole in 8 mL of ethanol and 1.2 mL of water. The reaction mixture was heated at 80°C under vigorous stirring for 10 minutes and the mixture was filtered on celite with ethanol washes and concentrated under reduced pressure. The crude material was diluted with 20 mL of ethyl acetate and washed with 20 mL of an aqueous saturated solution of sodium chloride. The organic layer was dried over sodium sulfate and concentrated under vacuum.

A solution of the residue in 4 mL of tetrahydrofuran was dropwise added to a solution of 382 µL of acetic anhydride (5 eq.) in 549 µL of formic acid (18 eq.) that had been premixed for 30 minutes at room temperature. The reaction mixture was stirred at room temperature for 1 hour then concentrated under vacuum. The residue was purified on silica, eluting with 5% of methanol in dichloromethane to afford 45 mg of racemic N-(3-cyclopropoxy-1-(1,1-difluoropropan-2-yl)-1H-pyrazol-4-yl)formamide. MS (method E) m/z 246 [M+1]+; t = 1.01 min.

### Intermediate 29: N-[3-(cyclopropoxy)-1-(methyl-d3)pyrazol-4-yl]formamide

### Step 1: Preparation of trimethyl-[2-[(4-nitropyrazol-1-yl)methoxy]ethyl]silane

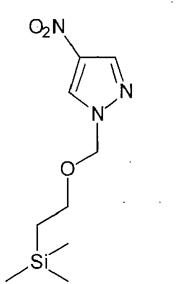

10 g (1 eq.) of 4-nitro-1H-pyrazole (commercially available) was dissolved in 100 mL of dry tetrahydrofuran under argon atmosphere. The mixture was cooled to -5°C and 4.24 g (1.2 eq.) of sodium hydride (60% in mineral oil) were added by portions. After stirring at room temperature for 10 min, the mixture was cooled to 0°C and 18 mL (1.15 eq.) of 2-(trimethylsilyl)ethoxymethyl chloride (SEM-Cl) were then added dropwise. The mixture was stirred at room temperature for 1.5 h and quenched by addition of ice and diethyl ether. The organic layer was separated and washed twice with water, dried over magnesium sulfate, and concentrated under vacuum. The residue was purified on silica gel, eluting with 20% of ethyl acetate in heptane, to afford 21.34 g of trimethyl-[2-[(4-nitropyrazol-1-yl)methoxy]ethyl]silane. MS (method E) m/z 242 [M-H]; t= 1.95 min.

### Step 2: Preparation of 2-[(3-chloro-4-nitro-pyrazol-1-yl)methoxy]ethyl-trimethyl-silane

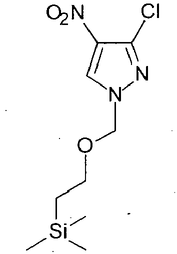

53 mL (1.2 eq.) of 1N lithium bis(trimethylsilyl)amide in tetrahydrofuran were dropwise added to a solution of 10.8 g (1 eq.) of trimethyl-[2-[(4-nitropyrazol-1-yl)methoxy]ethyl]silane in 80 mL of dry tetrahydrofuran at -78°C. The mixture was allowed to stir at -50°C for 30 minutes, then cooled back to -78°C and a solution of 13.66 g (1.3 eq.) of perchloroethane in 50 mL of anhydrous tetrahydrofuran was then dropwise added. After stirring at -70°C for 2 hours, the mixture was quenched with a 10% aqueous citric acid solution and diethyl ether. The organic layer was separated and washed with 10% aqueous citric acid solution, water and brine, dried over sodium sulfate and sodium bicarbonate and concentrated under vacuum. The residue was purified on silica gel, eluting with 5% of ethyl acetate in cyclohexane, to afford 10 g of 2-[(3-chloro-4-nitro-pyrazol-1-yl)methoxy]ethyl-trimethyl-silane. MS (method E), no ionization, t = 1.60 min.

### Step 3: Preparation of 2-[[3-(cyclopropoxy)-4-nitro-pyrazol-1-yl]methoxy]ethyl-trimethylsilane

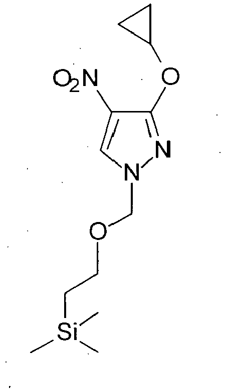

A suspension of 576 mg (2 eq.) of sodium hydride (60% in mineral oil) in 20 mL of anhydrous tetrahydrofuran was cooled to -10°C and 837 mg (2 eq.) of cyclopropanol was added. After stirring for 30 minutes, 2 g (1 eq.) of 2-[(3-chloro-4-nitro-pyrazol-1-yl)methoxy]ethyl-trimethyl-silane in 10 mL of anhydrous tetrahydrofuran was added. The mixture was stirred at 5°C for 30 minutes and was quenched with 10% aqueous citric acid solution and diethyl ether. The organic layer was separated and washed twice with water, dried over magnesium sulfate and concentrated under vacuum, to afford 2.1 g of 2-[[3-(cyclopropoxy)-4-nitro-pyrazol-1-yl]methoxy]ethyl-trimethyl-silane. The residue was used at the next step without further purification. MS (method H), no ionization, t= 1.25 min.

### Step 4: Preparation of [3-(cyclopropoxy)-4-nitro-pyrazol-1-yl]methanol

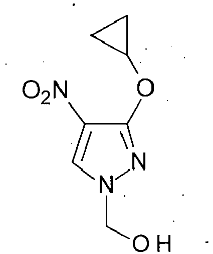

100 mL (4.8.eq.) of 1N hydrochloric solution was added to a solution of 6.28 g (1 eq.) of 2-[[3-(cyclopropoxy)-4-nitro-pyrazol-1-yl]methoxy]ethyl-trimethyl-silane in 200 mL of dry acetonitrile. After heating at 40°C for 3 hours, the reaction was cooled to room temperature and the solvent was removed under vacuum. The aqueous layer was extracted four times with dichloromethane. The combined organic layers were dried over sodium sulfate and concentrated under vacuum. The residue was purified on silica gel, eluting with a gradient of 0 to 50% of ethyl acetate in cyclohexane, to afford 4.72 g of [3-(cyclopropoxy)-4-nitro-pyrazol-1-yl]methanol MS (method G), m/z 200 [M+1]+; t=1.49 min.

### Step 5: Preparation of 3-(cyclopropoxy)-4-nitro-1H-pyrazole

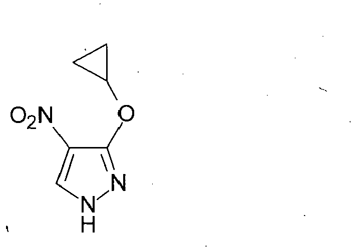

A solution of 2.86 g (1 eq.) of 3-(cyclopropoxy)-4-nitro-pyrazol-1-yl]methanol in 20 mL of methanol and 100 mL of 7N of NH₃ in MeOH was stirred at room temperature overnight. The reaction mixture was concentrated under vacuum to afford 2.5 g of 3-(cyclopropoxy)-4-nitro-1H-pyrazole. MS m/z 170 [M+1]+; t=0.88 min.

### Step 6: Preparation of 3-(cyclopropoxy)-4-nitro-1-(methyl-d3)pyrazole

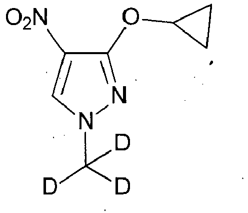

46 µL (1.5 eq.) of iodomethane-d3 (commercially available) and 308 mg (2 eq.) of cesium carbonate were added to a solution of 80 mg (1 eq.) of 3-(cyclopropoxy)-4-nitro-1H-pyrazole in 10 mL of dry acetonitrile. The mixture was heated at 80°C for 1 hour. After cooling to room temperature, the mixture was filtered with acetonitrile washed and the solvent was concentrated. The residue was purified on silica gel, eluting with a gradient of 0 to 40% of ethyl acetate in cyclohexane, to afford 75 mg of 3-(cyclopropoxy)-4-nitro-1-(methyl-d3)pyrazole. MS (method E), m/z 187 [M+1]+; t= 0.94 min.

### Step 7: Preparation of N-[3-(cyclopropoxy)-1-(methyl-d3)pyrazol-4-yl]formamide

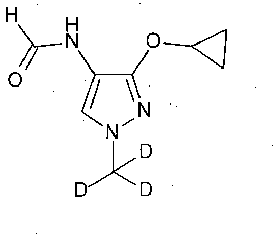

112.5 mg (5.0 eq.) of dust iron and 108.0 mg (5.0 eq.) of ammonium chloride were added to a solution of 75 mg (1 eq.) of 3-(cyclopropoxy)-4-nitro-1-(methyl-d3)pyrazole in a mixture of 8 mL of ethanol and 2 mL of water. The mixture was heated at 80°C for 30 minutes, then allowed to cool to room temperature. The reaction mixture was filtered on celite with ethanol washed and the filtrate concentrated under reduced pressure. The residue was taken in 10 mL of dry tetrahydrofuran and dropwise added to a solution of 190 µL (5 eq.) of acetic anhydride in 273 µL (18 eq.) of formic acid that had been premixed for 30 minutes at room temperature. The reaction mixture was stirred at room temperature for 1 hour then concentrated under vacuum. The residue was purified on silica gel, eluting with a gradient of 0 to 10% of methanol in dichloromethane, to afford 25 mg of N-[3-(cyclopropoxy)-1-(methyl-d3)pyrazol-4-yl]formamide. MS (method E) m/z 214 [M+1]+; t =1.37 min.

### Intermediate 30: N-[1-(1-cyano-1-methyl-ethyl)-3-(cyclopropoxy)pyrazol-4-yl]formamide

### Step 1: Preparation of methyl 2-[3-(cyclopropoxy)-4-nitro-pyrazol-1-yl]-2-methylpropanoate

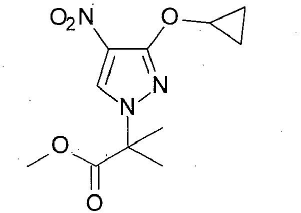

2.6 g (2.5 eq.) of potassium carbonate was added to a solution of 1.5 g (1 eq.) of [3-(cyclopropoxy)-4-nitro-pyrazol-1-yl]methanol (intermediate 29, step 4) in 45 mL of dry N,N-dimethylformamide. The reaction mixture was heated at 70°C for 2 hours, followed by the addition of 1.3 mL (1.3 eq.) of methyl 2-bromo-2-methylpropanoate. The resulting mixture was stirred for an additional 3 hours at 70°C before water was added to quench the reaction. The aqueous layer was extracted with ethyl acetate/ diethyl ether. The organic layer was dried over sodium sulfate, filtered, and concentrated under vacuum. The residue was purified on silica gel, eluting with a gradient of 0 to 10% of ethyl acetate in cyclohexane, to afford 1.78 g of methyl 2-[3-(cyclopropoxy)-4-nitro-pyrazol-1-yl]-2-methyl-propanoate as a colorless oil. MS (method E) m/z 270 [M+1]+; t=1.63 min.

### Step 2: Preparation of 2-[3-(cyclopropoxy)-4-nitro-pyrazol-1-yl]-2-methyl-propanenitrile

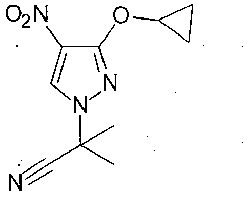

A solution of 1.78 g (1 eq.) of methyl 2-[3-(cyclopropoxy)-4-nitro-pyrazol-1-yl]-2-methyl-propanoate in 60 mL of 7N of NH₃ in MeOH was stirred at room temperature for 2 days, then the reaction mixture was concentrated under vacuum. The residue was taken in 30 mL of dry tetrahydrofuran and were added 1 mL (1.1 eq.) of trifluoroacetic anhydride and 2 mL (2.2 eq.) of triethylamine at 0°C. The reaction mixture was stirred for 1 hour before water and diethyl ether were added to quench the reaction. The aqueous layer was separated and extracted with diethyl ether. The combined organic layers were dried over sodium sulfate, filtered, and concentrated under vacuum, to afford 1.35 g of 2-[3-(cyclopropoxy)-4-nitro-pyrazol-1-yl]-2-methyl-propanenitrile. The residue was used without further purification. MS (method E) m/z 237 [M+1]+; t=1.25 min.

### Step 3: Preparation of N-[1-(1-cyano-1-methyl-ethyl)-3-(cyclopropoxy)pyrazol-4-yl]formamide

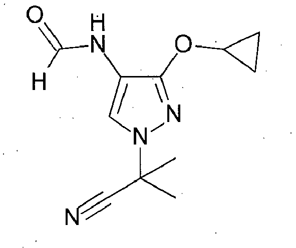

58.0 mg (5.0 eq.) of dust iron and 59.0 mg (5.0 eq.) of ammonium chloride were added to a solution of 50 mg (1 eq.) of 2-[3-(cyclopropoxy)-4-nitro-pyrazol-1-yl]-2-methylpropanenitrile in 4 mL of ethanol and 2 mL of water. The mixture was heated at 80°C for 30 minutes, then allowed to cool to room temperature. The reaction mixture was filtered on celite with ethanol washes and the filtrate concentrated under reduced pressure. The residue was taken in 10 mL of dry tetrahydrofuran and dropwise added to a solution of 100 µL (5 eq.) of acetic anhydride in 144 µL (18 eq.) of formic acid that had been premixed for 30 minutes at room temperature. The reaction mixture was stirred at room temperature for 1 hour then concentrated under vacuum. The residue was purified on silica gel, eluting with a gradient of 0 to 10% of methanol in dichloromethane, to afford 46 mg of N-[1-(1-cyano-1-methyl-ethyl)-3-(cyclopropoxy)pyrazol-4-yl]formamide. MS m/z 235 [M+1]+; t=1.03 min.

### Intermediate 31: N-[1-(1-cyano-1-methyl-ethyl)-3-isopropoxy-pyrazol-4-yl]formamide

### Step 1: Preparation of methyl 2-(3-isopropoxy-4-nitro-pyrazol-1-yl)-2-methyl-propanoate

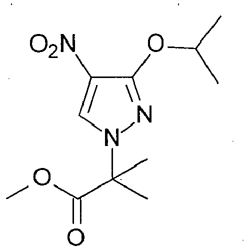

(1 eq.) of 3-isopropoxy-4-nitro-1H-pyrazole (commercially available) was dissolved in 70 mL of dry N,N-dimethylformamide under argon atmosphere. The mixture was cooled to -5°C and 941 mg (1.35 eq.) of sodium hydride (60% in mineral oil) was added by portions. The reaction mixture was stirred at 0°C for 30 minutes, and then 3.04 mL (1.35 eq.) of methyl 2-bromo-2-methylpropanoate was added. The resulting mixture was stirred for 2 days before water was added to quench the reaction. The aqueous layer was extracted twice with a 50/50 mixture of ethyl acetate and diethyl ether. The organic layer was washed twice with water, dried over sodium sulfate, filtered and concentrated under vacuum. The residue was purified on silica gel, eluting with a gradient of 0 to 60% of ethyl acetate in cyclohexane, to afford 3.3 g of methyl 2-(3-isopropoxy-4-nitro-pyrazol-1-yl)-2-methyl-propanoate. MS (method E) m/z 272 [M+1]+; t= 1.76 min.

### Step 2: Preparation of 2-(3-Isopropoxy-4-nitro-pyrazol-1-yl)-2-methyl-propanenitrile

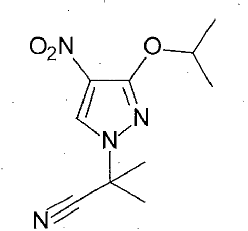

A solution of 3.g (1 eq.) of methyl 2-(3-isopropoxy-4-nitro-pyrazol-1-yl)-2-methylpropanoate in 100 mL of 7N of NH₃ in MeOH was heated at 100°C for 18 hours, then the reaction mixture was cooled to room temperature and concentrated under vacuum. The residue was taken in 50 mL of anhydrous tetrahydrofuran, cooled to 0°C and 2.06 mL (1.2 eq.) of trifluoroacetic anhydride and 4.24 mL (2.5 eq.) of triethylamine were added. The reaction mixture was stirred for 1 hour at room temperature before water and diethyl ether were added to quench the reaction. The aqueous layer was separated and extracted with diethyl ether. The organic layer was dried over sodium sulfate, filtered, and concentrated under vacuum. The residue was purified on silica gel eluting of 10 to 15% ethyl acetate in cyclohexane to afford, after concentration under vacuum, 2.6 g of 2-(3-isopropoxy-4-nitro-pyrazol-1-yl)-2-methyl-propanenitrile.
MS (method F): m/z 239 [M+1]+; t= 1.67 min.

### Step 3: Preparation of N-[1-(1-cyano-1-methyl-ethyl)-3-isopropoxy-pyrazol-4-yl]formamide

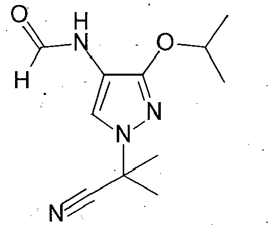

3.05 g of iron (5 eq.) was added to a solution of 2.6 g of 2-(3-isopropoxy-4-nitro-pyrazol-1-yl)-2-methyl-propanenitrile in 90 mL of ethanol, 10 mL of water, and 5 mL of acetic acid at 80°C. The reaction mixture was stirred vigorously at 80°C for 20 minutes and the mixture was cooled and filtered on celite with ethanol washes and concentrated under reduced pressure. The crude material was diluted with 100 mL of ethyl acetate and washed with 50 mL of an aqueous saturated solution of sodium chloride. The organic layer was dried over sodium sulfate and concentrated under vacuum.

A solution of the residue in 15 mL of tetrahydrofuran was dropwise added to a solution of 4.12 mL of acetic anhydride (4 eq.) in 3.35 mL of formic acid (8 eq.) that had been premixed for 30 minutes at room temperature. The reaction mixture was stirred at room temperature for 1 hour, then quenched with 100 mL of ethyl acetate and 100 mL of a 10% aqueous sodium carbonate under vigorous stirring for 30 min. The organic layer was separated and washed twice with 50 mL of 10% aqueous sodium carbonate then concentrated under vacuum. The residue was taken in 100 mL of toluene and concentrated under vacuum. The residue was triturated in 100 mL of a 70/30 pentane/ether mixture and the solid filtered to afford 2.06 g of N-[1-(1-cyano-1-methylethyl)-3-isopropoxy-pyrazol-4-yl]formamide. MS (method F): m/z 237 [M+1]+; t= 1.37 min.

### Intermediate 32: N-(3-((1-acetyl-2,2-dimethylazetidin-3-yl)oxy)-1-methyl-1H-pyrazol-4-yl)formamide (racemic)

### Step 1: Preparation of tert-butyl 2,2-dimethyl-3-((1-methyl-4-nitro-1H-pyrazol-3-yl)oxy)azetidine-1-carboxylate (racemic)

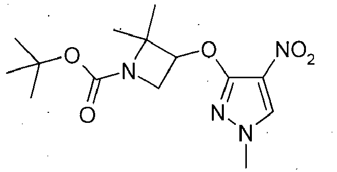

482 mg (1.3 eq.) of racemic tert-butyl 3-hydroxy-2,2-dimethylazetidine-1-carboxylate and 1.5 g (2 eq.) of cesium carbonate were added to a solution of 400 mg (1 eq.) of 1-methyl-3,4-dinitro-1H-pyrazole (commercially available) in 8 mL of acetonitrile. The mixture was heated at 80°C for 5 hours then allowed to cool to room temperature and poured onto ethyl acetate and water. The aqueous layer was separated and extracted twice with ethyl acetate. The combined organic layers were washed with 20 mL of saturated sodium chloride solution, dried over magnesium sulfate, and concentrated under vacuum. The residue was purified on silica gel, eluting with a gradient of 0 to 20% of ethyl acetate in dichloromethane, to afford 460 mg of racemic tert-butyl 2,2-dimethyl-3-((1-methyl-4-nitro-1H-pyrazol-3-yl)oxy)azetidine-1-carboxylate.
MS (method B) m/z 237 [M+1]+; t=1.69 min

### Step 2: Preparation of 3-((2,2-dimethylazetidin-3-yl)oxy)-1-methyl-4-nitro-1H-pyrazole (racemic)

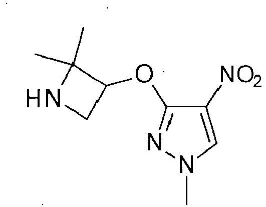

5.5 mL (19 eq.) of trifluoroacetic acid was added to a solution of 1.2g (1 eq.) of racemic tert-butyl 2,2-dimethyl-3-((1-methyl-4-nitro-1H-pyrazol-3-yl)oxy)azetidine-1-carboxylate in 30 mL of dichloromethane. The mixture was stirred for 2 hours at room temperature. The mixture was concentrated under vacuum then dissolved in 10 mL of methanol and treated with 30 mL of 7M methanolic ammonia solution for 1 hour and concentrated under vacuum. The residue was purified on silica gel, eluting with a gradient of 0 to 5% of a 7M methanolic ammonia solution in dichloromethane, to afford 460 mg of racemic 3-((2,2-dimethylazetidin-3-yl)oxy)-1-methyl-4-nitro-1H-pyrazole. MS (method B) m/z 227 [M+1]+; t=0.22-0.27 min.

### Step 3: Preparation of 1-(2,2-dimethyl-3-((1-methyl-4-nitro-1H-pyrazol-3-yl)oxy)azetidin-1-yl)ethan-1-one (racemic)

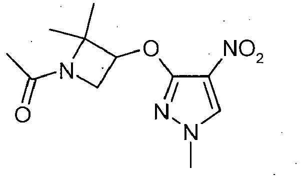

1.14 mL (4 eq.) of triethylamine was added to a solution of 460 mg (1 eq.) of racemic 3-((2,2-dimethylazetidin-3-yl)oxy)-1-methyl-4-nitro-1H-pyrazole in 23 mL of tetrahydrofuran. The mixture was cooled to 0-5°C, and 292 µL (2 eq). of acetyl chloride was added. The mixture was stirred at 0°C for 5 minutes and then at room temperature for 30 minutes. The mixture was poured onto ethyl acetate and water. The aqueous layer was separated and extracted twice with ethyl acetate. The combined organic layers were washed with 20 mL of a saturated sodium chloride solution, dried over magnesium sulfate, and concentrated under vacuum. The residue was purified on silica gel, eluting with a gradient of 0 to 5% of methanol in dichloromethane, to afford 360 mg of racemic 1-(2,2-dimethyl-3-((1-methyl-4-nitro-1H-pyrazol-3-yl)oxy)azetidin-1-yl)ethan-1-one. MS (method B) m/z 269 [M+1]+; t=1.13 min

### Step 4: Preparation of 1-(3-((4-amino-1-methyl-1H-pyrazol-3-yl)oxy)-2,2-dimethylazetidin-1-yl)ethan-1-one (racemic)

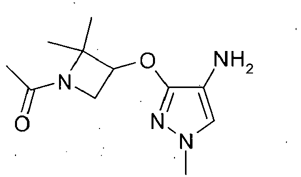

In a microwave vial, a solution of 310 mg (1 eq.) of racemic 1-(2,2-dimethyl-3-((1-methyl-4-nitro-1H-pyrazol-3-yl)oxy)azetidin-1-yl)ethan-1-one in 12 mL of methanol was treated with 515 mg of ammonium formate (7 eq.) and 86 mg of palladium on carbon (10%). The reaction mixture was heated at 70°C for 10 minutes. The mixture was filtered on celite with methanol washes and concentrated under reduced pressure. The residue was taken into dichloromethane, filtered and the filtrate concentrated under reduced pressure to afford 260 mg of racemic 1-(3-((4-amino-1-methyl-1H-pyrazol-3-yl)oxy)-2,2-dimethylazetidin-1-yl)ethan-1-one. MS (method A) m/z 239 [M+1]+; retention time: dead volume

Step 5: Preparation of N-(3-((1-acetyl-2,2-dimethylazetidin-3-yl)oxy)-1-methyl-1H-pyrazol-4-yl)formamide (racemic)

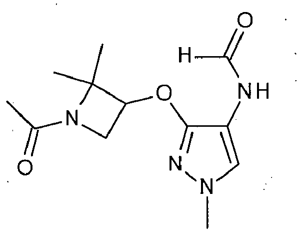

A solution of 264 mg (1 eq.) of racemic 1-(3-((4-amino-1-methyl-1H-pyrazol-3-yl)oxy)-2,2-dimethylazetidin-1-yl)ethan-1-one in 2.6 mL of tetrahydrofuran was dropwise added to a cooled (0°C) solution of 416 µL of acetic anhydride (3 eq.) in 374 µL of formic acid (6 eq.) that had been premixed for 30 minutes at room temperature. The reaction mixture was stirred at 5°C for 30 minutes, then concentrated under vacuum. The residue was taken in toluene and concentrated under vacuum. The residue was purified on silica gel, eluting with a gradient of 0 to 5% of methanol in dichloromethane, to afford 250 mg of racemic N-(3-((1-acetyl-2,2-dimethylazetidin-3-yl)oxy)-1-methyl-1H-pyrazol-4-yl)formamide. MS (method B) m/z 267 [M+1]+; t=0.92 min.

### Intermediate 33: methyl -2-(4-formamido-3-(oxetan-3-yloxy)-1H-pyrazol-1-yl)propanoate (racemic)

### Step 1: Preparation of methyl -2-(4-nitro-3-(oxetan-3-yloxy)-1H-pyrazol-1-yl)propanoate (racemic)

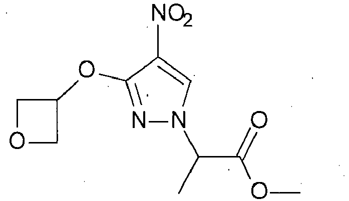

923 µL (1 eq.) of racemic methyl-2-bromopropionate was added to a solution of 1500 mg ( 1eq.) of 4-nitro-3-(oxetan-3-yloxy)-1H-pyrazole (Intermediate 13, step 3) and 5280 mg (2eq.) of cesium carbonate in 45 mL of acetonitrile. The mixture was heated at 80°C for 2.5 hours then allowed to cool to room temperature. The mixture was poured onto ethyl acetate and water. The aqueous layer was separated and extracted twice with ethyl acetate. The combined organic layers were washed with 20 mL of saturated sodium chloride solution, dried over magnesium sulfate, and concentrated under vacuum. The residue was purified on silica gel, eluting with a gradient of 10 to 50% of ethyl acetate in heptane, to afford 594 mg of racemic methyl -2-(4-nitro-3-(oxetan-3-yloxy)-1H-pyrazol-1-yl)propanoate. MS method B: m/z 272[M+ 1]+; t=1.19 min.

### Step 2: Preparation of methyl -2-(4-amino-3-(oxetan-3-yloxy)-1H-pyrazol-1-yl)propanoate (racemic)

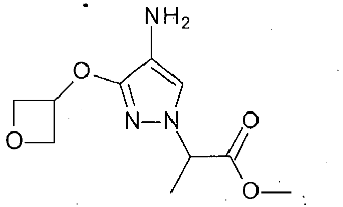

335 mg of iron (3 eq.) and 321 mg of ammonium chloride (3 eq.) were added to a solution of 542 mg (1 eq.) of racemic methyl 2-(4-nitro-3-(oxetan-3-yloxy)-1H-pyrazol-1-yl)propanoate in 20 mL of ethanol, 5 mL of water. The reaction mixture was stirred vigorously at 90°C for 2.5 hours and the mixture was cooled to room temperature, filtered on celite with ethanol washes and concentrated under reduced pressure. The crude material was diluted with 100 mL of ethyl acetate and washed with 50 mL of an aqueous saturated solution of sodium chloride. The organic layer was dried over magnesium sulfate and concentrated under vacuum to afford 454 mg of racemic methyl -2-(4-amino-3-(oxetan-3-yloxy)-1H-pyrazol-1-yl)propanoate which will be used in the next step without further purification.

### Step 3: Preparation of methyl -2-(4-formamido-3-(oxetan-3-yloxy)-1H-pyrazol-1-yl)propanoate (racemic)

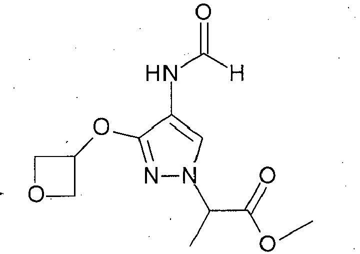

A solution of 454 mg (1 eq.) of racemic methyl 2-(4-amino-3-(oxetan-3-yloxy)-1H-pyrazol-1-yl)propanoate in 14mL of tetrahydrofuran was dropwise added at 5°C to a solution of 0.71 mL of acetic anhydride (4 eq.) in 0.57 mL of formic acid (8 eq.) that had been premixed for 55 minutes at room temperature. The reaction mixture was stirred at 5°C for 5 minutes, at room temperature for 1.5 hours, and then concentrated under vacuum. The residue was purified on silica gel, eluting with a gradient of 10 to 30% of acetone in dichloromethane, to afford 366 mg of racemic methyl-2-(4-formamido-3-(oxetan-3-yloxy)-1H-pyrazol-1-yl)propanoate. MS method A: m/z 270[M+ 1]+; t= 1.26 min.

### Intermediate 34: Methyl 3-(cyclopropoxy)-4-formamido-benzoate

### Step 1: Preparation of methyl 3-(cyclopropoxy)-4-nitro-benzoate

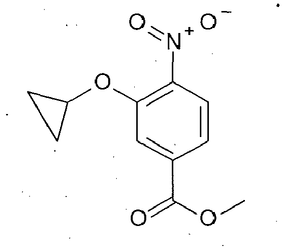

A solution of 0.843 mL (2 eq) of cyclopropanol in 35 mL of anhydrous tetrahydrofuran under argon was stirred to 0°C. A solution of 7.83 mL (1.2 eq.) of [bis(trimethylsilyl)amino]lithium (1M in THF) was added dropwise. The resulting mixture was stirred at 0°C for 30 minutes, and then 1.3 g (1 eq.) of methyl 3-fluoro-4-nitrobenzoate in 40 mL of anhydrous tetrahydrofuran was added dropwise. The mixture was stirred at room temperature for 4 hours, cooled to 0°C, and quenched with 50 mL of a saturated solution of ammonium chloride and extracted with ethyl acetate (3 times with 50 mL). The combined organic layers were dried over magnesium sulfate and filtered. The filtrate was concentrated under vacuum and purified on silica gel eluting with 20% of ethyl acetate in heptane to afford 380 mg of methyl 3-(cyclopropoxy)-4-nitro-benzoate. MS (method H): m/z 238 [M+1]+; t=1.53 min.

### Step 2: Preparation of methyl 4-amino-3-(cyclopropoxy)benzoate

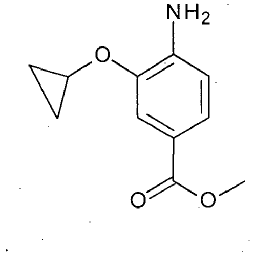

186 mg (3 eq.) of ammonium chloride and 194 mg (3 eq.) of iron were added to a stirring solution of 275 mg (1 eq.) of methyl 3-(cyclopropoxy)-4-nitro-benzoate in 7 mL of ethanol and 2 mL of water. The reaction mixture was heated at 90°C for 1 hour. The mixture was filtered on celite with ethanol washes and concentrated under reduced pressure. The residue was taken into ethyl acetate and washed with water. The organic layer was dried over magnesium sulfate and filtered. The filtrate was concentrated under vacuum, and purified on silica gel eluting with a gradient of 2 to 5 % of methanol in dichloromethane to afford 280 mg of 4-amino-3-(cyclopropoxy)benzoate. MS (method H) m/z 208 [M+1]+; t=1.69 min.

### Step 3: Preparation of methyl 3-(cyclopropoxy)-4-formamido-benzoate

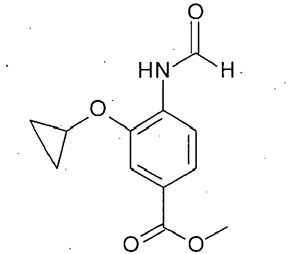

A solution of 280 mg (1 eq.) of methyl 4-amino-3-(cyclopropoxy)benzoate in 4 mL of tetrahydrofuran was dropwise added to a solution of 0.510 mL of acetic anhydride (4 eq.) in 0.414 mL of formic acid (8 eq.) that had been premixed for 30 minutes at room temperature. The reaction mixture was stirred at room temperature for 2 hours 30 minutes, and then concentrated under vacuum. The residue was taken into ethyl acetate and washed with a saturated solution of sodium bicarbonate and then water. The organic layer was dried over magnesium sulfate and filtered. The filtrate was concentrated under vacuum to afford 250 mg of methyl 3-(cyclopropoxy)-4-formamido-benzoate. MS (method A) m/z 236 [M+1]+; t=1.99 min.

### Intermediate 35: Methyl 4-formamido-3-(oxetan-3-yloxy)benzoate

### Step 1: Preparation of methyl 4-nitro-3-(oxetan-3-yloxy)benzoate and 4-nitro-3-(oxetan-3-yloxy)benzoic acid

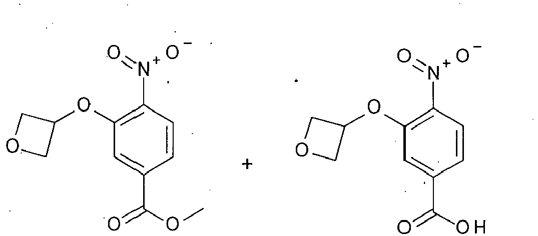

1.4 g (1.9 eq. 60% on oil dispersion) of sodium was added portionwise to a solution of 2.23 g (2 eq.) of oxetane-3-ol in 50 mL of anhydrous tetrahydrofuran, cooled to 0°C. The reaction mixture was stirred at 0°C for 1 hour, and then a solution of 3 g (1 eq.) of methyl 3-fluoro-4-nitro-benzoate in 15 mL of anhydrous tetrahydrofuran was added. The mixture was warmed to room temperature and stirred at room temperature for 1 hour. The reaction mixture was poured onto water and extracted three times with 50mL of ethyl acetate. The organic layer was dried over magnesium sulfate and filtered. The filtrate was concentrated in vacuum. The residue was purified on silica gel eluting with a gradient of 20 to 50 % of ethyl acetate in heptane to afford 900 mg of methyl 4-nitro-3-(oxetan-3-yloxy)benzoate. MS (method A) m/z 254 [M+1]+; t=1.97 min. The aqueous layer was acidified with a 5N solution of hydrochloric acid to pH=5 then extracted three times with 50 mL of ethyl acetate. The combined organic layers were dried over magnesium sulfate, filtered, and concentrated under vacuum. The residue was purified on silica gel, eluting with a gradient of 5 to 10% of methanol in dichloromethane to afford 1.5 g of 4-nitro-3-(oxetan-3-yloxy)benzoic acid. MS (method A) m/z 240 [M+1]+; t=1.69 min.

### Step 2: Preparation of methyl 4-amino-3-(oxetan-3-yloxy)benzoate

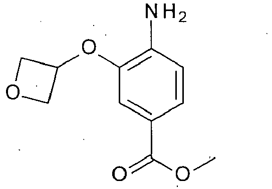

In a microwave vial, a solution of 500 mg (1 eq.) of methyl 4-nitro-3-(oxetan-3-yloxy)benzoate in 10 mL of methanol was treated with 355 mg of ammonium formate (2.8 eq.) and 210 mg (0.1 eq.) of palladium on carbon (10%). The reaction mixture was heated at 85°C for 15 minutes. The mixture was filtered on celite with methanol washes and concentrated under reduced pressure to afford 440 mg of methyl 4-amino-3-(oxetan-3-yloxy)benzoate. MS (method A) m/z 254 [M+1]+; t=1.55 min.

### Step 3: Preparation of methyl 4-formamido-3-(oxetan-3-yloxy)benzoate

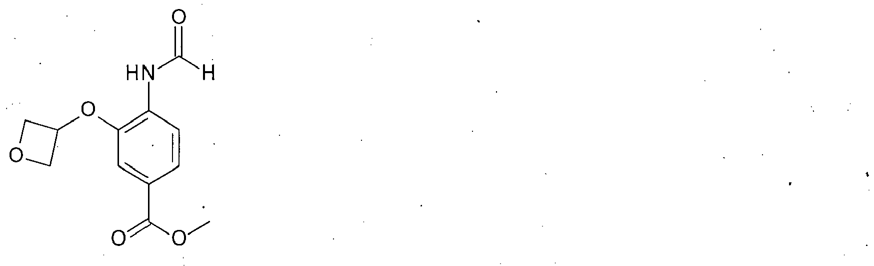

A solution of 440 mg (1 eq.) of methyl 4-amino-3-(oxetan-3-yloxy)benzoate in 4 mL of tetrahydrofuran was dropwise added to a solution of 0.75 mL of acetic anhydride (4 eq.) in 0.68 mL of formic acid (8 eq.) that had been premixed for 30 minutes at room temperature. The reaction mixture was stirred at room temperature for 30 min, and then concentrated under vacuum to afford 435 mg of methyl 4-formamido-3-(oxetan-3-yloxy)benzoate. MS (method A) m/z 252 [M+1]+; t=1.59 min.

### Intermediate 36: (N-(3-((2-oxaspiro[3.3]heptan-5-yl)oxy)-1-methyl-1H-pyrazol-4-yl)formamide

### Step 1: Preparation of 3-((2-oxaspiro[3.3]heptan-5-yl)oxy)-1-methyl-4-nitro-1H-pyrazole

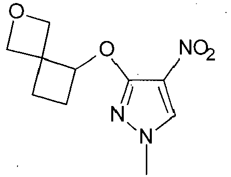

430 mg (1.3 eq.) of 2-oxaspiro[3.3]heptan-5-ol (commercially available) and 1.9 g (2 eq.) of cesium carbonate were added To a solution of 500 mg (1 eq.) of 1-methyl-3,4-dinitro-1H-pyrazole (commercially available) in 25 mL of acetonitrile. The mixture was heated at 70°C overnight, and then allowed to cool to room temperature and poured onto ethyl acetate and water. The aqueous layer was separated and extracted three times with ethyl acetate. The combined organic layers were dried over magnesium sulfate and concentrated under vacuum. The residue was purified on silica gel, eluting with 50% of ethyl acetate in heptane, to afford 595 mg of 3-((2-oxaspiro[3.3]heptan-5-yl)oxy)-1-methyl-4-nitro-1H-pyrazole. MS (method A) m/z 240 [M+1]+; t=1.67 min.

### Step' 2: Preparation of N-(3-((2-oxaspiro[3.3]heptan-5-yl)oxy)-1-methyl-1H-pyrazol-4-yl)formamide

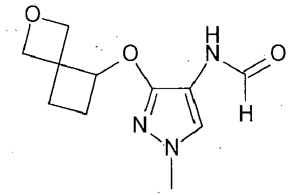

In a microwave vial, a solution of 600 mg (1 eq.) of 3-((2-oxaspiro[3.3]heptan-5-yl)oxy)-1-methyl-4-nitro-1H-pyrazole in 16 mL of methanol was treated with 448 mg of ammonium formate (2.8 eq.) and 267 mg (0.1 eq.) of palladium on carbon (10%). The reaction mixture was heated at 70°C for 10 minutes. The mixture was filtered on celite with methanol washes and concentrated under reduced pressure. The residue was dissolved in 2 mL of tetrahydrofuran and the solution was dropwise added to a cooled (0°C) solution of 946 µL of acetic anhydride (4 eq.) in 1.73 mL of formic acid (18 eq.) that had been premixed for 30 minutes at room temperature. The reaction mixture was stirred at 5°C for 5 minutes, at room temperature for 1 hour, and then concentrated under vacuum. The residue was taken up with 50 mL of toluene and concentrated under vacuum to afford 395 mg of N-(3-((2-oxaspiro[3.3]heptan-5-yl)oxy)-1-methyl-1H-pyrazol-4-yl)formamide. MS (method A) m/z 238 [M+1]+; t=1.39 min.

### Intermediate 37: N-(1-(methoxymethyl)-3-(((2R,3S)-2-methyloxetan-3-yl)oxy)-1H-pyrazol-4-yl)formamide and N-(1-(methoxymethyl)-3-(((2S,3R)-2-methyloxetan-3-yl)oxy)-1H-pyrazol-4-yl)formamide (racemic trans)

### Step 1: Preparation of 1-(methoxymethyl)-3,4-dinitro-1H-pyrazole

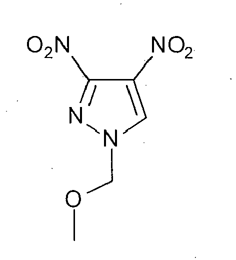

357 mg (1.4 eq.) of chloro(methoxy)methane and 883 mg (2 eq.) of potassium carbonate were added to a solution of 0.5 g (1 eq.) of 3,4-dinitro-1H-pyrazole (commercially available) in 15 mL of acetonitrile. The mixture was stirred at room temperature for 2 hours then poured onto ethyl acetate and water. The aqueous layer was separated and extracted three times with ethyl acetate. The combined organic layers were dried over magnesium sulfate and concentrated under vacuum. The residue was purified on silica gel, eluting with 20 to 50% of ethyl acetate in heptane, to afford 485 mg of 1-(methoxymethyl)-3,4-dinitro-1H-pyrazole. ¹H NMR (400 MHz, DMSO-d6) δ in ppm: 3.35 (s, 3 H); 5.5 (s, 2H); 9.32 (s, 1H).

### Step 2: Preparation of 1-(methoxymethyl)-3-(((2R,3S)-2-methyloxetan-3-yl)oxy)-4-nitro-1H-pyrazole and 1-(methoxymethyl)-3-(((2S,3R)-2-methyloxetan-3-yl)oxy)-4-nitro-1H-pyrazole (racemic trans)

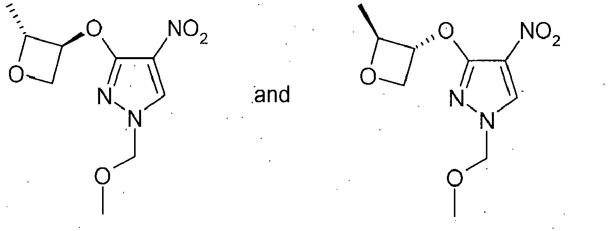

714 mg (1.25 eq.) of 2-methyloxetan-3-ol and 4.23 g (2 eq.) of cesium carbonate were added to a solution of 1310 mg (1 eq.) of 1-(methoxymethyl)-3,4-dinitro-1H-pyrazole in 17 mL of acetonitrile. The mixture was heated at 80°C for 2 hours then allowed to cool to room temperature and poured onto ethyl acetate and water. The aqueous layer was separated and extracted three times with ethyl acetate. The combined organic layers were dried over magnesium sulfate and concentrated under vacuum. The residue was purified on silica gel, eluting with 10% to 50% of ethyl acetate in heptane, to afford 501 mg of a mixture of 1-(methoxymethyl)-3-(((2R,3S)-2-methyloxetan-3-yl)oxy)-4-nitro-1H-pyrazole and 1-(methoxymethyl)-3-(((2S,3R)-2-methyloxetan-3-yl)oxy)-4-nitro-1H-pyrazole characterized by NMR as racemic *trans* (MS (method A) m/z 244 [M+1]+; t=1.13 min) and 940 mg of a mixture of 1-(methoxymethyl)-3-(((2S,3S)-2-methyloxetan-3-yl)oxy)-4-nitro-1H-pyrazole and 1-(methoxymethyl)-3-(((2R,3R)-2-methyloxetan-3-yl)oxy)-4-nitro-1H-pyrazole characterized by NMR as racemic *cis* (MS (method A) m/z 244 [M+1]+; t=1.17 min).

### Step 3: Preparation of 1-(methoxymethyl)-3-(((2R,3S)-2-methyloxetan-3-yl)oxy)-1H-pyrazol-4-amine and 1-(methoxymethyl)-3-(((2S,3R)-2-methyloxetan-3-yl)oxy)-1H-pyrazol-4-amine (racemic trans)

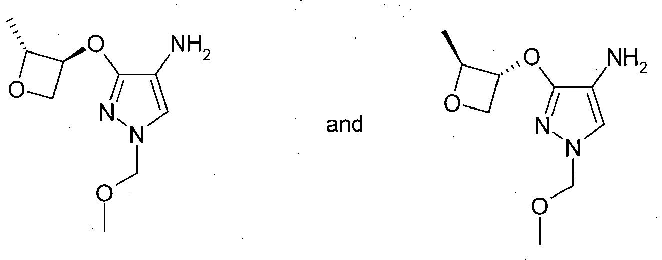

In a microwave vial, a solution of 501 mg (1 eq.) of a racemic mixture of 1-(methoxymethyl)-3-(((2R,3S)-2-methyloxetan-3-yl)oxy)-4-nitro-1H-pyrazole and 1-(methoxymethyl)-3-(((2S,3R)-2-methyloxetan-3-yl)oxy)-4-nitro-1H-pyrazole in 15 mL of methanol was treated with 371 mg of ammonium formate (2.8 eq.) and 220 mg (0.05 eq.) of palladium on carbon (10%). The reaction mixture was heated at 70°C for 15 minutes. The mixture was filtered on celite with methanol washes and concentrated under reduced pressure. The crude material was taken into the next step without further purification.

Step 4: Preparation of N-(1-(methoxymethyl)-3-(((2R,3S)-2-methyloxetan-3-yl)oxy)-1H-pyrazol-4-yl)formamide and N-(1 -(methoxymethyl)-3-(((2S,3R)-2-methyloxetan-3-yl)oxy)-1H-pyrazol-4-yl)formamide (racemic *trans*)

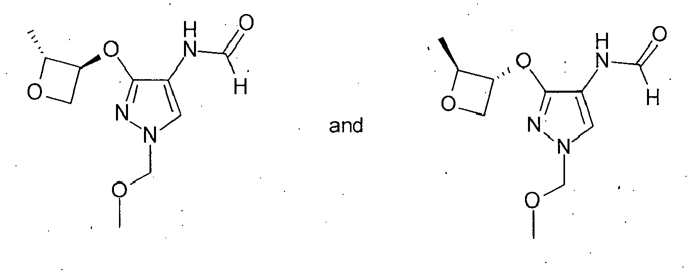

A solution of 439 mg (1 eq.) of a racemic mixture of 1-(methoxymethyl)-3-(((2R,3S)-2-methyloxetan-3-yl)oxy)-1H-pyrazol-4-amine and 1-(methoxymethyl)-3-(((2S,3R)-2-methyloxetan-3-yl)oxy)-1H-pyrazol-4-amine in 2 mL of tetrahydrofuran was dropwise added to a solution of 779 µL of acetic anhydride (4 eq.) in 1.76 mL of formic acid (18 eq.) that had been premixed for 30 minutes at room temperature. The reaction mixture was stirred for 1 hour at 0°C. The residue was concentrated under vacuum and purified on silica gel eluting with heptane/AcOEt/ 7NNH₃ in MeOH (50/45/5) to afford 387 mg of a mixture of N-(1-(methoxymethyl)-3-(((2R,3S)-2-methyloxetan-3-yl)oxy)-1H-pyrazol-4-yl)formamide and N-(1-(methoxymethyl)-3-(((2S,3R)-2-methyloxetan-3-yl)oxy)-1H-pyrazol-4-yl)formamide (racemic *trans).* MS (method B) m/z 242 [M+ 1]+; t=0.85 min (racemic *trans).*

### Intermediate 38: N-(3-isopropoxy-1-methyl-1H-pyrazol-4-yl)formamide

### Step 1: Preparation of 3-isopropoxy-1-methyl-4-nitro-1H-pyrazole

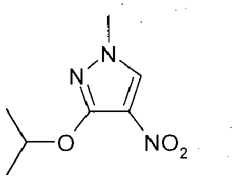

438 µL (1.2 eq.) of iodomethane and 1.61 g (2 eq.) of potassium carbonate were added to a solution of 1 g (1 eq.) of 3-isopropoxy-4-nitro-1H-pyrazole (commercially available) in 30 mL of N-methylpyrrolidinone. The mixture was heated at 70°C for 1 hour and poured onto a mixture of 100 mL of ethyl acetate and 100 mL of diethyl ether. The organic layer was washed 3 times with 100 mL of water, and then dried over sodium sulfate, and concentrated under vacuum. The residue was purified on silica gel, eluting with a 85/15 cyclohexane/ethyl acetate mixture, to afford 1g of 3-isopropoxy-1-methyl-4-nitro-1H-pyrazole as a white solid. MS (method E) m/z 186 [M+1]+; t= 1.08 min.

### Step 2: Preparation of 3-isopropoxy-1-methyl-1H-pyrazol-4-amine

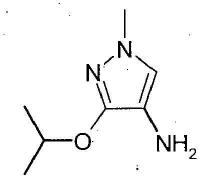

1.51 g of iron (5 eq.) was added to a solution of 1g of 3-isopropoxy-1-methyl-4-nitro-1H-pyrazole in 50 mL of ethanol, 1 mL of water and 10 mL of acetic acid at 80°C. The reaction mixture was stirred vigorously at 80°C for 20 minutes and the mixture was cooled to room temperature, filtered on celite with ethanol washes, and concentrated under reduced pressure. The crude material was diluted with 100 mL of ethyl acetate and washed with 50mL of an aqueous saturated solution of sodium carbonate. The organic layer was dried over sodium sulfate and concentrated under vacuum. The residue was purified on silica gel, eluting with 5% of methanol in dichloromethane to afford 650 mg of 3-isopropoxy-1-methyl-1H-pyrazol-4-amine. MS (method E); m/z 156 [M+1]+; t= 0.78 min.

### Step 3: Preparation of N-(3-isopropoxy-1-methyl-1H-pyrazol-4-yl)formamide

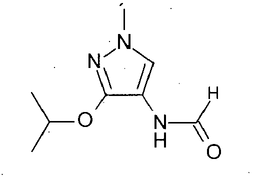

A solution of 3g of 3-isopropoxy-1-methyl-1H-pyrazol-4-amine in 10 mL of tetrahydrofuran was dropwise added to a solution of 5.91 mL of acetic anhydride (4 eq.) in 4.8 mL of formic acid (8 eq.) that had been premixed for 30 minutes at room temperature. The reaction mixture was stirred, at room temperature for 1 hour then quenched with 100 mL of ethyl acetate and 100 mL of an 10% aqueous sodium carbonate under vigorous stirring for 30 min. The organic layer was separated and washed twice with 50 mL of 10% aqueous sodium carbonate then concentrated under vacuum. The residue was purified on silica gel, eluting with a 70/30 cyclohexane/ethyl acetate mixture, to afford 2.18 g of N-(3-isopropoxy-1-methyl-1H-pyrazol-4-yl)formamide. MS (method E); m/z 184 [M+1]+; t=0.80 min.

### Intermediate 39: (R)-N-(1-(1-cyanoethyl)-3-isopropoxy-1H-pyrazol-4-yl)formamide and (S)-N-(1-(1-cyanoethyl)-3-isopropoxy-1H-pyrazol-4-yl)formamide

### Step 1: Preparation of (R)-2-(3-isopropoxy-4-nitro-1H-pyrazol-1-yl)propanenitrile and (S)-2-(3-isopropoxy-4-nitro-1H-pyrazol-1-yl)propanenitrile

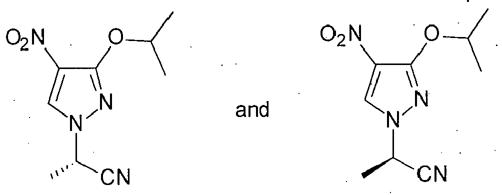

1.21 mL (1.2 eq.) of 2-bromopropanenitrile and 3.23 g (2 eq.) of potassium carbonate were added to a solution of 2 g (1 eq.) of 3-isopropoxy-4-nitro-1H-pyrazole (commercially available) in 20 mL of 1-methyl-2-pyrrolidinone (NMP). The mixture was stirred at room temperature for 1 hour, then poured onto ethyl acetate and water. The aqueous layer was separated and extracted three times with ethyl acetate. The combined organic layers were dried over magnesium sulfate and concentrated under reduced pressure. The residue was purified on silica gel, eluting with 50% of ethyl acetate in heptane, to afford 2.2 g of a racemic mixture of (R)-2-(3-isopropoxy-4-nitro-1H-pyrazol-1-yl)propanenitrile and (S)-2-(3-isopropoxy-4-nitro-1H-pyrazol-1-yl)propanenitrile. MS (method B) m/z 225 [M+1]+; t=1.34 min.

### Step 2: Preparation of (R)-2-(4-amino-3-isopropoxy-1H-pyrazol-1-yl)propanenitrile and (S)-2-(4-amino-3-isopropoxy-1H-pyrazol-1-yl)propanenitrile

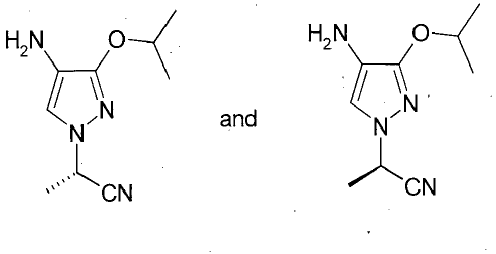

680 mg of ammonium chloride (3 eq.) and 710 mg of iron (3 eq.) were added to a solution of 950 mg (1 eq.) of a racemic mixture of (R)-2-(3-isopropoxy-4-nitro-1H-pyrazol-1-yl)propanenitrile and (S)-2-(3-isopropoxy-4-nitro-1H-pyrazol-1-yl)propanenitrile in 25 mL of ethanol and 7 mL of water. The reaction mixture was heated at 90°C for 7 hours then allowed to cool to room temperature. The mixture was filtered on celite with ethanol washes and concentrated under reduced pressure. The residue was taken into ethyl acetate, washed with water, dried over magnesium sulfate, and concentrated under reduced pressure. The crude material was purified on silica gel, eluting with 5% of methanol in dichloromethane, to give 850 mg of a racemic mixture of (R)-2-(4-amino-3-isopropoxy-1H-pyrazol-1-yl)propanenitrile and (S)-2-(4-amino-3-isopropoxy-1H-pyrazol-1-yl)propanenitrile. MS (method B) m/z 195 [M+1]+; t=0.59 min.

### Step 3: Preparation of (R)-N-(1-(1-cyanoethyl)-3-isopropoxy-1H-pyrazol-4-yl)formamide and (S)-N-(1-(1-cyanoethyl)-3-isopropoxy-1H-pyrazol-4-yl)formamide

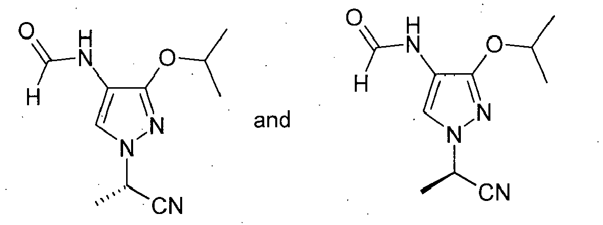

A solution of 1.4 g (1 eq.) of a racemic mixture of (R)-2-(4-amino-3-isopropoxy-1H-pyrazol-1-yl)propanenitrile and (S)-2-(4-amino-3-isopropoxy-1H-pyrazol-1-yl)propanenitrile in 15 mL of tetrahydrofuran was added dropwise to a cooled (0°C) solution of 2.72 mL of acetic anhydride (4 eq.) in 2.21 mL of formic acid (8 eq.) that had been premixed for 30 minutes at room temperature. The reaction mixture was stirred at room temperature for 30 minutes then concentrated under reduced pressure. The residue was taken into ethyl acetate and washed with a saturated aqueous sodium hydrogenocarbonate solution, dried over magnesium sulfate and concentrated under reduced pressure. The crude material was purified on silica gel, eluting by 2% of methanol in dichloromethane. The resulting oil was triturated in diisopropyl ether and the precipitate filtered to afford 1.1 g of a racemic mixture of (R)-N-(1-(1-cyanoethyl)-3-isopropoxy-1H-pyrazol-4-yl)formamide and (S)-N-(1-(1-cyanoethyl)-3-isopropoxy-1H-pyrazol-4-yl)formamide. MS (method B) m/z 223 [M+ 1]+; t=2.27 min.

' Chiral separation performed on 1.1 g of the racemic mixture gave 488 mg of the first eluting isomer and 503 mg of the second eluting isomer (conditions: column Chiralpak AY, 20 µm, 230×100 mm; liquid phase: heptane 90/ ethyl alcohol 10; flow rate 400 mL/min).
Peak 1 (1^{st} isomer): MS (method B) m/z 223 [M+ 1]+; t=2.27 min.
Peak 2 (2^{nd} isomer): MS (method B) m/z 223 [M+ 1]+; t=2.27 min.

### Intermediate 40: N-(1-(1-cyanoethyl)-3-cyclopropoxy-1H-pyrazol-4-yl)formamide

### Step 1: Preparation of (R)-2-(3-cycloproppxy-4-nitro-1H-pyrazol-1-yl)propanenitrile and (S)-2-(3-cyclopropoxy-4-nitro-1H-pyrazol-1-yl)propanenitrile

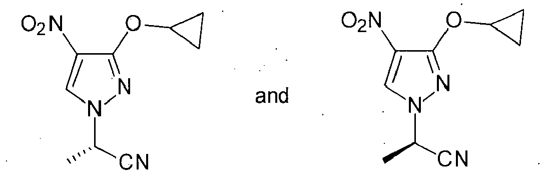

491 µL (1.2 eq.) of 2-bromopropanenitrile and 1.31 g (2 eq.) of potassium carbonate were added to a solution of 0.8 g (1 eq.) of 3-cyclopropoxy-4-nitro-1H-pyrazole (intermediate 29, step 5) in 8 mL of N,N-dimethylformamide (DMF). The mixture was stirred at room temperature for 3.5 hours then poured onto ethyl acetate and water. The aqueous layer was separated and extracted three times with ethyl acetate. The combined organic layers were dried over magnesium sulfate and concentrated under reduced pressure. The residue was purified on silica gel, eluting with 50% of ethyl acetate in heptane, to afford 755 mg of a racemic mixture of (R)-2-(3-cyclopropoxy-4-nitro-1H-pyrazol-1-yl)propanenitrile and (S)-2-(3-cyclopropoxy-4-nitro-1H-pyrazol-1-yl)propanenitrile._MS (method A) m/z 223 [M+1]+; t=1.96 min.

### Step 2: Preparation of (R)-2-(4-amino-3-cyclopropoxy-1H-pyrazol-1-yl)propanenitrile and (S)-2-(4-amino-3-cyclopropoxy-1H-pyrazol-1-yl)propanenitrile

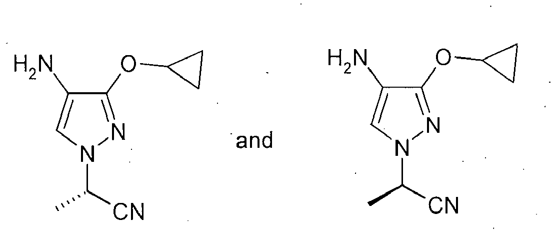

545 mg of ammonium chloride (3 eq.) and 570 mg of iron (3 eq.) were added to a solution of 755 mg (1 eq.) of a racemic mixture of (R)-2-(3-cyclopropoxy-4-nitro-1H-pyrazol-1-yl)propanenitrile and (S)-2-(3-cyclopropoxy-4-nitro-1H-pyrazol-1-yl)propanenitrile in 20 mL of ethanol and 5 mL of water. The reaction mixture was heated at 90°C for 1.5 hours, then allowed to cool to room temperature. The mixture was filtered on celite with ethanol washes and concentrated under reduced pressure. The residue was taken into ethyl acetate and washed with water, dried over magnesium sulfate and concentrated under reduced pressure. The crude material was taken into the next step without further purification.

### Step 3: Preparation of (R)-N-(1-(1-cyanoethyl)-3-cyclopropoxy-1H-pyrazol-4-yl)formamide and (S)-N-(1-(1-cyanoethyl)-3-cyclopropoxy-1H-pyrazol-4-yl)formamide

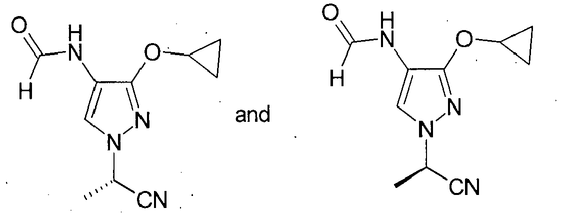

A solution of 0.65 g (1 eq.) of (R)-2-(4-amino-3-cyclopropoxy-1H-pyrazol-1-yl)propanenitrile and (S)-2-(4-amino-3-cyclopropoxy-1H-pyrazol-1-yl)propanenitrile in 7 mL of tetrahydrofuran was added dropwise to a cooled (0°C) solution of 1.28 mL of acetic anhydride (4 eq.) in 2.33 mL of formic acid (18 eq.) that had been premixed for 30 minutes at room temperature. The reaction mixture was stirred at room temperature for 30 minutes then concentrated under reduced pressure to afford 620 mg of a racemic mixture of (R)-N-(1-(1-cyanoethyl)-3-cyclopropoxy-1H-pyrazol-4-yl)formamide and (S)-N-(1-(1-cyanoethyl)-3-cyclopropoxy-1H-pyrazol-4-yl)formamide. MS (method A) m/z 221 [M+1]+; t=1.42 min.

### Intermediate 41: N-(3-cyclopropoxy-1-isopropyl-1H-pyrazol-4-yl)formamide

### Step 1: Preparation of 3-cyclopropoxy-1-isopropyl-4-nitro-1H-pyrazole

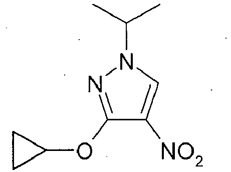

603 mg (1.2 eq.) of 2-iodopropane and 817 mg (2 eq.) of potassium carbonate were added to a solution of 0.5 g (1 eq.) of 3-cyclopropoxy-4-nitro-1H-pyrazole (intermediate 29, step 5) in 15 mL of N,N-dimethylformamide (DMF). The mixture was heated at 80°C for 1 hour and poured onto a mixture of 100 mL of ethyl acetate and 100 mL of diethyl ether. The organic layer was washed 3 times with 100 mL of water, dried over sodium sulfate, and concentrated under vacuum. The residue was purified on silica gel, eluting with a 80/20 heptane/ethyl acetate mixture, to afford 273 mg of 3-cyclopropoxy-1-isopropyl-4-nitro-1H-pyrazole. MS (method A) m/z 212 [M+1]+; t= 2.17 min.

### Step 2: Preparation of 3-cyclopropoxy-1-isopropyl-1H-pyrazol-4-amine

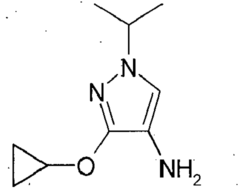

In a microwave vial, a solution of 273 mg (1 eq.) of 3-cyclopropoxy-1-isopropyl-4-nitro-1H-pyrazole in 8 mL of methanol was treated with 236 mg of ammonium formate (2.8 eq.) and 138 mg (0.1 eq.) of palladium on carbon (10%). The reaction mixture was heated at 70°C for 10 minutes. The mixture was filtered on celite with methanol washes and concentrated under reduced pressure. The crude material was taken into the next step without further purification.

### Step 3: Preparation of N-(3-cyclopropoxy-1-isopropyl-1H-pyrazol-4-yl)formamide

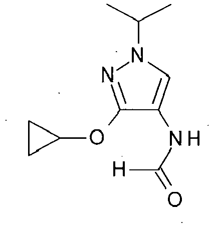

A solution of 234 mg of 3-cyclopropoxy-1-isopropyl-1H-pyrazol-4-amine in 2 mL of tetrahydrofuran was dropwise added to a solution of 488 µL of acetic anhydride (4 eq.) in 892 µL of formic acid (18 eq.) that had been premixed for 30 minutes and 0°C. The reaction mixture was stirred at 0°C for 1 hour then concentrated under vacuum. The residue was purified on silica gel, eluting with a 50/50 heptane/ethyl acetate mixture, to afford 155 mg of N-(3-cyclopropoxy-1-isopropyl-1H-pyrazol-4-yl)formamide. MS (method A); m/z 210 [M+1]+; t=1.55 min.

### Intermediate 42: N-(3-(3,3-difluorocyclobutoxy)-1-methyl-1H-pyrazol-4-yl)formamide

### Step 1: Preparation of 5-(3,3-difluorocyclobutoxy)-4-nitro-1-((2-(trimethylsilyl)ethoxy)methyl)-1H-pyrazole

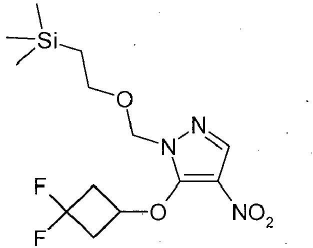

A suspension of 288 mg (2 eq.) of sodium hydride (60% in mineral oil) in 10 mL of dry tetrahydrofuran was cooled to -10°C, and 779 mg (2 eq.) of 3,3-difluorocyclobutan-1-ol was added. After stirring for 30 min, 1 g (1 eq.) of 2-[(3-chloro-4-nitro-pyrazol-1-yl)methoxy]ethyl-trimethyl-silane (intermediate 29, step 2) in 5 mL of dry tetrahydrofuran was added. The mixture was allowed to stir at 5°C for 30 minutes. and was quenched with 10% aqueous citric acid solution and diethyl ether. The organic layer was separated and washed twice with water, dried over magnesium. sulfate and concentrated under vacuum, to afford 1.5 g of 5-(3,3-difluorocyclobutoxy)-4-nitro-1-((2-(trimethylsilyl)ethoxy)methyl)-1H-pyrazole. The crude material was used at the next step without further purification. MS (method A):no ionization; t = 3.13 min.

### Step 2: Preparation of 3-(3,3-difluorocyclobutoxy)-1-methyl-4-nitro-1H-pyrazole

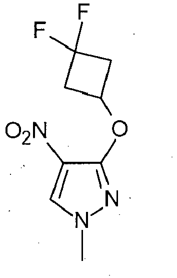

32 mL (7.5 eq.) of 1N hydrochloric solution was added to a solution of 1.5 g (1 eq.) of 5-(3,3-difluorocyclobutoxy)-4-nitro-1-((2-(trimethylsilyl)ethoxy)methyl)-1H-pyrazole in 32 mL of dry acetonitrile. After heating to 40°C for 4 hours, the reaction was cooled to room temperature and neutralized to pH 7 by addition of 32 mL of a 1N solution of sodium hydroxide in water. The reaction mixture was extracted twice with 50 mL of ethyl acetate. The combined organic layers were dried over magnesium sulfate and concentrated under vacuum to afford 711 mg of a yellow solid which was dissolved in 25 mL of N-methylpyrrolidinone. 244 µL (1.2 eq.) of iodomethane and 897 mg (2 eq.) of potassium carbonate were added to this solution. The mixture was heated at 80°C for 2 hours and poured onto a mixture of 200 mL of ethyl acetate and 200 mL of diethyl ether. The organic layer was washed 3 times with 100 mL of water then dried over magnesium sulfate and concentrated under vacuum. The residue was purified on silica gel, eluting with an 80/20 to 50/50 heptane/ethyl acetate mixture, to afford 725 mg of 3-(3,3-difluorocyclobutoxy)-1-methyl-4-nitro-1H-pyrazole. MS (method B)m/z 234 [M+1]+; t= 1.25 min.

### Step 3: Preparation of 3-(3,3-difluorocyclobutoxy)-1-methyl-1H-pyrazol-4-amine

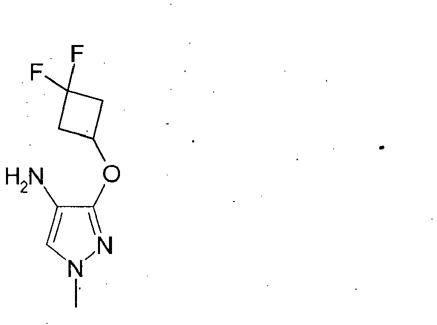

946 mg of ammonium chloride (5 eq.) and 988 mg of iron (5 eq.) were added to a solution of 825 mg (1 eq.) of 3-(3,3-difluorocyclobutoxy)-1-methyl-4-nitro-1H-pyrazole in 25 mL of ethanol and 3.8 mL of water. The reaction mixture was heated at 80°C for 1 hour then allowed to cool to room temperature. The mixture was filtered on celite with ethanol washes and concentrated under reduced pressure. The residue was taken into ethyl acetate and washed with water, dried over magnesium sulfate and concentrated under reduced pressure to afford 579 mg of 3-(3,3-difluorocyclobutoxy)-1-methyl-1H-pyrazol-4-amine as an orange gum. The crude material was taken into the next step without further purification.

### Step 4: Preparation of N-(3-(3,3-difluorocyclobutoxy)-1-methyl-1H-pyrazol-4-yl)formamide

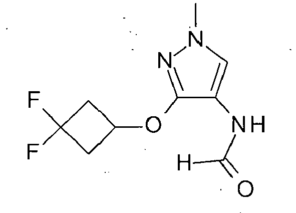

A solution of 579 mg of 3-(3,3-difluorocyclobutoxy)-1-methyl-1H-pyrazol-4-amine in 7 mL of tetrahydrofuran was dropwise added to a cooled to 0°C solution of 1.08 mL of acetic anhydride (4 eq.) in 875 µL of formic acid (8 eq.) that had been premixed for 30 minutes. The reaction mixture was stirred at 0°C for 10 minutes, and then for 30 minutes at room temperature and concentrated under vacuum. The residue was purified on silica gel, eluting with a 50/50 heptane /ethyl acetate mixture, to afford 374 mg of N-(3-(3,3-difluorocyclobutoxy)-1-methyl-1H-pyrazol-4-yl)formamide. MS (method B); m/z 232 [M+1]+; t=0.97 min.

### Intermediate 43: 2-(Methylthio)-7H-pyrrolo[2,3-d]pyrimidine-6-carbonitrile

### Step 1: Preparation of 2-(methylthio)-7H-pyrrolo[2,3-d]pyrimidine-6-carboxamide

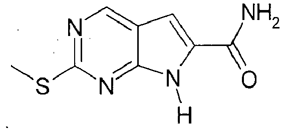

2 g (1 eq.) of methyl 2-(methylthio)-7H-pyrrolo[2,3-d]pyrimidine-6-carboxylate (Intermediate 1) in 200 mL of a 7N solution of ammoniac in methanol in a sealed tube were stirred for 3 days at room temperature. The mixture was then concentrated under reduced pressure. The residue was triturated in 50 mL of diethyl ether, and the unsoluble was filtered and dried under vacuum to afford 1.8 g of 2-(methylthio)-7H-pyrrolo[2,3-d]pyrimidine-6-carboxamide. MS (method B) m/z 209 [M+1]+; t=0.88 min.

### Step 2: Preparation of 2-(methylthio)-7H-pyrrolo[2,3-d]pyrimidine-6-carbonitrile

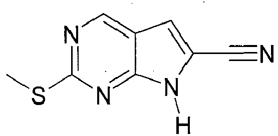

4.25 mL (3.6 eq.) of trifluoroacetic anhydride and 5.31 mL (4.5 eq.) of triethylamine were added to a solution of 1.75 g of 2-(methylthio)-7H-pyrrolo[2,3-d]pyrimidine-6-carboxamide (1 eq.) in 31 mL of anhydrous tetrahydrofuran at 0°C under argon. The mixture was stirred for 1 hour from 0°C to room temperature then poured into 200 mL of water. The precipitate was filtered under vacuum and triturated with diethyl ether to afford 1.3 g of 2-(methylthio)-7H-pyrrolo[2,3-d]pyrimidine-6-carbonitrile. MS (method A) m/z 191 [M+ 1]+; t=1.72 min.

### Intermediate 44: N-(1-ethyl-3-(oxetan-3-yloxy)-1H-pyrazol-4-yl)formamide

### Step 1: Preparation of 1-ethyl-4-nitro-3-(oxetan-3-yloxy)-1H-pyrazole

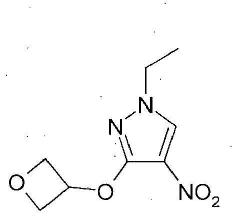

228 µL (1.5 eq.) of iodoethane and 1.23 g (2 eq.) of cesium carbonate were added to a solution of 350 mg (1 eq.) of 4-nitro-3-(oxetan-3-yloxy)-1H-pyrazole (Intermediate 13, step 3) in 10 mL of N,N-dimethylformamide. The mixture was stirred at room temperature for 1 hour and poured onto a mixture of 100 mL of ethyl acetate and 100 ml of diethyl ether. The organic layer was washed three times with 150 mL of water, dried over sodium sulfate, and concentrated under vacuum. The residue was purified on silica gel, eluting with a gradient of 0 to 80% of ethyl acetate in cyclohexane, to afford 374 mg of 1-ethyl-4-nitro-3-(oxetan-3-yloxy)-1H-pyrazole. MS (method E) m/z 214 [M+ 1]+; t=0.97 min.

### Step 2: Preparation of N-(1-ethyl-3-(oxetan-3-yloxy)-1H-pyrazol-4-yl)formamide

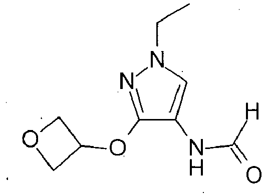

200 mg of N-(1-ethyl-3-(oxetan-3-yloxy)-1H-pyrazol-4-yl)formamide was prepared according to the basic procedure described in Intermediate 26, step2, starting with 373 mg of 1-ethyl-4-nitro-3-(oxetan-3-yloxy)-1H-pyrazole. MS (method E) m/z 212 [M+ 1]+; t=0.78 min.

### Intermediate 45: N-[3-isopropoxy-1-(methyl-d3)pyrazol-4-yl]formamide

Step 1: Preparation of 3-isopropoxy-4-nitro-1-(methyl-d3)pyrazole

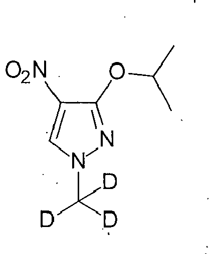

### 1.43 g of 3-isopropoxy-4-nitro-1-(methyl-d3)pyrazole was prepared according to the basic procedure described in Intermediate 26, Step 1, starting with 1.5 g of 3-isopropoxy-4-nitro-1H-pyrazole. MS (method E) m/z 189 [M+1]+; t= 1.11 min.

### Step 2: Preparation of N-[3-isopropoxy-1-(methyl-d3)pyrazol-4-yl]formamide

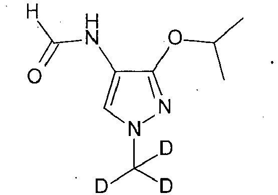

170 mg of ammonium chloride (5 eq.) and 178 mg of iron (5 eq.) were added to a solution of 120 mg of 3-isopropoxy-4-nitro-1-(methyl-d3)pyrazole in 8 mL of ethanol and 1.2 mL of water. The reaction mixture was heated at 80°C under vigorous stirring for 10 minutes and the mixture was filtered on celite with ethanol washes and concentrated under reduced pressure. The crude material was diluted with 20 mL of ethyl acetate and washed with 20mL of an aqueous saturated solution of sodium chloride. The organic layer was dried over sodium sulfate and concentrated under vacuum to form a residue.

A solution of the residue in 3 mL of tetrahydrofuran was dropwise added to a solution of 301 µL of acetic anhydride (5 eq.) in 433 µL of formic acid (18 eq.) that had been premixed for 30 minutes at room temperature. The reaction mixture was stirred at room temperature for 1 hour then concentrated under vacuum. The residue was purified on silica gel, eluting with 5% of methanol in dichloromethane to afford 95 mg of N-[3-isopropoxy-1-(methyl-d3)pyrazol-4-yl]formamide. MS (method E) m/z 187 [M+1]+; t= 0.83 min.

### Intermediate 46: (S)-N-(3-(2-butoxy)-1-methyl-1H-pyrazol-4-yl)formamide

### Step 1: Preparation of (S)-5-(2-butoxy)-4-nitro-1-((2-(trimethylsilyl)ethoxy)methyl)-1H-pyrazole

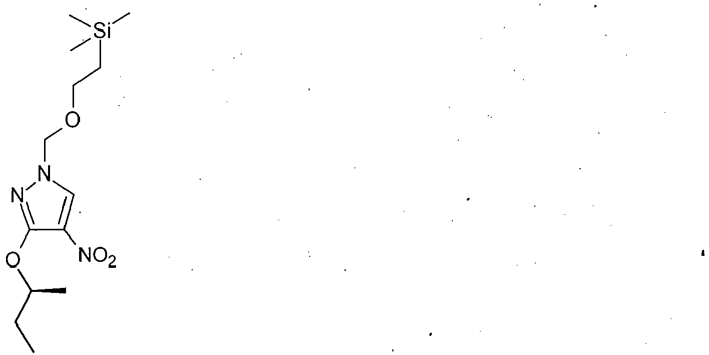

785 mg of (S)-5-(2-butoxy)-4-nitro-1-((2-(trimethylsilyl)ethoxy)methyl)-1H-pyrazole was prepared according to the basic procedure described in step 3 of Intermediate 29, starting with 1 g of 5-chloro-4-nitro-1-((2-(trimethylsilyl)ethoxy)methyl)-1H-pyrazole. MS (method F) m/z no ionization; t= 2.14 min.

### Step 2: Preparation of (S)-(5-(2-butoxy)-4-nitro-1H-pyrazol-1-yl)methanol

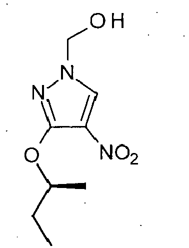

Following the basic procedure described in step 4 of Intermediate 29, 785 mg of (S)-5-(2-butoxy)-4-nitro-1-((2-(trimethylsilyl)ethoxy)methyl)-1H-pyrazole was converted to 495 mg of (S)-(5-(2-butoxy)-4-nitro-1H-pyrazol-1-yl)methanol. The crude material was taken into the next step without further purification.

### Step 3: Preparation of (S)-3-(2-butoxy)-1-methyl-4-nitro-1H-pyrazole

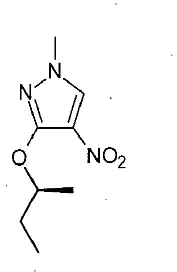

230 µL (1.1 eq.) of iodomethane and 1.16 g of potassium carbonate (2.5 eq.) were added to a solution of 495 mg (1 eq.) of (S)-(5-(2-butoxy)-4-nitro-1H-pyrazol-1-yl)methanol in 30 mL of N, N-dimethylformamide. The mixture was stirred at 60°C for 2 hours then cooled to room temperature and poured onto a mixture of 100 mL of ethyl acetate and 100 mL of diethyl ether. The organic layer was washed three times with 150 mL of water then was dried over sodium sulfate and concentrated under vacuum. The residue was purified on silica gel, eluting with a gradient of 0 to 60% of ethyl acetate in cyclohexane, to afford 367 mg of (S)-3-(2-butoxy)-1-methyl-4-nitro-1H-pyrazole. MS (method E) m/z 200 [M+1]+; t= 1.23 min.

### Step 4: Preparation of (S)-N-(3-(2-butoxy)-1-methyl-1H-pyrazol-4-yl)formamide

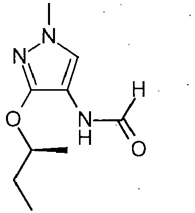

Following the basic procedure described in step 7 of Intermediate 29, 375 mg of (S)-3-(2-butoxy)-1-methyl-4-nitro-1H-pyrazole was converted to 179 mg of (S)-N-(3-(2-butoxy)-1-methyl-1H-pyrazol-4-yl)formamide. MS (method E) m/z 198 [M+1]+; t= 0.95 min.

### Intermediate 47: (R)-N-(3-(2-butoxy)-1-methyl-1H-pyrazol-4-yl)formamide

### Step 1: Preparation of (R)-5-(2-butoxy)-4-nitro-1-((2-(trimethylsilyl)ethoxy)methyl)-1H-pyrazole

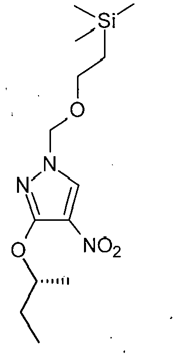

Following the basic procedure described in step 3 of Intermediate 29, 1 g of 5-chloro-4-nitro-1-((2-(trimethylsilyl)ethoxy)methyl)-1H-pyrazole was converted to 889 mg of (R)-5-(2-butoxy)-4-nitro-1-((2-(trimethylsilyl)ethoxy)methyl)-1H-pyrazole. MS (method F) m/z no ionization; t= 2.14 min.

### Step 2: Preparation of (R)-(5-(2-butoxy)-4-nitro-1H-pyrazol-1-yl)methanol

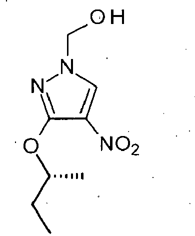

Following the basic procedure described in step 4 of Intermediate 29, 890 mg of (R)-5-(2-butoxy)-4-nitro-1-((2-(trimethylsilyl)ethoxy)methyl)-1H-pyrazole was converted to 607 mg of (R)-(5-(2-butoxy)-4-nitro-1H-pyrazol-1-yl)methanol. MS (method E) m/z 216 [M+1]+; t= 1.09 min.

### Step 2: Preparation of (R)-3-(2-butoxy)-1-methyl-4-nitro-1H-pyrazole

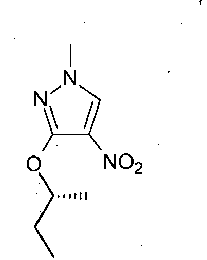

Following the basic procedure described in step 3 of Intermediate 46, 620 mg of (R)-(5-(2-butoxy)-4-nitro-1H-pyrazol-1-yl)methanol was converted to 440 mg of (R)-3-(2-butoxy)-1-methyl-4-nitro-1H-pyrazole. MS (method E) m/z 200 [M+1]+; t= 1.23 min.

### Step 3: Preparation of (R)-N-(3-(2-butoxy)-1-methyl-1H-pyrazol-4-yl)formamide

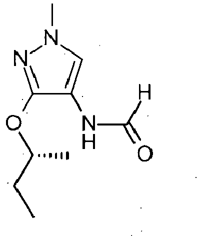

Following the basic procedure described in step 4 of Intermediate 46, 440 mg of (R)-3-(2-butoxy)-1-methyl-4-nitro-1H-pyrazole was converted to 175 mg of (R)-N-(3-(2-butoxy)-1-methyl-1H-pyrazol-4-yl)formamide. MS (method E) m/z 198 [M+1]+; t= 0.95 min.

### Intermediate 48: (N-(2,2-dimethyl-3,4-dihydro-2H-pyrano[3,2-b]pyridin-8-yl)formamide

### Step 1: Preparation of 2,2-dimethyl-2,3-dihydro-4H-pyrano[3,2-b]pyridin-4-one

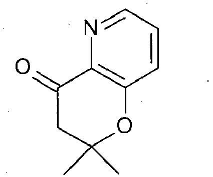

4.52 mL of pyrrolidine (1.5 eq.) was added to a solution of 5 g (1 eq.) of 1-(3-hydroxypyridin-2-yl)ethan-1-one in 100 mL of toluene in a closed vessel. The mixture was stirred for 15 minutes at room temperature. Then, 5.4 mL (2 eq) of acetone was added and the reaction mixture was stirred at 40°C for 24 hours. Another 4 mL (1.5 eq.) of acetone was added and the mixture was stirred again for 15 hours at room temperature then filtered over a pad of silica gel eluting with a gradient of 0 to 100% of ethyl acetate in cyclohexane, to afford 3.29 g of 2,2-dimethyl-2,3-dihydro-4H-pyrano[3,2-b]pyridin-4-one. MS (method F) m/z 178 [M+1]+; t=1.63 min.

### Step 2: Preparation of 2,2-dimethyl-3,4-dihydro-2H-pyrano[3,2-b]pyridin-4-ol

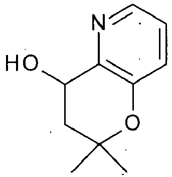

A solution of 1 g (1 eq.) of 2,2-dimethyl-2,3-dihydro-4H-pyrano[3,2-b]pyridin-4-one in 10 mL of ethanol was added dropwise to a solution of 160 mg (0.75 eq) of sodium borohydride in 5 mL of ethanol under stirring. The reaction mixture was stirred under reflux for 2 hours, and then cooled to room temperature, and concentrated under vacuum. The residue was dissolved in 200 mL of ethyl acetate and washed with 150 mL of a saturated aqueous solution of sodium hydrogen carbonate. The organic layer was dried over sodium sulfate, and then concentrated under vacuum, and the residue purified on silica gel, eluting with a gradient of 0 to 5% methanol in dichloromethane, to afford 820 mg of 2,2-dimethyl-3,4-dihydro-2H-pyrano[3,2-b]pyridin-4-ol. MS (method E) m/z 180 [M+1]+; t=0.34 min.

### Step 3: Preparation of 2,2-dimethyl-2H-pyrano[3,2-b]pyridine

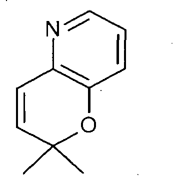

A solution of 820 mg (1 eq.) of 2,2-dimethyl-3,4-dihydro-2H-pyrano[3,2-b]pyridin-4-ol and 87 mg (0.1 eq.) of para-toluene sulfonic acid hydrate in 50 mL of toluene in a Dean & Stark apparatus was refluxed for 5 hours with regular elimination of water. After cooling to room temperature and concentration under vacuum, the residue was purified on silica gel, eluting with a gradient of 0 to 5% methanol in dichloromethane, to afford 442 mg of 2,2-dimethyl-2H-pyrano[3,2-b]pyridine. MS (method E) m/z 162 [M+1]+; t=0.83 min.

### Step 4: Preparation of 2,2-dimethyl-3,4-dihydro-2H-pyrano[3,2-b]pyridine

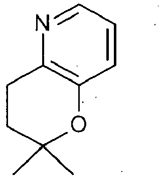

A solution of 440 mg (1 eq.) of 2,2-dimethyl-2H-pyrano[3,2-b]pyridine in 15 mL of methanol was treated with 286 mg of palladium 10% weight on carbon (0.1 eq.) under 2.5 bars of hydrogen for 1.5 hours in a Parr apparatus. After filtration on decalite, rinsing with 30 mL of methanol and concentration under vacuum, the residue was purified on silica gel, eluting with a gradient of 0 to 70% ethyl acetate in cyclohexane, to afford 445 mg of 2,2-dimethyl-3,4-dihydro-2H-pyrano[3,2-b]pyridine. MS (method E) m/z 164 [M+1]+; t=0.68 min.

### Step 5: Preparation of 2,2-dimethyl-3,4-dihydro-2H-pyrano[3,2-b]pyridine 5-oxide

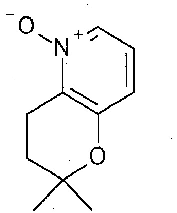

1.78 g (2.1 eq.) of 3-chloroperbenzoic acid (70% weight purity) was added to a solution of 560 mg (1 eq.) of 2,2-dimethyl-3,4-dihydro-2H-pyrano[3,2-b]pyridine in 250 mL of dichloromethane at 0°C The reaction mixture was stirred for 18 hours at room temperature, and then washed with 100 ml of a saturated aqueous sodium carbonate solution, and the organic layer was dried over sodium sulfate and concentrated under vacuum. The residue was purified on silica gel, eluting with a gradient of 0 to 5% methanol in dichloromethane, to afford 290 mg of 2,2-dimethyl-3,4-dihydro-2H-pyrano[3,2-b]pyridine 5-oxide. MS (method E) m/z 359[M+1]+; t=0.95 min.

### Step 6: Preparation of 2,2-dimethyl-8-nitro-3,4-dihydro-2H-pyrano[3,2-b]pyridine and 2,2-dimethyl-8-nitro-3,4-dihydro-2H-pyrano[3,2-b]pyridine 5-oxide

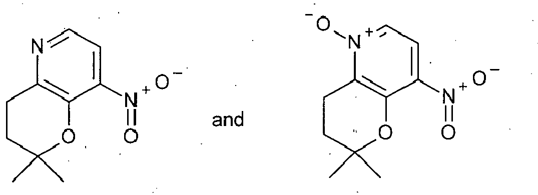

2 mL (28.6 eq.) of nitric acid was added dropwise to a solution of 280 mg (1 eq.) of 2,2-dimethyl-3,4-dihydro-2H-pyrano[3,2-b]pyridine 5-oxide in 2 mL of acetic acid. The reaction mixture was stirred for 5 hours at 85°C, and then cooled to room temperature, and poured onto a mixture of 200 g of ice and 10 mL of a 12N aqueous sodium hydroxide solution. The aqueous layer was saturated with sodium chloride and extracted three times with 150 mL of ethyl acetate. The combined organic layers were dried over sodium sulfate and concentrated under vacuum to afford 290 mg of an inseparable mixture of 2,2-dimethyl-8-nitro-3,4-dihydro-2H-pyrano[3,2-b]pyridine and 2,2-dimethyl-8-nitro-3,4-dihydro-2H-pyrano[3,2-b]pyridine 5-oxide. MS (method E) m/z 209 [M+1]+; t=1.26 min and m/z 225 [M+1]+; t=1.03 min.

### Step 7: Preparation of N-(2,2-dimethyl-3,4-dihydro-2H-pyrano[3,2-b]pyridin-8-yl)formamide

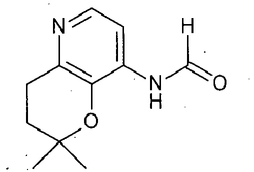

Following the basic procedure described in step 3 of Intermediate 31, 290 mg of 2,2-dimethyl-8-nitro-3,4-dihydro-2H-pyrano[3,2-b]pyridine and 2,2-dimethyl-8-nitro-3,4-dihydro-2H-pyrano[3,2-b]pyridine 5-oxide were reduced and formylated to afford 150 mg of N-(2,2-dimethyl-3,4-dihydro-2H-pyrano[3,2-b]pyridin-8-yl)formamide. MS (method E) m/z 207 [M+1]+; t=1.07 min.

### Intermediate 49: N-(3-isopropoxy-1-[(1R)-1,1,1-trifluoropropan-2-yl]-1H-pyrazol-4-yl)formamide and N-(3-isopropoxy-1-[(1S)-1,1,1-trifluoropropan-2-yl]-1H-pyrazol-4-yl)formamide

### Step 1: Preparation of 3-isopropoxy-4-nitro-1-(1,1,1-trifluoropropan-2-yl)-1H-pyrazole (racemic)

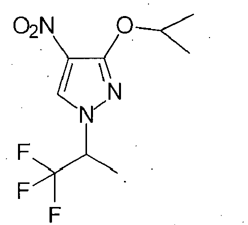

1.74 g (1.5 eq.) of racemic 1,1,1-trifluoropropan-2-yl 1,1,2,2,3,3,4,4,4-nonafluorobutane-1-sulfonate (commercially available) and 1.22 g (3 eq.) of potassium carbonate were added to a solution of 0.5 g (1 eq.) of 3-isopropoxy-4-nitro-1H-pyrazole (commercially available) in 2 mL of dimethylformamide. The mixture was heated at 80°C for 1.5 hours, and then allowed to cool to room temperature, and poured onto ethyl acetate and water. The aqueous layer was separated and extracted three times with ethyl acetate. The combined organic layers were dried over magnesium sulfate and concentrated under vacuum. The residue was taken into diethyl ether and water. The organic layer was separated, washed with water, dried over magnesium sulfate and concentrated under vacuum to give 360 mg of racemic 3-isopropoxy-4-nitro-1-(1,1,1-trifluoropropan-2-yl)-1H-pyrazole. MS (method D) m/z 268 [M+1]+; t=1.29 min.

### Step 2: Preparation of N-(3-isopropoxy-1-(1,1,1-trifluoropropan-2-yl)-1H-pyrazol-4-yl)formamide (racemic)

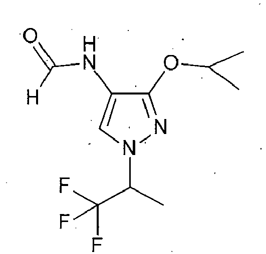

A solution of 550 mg (1 eq.) of racemic 3-isopropoxy-4-nitro-1-(1,1,1-trifluoropropan-2-yl)-1H-pyrazole in 80 mL of methanol was hydrogenated for 1 hour under 2.5 bar of hydrogen in presence of 100 mg (0.05 eq.) ofpalladium on carbon (10%). The mixture was filtered on decalite with dichloromethane washes and concentrated under reduced pressure. A solution of the crude material dissolved in 12 mL of tetrahydrofuran was dropwise added to a cooled (0°C) solution of 777 µL of acetic anhydride (4 eq.) in 632 µL of formic acid (8 eq.) that had been premixed for 30 minutes at room temperature. The reaction mixture was stirred at 0°C for 1 hour allowing it to warm up to room temperature, then poured onto a 10% aqueous sodium carbonate solution and stirred for 15 minutes. The aqueous layer was separated and extracted twice with ethyl acetate. The combined organic layers were dried over magnesium sulfate and concentrated under reduced pressure. The residue was purified on silica gel, eluting with a gradient from 0 to 20% of ethyl acetate in heptane, to afford 510 mg of racemic N-(3-isopropoxy-1-(1,1,1-trifluoropropan-2-yl)-1H-pyrazol-4-yl)formamide. MS (method D) m/z 266 [M+1]+; t=0.99 min.

### Intermediate 50: (3S,4S)-4-Methoxytetrahydrofuran-3-ol

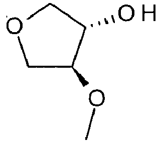

7.5 g of lipase AMANO AK (Ref. ALDRICH: Amano lipase from *Pseudomonas fluorescens* 20.000 U/g; catalog Number: 534730-50G) was added to a solution of 30 g (1 eq.) of a racemic mixture of (3S,4S)-4-methoxytetrahydrofuran-3-ol and (3R,4R)-4-methoxytetrahydrofuran-3-ol (Intermediate 17 step 1) and 150 mL of vinyl acetate in 300 mL of heptane. The suspension was stirred for 5 days at 22°C then filtered over a pad of decalite. The filter cake was rinsed twice with 50 mL of ethyl acetate and the filtrate was concentrated under reduced pressure. The residue was purified on silica gel, eluting with 30 to 90% of ethyl acetate in heptane to afford 11.72 g of (3S,4S)-4-methoxytetrahydrofuran-3-ol.
¹H NMR (400 MHz, CDCl₃) δ in ppm: 2.61 .(broad s, 1 H); 3.39 (s, 3 H); 3.76 (m, 3 H); 3.94 (dd, J=4.04 and 10 Hz, 1 H); 4.05 (dd, J=4.3 and 9.7 Hz, 1 H); 4.29 (m, 1 H).

### Intermediate 51: (R)-N-(2-methyl-3,4-dihydro-2H-[1,4]dioxepino[2,3-b]pyridin-9-yl)formamide

### Step 1: Preparation of (S)-4-((tert-butyldimethylsilyl)oxy)butan-2-ol

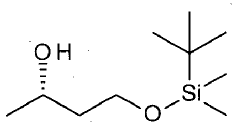

4.07 g (1.1 eq.) of imidazole was added to a solution of 5 g (1 eq.) of (S)-butane-1,3-diol in 50 ml of N,N-dimethylformamide under argon. The mixture was cooled to 0°C and 9.2g (1.1 eq.) of tert-butyl dimethylsilyl chloride was added. The reaction mixture was stirred for 24 hours at room temperature, and then diluted with 150 mL of diethyether and 100 mL of water. The organic layer was washed three times with 50 mL of water, dried over sodium sulfate and concentrated under vacuum. The residue was purified on silica gel, eluting with a gradient of 0 to 10% ethyl acetate in cyclohexane, to afford 9 g of (S)-4-((tert-butyldimethylsilyl)oxy)butan-2-ol.
1H NMR (400 MHz, CDCl3) δ in ppm: 0,1 (s, 6 H); 0,89 (s, 9 H); 1,19 (d, J=6.3 Hz, 3 H); 1,64 (m, 2 H); 3.30 (broad s, 1 H); 3,81 (m, 1 H); 3,88 (m, 1 H); 4,02 (m, 1 H).

### Step 2: Preparation of (R)-3-((2-chloropyridin-3-yl)oxy)butan-1-ol

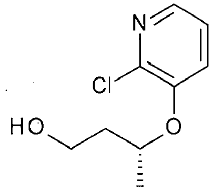

9.11 g (1.1 eq.) of triphenylphosphine and 6.45 mL (1.05 eq.) of diisopropylazodicarboxylate were added successively dropwise to a solution of 6.78 g (1.05 eq.) of (S)-4-((tert-butyldimethylsilyl)oxy)butan-2-ol and 4.09 g (1 eq.) of 2-chloropyridin-3-ol in 80 mL of tetrahydrofuran at 0°C. The reaction mixture was stirred 1 hour from 0°C to room temperature, and then diluted with 150 mL of diethyl ether and 100 mL of water. The organic layer was washed with 50 mL of water, dried over sodium sulfate, and concentrated under vacuum. The residue was purified on silica gel, eluting with a gradient of 0 to 10% of ethyl acetate in cyclohexane, to afford 8.24 g of sylilated intermediate. The resulting compound was dissolved in 250 mL of acetonitrile and 50 mL of an aqueous 1N hydrogen chloride solution and the reaction mixture stirred for 1 hour then carefully poured onto 200 mL of saturated aqueous sodium hydrogen carbonate solution and 400 mL of dichloromethane. The organic layer was dried over sodium sulfate and concentrated under vacuum. The residue was purified on silica gel, eluting with a gradient of 0 to 70% ethyl acetate in cyclohexane, to afford 4.4 g of (R)-3-((2-chloropyridin-3-yl)oxy)butan-1-ol. MS (method F) m/z 202 [M+1]+; t=1.17 min.

### Step 3: Preparation of (R)-2-methyl-3,4-dihydro-2H-[1,4]dioxepino[2,3-b]pyridine

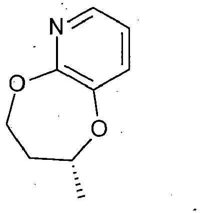

A solution of 2.5 g (1 eq.) of (R)-3-((2-chloropyridin-3-yl)oxy)butan-1-ol in 40 mL of 1-methyl-2-pyrrolidinone was added in 30 minutes to a suspension of 644 mg (1.3 eq.) of sodium hydride (60% weight in mineral oil) in 50 mL of 1-methyl-2-pyrrolidinone at 95°C under argon. The reaction mixture was stirred for 2 hours at 90-100 °C, and then cooled to room temperature, and poured onto 200 mL of a 50/50 mixture of diethyl ether and ethyl acetate and 100 mL of water. The aqueous layer was extracted with 50 mL of ethyl acetate and the organic layers were combined and washed four times with 50 mL of water, and then dried over sodium sulfate and concentrated under vacuum. The residue was purified on silica gel, eluting with a gradient of 0 to 50% ethyl acetate in cyclohexane, to afford 1 g of (R)-2-methyl-3,4-dihydro-2H-[1,4]dioxepino[2,3-b]pyridine. MS (method F) m/z 166 [M+1]+; t=1.11 min.

### Step 4: Preparation of (R)-2-methyl-3,4-dihydro-2H-[1,4]dioxepino[2,3-b]pyridine-9-carboxylic acid

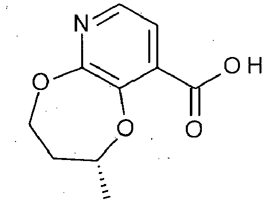

5.7 mL (1.5 eq.) of a 1.6 N butyllithium solution in heptane was slowly added to a solution of 1g (1 eq.) of (R)-2-methyl-3,4-dihydro-2H-[1,4]dioxepino[2,3-b]pyridine in 30 mL of anhydrous tetrahydrofuran at -60°C under argon while maintaining the temperature under -55°C. The reaction mixture was stirred for 1 hour 30 minutes at -60°C. The temperature was allowed to warm up gently to room temperature under stirring then diluted with 50 mL of diethyl ether and 20 mL of water. The organic layer was discarded and the basic aqueous layer acidified with 10 mL of a 10% citric acid aqueous solution. After extraction 5 times with a 75/25 mixture of dichloromethane/ isopropanol, the organic layers were combined, dried over sodium sulfate, and concentrated under vacuum. The residue was triturated in 10 mL of a 60/40 mixture of pentane/diethyl ether to afford 390 mg of (R)-2-methyl-3,4-dihydro-2H-[1,4]dioxepino[2,3-b]pyridine-9-carboxylic acid. MS (method F) m/z 210 [M+1]+; t=0.81 min.

### Step 5: Preparation of tert-butyl (R)-(2-methyl-3,4-dihydro-2H-[1,4]dioxepino[2,3-b]pyridin-9-yl)carbamate

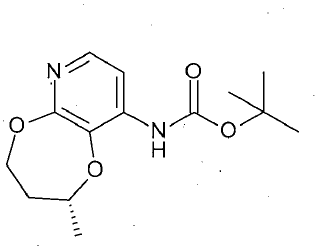

316 µL (1.2 eq.) of triethylamine and 503 µL (1.2 eq.) of diphenylphosphoryl azide were added to a solution of 395 mg (1 eq.) of of (R)-2-methyl-3,4-dihydro-2H-[1,4]dioxepino[2,3-b]pyridine-9-carboxylic acid in 10 mL of anhydrous toluene and 10 mL of tert-butanol under argon. The reaction mixture was stirred for 2 hours 30 minutes at room temperature. Then 7.6 mg of copper(II) chloride (0.03 eq.) and another 10 mL of tert-butanol was added and the mixture was stirred at 110°C for 1 hour 30 minutes, and then cooled to room temperature, and diluted with 30 mL of diethyl ether, 30 mL of ethyl acetate and 20 mL of water. The organic layer was washed with 10 mL of water and 10 mL of brine, dried over sodium sulfate, and concentrated under vacuum. The residue was purified on silica gel, eluting with a gradient of 0 to 30% of ethyl acetate in cyclohexane, to afford 450 mg of tert-butyl (R)-(2-methyl-3,4-dihydro-2H-[1,4]dioxepino[2,3-b]pyridin-9-yl)carbamate. MS (method F) m/z 281 [M+1]+; t=1.52 min.

### Step 6: Preparation of (R)-N-(2-methyl-3,4-dihydro-2H-[1,4]dioxepino[2,3-b]pyridin-9-yl)formamide

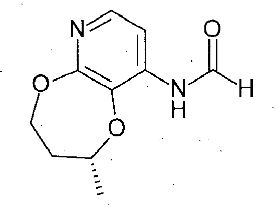

A solution of 450 mg (1 eq.) of tert-butyl (R)-(2-methyl-3,4-dihydro-2H-[1,4]dioxepino[2,3-b]pyridin-9-yl)carbamate in 10 mL of dichloromethane and 10 mL of trifluoroacetic acid was stirred at room temperature for 1 hour then concentrated under vacuum. The residue was azeotroped three times with a 50/50 mixture of toluene/tetrahydrofuran then taken in 5 mL of tetrahydrofuran and added to a premix solution of 492 µL of formic acid (8 eq.) and 606 µL of acetic anhydride (4 eq.). After 2 hours of stirring at room temperature, the reaction mixture was poured onto 20 mL of dichloromethane and 10 mL of a 10% aqueous sodium carbonate solution. The biphasic mixture was vigorously stirred for 10 minutes and the aqueous layer extracted with another 20 mL of dichloromethane. The organic layers were combined, dried over sodium sulfate, and concentrated under vacuum. The residue was triturated in 10 mL of a 50/50 mixture of pentane/diethyl ether to afford 238 mg of (R)-N-(2-methyl-3,4-dihydro-2H-[1,4]dioxepino[2,3-b]pyridin-9-yl)formamide.
MS (method F) m/z 209 [M+1]+; t=0.84 min.

### EXAMPLES

The following examples are provided to describe the disclosure in further details. These examples illustrate suitable methods of synthesis of representative compounds of this disclosure. The absolute configuration for some of the compounds described in Examples has been assigned unless specifically indicated. When a compound described in Examples was obtained as single diastereoisomer or enantiomer but absolute configuration was not determined, said compound is indicated as "absolute configuration unknown". When a compound described in Examples was obtained as a mixture, said compound is indicated as "mixture".

### Example 1: 2-[[3-(1-acetylazetidin-3-yl)oxy-1-methyl-pyrazol-4-yl]amino]-7-[(3R,4R)-4-methyltetrahydrofuran-3-yl]pyrrolo[2,3-d]pyrimidine-6-carbonitrile

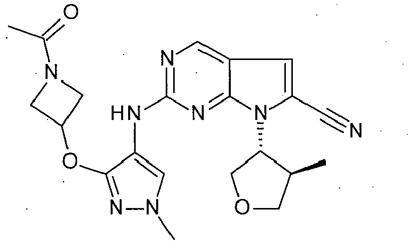

### Step 1: Preparation of 2-methylsulfanyl-7-[(3R,4R)-4-methyltetrahydrofuran-3-yl]pyrrolo[2,3-d]pyrimidine-6-carboxylic acid

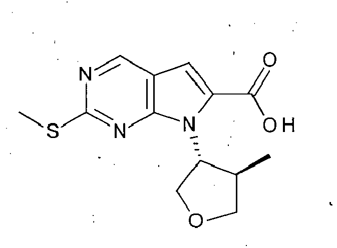

3.6 g (1 eq.) of methyl 2-(methylthio)-7H-pyrrolo[2,3-d]pyrimidine-6-carboxylate (intermediate 1) was added to a solution of 10.41 g (3 eq.) of diisopropyl azodicarboxylate (DIAD) and 12.82 g (3 eq.) of triphenylphosphine in 180 mL of anhydrous tetrahydrofuran cooled at -10°C. The mixture was stirred at 0°C for 1 hour then 4.39 g of (3S,4S)-4-methyltetrahydrofuran-3-ol (purity 87%) (2.4 eq.) (intermediate 2) were added. The mixture was stirred at room temperature for one night and concentrated under reduced pressure. The residue was dissolved in 160 mL of tetrahydrofuran and 1.97 g (5 eq.) of lithium hydroxide in 18 mL of water was added. The mixture was stirred for 1 hour at room temperature then diluted with 200 mL of ethyl acetate and 50 mL of water. NaOH 2N was added to reach pH10. The two phases were separated. The aqueous layer was acidified to pH 1 with HCI 5N. The precipitate formed upon acidification was filtered-off, washed with water, and dried under vacuum (overnight) then at 40°C for 3 hours to afford 1.4 g of 2-methylsulfanyl-7-[(3R,4R)-4-methyltetrahydrofuran-3-yl]pyrrolo[2,3-d]pyrimidine-6-carboxylic acid as a beige solid. The organic layer was dried over magnesium sulfate, concentrated under vacuum and purified on silica, eluting with 50% of ethyl acetate in heptane then 20% of methanol in DCM to afford additional 2.9 g of 2-methylsulfanyl-7-[(3R,4R)-4-methyltetrahydrofuran-3-yl]pyrrolo[2,3-d]pyrimidine-6-carboxylic acid as a beige solid. MS (method A) m/z 294 [M+ 1]+; t=1.72 min.

### Step 2: Preparation of 2-methylsulfanyl-7-[(3R,4R)-4-methyltetrahydrofuran-3-yl]pyrrolo[2,3-d]pyrimidine-6-carboxamide

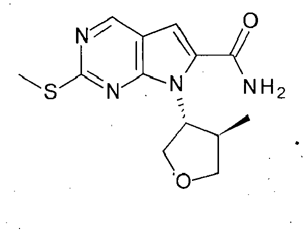

1.46 g (2 eq.) of di(1H-imidazol-1-yl)methanone (CDI) was added to a solution of 1.32 g of 2-methylsulfanyl-7-[(3R,4R)-4-methyltetrahydrofuran-3-yl]pyrrolo[2,3-d]pyrimidine-6-carboxylic acid (1 eq.) in 15 mL of dimethylformamide. The mixture was stirred for 1 hour at room temperature and 3 mL of a 28% ammonium hydroxide solution was added. The reaction mixture was stirred at room temperature for 1 hour. It was then diluted with 100 mL of ethyl acetate and washed with water. The organic layer was washed with 50 mL of water and 50 mL of brine, dried over magnesium sulfate and concentrated under reduced pressure. The crude material was triturated in diisopropyl ether and the solid filtered to afford 1.24 g of 2-methylsulfanyl-7-[(3R,4R)-4-methyltetrahydrofuran-3-yl]pyrrolo[2,3-d]pyrimidine-6-carboxamide as a beige solid. MS (method A) m/z 293 [M+ 1]+; t=1.74 min.

### Step 3: Preparation of 2-methylsulfanyl-7-[(3R,4R)-4-methyltetrahydrofuran-3-yl]pyrrolo[2,3-d]pyrimidine-6-carbonitrile

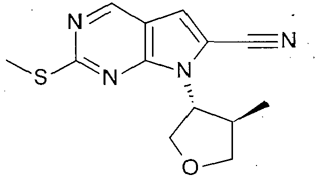

2.42 mL (3.6 eq.) of trifluoroacetic anhydride was added to a solution of 1.4 g of 2-methylsulfanyl-7-[(3R,4R)-4-methyltetrahydrofuran-3-yl]pyrrolo[2,3-d]pyrimidine-6-carboxamide (1 eq.) and 3.04 mL (4.5 eq.) of triethylamine in 20 mL of anhydrous tetrahydofuran at 0°C under argon. The mixture was stirred for 1 hour from 0°C to room temperature and quenched with 100 mL of ethyl acetate, 100 mL of diethyl ether and 100 mL of water under vigorous stirring. The organic layer was washed with 50 mL of water and 50 mL of brine, dried over magnesium sulfate, and concentrated under reduced pressure. The residue was purified on silica, eluting with 0-50% of ethyl acetate in cyclohexane to afford 1.3 g of 2-methylsulfanyl-7-[(3R,4R)-4-methyltetrahydrofuran-3-yl]pyrrolo[2,3-d]pyrimidine-6-carbonitrile. MS (method A) m/z 275 [M+ 1]+; t=2.28 min.

### Step 4: Preparation of 2-methylsulfonyl-7-[(3R,4R)-4-methyltetrahydrofuran-3-yl]pyrrolo[2,3-d]pyrimidine-6-carbonitrile.

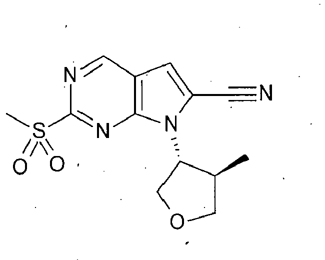

2.4 g of 3-chloroperbenzoic acid (77% purity; 2.1 eq.) was added to a solution of 1.4 g (1 eq.) of 2-methylsulfanyl-7-[(3R,4R)-4-methyltetrahydrofuran-3-yl]pyrrolo[2,3-d]pyrimidine-6-carbonitrile in 50 mL of dichloromethane at 0°C. The mixture was stirred for 1 hour at 0°C and 1 hour at room temperature then quenched with 50 mL of dichloromethane and 50 mL of a 10% aqueous sodium carbonate solution. The organic layer was washed successively with 50 mL of aqueous saturated sodium carbonate solution, 50 mL of water and 50 mL of brine, and then dried over magnesium sulfate, and concentrated under reduced pressure. The residue was purified on silica, eluting with 0-10% of methanol in dichloromethane to afford 1.2 g of 2-methylsulfonyl-7-[(3R,4R)-4-methyltetrahydrofuran-3-yl]pyrrolo[2,3-d]pyrimidine-6-carbonitrile. MS (method A) m/z 307 [M+ 1]+; t=1.64 min.

### Step 5: Preparation of 2-[[3-(1-acetylazetidin-3-yl)oxy-1-methyl-pyrazol-4-yl]amino]-7-[(3R,4R)-4-methyltetrahydrofuran-3-yl]pyrrolo[2,3-d]pyrimidine-6-carbonitrile

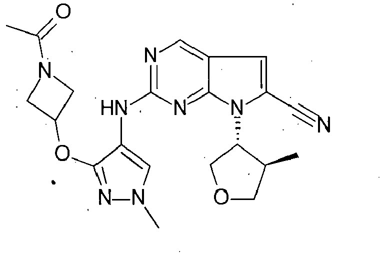

158 mg (2.5 eq.) of tert-butylimino-tri(pyrrolidino)phosphorane (BTPP) was added to a solution of 60 mg (1 eq.) of 2-methylsulfonyl-7-[(3R,4R)-4-methyltetrahydrofuran-3-yl]pyrrolo[2,3-d]pyrimidine-6-carbonitrile and 56 mg (1.2 eq.) of N-(3-((1-acetylazetidin-3-yl)oxy)-1-methyl-1H-pyrazol-4-yl)formamide (intermediate 9) in 4 mL of dimethylformamide and the mixture was stirred at room temperature for 4 hours, and then diluted with 50 mL of ethyl acetate and 50 mL of water. The organic layer was washed twice with 50 mL of water then dried over magnesium sulfate and concentrated under reduced pressure. The residue was taken in 15 mL of methanol and 30 mL of a 7N methanolic ammonia solution and stirred for 1 hour then concentrated under reduced pressure. The residue was purified on silica, eluting with a mixture of heptane/AcOEt/7N NH₃ in MeOH from the ratio (80/18/2) to (50/45/5) to afford 46 mg of 2-[[3-(1-acetylazetidin-3-yl)oxy-1-methyl-pyrazol-4-yl]amino]-7-[(3R,4R)-4-methyltetrahydrofuran-3-yl]pyrrolo[2,3-d]pyrimidine-6-carbonitrile.
¹H NMR (400 MHz, DMSO-d6) δ in ppm: 1.05 (d, J=7 Hz, 3 H); 1.77 (s, 3 H); 2.91 (m, 1 H); 3.47 (m, 1 H); 3.68 (s, 3 H); 3.82 (m, 1 H); 4.07-4.24 (m, 4 H); 4.31 (br s, 1 H); 4.44 (m, 1 H); 4.77 (br s, 1 H); 5.08 (m, 1 H); 7.47 (s, 1 H); 8.11 (br s, 1 H); 8.83 (s, 1 H); 9.01 (br s, 1 H). MS (method B) m/z 437 [M+ 1]+; t=1.34 min.

Example 2 and Example 3: 2-[[1-Methyl-3-[(3R,4S)-4-methyltetrahydrofuran-3-yl]oxy-1H-pyrazol-4-yl]amino]-7-[(3R,4R)-4-methyltetrahydrofuran-3-yl]pyrrolo[2,3-d]pyrimidine-6-carbonitrile and 2-[[1-methyl-3-[(3S,4R)-4-methyltetrahydrofuran-3-yl]oxy-1H-pyrazol-4-yl]amino]-7-[(3R,4R)-4-methyltetrahydrofuran-3-yl]pyrrolo[2,3-d]pyrimidine-6-carbonitrile

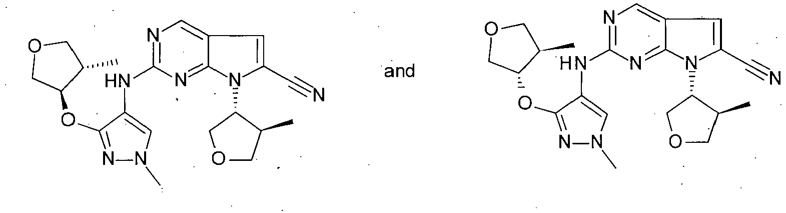

Generally following the procedure described in Step 5 of Example 1, 2-methylsulfonyl-7-[(3R,4R)-4-methyltetrahydrofuran-3-yl]pyrrolo[2,3-d]pyrimidine-6-carbonitrile (80 mg) and a racemic mixture of N-(1-methyl-3-(((3S,4R)-4-methyltetrahydrofuran-3-yl)oxy)-1H-pyrazol-4-yl)formamide and N-(1-methyl-3-(((3R,4S)-4-methyltetrahydrofuran-3-yl)oxy)-1H-pyrazol-4-yl)formamide (60 mg) (Intermediate 5) gave an isomeric mixture of 2-[[1-methyl-3-[(3R,4S)-4-methyltetrahydrofuran-3-yl]oxy-1H-pyrazol-4-yl]amino]-7-[(3R,4R)-4-methyltetrahydrofuran-3-yl]pyrrolo[2,3-d]pyrimidine-6-carbonitrile and 2-[[1-methyl-3-[(3S,4R)-4-methyltetrahydrofuran-3-yl]oxy-1H-pyrazol-4-yl]amino]-7-[(3R,4R)-4-methyltetrahydrofuran-3-yl]pyrrolo[2,3-d]pyrimidine-6-carbonitrile (95 mg) (Referred herein as 2-[[1-methyl-3-[(trans)-4-methyltetrahydrofuran-3-yl]oxy-1H-pyrazol-4-yl]amino]-7-[(3R,4R)-4-methyltetrahydrofuran-3-yl]pyrrolo[2,3-d]pyrimidine-6-carbonitrile).

Chiral separation performed on 82 mg of the isomeric mixture gave 33 mg of the first eluting isomer and 29 mg of the second eluting isomer (conditions: column Chiralcel OZ-H, 5 µm, 250x30 mm; liquid phase: heptane 25/ethyl alcohol 75/triethylamine 0.1%; flow rate: 40 mL/min).

Peak 1 (1^{st} isomer): ¹H NMR (400 MHz, DMSO-d6) δ in ppm: 1.02 (d, J=7 Hz, 3 H); 1.05 (d, J=7 Hz, 3 H); 2.41 (m, 1 H); 2.92 (m, 1 H); 3.33 (m, 1 H); 3.47 (t, J=9 Hz, 2 H); 3.68 (s, 3 H); 3.80 (d, J=10 Hz, 1 H); 3.90-3.99 (m, 2 H); 4.12-4.24 (m, 2 H); 4.29 (m, 1 H); 4.68 (m, 1 H); 4.77 (m, 1 H); 7.47 (s, 1 H); 8.10 (br s, 1 H); 8.82 (s, 2 H). MS (method B) m/z 424 [M+1]+; t=1.56 min - Example 2 (absolute configuration unknown)

Peak 2 (2^{nd} isomer): ¹H NMR (400 MHz, DMSO-d6) δ in ppm: 1.02 (d, J=7 Hz, 3 . H); 1.05 (d, J=7 Hz, 3 H); 2.41 (m, 1 H); 2.92 (m, 1 H); 3.33 (m, 1 H); 3.47 (t, J=9 Hz, 2 H); 3.68 (s, 3 H); 3.80 (d, J=10 Hz, 1 H); 3.90-3.99 (m, 2 H); 4.12-4.24 (m, 2 H); 4.29 (m, 1 H); 4.68 (m, 1 H); 4.77 (m, 1 H); 7.47 (s, 1 H); 8.10 (br s, 1 H); 8.82 (s, 2 H). MS (method B) m/z 424 [M+ 1]+; t=1.56 min -Example 3 (absolute configuration unknown)

Example 4 and Example 5: 2-[[1-[(1R)-2,2-Difluoro-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile and 2-[[1-[(1S)-2,2-difluoro-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile

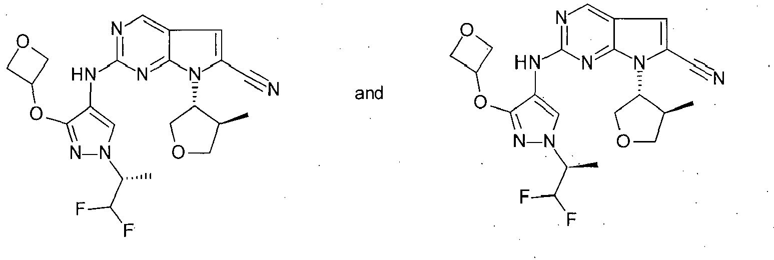

Generally following the procedure described in Step 5 of Example 1, 2-methylsulfonyl-7-[(3R,4R)-4-methyltetrahydrofuran-3-yl]pyrrolo[2,3-d]pyrimidine-6-carbonitrile (80 mg) and N-[1-[2,2-difluoro-1-methyl-ethyl]-3-(oxetan-3-yloxy)pyrazol-4-yl]formamide (69 mg) (Intermediate 25) gave an isomeric mixture of 2-[[1-[(1R)-2,2-difluoro-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile and 2-[[1-[(1S)-2,2-difluoro-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile (69 mg) (Referred herein as 2-[[1-(2,2-difluoro-1-methyl-ethyl)-3-(oxetan-3-yloxy)pyrazol-4-yl]amino]-7-[(3R,4R)-4-methyltetrahydrofuran-3-yl]pyrrolo[2,3-d]pyrimidine-6-carbonitrile).

Chiral separation performed on 69 mg of the isomeric mixture gave 29 mg of the first eluting isomer and 30 mg of the second eluting isomer (conditions: column Phenomenex cellulose-4, 5 µm, 250x30 mm; liquid phase: heptane 55/ethyl alcohol 45/triethylamine 0.1%; flow rate: 45 mL/min).

Peak 1 (1^{st} isomer): ¹H NMR (400 MHz, DMSO-d6) δ in ppm: 1.03 (d, J=7 Hz, 3 H); 1.48 (d, J=7 Hz, 3 H); 2.88 (m, 1 H); 3.44(t, J=9 Hz, 1 H); 4.15 (t, J=9 Hz, 2 H); 4.32 (m, 1 H); 4.51 (m, 1 H); 4.59 (m, 2 H); 4.76 (m, 1 H); 4.82 (t, J=7 Hz, 2 H); 5.35 (m, 1 H); 6.15 (dt, J=4 and 56 Hz, 1 H); 7.49 (s, 1 H); 8.17 (br s, 1 H); 8.85 (s, 1 H); 9.03 (br s, 1 H). MS (method B) m/z 460 [M+ 1]+; t=1.65 min - Example 4 (absolute configuration unknown)

Peak 2 (2^{nd} isomer): ¹H NMR (400 MHz, DMSO-d6) δ in ppm: 1.04 (d, J=7 Hz, 3 H); 1.49 (d, J=7 Hz, 3 H); 2.88 (quin, J=7 Hz, 1 H); 3.44 (t, J=9 Hz, 1 H); 4,09 - 4,21 (m, 2 H); 4.31 (m, 1 H); 4.51 (m, 1 H); 4.58 (t, J=6 Hz, 2 H); 4.76 (m, 1 H); 4.82 (t, J=7 Hz, 2 H); 5.35 (quin, J=6 Hz, 1 H); 6.13 (td, J=4 and 56 Hz, 1 H); 7.49 (s, 1 H); 8.17 (br s, 1 H); 8.85 (s, 1 H); 9.03 (br s, 1 H). MS (method B) m/z 460 [M+ 1]+; t=1.65 min - Example 5 (absolute configuration unknown)

Example 6 and Example 7: 2-((1-Methyl-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile and 2-((1-methyl-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile

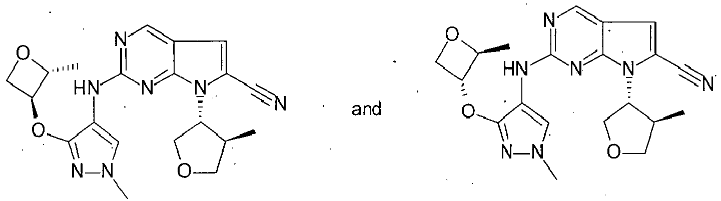

Generally following the procedure described in Step 5 of Example 1, 2-methylsulfonyl-7-[(3R,4R)-4-methyltetrahydrofuran-3-yl]pyrrolo[2,3-d]pyrimidine-6-carbonitrile (400 mg) and a racemic mixture of N-(1-methyl-3-(((2R,3S)-2-methyloxetan-3-yl)oxy)-1H-pyrazol-4-yl)formamide and N-(1-methyl-3-(((2S,3R)-2-methyloxetan-3-yl)oxy)-1H-pyrazol-4-yl)formamide (290 mg) (Intermediate 19) gave an isomeric mixture of 2-((1-methyl-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile and 2-((1-methyl-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile (421 mg) (Referred herein as 2-((1-methyl-3-(((trans)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile).

Chiral separation performed on 406 mg of the isomeric mixture gave 199 mg of the first eluting isomer and 187 mg of the second eluting isomer (conditions: column Chiralpak AD-H, 20 µm, 350x76 mm; liquid phase: heptane 50/ethyl alcohol 50/triethylamine 0.1%; flow rate: 400 mL/min).

Peak 1 (1^{st} isomer): ¹H NMR (400,MHz, DMSO-d6) δ in ppm: 1.06 (d, J=7 Hz, 3 H); 1.42 (d, J=6 Hz, 3 H); 2.88 ― 2.97 (m, 1 H); 3.47 (t, J=9 Hz, 1 H); 3.67 (s, 3 H); 4.05 - 4.36 (m, 3 H); 4.38 ― 4.51 (m, 1 H); 4.66 (t, J=7 Hz, 1 H); 4.75 - 4.84 (m, 2 H); 4.84 - 4.93 (m, 1 H); 7.48 (s, 1 H); 8.09 (br s, 1 H); 8.83 (s, 1 H); 8.95 (br s, 1 H). MS (method B) m/z 410 [M+ 1]+; t=1.57 min -Example 6 (absolute configuration unknown)

Peak 2 (2^{nd} isomer): ¹H NMR (400 MHz, DMSO-d6) δ in ppm: 1.06 (d, J=7 Hz, 3 H); 1.42 (d, J=6 Hz, 3 H); 2.84 - 2.97 (m, 1 H); 3.47 (t, J=9 Hz, 1 H); 3.67 (s, 3 H); 4.05 ― 4.34 (m, 2 H); 4.38 - 4.45 (m, 1 H); 4.66 (t, J=7 Hz, 1 H); 4.72 - 4.82 (m, 2 H); 4.89 (m, 1 H); 7.48 (s, 1 H); 8.08 (br s, 1 H); 8,83 (s, 1 H); 8,95(br s, 1 H). MS (method B) m/z410 [M+ 1]+; t=1.56 min -Example 7 (absolute configuration unknown)

### Example 8: 2-[[1-Methyl-3-(oxetan-3-yloxy)pyrazol-4-yl]amino]-7-[(3R,4R)-4-methyltetrahydrofuran-3-yl]pyrrolo[2,3-d]pyrimidine-6-carbonitrile

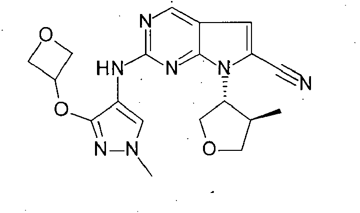

459 mg (2.5 eq.) of tert-butylimino-tri(pyrrolidino)phosphorane (BTPP) was added to a solution of 180 mg (1 eq.) of 2-methylsulfonyl-7-[(3R,4R)-4-methyltetrahydrofuran-3-yl]pyrrolo[2,3-d]pyrimidine-6-carbonitrile which was made generally following the procedure described in Step 1-4 of Example 1 and 139 mg (1.2 eq.) of N-[1-methyl-3-(oxetan-3-yloxy)pyrazol-4-yl]formamide (intermediate 4) in 20 mL of dimethylformamide and the mixture was stirred at room temperature for 1.5 hours then diluted with 50 mL of ethyl acetate and 50 mL of water. The organic layer was washed with 2 times 50 mL of water, dried over magnesium sulfate, and concentrated under reduced pressure. The residue was taken in 5 mL of methanol and 5 mL of a 7N methanolic ammonia solution, stirred for 30 minutes then concentrated under reduced pressure. The residue was purified on silica, eluting with a mixture of heptane/AcOEt from the ratio (100/0) to (0/100) to give 181 mg of 2-[[1-methyl-3-(oxetan-3-yloxy)pyrazol-4-yl]amino]-7-[(3R,4R)-4-methyltetrahydrofuran-3-yl]pyrrolo[2,3-d]pyrimidine-6-carbonitrile (181 mg).
¹H NMR (400 MHz, DMSO-d6) δ in ppm: 1.05 (d, J=7 Hz, 3 H); 2.95 (m, 1 H); 3.47 (t, J=9 Hz, 1 H); 3.66 (s, 3 H); 4.11 - 4.24 (m, 2 H); 4,30 (br s, 1 H); 4.58 (m, 2 H); 4.78 (m, 1 H); 4.82 (t, J=7 Hz, 2 H); 5.32 (m, 1 H); 7.48 (s, 1 H); 8.08 (br s, 1 H); 8.83 (s, 1 H); 8.99 (br s, 1 H). MS (method B) m/z 396 [M+ 1]+; t=1.46 min.

### Example 9: 2-[[1-[2-Hydroxy-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile

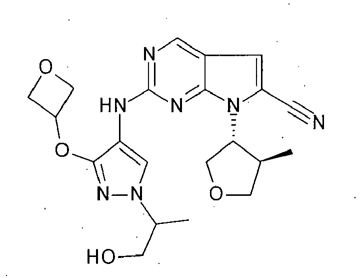

### Step 1: Preparation of methyl 2-[4-[[6-cyano-7-[(3R,4R)-4-methyltetrahydrofuran-3-yl]pyrrolo[2,3-d]pyrimidin-2-yl]amino]-3-(oxetan-3-yloxy)pyrazol-1-yl]propanoate

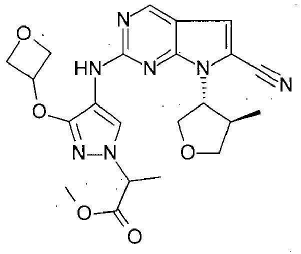

Generally following the procedure described in Step 5 of Example 1, 2-methylsulfonyl-7-[(3R,4R)-4-methyltetrahydrofuran-3-yl]pyrrolo[2,3-d]pyrimidine-6-carbonitrile (200 mg) and racemic methyl 2-(4-formamido-3-(oxetan-3-yloxy)-1H-pyrazol-1-yl)propanoate (185mg) (Intermediate 33) gave methyl 2-[4-[[6-cyano-7-[(3R,4R)-4-methyltetrahydrofuran-3-yl]pyrrolo[2,3-d]pyrimidin-2-yl]amino]-3-(oxetan-3-yloxy)pyrazol-1-yl]propanoate (170 mg) which was used in the next without further purification.

### Step 2: Preparation of 2-[[1-[2-hydroxy-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile (Example 9)

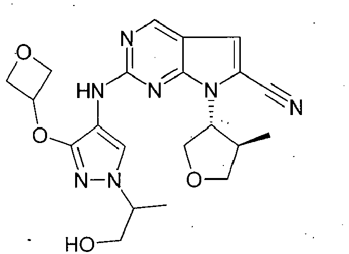

Sodium borohydride (64 mg, 7 eq.) was added at 0°C to a solution of methyl 2-[4-[[6-cyano-7-[(3R,4R)-4-methyltetrahydrofuran-3-yl]pyrrolo[2,3-d]pyrimidin-2-yl]amino]-3-(oxetan-3-yloxy)pyrazol-1-yl]propanoate (110 mg, 1 eq.) in 5 mL of methanol. The reaction mixture was stirred at 0°C for 1 hour, quenched with 10 mL of a saturated solution of ammonium chloride and extracted with ethyl acetate. The organic layer was washed with 50 mL of water and 50 mL of brine, dried over magnesium sulfate, and concentrated under reduced pressure. The residue was purified on silica, eluting with a mixture of heptane/AcOEt/7N methanolic ammonia solution from the ratio (80/18/2) to (50/45/5) to afford 24 mg of 2-[[1-[2-hydroxy-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile (Example 9).
¹H NMR (400 MHz, DMSO-d6) δ in ppm: 1.04 (d, J=6 Hz, 3 H); 1.33 (d, J=7 Hz, 3 H); 2.92 (m, 1 H); 3.45 (m, 1 H); 3.51 (m, 1 H); 3.63 (m, 1 H); 4.04-4.22 (m, 3 H); 4.30 (m, 1 H); 4.59 (m, 2 H); 4.75 -4.89 (m, 4 H); 5.33 (m, 1 H); 7.47 (s, 1 H); 8.07 (br s, 1 H); 8.83 (s, 1 H); 8.91 (br s, 1 H). MS (method B) m/z 440 [M+1]+; t=1.36 min.

### Example 10 and Example 11: 7-[(3R,4R)-4-Methyltetrahydrofuran-3-yl]-2-[[1-(oxetan-3-yl)-3-[(1R)-2,2,2-trifluoro-1-methyl-ethoxy]pyrazol-4-yl]amino]pyrrolo[2,3-d]pyrimidine-6-carbonitrile and 7-[(3R,4R)-4-methyltetrahydrofuran-3-yl]-2-[[1-(oxetan-3-yl)-3-[(1S)-2,2,2-trifluoro-1-methyl-ethoxy]pyrazol-4-yl]amino]pyrrolo[2,3-d]pyrimidine-6-carbonitrile

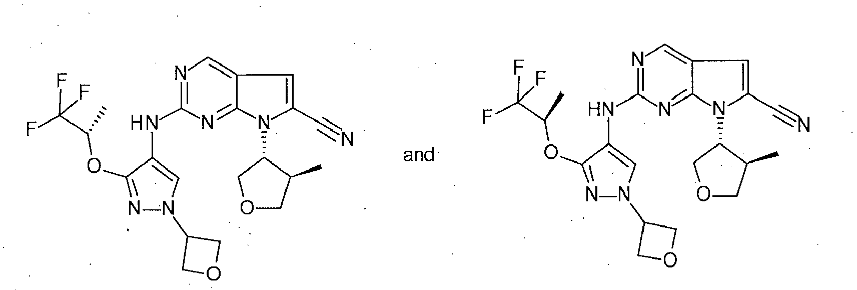

Generally following the procedure described in Step 5 of Example 1, 2-methylsulfonyl-7-[(3R,4R)-4-methyltetrahydrofuran-3-yl]pyrrolo[2,3-d]pyrimidine-6-carbonitrile (100 mg) and a racemic mixture of N-[1-(oxetan-3-yl)-3-[(1R)-2,2,2-trifluoro-1-methyl-ethoxy]pyrazol-4-yl]formamide and N-[1-(oxetan-3-yl)-3-[(1S)-2,2,2-trifluoro-1-methyl-ethoxy]pyrazol-4-yl]formamide (96 mg) (Intermediate 23) gave an isomeric mixture of 7-[(3R,4R)-4-methyltetrahydrofuran-3-yl]-2-[[1-(oxetan-3-yl)-3-[(1R)-2,2,2-trifluoro-1-methyl-ethoxy]pyrazol-4-yl]amino]pyrrolo[2,3-d]pyrimidine-6-carbonitrile and 7-[(3R,4R)-4-methyltetrahydrofuran-3-yl]-2-[[1-(oxetan-3-yl)-3-[(1S)-2,2,2-trifluoro-1-methyl-ethbxy]pyrazol-4-yl]amino]pyrrolo[2,3-d]pyrimidine-6-carbonitrile (129 mg) (Referred herein as 7-[(3R,4R)-4-methyltetrahydrofuran-3-yl]-2-[[1-(oxetan-3-yl)-3-(2,2,2-trifluoro-1-methyl-ethoxy)pyrazol-4-yl]amino]pyrrolo[2,3-d]pyrimidine-6-carbonitrile).

Chiral separation performed on 93 mg of the isomeric mixture gave 39 mg of the first eluting isomer and 40 mg of the second eluting isomer (conditions: column Chiralpak AY-H, 5 µm, 250x4.6 mm; liquid phase: heptane 80/ethyl alcohol 20/triethylamine 0.1%; flow rate: 45 mL/min).

Peak 1 (1^{st} isomer): ¹H NMR (400 MHz, DMSO-d6) δ in ppm: 1.05 (d, J=7 Hz, 3 H); 1.48 (d, J=6 Hz, 3 H); 2.90 (m, 1 H); 3.45 (t, J=9 Hz, 1 H); 4.18 (m, 2 H); 4.30 (m, 1 H); 4.76 (m, 1 H); 4.85-4.93 (m, 4 H); 5.30-5.42 (m, 2 H); 7.48 (s, 1 H); 8.27 (br s, 1 H); 8.84 (s, 1 H); 8.94 (br s, 1 H). MS (method B) m/z 478 [M+1]+; t= 1.76 min - Example 10 (absolute configuration unknown)

Peak 2 (2^{nd} isomer): ¹H NMR (400 MHz, DMSO-d6) δ in ppm: 1.04 (d, J=7 Hz, 3 H); 1.48 (d, J=6 Hz, 3 H); 2.90 (quin, J=7 Hz, 1 H); 3.44 (t, J=9 Hz, 1 H); 4.07 - 4,23 (m, 2 H); 4.29 (m, 1 H); 4.72 (m, 1 H); 4.82 - 4,98 (m, 4 H); 5.26 - 5.47 (m, 2 H); 7.48 (s, 1 H); 8,26 (br s, 1 H); 8.84 (s, 1 H); 8.94 (br s, 1 H). MS (method B) m/z 478 [M+1]+; t=1.76 min ― Example 11 (absolute configuration unknown)

### Example 12 and Example 13: 2-[[3-[(3R,4R)-4-Methoxytetrahydrofuran-3-yl]oxy-1-methyl-pyrazol-4-yl]amino]-7-[(3R,4R)-4-methyltetrahydrofuran-3-yl]pyrrolo[2,3-d]pyrimidine-6-carbonitrile and 2-[[3-[(3S,4S)-4-methoxytetrahydrofuran-3-yl]oxy-1-methyl-pyrazol-4-yl]amino]-7-[(3R,4R)-4-methyltetrahydrofuran-3-yl]pyrrolo[2,3-d]pyrimidine-6-carbonitrile

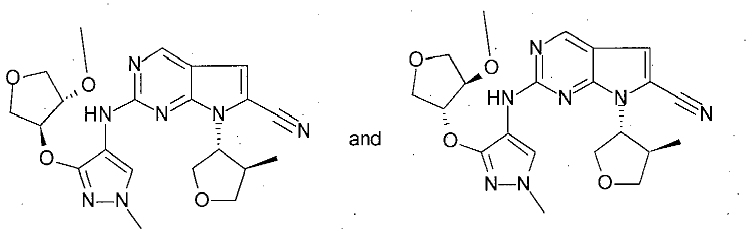

Generally following the procedure described in Step 5 of Example 1, 2-methylsulfonyl-7-[(3R,4R)-4-methyltetrahydrofuran-3-yl]pyrrolo[2,3-d]pyrimidine-6-carbonitrile (60 mg) and a racemic mixture of N-(3-(((3R,4R)-4-methoxytetrahydrofuran-3-yl)oxy)-1-methyl-1H-pyrazol-4-yl)formamide and N-(3-(((3S,4S)-4-methoxytetrahydrofuran-3-yl)oxy)-1-methyl-1H-pyrazol-4-yl)formamide (48 mg) (Intermediate 17) gave an isomeric mixture of 2-[[3-[(3R,4R)-4-methoxytetrahydrofuran-3-yl]oxy-1-methyl-pyrazol-4-yl]amino]-7-[(3R,4R)-4-methyltetrahydrofuran-3-yl]pyrrolo[2,3-d]pyrimidine-6-carbonitrile and 2-[[3-[(3S,4S)-4-methoxytetrahydrofuran-3-yl]oxy-1-methyl-pyrazol-4-yl]amino]-7-[(3R,4R)-4-methyltetrahydrofuran-3-yl]pyrrolo[2,3-d]pyrimidine-6-carbonitrile (70 mg) (Referred herein as 2-[[3-[(trans)-4-methoxytetrahydrofuran-3-yl]oxy-1-methyl-pyrazol-4-yl]amino]-7-[(3R,4R)-4-methyltetrahydrofuran-3-yl]pyrrolo[2,3-d]pyrimidine-6-carbonitrile).

Chiral separation performed on 61 mg of the isomeric mixture gave 24 mg of the first eluting isomer and 23 mg of the second eluting isomer (conditions: column Phenomenex Lux Amylose-1, 250x30 mm; liquid phase: heptane 20/ethyl alcohol 80/triethylamine 0.1%; flow rate: 40 mL/min).

Peak 1 (1^{st} isomer): ¹H NMR (400 MHz, DMSO-d6) δ in ppm: 1.06 (d, J=7 Hz, 3 H); 2.92 (spt, J=7 Hz, 1 H); 3.34 (m masked, 3 H); 3.48 (t, J=9 Hz, 1 H); 3.67 (m, 1 H); 3.70 (s, 3 H); 3.84 (d, J=10 Hz, 1 H); 3.93 (m, 2 H); 4.04 (m, 1 H); 4.16 (t, J=9 Hz, 1 H); 4.22 (t, J=8 Hz, 1 H); 4.34 (br s, 1 H); 4.78 (dt, J=1 and 7 Hz, 1 H); 4.96 (d, J=4 Hz, 1 H); 7.48 (s, 1 H); 8.13 (br s, 1 H); 8.83 (s, 1 H); 8.93 (br s, 1 H). MS (method B) m/z 440 [M+1]+; t=1.5 min - Example 12 (absolute configuration unknown)

Peak 2 (2^{nd} isomer): ¹H NMR (400 MHz, DMSO-d6) δ in ppm: 1.06 (d, J=7 Hz, 3 H); 2.92 (spt, J=7. 1 H); 3.33 (m masked 3 H); 3.48 (t, J=9 Hz, 1 H); 3.67 (m, 1 H); 3.70 (s, 3 H); 3.84 (d, J=10 Hz, 1 H); 3.93 (m, 2 H); 4.04 (d, J=4 Hz, 1 H); 4.17 (t, J=9 Hz, 1 H); 4.22 (t, J=8Hz, 1 H); 4.31 (br s, 1 H); 4.78(m, 1 H); 4.96 (d, J=4 Hz, 1 H); 7.48 (s, 1 H); 8.14 (br s, 1 H); 8.83 (s, 1 H); 8.92 (br s, 1 H). MS (method B) m/z 440 [M+1]+; t=1.5 min - Example 13 (absolute configuration unknown)

### Example 14: 2-[[1-(Methylsulfonylmethyl)-3-(oxetan-3-yloxy)pyrazol-4-yl]amino]-7-[(3R,4R)-4-methyltetrahydrofuran-3-yl]pyrrolo[2,3-d]pyrimidine-6-carbonitrile

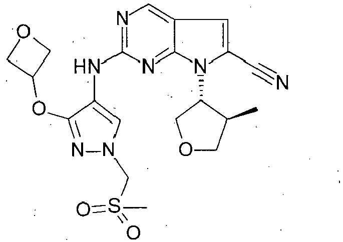

Generally following the procedure described in Step 5 of Example 1, 2-methylsulfonyl-7-[(3R,4R)-4-methyltetrahydrofuran-3-yl]pyrrolo[2,3-d]pyrimidine-6-carbonitrile (100 mg) and N-(1-((methylsulfonyl)methyl)-3-(oxetan-3-yloxy)-1H-pyrazol-4-yl)formamide (48 mg) (Intermediate 13) gave 2-[[1-(methylsulfonylmethyl)-3-(oxetan-3-yloxy)pyrazol-4-yl]amino]-7-[(3R,4R)-4-methyltetrahydrofuran-3-yl]pyrrolo[2,3-d]pyrimidine-6-carbonitrile (129 mg) (Example 14).

¹H NMR (400 MHz, DMSO-d6) δ in ppm: 1.04 (d, J=7 Hz, 3 H); 2.88 (m, 1 H); 2.99 (s, 3 H); 3.45 (t, J=9 Hz, 1 H); 4.15 (t, J=9 Hz, 1 H); 4.20-4.30 (m, 2 H); 4.5 (m, 2 H); 4.80-4.90 (m, 3 H); 5.34-5.54 (m, 3 H); 7.52 (s, 1 H); 8.27 (br s, 1 H); 8.88 (s, 1 H); 9.20 (br s, 1 H). MS (method B) m/z 474 [M+1]+; t= 1.36 min.

Example 15 and Example 16: 2-((1-(Methyl-d3)-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile and 2-((1-(methyl-d3)-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile

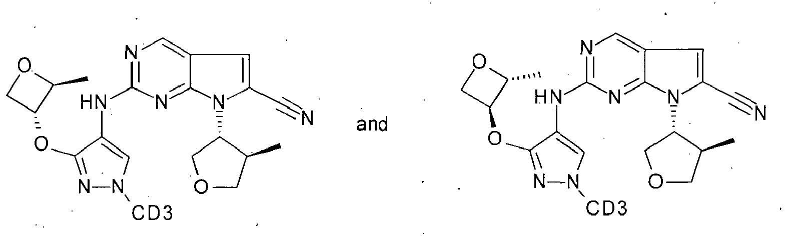

Generally following the procedure described in Step 5 of Example 1, 2-methylsulfonyl-7-[(3R,4R)-4-methyltetrahydrofuran-3-yl]pyrrolo[2,3-d]pyrimidine-6-carbonitrile (245 mg) and a racemic mixture of N-(1-(methyl-d3)-3-(((2S,3R)-2-methyloxetan-3-yl)oxy)-1H-pyrazol-4-yl)formamide and N-(1-(methyl-d3)-3-(((2R,3S)-2-methyloxetan-3-yl)oxy)-1H-pyrazol-4-yl)formamide (189 mg) (Intermediate 21) gave an isomeric mixture of 2-((1-(methyl-d3)-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile and 2-((1-(methyl-d3)-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile (267 mg) (Referred herein as 2-((1-(methyl-d3)-3-(((trans)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile).

Chiral separation performed on 262 mg of the isomeric mixture gave 113 mg of the first eluting isomer and 96 mg of the second eluting isomer (conditions: column Chiralpak AD-H, 20 µm, 350x76 mm; liquid phase: heptane 50/ethyl alcohol 50 /triethylamine 0.1%; flow rate: 400 mL/min then column Amylose-1.5 µm, 250x30 mm; liquid phase: heptane 80/ethyl alcohol 20/ triethylamine 0.1%; flow rate: 45 mL/min).

Peak 1 (1^{st} isomer): ¹H NMR (400 MHz, DMSO-d6) δ in ppm: 1.05 (d, J=7 Hz, 3 H); 1.41 (d, J=6 Hz, 3 H); 2.92 (dquin, J=8 and 15 Hz, 1 H); 3.46 (t, J=9 Hz, 1 H); 4.10-4.24 (m, 2 H); 4.30 (m, 1 H); 4.41 (dd, J=6 and 7 Hz, 1 H); 4.65 (t, J=6 Hz, 1 H); 4.72-4.83 (m, 2 H); 4.89 (m, 1 H); 7.47 (s, 1 H); 8.07 (br s, 1 H); 8.82 (s, 1 H); 8.96 (br s, 1 H). MS (method B) m/z 413 [M+1]+; t=1.5 min - Example 15 (absolute configuration unknown)

Peak 2 (2^{nd} isomer): ¹H NMR (400 MHz, DMSO-d6) δ in ppm: 1.05 (d, J=7 Hz, 3 H); 1.41 (d, J=6 Hz, 3 H); 2.92 (dquin, J=7 and 15 Hz, 1 H); 3.46 (t, J=9 Hz, 1 H); 4.16 (q, J=9 Hz, 2 H); 4.29 (m, 1 H); 4.40 (dd, J=6 and 7 Hz, 1 H); 4.66 (t, J=7 Hz, 1 H); 4.71 - 4.84 (m, 2 H); 4.89 (q, J=6 Hz, 1 H); 7.47 (s, 1 H); 8.06 (br s, 1 H); 8.82 (s, 1 H); 8.95 (br s, 1 H). MS (method B) m/z 413 [M+1]+; t=1.49 min - Example-16 (absolute configuration unknown)

### Example 17 and Example 18: 2-((3-(((R)-1-Acetyl-2,2-dimethylazetidin-3-yl)oxy)-1-methyl-1H-pyrazol-4-yl)amino)-7-((3R,4R)-4-methyltetrahydrofuran-3-yl)-7H-pyrrolo[2,3-d]pyrimidine-6-carbonitrile and 2-((3-(((S)-1-acetyl-2,2-dimethylazetidin-3-yl)oxy)-1-methyl-1H-pyrazol-4-yl)amino)-7-((3R,4R)-4-methyltetrahydrofuran-3-yl)-7H-pyrrolo[2,3-d]pyrimidine-6-carbonitrile

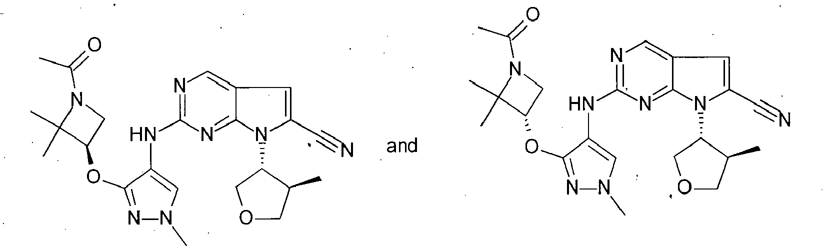

Generally following the procedure described in Step 5 of Example 1, 2-methylsulfonyl-7-[(3R,4R)-4-methyltetrahydrofuran-3-yl]pyrrolo[2,3-d]pyrifmidine-6-carbonitrile (110 mg) and a racemic mixture of (R)-N-(3-((1-acetyl-2,2-dimethylazetidin-3-yl)oxy)-1-methyl-1H-pyrazol-4-yl)formamide and (S)-N-(3-((1-acetyl-2,2-dimethylazetidin-3-yl)oxy)-1-methyl-1H-pyrazol-4-yl)formamide (87 mg) (Intermediate 32) gave an isomeric mixture of 2-((3-(((R)-1-acetyl-2,2-dimethylazetidin-3-yl)oxy)-1-methyl-1H-pyrazol-4-yl)amino)-7-((3R,4R)-4-methyltetrahydrofuran-3-yl)-7H-pyrrolo[2,3-d]pyrimidine-6-carbonitrile and 2-((3-(((S)-1-acetyl-2,2-dimethylazetidin-3-yl)oxy)-1-methyl-1H-pyrazol-4-yl)amino)-7-((3R,4R)-4-methyltetrahydrofuran-3-yl)-7H-pyrrolo[2,3-d]pyrimidine-6-carbonitrile (99 mg) (Referred herein as 2-((3-((1-acetyl-2,2-dimethylazetidin-3-yl)oxy)-1-methyl-1H-pyrazol-4-yl)amino)-7-((3R,4R)-4-methyltetrahydrofuran-3-yl)-7H-pyrrolo[2,3-d]pyrimidine-6-carbonitrile).

Chiral separation performed on 94 mg of the isomeric mixture gave 34 mg of the first eluting isomer and 35 mg of the second eluting isomer (conditions: column Chiralcel OD-H, 5 µm, 250x4.6 mm; liquid phase: heptane 50/ethyl alcohol 50/triethylamine 0.1%; flow rate: 42 mL/min).

Peak 1 (1^{st} isomer): ¹H NMR (400 MHz, DMSO-d6) δ in ppm: 1.05 (d, J=7 Hz, 3 H); 1.37 (s, 3 H); 1.52 (s, 3 H); 1.70 (s, 3 H); 2.92 (spt, J=7 Hz, 1 H); 3.47 (t, J=9 Hz, 1 H); 3.67 (s, 3 H); 3.96 (dd, J=5 and 10 Hz, 1 H); 4.16 (t, J=9 Hz, 1 H); 4.22 (td, J=3 and 8 Hz, 1 H); 4.26-4.40 (m, 2 H); 4.76 (m, 1 H); 4.80 (m, 1 H); 7.47 (s, 1 H); 8.10 (br s, 1 H); 8.83 (s, 1 H); 8.96 (br s, 1 H). MS (method B) m/z 465 [M+1]+; t=1.48 min - Example 17 (absolute configuration unknown)

Peak 2 (2^{nd} isomer): ¹H NMR (400 MHz, DMSO-d6) δ in ppm: 1.05 (d, J=7 Hz, 3 H); 1.37 (s, 3 H); 1.52 (s, 3 H); 1.70 (s, 3 H); 2.92 (spt, J=7 Hz, 1 H); 3.47 (t, J=9 Hz, 1 H); 3.67 (s, 3 H); 3.96 (dd, J=5 and 10 Hz, 1 H); 4.16 (t, J=9 Hz, 1 H); 4.22 (td, J=3 and 8 Hz, 1 H); 4.26-4.40 (m, 2 H); 4.76 (m, 1 H); 4.80 (m, 1 H); 7.47 (s, 1 H); 8.13 (br s, 1 H); 8.83 (s, 1 H); 8.98 (br s, 1 H). MS (method B) m/z 465 [M+1]+; t=1,48 min - Example 18 (absolute configuration unknown)

### Example 19: 2-((1-(Methyl-d3)-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile

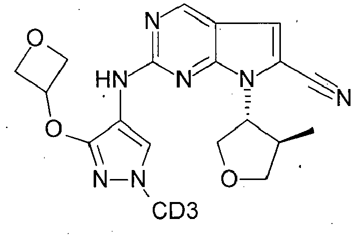

Generally following the procedure described in Step 5 of Example 1, 2-methylsulfonyl-7-[(3R,4R)-4-methyltetrahydrofuran-3-yl]pyrrolo[2,3-d]pyrimidine-6-carbonitrile (40 mg) and N-(1-(methyl-d3)-3-(oxetan-3-yloxy)-1H-pyrazol-4-yl)formamide (32 mg) (Intermediate 26) gave 2-((1-(methyl-d3)-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile (35 mg).
¹H NMR (400 MHz, DMSO-d6) δ in ppm: 1.05 (d, J=6.8 Hz, 3 H), 2.92 (m, 1 H), 3.47 (t, J=8.8 Hz, 1 H), 4.12-4.35 (m, 3 H), 4.57 (m, 2 H), 4.72-4.84 (m, 3 H), 5.31 (tt, J=6.0 and 5.0 Hz, 1 H), 7.46 (s, 1 H), 8:04 (br s, 1 H), 8.82 (s, 1 H), 8.92 (br s, 1 H). MS (method D) m/z 399 [M+1]+; t=1.03 min.

### Example 20: 2-((4-(2-Hydroxypropan-2-yl)-2-(oxetan-3-yloxy)phenyl)amino)-7-((3R,4R)-4-methyltetrahydrofuran-3-yl)-7H-pyrrolo[2,3-d]pyrimidine-6-carbonitrile

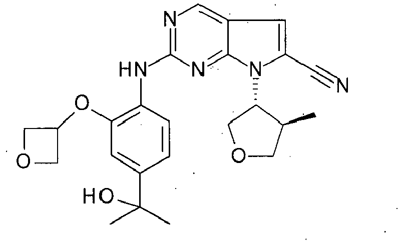

### Step 1: Preparation of methyl 4-((6-cyano-7-((3R,4R)-4-methyltetrahydrofuran-3-yl)-7H-pyrrolo[2,3-d]pyrimidin-2-yl)amino)-3-(oxetan-3-yloxy)benzoate

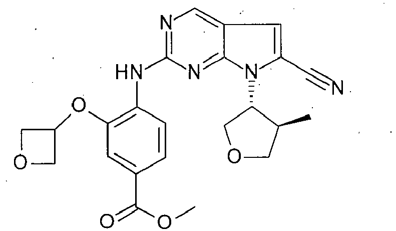

Generally following the procedure described in Step 5 of Example 1, 2-methylsulfonyl-7-[(3R,4R)-4-methyltetrahydrofuran-3-yl]pyrrolo[2,3-d]pyrimidine-6-carbonitrile (100 mg) and methyl 4-formamido-3-(oxetan-3-yloxy)benzoate (90 mg) (Intermediate 35) gave methyl 4-((6-cyano-7-((3R,4R)-4-methyltetrahydrofuran-3-yl)-7H-pyrrolo[2,3-d]pyrimidin-2-yl)amino)-3-(oxetan-3-yloxy)benzoate (146 mg). MS (method A) m/z 450 [M+1]+; t=2.54 min.

### Step 2: Preparation of 2-((4-(2-hydroxypropan-2-yl)-2-(oxetan-3-yloxy)phenyl)amino)-7-((3R,4R)-4-methyltetrahydrofuran-3-yl)-7H-pyrrolo[2,3-d]pyrimidine-6-carbonitrile

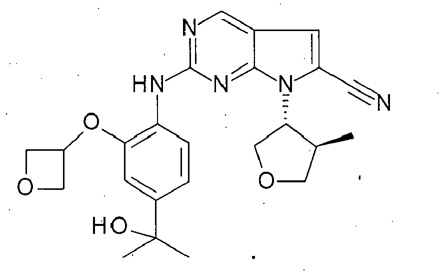

467 µL (10 eq.) of methyl magnesium bromide 3M in tetrahydrofuran was added to a solution of 63 mg (1 eq.) of methyl 4-((6-cyano-7-((3R,4R)-4-methyltetrahydrofuran-3-yl)-7H-pyrrolo[2,3-d]pyrimidin-2-yl)amino)-3-(oxetan-3-yloxy)benzoate in 4.5 mL of tetrahydrofuran at 0°C. The reaction mixture was stirred for 30 minutes at 0°C then 4 eq. of methyl magnesium bromide 3M in tetrahydrofuran was added and the reaction mixture stirred at 0°C for 30 minutes. The reaction mixture was quenched with a saturated aqueous solution of ammonium chloride and extracted with ethyl acetate. The organic layer was washed with 50 mL of water and 50 mL of brine, dried over magnesium sulfate, and concentrated under reduced pressure. The residue was purified on silica, eluting with a mixture of heptane/AcOEt/7N methanolic ammonia solution (80/18/2) to afford 35 mg of 2-((4-(2-hydroxypropan-2-yl)-2-(oxetan-3-yloxy)phenyl)amino)-7-((3R,4R)-4-methyltetrahydrofuran-3-yl)-7H-pyrrolo[2,3-d]pyrimidine-6-carbonitrile as a yellow solid.
¹H NMR (400 MHz, DMSO-d6) δ in ppm: 1.08 (d, J=7 Hz, 3 H); 1.42 (s, 6 H); 2.95 (m, 1 H); 3.48 (t, J=9 Hz, 1 H); 4.12-4.31 (m, 3 H); 4.63 (dd, J=5 and 7 Hz, 2 H); 4.86 (q, J=8 Hz, 1 H); 4.93 (t, J=7 Hz, 2 H); 4.98 (s, 1 H); 5.35 (m, 1 H); 6.73 (d, J=2 Hz, 1 H); 7.07 (dd, J=2 and 8 Hz, 1 H); 7.55 (s, 1 H); 8.25 (d, J=8 Hz, 1 H); 8.43 (s, 1 H); 8.91 (s, 1 H). MS (method B) m/z 450 [M+1]+; t=1.54 min.

### Example 21 and Example 22: 2-((1-Methyl-3-(((R)-1,1,1-trifluoropropan-2-yl)oxy)-1H-pyrazol-4-yl)amino)-7-((3R,4R)-4-methyltetrahydrofuran-3-yl)-7H-pyrrolo[2,3-d]pyrimidlne-6-carbonitrile and 2-((1-methyl-3-(((S)-1,1,1-trifluoropropan-2-yl)oxy)-1H-pyrazol-4-yl)amino)-7-((3R,4R)-4-methyltetrahydrofuran-3-yl)-7H-pyrrolo[2,3-d]pyrimidine-6-carbonitrile

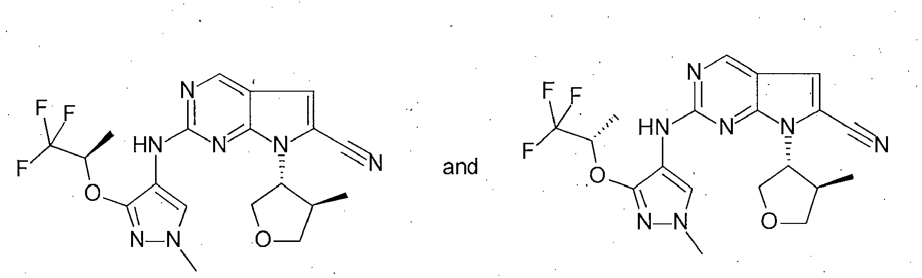

Generally following the procedure described in Step 5 of Example 1, 2-methylsulfonyl-7-[(3R,4R)-4-methyltetrahydrofuran-3-yl]pyrrolo[2,3-d]pyrimidine-6-carbonitrile (100 mg) and N -(1-methyl-3-((1,1,1-trifluoropropan-2-yl)oxy)-1H-pyrazol-4-yl)formamide (82 mg) (Intermediate 7) gave an isomeric mixture of 2-((1-methyl-3-(((R)-1,1,1-trifluoropropan-2-yl)oxy)-1H-pyrazol-4-yl)amino)-7-((3R,4R)-4-methyltetrahydrofuran-3-yl)-7H-pyrrolo[2,3-d]pyrimidine-6-carbonitrile and 2-((1-methyl-3-(((S)-1,1,1-trifluoropropan-2-yl)oxy)-1H-pyrazol-4-yl)amino)-7-((3R,4R)-4-methyltetrahydrofuran-3-yl)-7H-pyrrolo[2,3-d]pyrimidine-6-carbonitrile (90 mg) (Referred herein as 2-((1-methyl-3-((1,1,1-trifluoroprbpan-2-yl)oxy)-1H-pyrazol-4-yl)amino)-7-((3R,4R)-4-methyltetrahydrofuran-3-yl)-7H-pyrrolo[2,3-d]pyrimidine-6-carbonitrile).

Chiral separation performed on 65 mg of the isomeric mixture gave 31 mg of the first eluting isomer and 35 mg of the second eluting isomer (conditions: column ChiralpaK AY-H, 5 µm, 250x30 mm; liquid phase: step gradient from heptane 70/ethyl alcohol 30/triethylamine 0.1% to heptane 30/ethyl alcohol 70/triethylamine 0.1%; flow rate: 40mL/min).

Peak 1 (1^{st} isomer): ¹H NMR (400 MHz, DMSO-d6) δ in ppm: 1.05 (d, J=7 Hz, 3 H); 1.43 (d, J=6 Hz, 3 H); 2.91 (spt, J=7 Hz, 1 H); 3.46 (t, J=9 Hz, 1 H); 3.71 (s, 3 H); 4.14 (t, J=8 Hz, 1 H); 4.21 (t, J=8 Hz, 1 H); 4.29 (m, 1 H); 4.79 (m, 1 H); 5.23 (spt, J=7 Hz, 1 H); 7.48 (s, 1 H); 8.08 (br s, 1 H); 8.82 (s, 1 H); 8.86 (br s, 1 H). MS (method B) m/z 436 [M+1]+; t=1.76 min - Example 21 (absolute configuration unknown)

Peak 2 (2^{nd} isomer): ¹H NMR (400 MHz, DMSO-d6) δ in ppm: 1.05 (d, J=7 Hz, 3 H); 1.43 (d, J=6 Hz, 3 H); 2.91 (spt, J=7 Hz, 1 H); 3.46 (t, J=9 Hz, 1 H); 3.70 (s, 3 H); 4.04 - 4.34 (m, 3 H); 4.75 (m, 1 H); 5.23 (spt, J=7 Hz, 1 H); 7.47 (s, 1 H); 8.06 (br s, 1 H); 8.82 (s, 1 H); 8.86 (s large, 1 H). MS (method B) m/z 436 [M+1]+; t=1.76 min - Example 22 (absolute configuration unknown)

### Example 23 and Example 24: 2-((1-(Methyl-d3)-3-(((2R,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile and 2-((1-(methyl-d3)-3-(((2S,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbon itrile

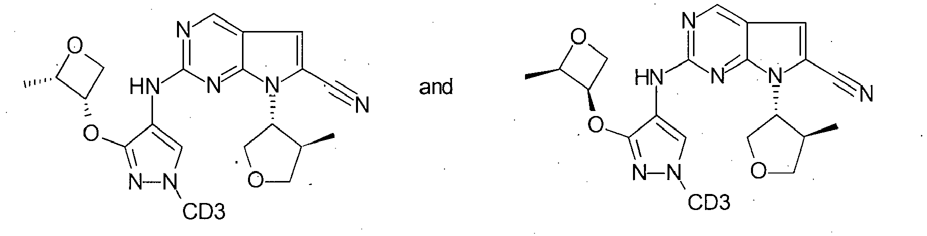

Generally following the procedure described in Step 5 of Example 1, 2-methylsulfonyl-7-[(3R,4R)-4-methyltetrahydrofuran-3-yl]pyrrolo[2,3-d]pyrimidine-6-carbonitrile (245 mg) and a racemic mixture of N-(1-(methyl-d3)-3-(((2S,3S)-2-methyloxetan-3-yl)oxy)-1H-pyrazol-4-yl)formamide and N-(1-(methyl-d3)-3-(((2R,3R)-2-methyloxetan-3-yl)oxy)-1H-pyrazol-4-yl)formamide (189 mg) (Intermediate 22) gave an isomeric mixture of 2-((1-(methyl-d3)-3-(((2R,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile and 2-((1-(methyl-d3)-3-(((2S,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile (235 mg) (Referred herein as 2-((1-(methyl-d3)-3-(((cis)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile).

Chiral separation performed on 230 mg of the isomeric mixture gave 100 mg of the first eluting isomer and 102 mg of the second eluting isomer (conditions: column ChiralpaK AD, 20 µm, 250X30 mm; liquid phase: step gradient from heptane 60/ethyl alcohol 40; flow rate: 400 mL/min).

Peak 1 (1^{st} isomer): ¹H NMR (400 MHz, DMSO-d6) δ in ppm: 1,05 (d, J=7 Hz, 3 H); 1,30 (d, J=6 Hz, 3 H); 2,93 (spt, J=7 Hz, 1 H); 3,47 (t, J=9 Hz, 1 H); 4,16 (t, J=10 Hz, 1 H); 4,21 (t, J=8 Hz, 1 H); 4,32 (m, 1 H); 4,46 (dd, J=5 and 7 Hz, 1 H); 4,74 (dd, J=6 and 7 Hz, 1 H); 4,81 (m, 1 H); 5,04 (quin, J=6 Hz, 1 H); 5,30 (q, J=6 Hz, 1 H); 7,48 (s, 1 H); 8,10 (br s , 1 H); 8,83 (s, 1 H); 8,99 (br s, 1 H). MS (method B) m/z 413 [M+1]+; t=1.46 min - Example 23 (absolute configuration unknown)

Peak 2 (2^{nd} isomer): ¹H NMR (400 MHz, DMSO-d6) δ in ppm: 1.05 (d, J=7 Hz, 3 H); 1.30 (d, J=6 Hz, 3 H); 2.90 (spt, J=7 Hz, 1 H); 3.46 (t, J=9 Hz, 1 H); 4.17 (m, 2 H); 4.3.1 (m, 1 H); 4.46 (dd, J=5 and 7 Hz, 1 H); 4.72 (dd, J=6 and 7 Hz, 1 H); 4.79 (m, 1 H); 5.03 (quin, J=6 Hz, 1 H); 5.29 (td, J=5 and 6 Hz, 1 H); 7.47 (s, 1 H); 8.09 (br s, 1 H); 8.83 (s, 1 H); 8.97 (br s, 1 H). MS (method B) m/z 413 [M+1]+; t=1.46 min - Example 24 (absolute configuration unknown)

Generally following the procedure described in Example 1, reacting 2-methylsulfonyl-7-[(3R,4R)-4-methyltetrahydrofuran-3-yl]pyrrolo[2,3-d]pyrimidine-6-carbonitrile with appropriate formamides gave Example 25 to 43 in Table I.

**Table I**

| Example | Name | Formamide | NMR ¹H NMR (400 MHz, DMSO-d6) | LC/MS MH+; t, Method |
|---|---|---|---|---|
| 25 | 2-((3-lsopropoxy-1-(2,2,2-trifluoroethyl)-1H-pyrazol-4-yl)amino)-7-((3R,4R)-4-methyltetrahydrofuran-3-yl)-7H-pyrrolo[2,3-d]pyrimidine-6-carbonitrile | Intermediate 8 | 1.04 (d, J=7 Hz, 3 H); 1.30 (d, J=6 Hz, 6 H); 2.87 (m, 1 H); 3.45 (t, J=9 Hz, 1 H); 4.12-4.33 (m, 3 H); 4.72-4,95 (m, 4 H); 7.50 (s, 1 H); 8.20 (br s, 1 H); 8.75 (br s, 1 H); 8.85 (s, 1 H) | 450; 1.95 B |
| 26 | 2-((3-lsopropoxy-1-me thyl-1H-pyrazol-4-yl) amino)-7-((3R,4R)-4-methyltetrahydrofuran-3-yl)-7H-pyrrolo[2,3-d]pyrimidine-6-carbonitrile | Intermediate 38 | 1.05 (d, J=7 Hz, 3 H); 1.27 (d, J=6 Hz, 6 H); 2.93 (spt, J=7 Hz, 1 H); 3.47 (t, J=9 Hz, 1 H); 3.67 (s, 3 H); 4.16 (t, J=9 Hz, 1 H); 4.21 (t, J=8 Hz, 1 H); 4.34 (m, 1 H); 4.60-4,90 (m, 2 H); 7.46 (s, 1 H); 8.03 (br s, 1 H); 8.53 (br s, 1 H); 8.81 (s, 1 H) | 382; 1.23 D |
| 27 | 2-((1-(2-Cyanopropan-2-yl)-3-(oxetan-3-yloxy)-1H-pyrazol-4-yl)amino)-7-((3R,4R)-4-methyltetrahydrofuran-3-yl)-7H-pyrrolo[2,3-d] pyrimidine-6-carbonitrile | Intermediate 27 | 1.04 (d, J=6.8 Hz, 3 H) 1.92 (s, 3 H), 1.93 (s, 3 H), 2.87 (dt, J=14.6, 7.3 Hz, 1 H), 3.43 (t, J=7.4 Hz, 1 H), 4.17 (t, J=8.8 Hz, 2 H), 4.37 (m, 1 H), 4.60 (m, 2 H), 4.75 (m, 1 H), 4.84 (ddd, J=7.3, 6.3, 1.5 Hz, 2 H), 5.41 (ddd, J=11.2, 6.1, 5.1 Hz, 1 H), 7.49 (s, 1 H), 8.23 (br s, 1 H), 8.87 (s, 1 H), 9.06 (br s, 1 H) | 449; 1.24 D |
| 28 | 2-((3-Cyclopropoxy-1-(methyl-d3)-1H-pyrazol-4-yl)amino)-7-((3R,4R)-4-methyltetrahydrofuran-3-yl)-7H-pyrrolo[2,3-d]pyrimidine-6-carbonitrile | Intermediate 29 | 0.60 - 0.89 (m, 4 H), 1.06 (d, J=6.7 Hz, 3 H), 2.93 (m, 1 H), 3.48 (t, J=8.7 Hz, 1 H), 4.06 (m, 1 H), 4.12-4.33 (m, 3 H), 4.77 (br q, J=7.7 Hz, 1 H), 7.45 (s, 1 H), 8.04 (br s, 1 H), 8.72 (br s, 1 H), 8.80 (s, 1 H) | 383; 1.27 D |
| 29 | 2-((1-(2-Cyanopropan-2-yl)-3-isopropoxy-1H-pyrazol-4-yl)amino)-7-((3R,4R)-4-methyltetrahydrofuran-3-yl)-7H-pyrrolo[2,3-d]pyrimidine-6-carbonitrile | Intermediate 31 | 1.04 (d, J=6.8 Hz, 3 H), 1.32 (d, J=6.1 Hz, 6 H), 1.94(s, 3 H), 1.95 (s, 3 H), 2.88 (m, 1 H), 3.44 (t, J=8.6 Hz, 1 H), 4.13 - 4.22 (m, 2 H), 4.38 (m, 1 H), 4.76 (m, 1 H), 4.82 (sep, J=6.1 Hz, 1 H), 7.49 (s, 1 H), 8.20 (br s, 1 H), 8.69 (br s, 1 H), 8.85 (s, 1 H) | 435; 1.55 D |
| 30 | 2-((3-Cyclopropoxy-1-(oxetan-3-yl)-1H-pyrazol-4-yl)amino)-7-((3R,4R)-4-methyltetrahydrofuran-3-yl)-7H-pyrrolo[2,3-d]pyrimidine-6-carbonitrile | Intermediate 11 | 0.64 - 0.75 (m, 4 H), 1.05 (d, J=6.7 Hz, 3 H), 2.92 (m, 1 H), 3.46 (t, J=8.7 Hz, 1 H), 4.12 - 4.24 (m, 3 H), 4.33 (m, 1 H), 4.76 (m, 1 H), 4.86 - 4.96 (m, 4 H), 5.37 (quin, J=7.1 Hz, 1 H), 7.46 (s, 1 H), 8.26 (br s, 1 H), 8.80 (br s, 1 H), 8.82 (s, 1 H) | 422; 1.28 D |
| 31 | 2-((3-Isopropoxy-1-(oxetan-3-yl)-1H-pyrazol-4-yl)amino)-7-((3R,4R)-4-methyltetrahydrofuran-3-yl)-7H-pyrrolo[2,3-d]pyrimidine-6-carbonitrile | Intermediate 12 | 1.05 (d, J=6.5 Hz, 3 H), 1.32 (d, J=6.3 Hz, 6 H), 2.92 (m, 1 H), 3.46 (t, J=8.7 Hz, 1 H), 4.13 -4.23 (m, 2 H), 4.32 (m, 1 H), 4.74 - 4.92 (m, 6 H), 5.34 (m, 1 H), 7.47 (s, 1 H), 8.22 (br s, 1 H), 8.61 (br s, 1 H), 8.83 (s, 1 H) | 424; 1.39 D |
| 32 | 2-((3-(2-Hydroxy-2-methylpropoxy)-1-methyl-1H-pyrazol-4-yl)amino)-7-((3R,4R)-4-methyltetrahydrofuran-3-yl)-7H-pyrrolo[2,3-d]pyrimidine -6-carbonitrile | Intermediate 6 | 1.06 (d, J=7 Hz, 3 H); 1.17 (s, 6 H); 2.94 (m, 1 H); 3.49 (t, J=9 Hz, 1 H); 3.67 (s, 3 H); 3.86 (s, 2 H); 4.20 (t, J=9 Hz, 1 H); 4.26 (t, J=8 Hz, 1 H); 4.41 (br s, 1 H); 4.80 (m large, 2 H); 7.48 (s, 1 H); 8.25 (br s, 1 H); 8.85 (s, 1 H); 9.22 (br s, 1 H) | 412; 1.46 B |
| 33 | 2-((1-(Methoxymethyl) -3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile | Intermediate 14 | 1.04 (d, J=7 Hz, 3 H); 2.92 (m, 1 H); 3.22 (s, 3 H); 3.45 (t, J=9 Hz, 1 H); 4.12 -4.22 (m, 2 H); 4.32 (m, 1 H); 4.60 (m, 2 H); 4.78 (m, 1 H); 4.84 (t, J=7 Hz, 2 H); 5.16 (m, 2 H); 5.38 (m, 1 H); 7.49 (s, 1 H); 8.32 (br s, 1 H); 8.86 (s, 1 H); 9.08 (br s, 1 H) | 426; 1.45 B |
| 34 | 2-((1-(Methylsulfonyl)-3-(oxetan-3-yloxy)-1H-pyrazol-4-yl)amino)-7 -((3R,4R)-4-methyltetrahydrofuran-3-yl)-7H-pyrrolo[2,3-d]pyrimidine-6-carbonitrile | Intermediate 18 | 1.08 (d, J=6.5 Hz, 3 H), 2.97 (m, 1 H), 3.29 (s, 3 H), 3.44 (t, J=8.9 Hz, 1 H), 4.14 (t, J=8.9 Hz, 1 H) 4.25 - 4.34 (m, 2 H), 4.67 (m, 2 H), 4.81 (m, 1 H), 4.90 (m, 2 H), 5.54 (tt, J=6.0, 4.8 Hz, 1 H), 7.55 (s, 1 H), 8.54 (s, 1 H), 8.96 (s, 1 H), 9.54 (br, 1 H) | 460; 1.13 D |
| 35 | 2-((3-Cyclopropoxy-1-(methoxymethyl)-1H-pyrazol-4-yl)amino)-7-((3R,4R)-4-methyltetrahydrofuran-3-yl)-7H-pyrrolo[2,3-d]pyrimidine-6-carbonitrile | Intermediate 15 | 0.63 - 0.75 (m, 4 H); 1.05 (d, J=7 Hz, 3 H); 2.93 (spt, J=8 Hz, 1 H); 3.26 (s, 3 H); 3.46 (t, J=9 Hz, 1 H); 4.09 - 4,27 (m, 3 H); 4.32 (br s, 1 H); 4.78 (m, 1 H); 5.16 - 5,26 (m, 2 H); 7.49 (s, 1 H); 8.32 (br s, 1 H); 8.84 (s, 1 H); 8.87 (br s, 1 H) | 410; 1.58 B |
| 36 | 2-((3-Cyclopropoxy-1-((methylsulfonyl)methyl)-1H-pyrazol-4-yl)amino)-7-((3R,4R)-4-methyltetrahydrofuran-3-yl)-7H-pyrrolo[2,3-d]pyrimidine-6-carbonitrile | Intermediate 16 | 0.65 0,77 (m, 4 H); 1.04 (d, J=7 Hz, 3 H); 2.89 (m, 1 H); 3.05 (s, 3 H); 3.45 (t, J=9 Hz, 1 H); 4.10 - 4.30 (m, 4 H); 4.87 (q, J=8 Hz, 1 H); 5.40 -5.60 (m, 2 H); 7.50 (s, 1 H); 8.27 (br s, 1 H); 8.85 (s, 1 H); 8.99 (br s, 1 H) | 458; 1.49 B |
| 37 | 2-((1-Cyclopropyl-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile | Intermediate 24 | 0.88 (m, 2 H); 0.96 (m, 2 H); 1.05 (d, J=7 Hz, 3 H); 2.94 (spt, J=7 Hz, 1 H); 3.43 - 3.53 (m, 2 H); 4.11 - 4.24 (m, 2 H); 4.32 (m, 1 H); 4.53 - 4.61 (m, 2 H); 4.71 (m, 1 H); 4.82 (t, J=7 Hz, 2 H); 5.33 (quin, J=6 Hz, 1 H); 7.48 (s, 1 H); 8.10 (br s, 1 H); 8.83 (s, 1 H); 8.99 (br s, 1 H) | 422; 3.0 C |
| 38 | 2-((1-Ethyl-3-(oxetan-3-yloxy)-1H-pyrazol-4-yl)amino)-7-((3R,4R)-4 -methyltetrahydrofuran-3-yl)-7H-pyrrolo[2,3-d]pyrimidine-6-carbonitrile | Intermediate 44 | 1.05 (d, J=7 Hz, 3 H); 1.32 (t, J=7 Hz, 3 H); 2.92 (spt, J=7 Hz, 1 H); 3.46 (t, J=9 Hz, 1 H); 3.93 (q, J=7 Hz, 2 H); 4.11 - 4.21 (m, 2 H); 4.31 (t, J=6 Hz, 1 H); 4.58 (dt, J=4 and 8 Hz, 2 H); 4.73 (m, 1 H); 4.81 (t, J=7 Hz, 2 H); 5.33 (quin, J=6 Hz, 1 H); 7.46 (s, 1 H); 8.07 (br s, 1 H); 8.82 (s, 1 H); 8.90 (br s, 1 H) | 410; 1.22 D |
| 39 | 2-[[1-(Methoxymethyl)-3-[(2R,3S)-2-methyloxetan-3-yl]oxy-pyrazol-4-yl]amino]-7-[(3R,4R)-4-methyltetrahydrofuran-3-yl]pyrrolo[2,3-d]pyrimidine-6-carbonitrile and 2-[[1-(methoxymethyl)-3-[(2S,3R)-2-methyloxetan-3-yl]oxy-pyrazol-4-yl]amino]-7-[(3R,4R)-4-methyltetrahydrofuran-3-yl]pyrrolo[2,3-d]pyrimidine-6-carbonitrile (Referred herein as 2-[[1-(methoxymethyl)-3-[(trans)-2-methyloxetan-3-yl]oxy-pyrazol-4-yl]amino]-7-[(3R,4R)-4-methyltetrahydrofuran-3-yl]pyrrolo[2,3-d]pyrimidine-6-carbonitrile) | Intermediate 37 | 1.05 (d, J=7 Hz, 3 H); 1.44 (dd, J=2 and 6 Hz, 3 H); 2.92 (dquin, J=7 and 15 Hz, 1 H); 3.24 (s, 3 H); 3.46 (t, J=9 Hz, 1 H); 4.12 -4.25 (m, 2 H); 4.32 (t, J=8 Hz, 1 H); 4.44 (dt, J=5 and 7 Hz, 1 H); 4.68 (t, J=7 Hz, 1 H); 4.73 - 4.87 (m, 2 H); 4.96 (q, J=6 Hz, 1 H); 5.11 - 5.22 (m, 2 H); 7.49 (s, 1 H); 8.29 (br s, 1 H); 8.86 (s, 1 H); 9.01 (br s, 1 H) | 440; 1.54 B |
| 40 | 2-[[1-Methyl-3-[[(7R)-2-oxaspiro[3.3]heptan-7-yl]oxy]pyrazol-4-yl]amino]-7-[(3R,4R)-4-methyltetrahydrofuran-3-yl]pyrrolo[2,3-d]pyrimidine-6-carbonitrile and 2-[[1-methyl-3-[[(7S)-2-oxaspiro[3.3]heptan-7-yl]oxy]pyrazol-4-yl]amino]-7-[(3R,4R)-4-methyltetrahydrofuran-3-yl]pyrrolo[2,3-d]pyrimidine-6-carbonitrile (Referred herein as 2-[[1-methyl-3-[(2-oxaspiro[3.3]heptan-7-yl)oxy]pyrazol-4-yl]amino]-7-[(3R,4R)-4-methyltetrahydrofuran-3-yl]pyrrolo[2,3-d]pyrimidine-6-carbonitrile) | Intermediate 36 | 1.05 (d, J=7 Hz, 3 H); 1.73 (m, 1 H); 1.79 (m, 1 H); 2.05 (m, 1 H); 2.16 (m, 1 H); 2.94 (m, 1 H); 3.48 (t, J=9 Hz, 1 H); 3.68 (s, 3 H); 4.17 (t, J=9 Hz, 1 H); 4.23 (t, J=8 Hz, 1 H); 4.33 (br s, 1 H); 4.41 (m, 2 H); 4.73 (m, 1 H); 4.76 - 4.87 (m, 2 H); 5.08 (br s, 1 H); 7.47 (s, 1 H); 8.15 (br s, 1 H); 8.82 (s, 1 H); 9.01 (br s, 1 H) | 436; 1.53 B |
| 41 | 2-((2-Cyclopropoxy-4-((dimethylamino)methyl)p henyl)amino)-7-((3R,4R)-4-methyltetrahydrofuran-3-yl)-7H-pyrrolo[2,3-d]pyrimidine-6-carbonitrile | Intermediate 10 | 0.65 - 0.94 (m, 4 H); 1.07 (d, J=7 Hz, 3 H); 2.17 (s, 6 H); 2.95 (m, 1 H); 3.38 (m, 2 H); 3.47 (t, J=8 Hz, 1 H); 3.92 (m, 1 H); 4.03 - 4.31 (m, 3 H); 4.84 (q, J=7 Hz, 1 H); 6.90 (d, J=8 Hz, 1 H); 7.26 (s, 1 H); 7.53 (s, 1 H); 8.16 (s, 1 H); 8.25 (d, J=8 Hz, 1 H); 8.89 (s, 1 H) | 433; 1.22 B |
| 42 | 2-((3-Isopropoxy-1-(m ethyl-d3)-1H-pyrazol-4 -yl)amino)-7-((3R,4R)-4-methyltetrahydrofuran-3-yl)-7H-pyrrolo[2;3-d]pyrimidine-6-carbonitrile | Intermediate 45 | 1.05 (d, J=6.5 Hz, 3 H), 1.27 (d, J=6.0 Hz, 6 H), 2.93 (m, 1 H), 3.47 (t, J=8.8 Hz, 1 H), 4.13-4.24 (m, 2 H), 4.29 (m, 1 H), 4.68 - 4.80 (m, 2 H), 7.46 (s, 1 H), 8.04 (br s, 1 H), 8.53 (br s, 1 H), 8.81 (s, 1 H) | 385; 1.37 D |
| 43 | 2-((3-Cyclopropoxy-1-((R)-1,1-difluoropropan-2-yl)-1H-pyrazol-4-yl)amino)-7-((3R,4R)-4-methyltetrahydrofuran-3-yl)-7H-pyrrolo[2,3-d]pyrimidine-6-carbonitrile and 2-((3-cyclopropoxy-1-((S)-1,1-difluoropropan-2-yl)-1H-pyrazol-4-yl)amino)-7-((3R,4R)-4-methyltetrahydrofuran-3-yl)-7H-pyrrolo[2,3-d]pyrimidine-6-carbonitrile (Referred herein as 2-((3-cyclopropoxy-1-(1,1-difluoropropan-2-yl)-1H-pyrazol-4-yl)amino)-7-((3R,4R)-4-methyltetrahydrofuran-3-yl)-7H-pyrrolo[2,3-d]pyrimidine-6-carbonitrile) | Intermediate 28 | 0.64 (m, 2 H), 0.71 (m, 2 H), 1.04 (d, J=6.8 Hz, 3 H), 1.53 (br d, J=6.8 Hz, 3 H), 2.89 (m, 1 H), 3.45 (br t, J=8.4 Hz, 1 H), 4.08-4.22 (m, 3 H), 4.33 (m, 1 H), 4.58 (m, 1H), 4.77 (m, 1 H), 6.17 (tdd, J H-F=55.7 Hz, J=3.6, 3.0 Hz, 1H), 7.47 (s, 1 H), 8.16 (br s, 1 H), 8.78 (br s, 1 H), 8.82 (s, 1 H) | 444; 1.42 D |

### Example 44: 2-((1-Methyl-3-(oxetan-3-yloxy)-1H-pyrazol-4-yl)amino)-7-((3S,4S)-4-methyltetrahydrofuran-3-yl)-7H-pyrrolo[2,3-d]pyrimidine-6-carbonitrile

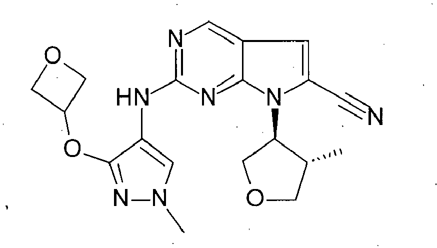

69 mg of 2-((1-methyl-3-(oxetan-3-yloxy)-1H-pyrazol-4-yl)amino)-7-((3S,4S)-4-methyltetrahydrofuran-3-yl)-7H-pyrrolo[2,3-d]pyrimidine-6-carbonitrile was prepared according to the procedure described in Example 1, starting with (3R,4R)-4-methyltetrahydrofuran-3-ol (Intermediate 3) instead of (3S,4S)-4-methyltetrahydrofuran-3-ol in step 1 and replacing N-(3-((1-acetylazetidin-3-yl)oxy)-1-methyl-1H-pyrazol-4-yl)formamide by N-[1-methyl-3-(oxetan-3-yloxy)pyrazol-4-yl]formamide (Intermediate 4) in step 5.
¹H NMR (400 MHz, DMSO-d6) δ in ppm: 1.05 (d, J=7 Hz, 3 H); 2.92 (dquin, J=8 and 15 Hz, 1 H); 3.47 (t, J=9 Hz, 1 H); 3.66 (s, 3 H); 4.08-4.39 (m, 3 H); 4.57 (dt, J=4 and 8 Hz, 2 H); 4.75 (m, 1 H); 4.81 (t, J=7 Hz, 2 H); 5.31 (quin, J=6 Hz, 1 H); 7.47 (s, 1 H); 8.08 (br s, 1 H); 8.83 (s, 1 H); 8.98 (br s, 1 H). MS (method B) m/z 396 [M+1]+; t=1.40 min.

### Example 45 and Example 46: 2-((1-Methyl-3-(oxetan-3-yloxy)-1H-pyrazol-4-yl)amino)-7-((3S,4R)-4-methyltetrahydrofuran-3-yl)-7H-pyrrolo[2,3-d]pyrimidine-6-carbonitrile and 2-((1-methyl-3-(oxetan-3-yloxy)-1H-pyrazol-4-yl)amino)-7-((3R,4S)-4-methyltetrahydrofuran-3-yl)-7H-pyrrolo[2,3-d]pyrimidine-6-carbonitrile

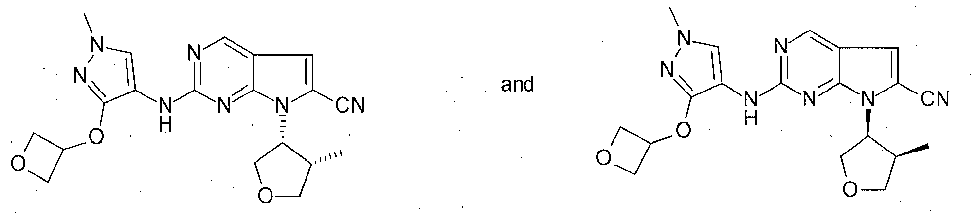

### Step 1: Preparation of racemic mixture of methyl 2-methylsulfanyl-7-[(3S,4R)-4-methyltetrahydrofuran-3-yl]pyrrolo[2,3-d]pyrimidine-6-carboxylate and methyl 2-methylsulfanyl-7-[(3R,4S)-4-methyltetrahydrofuran-3-yl]pyrrolo[2,3-d]pyrimidine-6-carboxylate

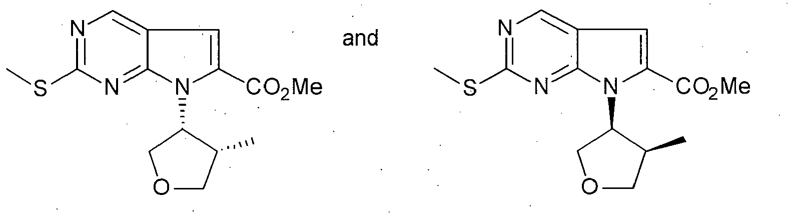

0.82 g of a racemic mixture of (3R,4S)-4-methyltetrahydrofuran-3-ol and (3S,4R)-4-methyltetrahydrofuran-3-ol (1.5 eq.) (commercially available) was added to a solution of 1.99g (1.4 eq.) of triphenylphosphine in 50 mL of tetrahydrofuran and 1g (1 eq.) of methyl 2-(methylthio)-7H-pyrrolo[2,3-d]pyrimidine-6-carboxylate (intermediate 1). The reaction mixture was cooled to 0°C and 1.6 mL of diisopropyl azodicarboxylate (DIAD) (1.5 eq.) was added. The mixture was stirred at room temperature for one night, concentrated under reduced pressure and purified on silica, eluting with 20% of ethyl acetate in heptane to afford 1 g of a racemic mixture of methyl 2-methylsulfanyl-7-[(3S,4R)-4-methyltetrahydrofuran-3-yl]pyrrolo[2,3-d]pyrimidine-6-carboxylate and methyl 2-methylsulfanyl-7-[(3R,4S)-4-methyltetrahydrofuran-3-yl]pyrrolo[2,3-d]pyrimidine-6-carboxylate. MS (method A) m/z 308 [M+1]+; t=2 min.

### Step 2: Preparation of racemic mixture of 2-methylsulfanyl-7-[(3S,4R)-4-methyltetrahydrofuran-3-yl]pyrrolo[2,3-d]pyrimidine-6-carboxamide and 2-methylsulfanyl-7⁻[(3R,4S)-4-methyltetrahydrofuran-3-yl]pyrrolo[2,3-d]pyrimidine-6-carboxamide

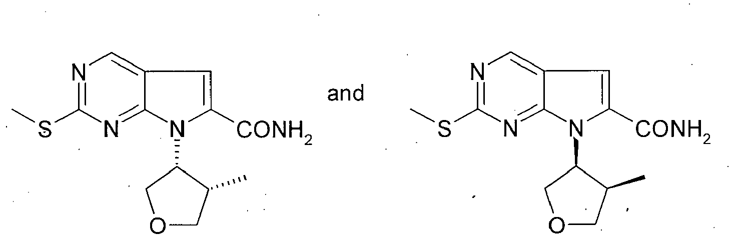

1 g (1 eq.) of a racemic mixture of methyl 2-methylsulfanyl-7-[(3S,4R)-4-methyltetrahydrofuran-3-yl]pyrrolo[2,3-d]pyrimidine-6-carboxylate and methyl 2-methylsulfanyl-7-[(3R,4S)-4-methyltetrahydrofuran-3-yl]pyrrolo[2,3-d]pyrimidine-6-carboxylate in 70 mL of a 7N solution of ammoniac in methanol in a sealed tube was stirred 3 days at room temperature. The mixture was then concentrated under reduced pressure and purified on silica, eluting with 20% of ethyl acetate in heptane to afford 0.715 g of a racemic mixture of 2-methylsulfanyl-7-[(3S,4R)-4-methyltetrahydrofuran-3-yl]pyrrolo[2,3-d]pyrimidine-6-carboxamide and 2-methylsulfanyl-7-[(3R,4S)-4-methyltetrahydrofuran-3-yl]pyrrolo[2,3-d]pyrimidine-6-carboxamide. MS (method A) m/z 293 [M+1]+; t=1.66 min.

### Step 3: Preparation of racemic mixture of 2-methylsulfanyl-7-[(3S,4R)-4-methyltetrahydrofuran-3-yl]pyrrolo[2,3-d]pyrimidine-6-carbonitrile and 2-methylsulfanyl-7-[(3R,4S)-4-methyltetrahydrofuran-3-yl]pyrrolo[2,3-d]pyrimidine-6-carbonitrile

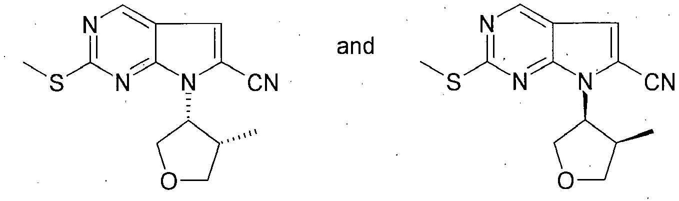

1.22 mL (3.6 eq.) of trifluoroacetic anhydride was added to a solution of 0.715 g of a racemic mixture of 2-methylsulfanyl-7-[(3S,4R)-4-methyltetrahydrofuran-3-yl]pyrrolo[2,3-d]pyrimidine-6-carboxamide and 2-methylsulfanyl-7-[(3R,4S)-4-methyltetrahydrofuran-3-yl]pyrrolo[2,3-d]pyrimidine-6-carboxamide, 1.55 mL (4.5 eq.) of triethylamine in 21 mL of anhydrous tetrahydrofuran at 0°C under argon. The mixture was stirred 4 hours at room temperature and quenched with 100 mL of ethyl acetate, and 100 mL of an aqueous saturated solution of sodium hydrogenocarbonate under vigorous stirring. The organic layer was washed with. 50 mL of water and 50 mL of brine, dried over magnesium sulfate, and concentrated under reduced pressure. The residue was purified on silica, eluting with 20% of ethyl acetate in heptane to afford 0.68 g of a racemic mixture of 2-methylsulfanyl-7-[(3S,4R)-4-methyltetrahydrofuran-3-yl]pyrrolo[2,3-d]pyrimidine-6-carbonitrile and 2-methylsulfanyl-7-[(3R,4S)-4-methyltetrahydrofuran-3-yl]pyrrolo[2;3-d]pyrimidine-6-carbonitrile. MS (method A) m/z 275 [M+1]+; t=2.19 min.

### Step 4: Preparation of racemic mixture of 2-methylsulfonyl-7-[(3S,4R)-4-methyltetrahydrofuran-3-yl]pyrrolo[2,3-d]pyrimidine-6-carbonitrile and 2-methylsulfonyl-7-[(3R,4S)-4-methyltetrahydrofuran-3-yl]pyrrolo[2,3-d]pyrimidine-6-carbonitrile

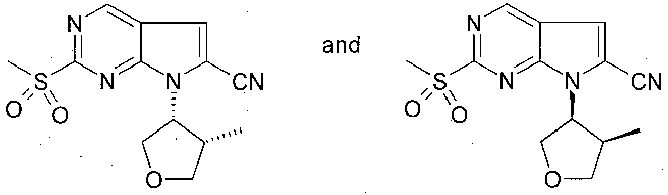

1.28 g of 3-chloroperbenzoic acid (77% purity; 2.3 eq.) was added to a solution of 0.68 g (1 eq.) of a racemic mixture of 2-methylsulfanyl-7-[(3S,4R)-4-methyltetrahydrofuran-3-yl]pyrrolo[2,3-d]pyrimidine-6-carbonitrile and 2-methylsulfanyl-7-[(3R,4S)-4-methyltetrahydrofuran-3-yl]pyrrolo[2,3-d]pyrimidine-6-carbonitrile in 20 mL of dichloromethane at 0°C. The mixture was stirred for 1 hour at room temperature then quenched with 50 mL of dichloromethane and 40 mL of a 10% aqueous sodium thiosulfate solution. The organic layer was washed successively with 40 mL of an aqueous saturated solution of sodium hydrogenocarbonate, 50 mL of brine, then dried over magnesium sulfate, and concentrated under reduced pressure to afford 0.58 g of a racemic mixture of 2-methylsulfonyl-7-[(3S,4R)-4-methyltetrahydrofuran-3-yl]pyrrolo[2,3-d]pyrimidine-6-carbonitrile and 2-methylsulfonyl-7-[(3R,4S)-4-methyltetrahydrofuran-3-yl]pyrrolo[2,3-d]pyrimidine-6-carbonitrile. MS (method A) m/z 307 [M+1]+; t=1.58 min.

### Step 5: Preparation of 2-((1-methyl-3-(oxetan-3-yloxy)-1H-pyrazol-4-yl)amino)-7-((3S,4R)-4-methyltetrahydrofuran-3-yl)-7H-pyrrolo[2,3-d]pyrimidine-6-carbonitrile and 2-((1-methyl-3-(oxetan-3-yloxy)-1H-pyrazol-4-yl)amino)-7-((3R,4S)-4-methyltetrahydrofuran-3-yl)-7H-pyrrolo[2,3-d]pyrimidine-6-carbonitrile Example 45 and Example 46

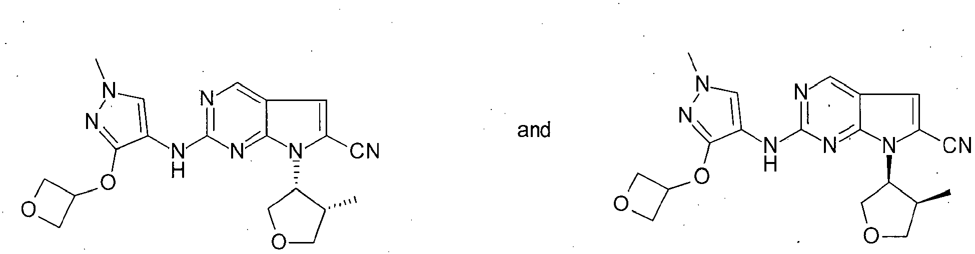

Generally following the procedure described in Step 5 of Example 1, a racemic mixture of 2-methylsulfonyl-7-[(3S,4R)-4-methyltetrahydrofuran-3-yl]pyrrolo[2,3-d]pyrimidine-6-carbonitrile and 2-methylsulfonyl-7-[(3R,4S)-4-methyltetrahydrofuran-3-yl]pyrrolo[2,3-d]pyrimidine-6-carbonitrile (155 mg) and N-(1-methyl-3-(oxetan-3-yloxy)-1H-pyrazol-4-yl)formamide (105 mg) (Intermediate 4) gave a racemic mixture of 2-((1-methyl-3-(oxetan-3-yloxy)-1H-pyrazol-4-yl)amino)-7-((3S,4R)-4-methyltetrahydrofuran-3-yl)-7H-pyrrolo[2,3-d]pyrimidine-6-carbonitrile and 2-((1-methyl-3-(oxetan-3-yloxy)-1H-pyrazol-4-yl)amino)-7-((3R,4S)-4-methyltetrahydrofuran-3-yl)-7H-pyrrolo[2,3-d]pyrimidine-6-carbonitrile (145 mg) (Referred herein as 2-((1-methyl-3-(oxetan-3-yloxy)-1H-pyrazol-4-yl)amino)-7-((cis)-4-methyltetrahydrofuran-3-yl)-7H-pyrrolo[2,3-d]pyrimidine-6-carbonitrile).

Chiral separation performed on 135 mg of the mixture gave 58 mg of the first eluting isomer and 59 mg of the second eluting isomer (conditions: column Phenomenex Lux Amylose-1, 5 µm, 250x4.6 mm; liquid phase: heptane 20/ethyl alcohol 80/triethylamine 0.1%; flow rate: 40 mL/min).

Peak 1 (1^{st} isomer): ¹H NMR (400 MHz, DMSO-d6) δ in ppm: 0.55 (d, J=7 Hz, 3 H); 2.67 (m, 1 H); 3.65 (s, 3 H); 3.70 (m, 1 H); 3.97 (t, J=8 Hz, 1 H); 4.31 (dd, J=8 and 10 Hz, 1 H); 4.57 (q, J=6 Hz, 2 H); 4.65 (m, 1 H); 4.81 (t, J=7 Hz, 2 H); 5.21 (m, 1 H); 5.31 (m, 1 H); 7.49 (s, 1 H); 8.05 (d, J=2 Hz, 1 H); 8.82 (s, 1 H); 8.91 (br s, 1 H). MS (method D) m/z 396 [M+1]+; t=2.63 min - Example 45 (absolute configuration unknown)

Peak 2 (2^{nd} isomer): ¹H NMR (400 MHz, DMSO-d6) δ in ppm: 0.55 (d, J=7 Hz, 3 H); 2.67 (m, 1 H); 3.65 (s, 3 H); 3.71 (m, 1 H); 3.97 (t, J=8 Hz, 1 H); 4.31 (dd, J=8 and 10 Hz, 1 H); 4.57 (q, J=6 Hz, 2 H); 4.66 (m, 1 H); 4.81 (t, J=7 Hz, 2 H); 5.21 (br s, 1 H); 5.31 (quin, J=6 Hz, 1 H); 7.49 (s, 1 H); 8.05 (br s, 1 H); 8.82 (s, 1 H); 8.92 (br s, 1 H). MS (method D) m/z 396 [M+1]+; t=2.63 min - Example 46 (absolute configuration unknown) Example 47: 2-((3-lsopropoxy-1-methyl-1H-pyrazol-4-yl)amino)-7-((3S,4R)-4-methyltetrahydrofuran-3-yl)-7H-pyrrolo[2,3-d]pyrimidine-6-carbonitrile and 2-((3-isopropoxy-1-methyl-1H-pyrazol-4-yl)amino)-7-((3R,4S)-4-methyltetrahydrofuran-3-yl)-7H-pyrrolo[2,3-d]pyrimidine-6-carbonitrile (racemic mixture)

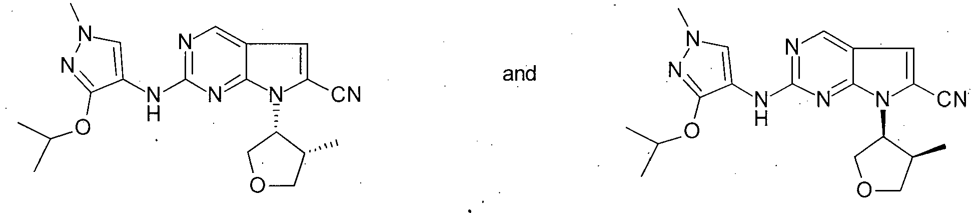

A racemic mixture of 2-methylsulfonyl-7-[(3S,4R)-4-methyltetrahydrofuran-3-yl]pyrrolo[2,3-d]pyrimidine-6-carbonitrile and 2-methylsulfonyl-7-[(3R,4S)-4-methyltetrahydrofuran-3-yl]pyrrolo[2,3-d]pyrimidine-6-carbonitrile (60 mg) (step 4 of Example 45), 38 mg (1.05 eq.) of N-(3-isopropoxy-1-methyl-1H-pyrazol-4-yl)formamide (Intermediate 38) and 192 mg (3 eq.) of cesium carbonate in 5 mL of dimethylformamide was heated at 60°C for 1 hour. The mixture was cooled to room temperature then diluted with 50 mL of ethyl acetate and 50 mL of water. The organic layer was washed twice with 50 mL of water then dried over magnesium sulfate and concentrated under reduced pressure. The residue was taken into 15 mL of a 7N methanolic ammonia solution and stirred for 30 minutes then concentrated under reduced pressure. The residue was purified on silica, eluting with a mixture of dichloromethane/methanol (98/2) to afford 43 mg of a racemic mixture of 2-((3-isopropoxy-1-methyl-1H-pyrazol-4-yl)amino)-7-((3S,4R)-4-methyltetrahydrofuran-3-yl)-7H-pyrrolo[2,3-d]pyrimidine-6-carbonitrile and 2-((3-isopropoxy-1-methyl-1H-pyrazol-4-yl)amino)-7-((3R,4S)-4-methyltetrahydrofuran-3-yl)-7H-pyrrolo[2,3-d]pyrimidine-6-carbonitrile (Referred herein as 2-((3-isopropoxy-1-methyl-1H-pyrazol-4-yl)amino)-7-((cis)-4-methyltetrahydrofuran-3-yl)-7H-pyrrolo[2,3-d]pyrimidine-6-carbonitrile)- Example 47.
¹H NMR (400 MHz, DMSO-d6) δ in ppm: 0.55 (d, J=7 Hz, 3 H); 1.26 (d, J=3 Hz, 3 H); 1.28 (d, J=3 Hz, 3 H); 2.68 (m, 1 H); 3.67 (s, 3 H); 3.72 (t, J=9 Hz, 1 H); 3.96 (m, 1 H); 4.31 (dd, J=8 and 10 Hz, 1 H); 4.59 - 4.81 (m, 2 H); 5.24 (m, 1 H); 7.48 (s, 1 H); 8.05 (br s, 1 H); 8.50 (br s, 1 H); 8.81 (s, 1 H). MS (method B) m/z 382 [M+1]+; t=1.51 min.

### Example 48, Example 49, Example 50 and Example 51: 2-((1-((R)-1-Cyanoethyl)-3-isopropoxy-1H-pyrazol-4-yl)amino)-7-((3S,4R)-4-methyltetrahydrofuran-3-yl)-7H-pyrrolo[2,3-d]pyrimidine-6-carbonitrile and 2-((1-((S)-1-cyanoethyl)-3-isopropoxy-1H-pyrazol-4-yl)amino)-7-((3S,4R)-4-methyltetrahydrofuran-3-yl)-7H-pyrrolo[2,3-d]pyrimidine-6-carbonitrile and 2-((1-((R)-1-cyanoethyl)-3-isopropoxy-1H-pyrazol-4-yl)amino)-7-((3R,4S)-4-methyltetrahydrofuran-3-yl)-7H-pyrrolo[2,3-d]pyrimidine-6-carbonitrile and 2-((1-((S)-1-cyanoethyl)-3-isopropoxy-1H-pyrazol-4-yl)amino)-7-((3R,4S)-4-methyltetrahydrofuran-3-yl)-7H-pyrrolo[2,3-d]pyrimidine-6-carbonitrile

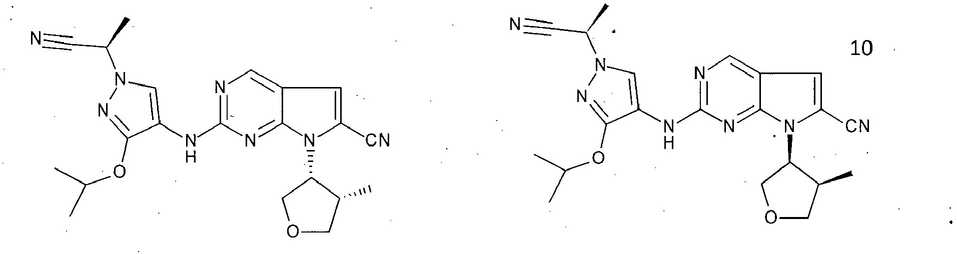

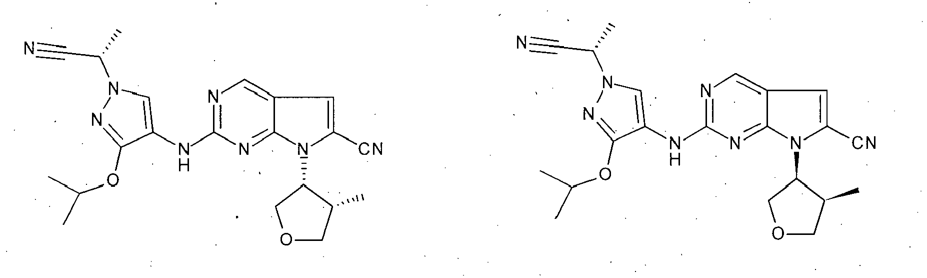

Following the procedure described in Example 47, a racemic mixture of 2-methylsulfonyl-7-[(3S,4R)-4-methyltetrahydrofuran-3-yl]pyrrolo[2,3-d]pyrimidine-6-carbonitrile and 2-methylsulfonyl-7-[(3R,4S)-4-methyltetrahydrofuran-3-yl]pyrrolo[2,3-d]pyrimidine-6-carbonitrile (200 mg) (step 4 of Example 45) and racemic N-(1-(1-cyanoethyl)-3-isopropoxy-1H-pyrazol-4-yl)formamide (152 mg) (Intermediate 39) gave an isomeric mixture of 2-((1-((R)-1-cyanoethyl)-3-isopropoxy-1H-pyrazol-4-yl)amino)-7-((3S,4R)-4-methyltetrahydrofuran-3-yl)-7H-pyrrolo[2,3-d]pyrimidine-6-carbonitrile and 2-((1-((S)-1-cyanoethyl)-3-isopropoxy-1H-pyrazol-4-yl)amino)-7-((3S,4R)-4-methyltetrahydrofuran-3-yl)-7H-pyrrolo[2,3-d]pyrimidine-6-carbonitrile and 2-((1-((R)-1-cyanoethyl)-3-isopropoxy-1H-pyrazol-4-yl)amino)-7-((3R,4S)-4-methyltetrahydrofuran-3-yl)-7H-pyrrolo[2,3-d]pyrimidine-6-carbonitrile and 2-((1-((S)-1-cyanoethyl)-3-isopropoxy-1H-pyrazol-4-yl)amino)-7-((3R,4S)-4-methyltetrahydrofuran-3-yl)-7H-pyrrolo[2,3-d]pyrimidine-6-carbonitrile (Referred herein as 2-((1-(1-cyanoethyl)-3-isopropoxy-1H-pyrazol-4-yl)amino)-7-((cis)-4-methyltetrahydrofuran-3-yl)-7H-pyrrolo[2,3-d]pyrimidine-6-carbonitrile)(160 mg).

Chiral separation was performed on 160 mg of the isomeric mixture. A first separation gave 84 mg of a mixture of 2 compounds and 66 mg of a mixture of 2 other compounds (conditions: column Chiralpak-IC, 20 µm, 350×76 mm; liquid phase: heptane 20/ethyl alcohol 80; flow rate: 400 mL/min).

The first mixture of 2 compounds (84 mg) was separated by chiral chromatography to give 36 mg of the first eluting isomer and 32 mg of the second eluting isomer (conditions: column Chiralpak-AY-H, 5 µm, 250x30 mm; liquid phase: heptane 80/ethyl alcohol 20; flow rate: 45 mL/min).

Peak 1 (1^{st} isomer): ¹H NMR (400 MHz, DMSO-d6) δ in ppm: 0.56 (d, J=7 Hz, 3 H); 1.32 (d, J=6 Hz, 6 H); 1.80 (d, J=7 Hz, 3 H); 2.73 (m, 1 H); 3.73 (t, J=9 Hz, 1 H); 3.99 (t, J=8 Hz, 1 H); 4.34 (dd, J=8 and 10 Hz, 1 H); 4.71 (m, 1 H); 4.81 (spt, J=7 Hz, 1 H); 5.22 (m, 1 H); 5.55 (q, J=7Hz, 1 H); 7.51 (s, 1 H); 8.31 (m, 1 H); 8.71 (br s, 1 H); 8.86 (s, 1 H). MS (method B) m/z 421 [M+1]+; t=1.7 min ― Example 48 (absolute configuration unknown)

Peak 2 (2^{nd} eluting isomer): ¹H NMR (400 MHz, DMSO-d6) δ in ppm: 0.56 (d, J=7 Hz, 3 H); 1.32 (dd, J=3 and 6 Hz, 6 H); 1.79 (d, J=7 Hz, 3 H); 2.71 (m, 1 H); 3.71 (t, J=9 Hz, 1 H); 3.99 (t, J=8 Hz, 1 H); 4.34 (dd, J=8 and 10 Hz, 1 H); 4.64 ― 4.90 (m, 2 H); 5.23 (m, 1 H); 5.54 (q, J=7 Hz, 1 H); 7.51 (s, 1 H); 8.30 (br s, 1 H); 8.71 (br s, 1 H); 8,86 (s, 1 H). MS (method B) m/z 421, [M+1]+; t=1.7 min ― Example 49 (absolute configuration unknown)

The second mixture of 2 compounds (66 mg) was separated by chiral chromatography to give 34 mg of the first eluting isomer and 39 mg of the second eluting isomer (conditions: column Chiralpak-AY-H, 5 µm, 250x30 mm; liquid phase: heptane 60/ethyl alcohol 40; flow rate: 45 mL/min).

Peak 1 (1^{st} eluting isomer): ¹H NMR (400 MHz, DMSO-d6) δ in ppm: 0.56 (d, J=7 Hz, 3 H); 1.31 (d, J=3 Hz, 3 H); 1.33 (d, J=3 Hz, 3 H); 1.79 (d, J=7 Hz, 3 H); 2.70 (m, 1 H); 3.71 (t, J=9 Hz, 1 H); 3.99 (t, J=8 Hz, 1 H); 4.33 (dd, J=9 and 11 Hz, 1 H); 4.70 (m, 1 H); 4.81 (spt, J=6 Hz, 1 H); 5.24 (m, 1 H); 5.54 (q, J=6 Hz, 1 H); 7.51 (s, 1 H); 8.30 (br s, 1 H); 8.71 (br s, 1 H); 8.86 (s, 1 H). MS (method B) m/z 421 [M+1]+; t=1.7min - Example 50 (absolute configuration unknown)

Peak 2 (2^{nd} eluting isomer): ¹H NMR (400 MHz, DMSO-d6) δ in ppm: 0.56 (d, J=7 Hz, 3 H); 1.32 (d, J=6 Hz, 6 H); 1.80 (d, J=7 Hz, 3 H); 2.70 (m, 1 H); 3.73 (t, J=9 Hz, 1 H); 3.99 (t, J=8 Hz, 1 H); 4.34 (t, J=9 Hz, 1 H); 4.62 - 4.92 (m, 2 H); 5.23 (m, 1 H); 5.55 (q, J=7 Hz, 1 H); 7.51 (s, 1 H); 8.32 (br s, 1 H); 8.71 (br s, 1 H); 8.86 (s, 1 H). MS (method B) m/z 421 [M+1]+; t=1.7min - Example 51 (absolute configuration unknown)

### Example 52 and Example 53: (R)-2-((1-Methyl-3-(oxetan-3-yloxy)-1H-pyrazol-4-yl)amino)-7-(tetrahydrofuran-3-yl)-7H-pyrrolo[2,3-d]pyrimidine-6-carbonitrile and (S)-2-((1-methyl-3-(oxetan-3-yloxy)-1H-pyrazol-4-yl)amino)-7-(tetrahydrofuran-3-yl)-7H-pyrrolo[2,3-d]pyrimidine-6-carbonitrile

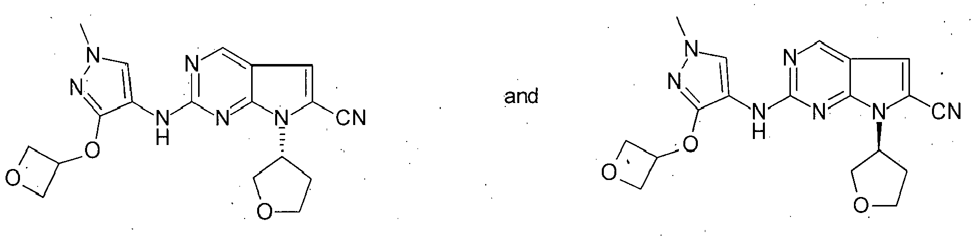

### Step 1: Preparation of 2-(methylthio)-7-(tetrahydrofuran-3-yl)-7H-pyrrolo[2,3-d]pyrimidine-6-carbonitrile

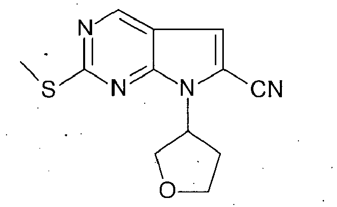

1.36 g of diisopropyl azodicarboxylate (DIAD) (3 eq.) was added to a solution of 1.67g (3 eq.) of triphenylphosphine in 20 mL of tetrahydrofuran and 0.4 g (1 eq.) of racemic 2-(methylthio)-7H-pyrrolo[2,3-d]pyrimidine-6-carbonitrile (intermediate 43) at 0°C. The reaction mixture was stirred at 0°C for 30 minutes then 0.56 g (3 eq.) of tetrahydrofuran-3-ol (commercially available) was added. The mixture was stirred at room temperature for one night, concentrated under reduced pressure and purified on silica, eluting with 10 to 50 % of ethyl acetate in heptane to afford 1.2 g of racemic 2-(methylthio)-7-(tetrahydrofuran-3-yl)-7H-pyrrolo[2,3-d]pyrimidine-6-carbonitrile (purity 50%). MS (method A) m/z 261 [M+1]+; t=1.54 min.

The crude material was taken into the next step without further purification.

### Step 2: Preparation of 2-(methylsulfonyl)-7-(tetrahydrofuran-3-yl)-7H-pyrrolo[2,3-d]pyrimidine-6-carbon itrile

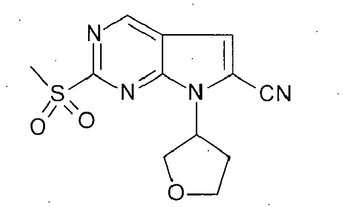

1.55 g of 3-chloroperbenzoic acid (77% purity; 3 eq.) were added to a solution of 1.2 g (1 eq.) of 2-(methylthio)-7-(tetrahydrofuran-3-yl)-7H-pyrrolo[2,3-d]pyrimidine-6-carbonitrile in 30 mL of dichloromethane at 0°C. The mixture was stirred for 1 hour at room temperature then quenched with 50 mL of dichloromethane and 40 mL of a 10% aqueous sodium thiosulfate solution. The organic layer was washed successively with 40 mL of an aqueous saturated solution of sodium hydrogenocarbonate solution, 50 mL of brine, then dried over magnesium sulfate, and concentrated under reduced pressure to afford 0. 8 g of racemic 2-(methylsulfonyl)-7-(tetrahydrofuran-3-yl)-7H-pyrrolo[2,3-d]pyrimidine-6-carbonitrile. The crude material was taken into the next step without further purification. MS (method A) m/z 293 [M+1]+; t=1.41 min.

### Step 3: Preparation of (R)-2-((1-methyl-3-(oxetan-3-yloxy)-1H-pyrazol-4-yl)amino)-7-(tetrahydrofuran-3-yl)-7H-pyrrolo[2,3-d]pyrimidine-6-carbonitrile and (S)-2-((1-methyl-3-(oxetan-3-yloxy)-1H-pyrazol-4-yl)amino)-7-(tetrahydrofuran-3-yl)-7H-pyrrolo[2,3-d]pyrimidine-6-carbonitrile Example 52 and Example 53

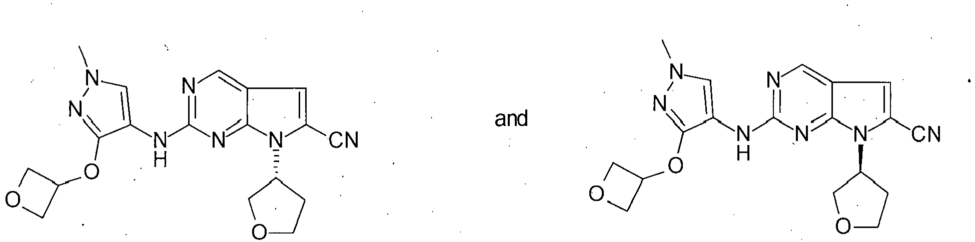

Generally following the procedure described in Step 5 of Example 1, racemic 2-(methylsulfonyl)-7-(tetrahydrofuran-3-yl)-7H-pyrrolo[2,3-d]pyrimidine-6-carbonitrile (200 mg) and N-[1-methyl-3-(oxetan-3-yloxy)pyrazol-4-yl]formamide (intermediate 4) gave a racemic mixture of (R)-2-((1-methyl-3-(oxetan-3-yloxy)-1H-pyrazol-4-yl)amino)-7-(tetrahydrofuran-3-yl)-7H-pyrrolo[2,3-d]pyrimidine-6-carbonitrile and (S)-2-((1-methyl-3-(oxetan-3-yloxy)-1H-pyrazol-4-yl)amino)-7-(tetrahydrofuran-3-yl)-7H-pyrrolo[2,3-d]pyrimidine-6-carbonitrile (80mg) (Referred herein as 2-((1-methyl-3-(oxetan-3-yloxy)-1H-pyrazol-4-yl)amino)-7-(tetrahydrofuran-3-yl)-7H-pyrrolo[2,3-d]pyrimidine-6-carbonitrile).

Chiral separation performed on 73 mg of the isomeric mixture gave 22 mg of the first eluting isomer and 21 mg of the second eluting isomer (conditions: column Chiralpak AY, 20 µm, 230x100 mm; liquid phase: heptane 20/ethyl alcohol 80; flow rate: 400 mL/min).

Peak 1 (1^{st} eluting isomer): ¹H NMR (400 MHz, DMSO-d6) δ in ppm: 2.44 (q, J=7 Hz, 2 H); 3.66 (s, 3 H); 3.83 (q, J=7 Hz, 1 H); 3.95-4.19 (m, 3 H); 4.56 (m, 2 H); 4.80 (t, J=7 Hz, 2 H); 5.28 (m, 2 H); 7.45 (s, 1 H); 7.94 (br s, 1 H); 8.80 (s, 1 H); 8.86 (br s, 1 H). MS (method B) m/z 382 [M+1]+; t=1.27min - Example 52 (absolute configuration unknown)

Peak 2 (2^{nd} eluting isomer): ¹H NMR (400 MHz, DMSO-d6) δ in ppm: 2.44 (q, J=7 Hz, 2 H); 3.66 (s, 3 H); 3.83 (q, J=7 Hz, 1 H); 3.92 - 4.20 (m, 3 H); 4.57 (m, 2 H); 4.80 (t, J=7 Hz, 2 H); 5.27 (m, 2 H); 7.45 (s, 1 H); 7.93 (br s, 1 H); 8.80 (s, 1 H); 8.86 (br s, 1 H). MS (method B) m/z 382 [M+1]+; t=1.27min - Example 53 (absolute configuration unknown)

### Example 54: 7-((3R,4R)-4-Methoxytetrahydrofuran-3-yl)-2-((1-(oxetan-3-yl)-3=(((R)-1,1,1-trifluoropropan-2-yl)oxy)-1H-pyrazol-4-yl)amino)-7H-pyrrolo[2,3-d]pyrimidine-6-carbonitrile and 7-((3R,4R)-4-methoxytetrahydrofuran-3-yl)-2-((1-(oxetan-3-yl)-3-(((S)-1,1,1-trifluoropropan-2-yl)oxy)-1H-pyrazol-4-yl)amino)-7H-pyrrolo[2,3-d]pyrimidine-6-carbonitrile (mixture)

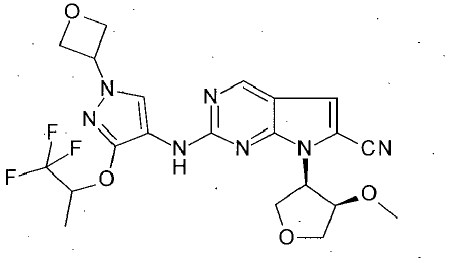

### Step 1: Preparation of 7-((3R,4R)-4-methoxytetrahydrofuran-3-yl)-2-(methylthio)-7H-pyrrolo[2,3-d]pyrimidine-6-carboxylic acid

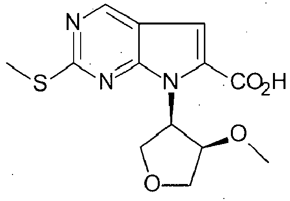

2.3 mL of diisopropyl azodicarboxylate (DIAD) (1.5 eq.) was added to a solution of 3.03 g (1.5 eq.) of triphenylphosphine, 1.7g (1 eq.) of methyl 2-(methylthio)-7H-pyrrolo[2,3-d]pyrimidine-6-carboxylate (intermediate 1) and 1.44 g of (3S,4S)-4-methoxytetrahydrofuran-3-ol (1.6 eq.) (intermediate 50) in 20 mL of tetrahydrofuran at 5°C. The mixture was heated at 60°C for 20 hours then cooled to 0°C and 11.4 mL (1.5 eq.) of a 1N aqueous sodium hydroxide solution was added. The reaction mixture was stirred for 2 hours at room temperature. 40 mL of water and 40 mL of diethyl ether were added. The aqueous layer was washed with 40 mL of diethyl ether then was acidified to pH 1 with HCI 1N. The 7-((3R,4R)-4-methoxytetrahydrofuran-3-yl)-2-(methylthio)-7H-pyrrolo[2,3-d]pyrimidine-6-carboxylic acid precipitated upon acidification, was filtered-off, washed with water and dried under vacuum to afford 930 mg of 7-((3R,4R)-4-methoxytetrahydrofuran-3-yl)-2-(methylthio)-7H-pyrrolo[2,3-d]pyrimidine-6-carboxylic acid. The crude material was used in the next step without further purification.

### Step 2: Preparation of methyl 7-((3R,4R)-4-methoxytetrahydrofuran-3-yl)-2-(methylthio)-7H-pyrrolo[2,3-d]pyrimidine-6-carboxylate

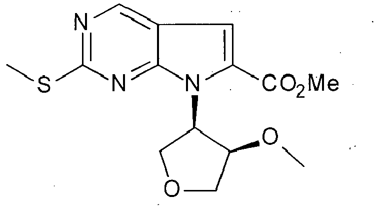

Sulfuric acid (1.34 mL, 1.4 eq.) was added dropwise to a solution of 1.93 g (1 eq.) of 7-((3R,4R)-4-methoxytetrahydrofuran-3-yl)-2-(methylthio)-7H-pyrrolo[2,3-d]pyrimidine-6-carboxylic acid in 35 mL of methanol. The reaction mixture was heated at 60°C for 16 hours. 0.6 mL of sulfuric acid (0.6 eq.) was added and the reaction mixture heated at 60°C for 24 hours. 0.6 mL of sulfuric acid (0.6 eq.) was added and the reaction mixture heated at 60°C for 4 hours and stirred at room temperature for 60 hours. The reaction mixture was poured into an ice cooled saturated sodium bicarbonate solution, stirred for 10 minutes and extracted with twice with 50 mL of ethyl acetate. The combined organic layers were washed twice with 50 mL of water, and then dried over magnesium sulfate, and concentrated under reduced pressure. The residue was purified on silica gel, eluting with a mixture of heptane/ethyl acetate (95/5 to 70/30) to afford 960 mg of a colorless oil which was triturated in diethyl ether. The solid was filtered-off and dried under vacuum to afford 660 mg of methyl 7-((3R,4R)-4-methoxytetrahydrofuran-3-yl)-2-(methylthio)-7H-pyrrolo[2,3-d]pyrimidine-6-carboxylate. MS (method B) m/z 324 [M+1]+; t=2.85 min.

### Step 3: Preparation of 7-((3R,4R)-4-methoxytetrahydrofuran-3-yl)-2-(methylthio)-7H-pyrrolo[2,3-d]pyrimidine-6-carboxamide

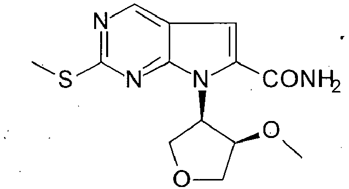

0.36 g (1 eq.) of methyl 7-((3R,4R)-4-methoxytetrahydrofuran-3-yl)-2-(methylthio)-7H-pyrrolo[2,3-d]pyrimidine-6-carboxylate in 75 mL of a 7N solution of ammoniac in methanol in a sealed tube was stirred for 88 hours at room temperature. The mixture was then concentrated under reduced pressure to afford 330 mg of 7-((3R,4R)-4-methoxytetrahydrofuran-3-yl)-2-(methylthio)-7H-pyrrolo[2,3-d]pyrimidine-6-carboxamide. The crude material was used in the next step without further purification.

### Step 4: Preparation of 7-((3R,4R)-4-methoxytetrahydrofuran-3-yl)-2-(methylthio)-7H-pyrrolo[2,3-d]pyrimidine-6-carbonitrile

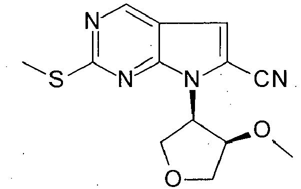

0.56 mL (3.6 eq.) of trifluoroacetic anhydride was added to a solution of 0.34 g of 7-((3R,4R)-4-methoxytetrahydrofuran-3-yl)-2-(methylthio)-7H-pyrrolo[2,3-d]pyrimidine-6-carboxamide (1 eq.), 0.7 mL (4.5 eq.) of triethylamine in 20 mL of anhydrous tetrahydrofuran at 0°C under argon. The mixture was stirred for 1 hour at room temperature and quenched with 20 mL of dichloromethane, and 20 mL of an aqueous saturated solution of sodium hydrogenocarbonate under vigorous stirring. The organic layer was washed with 50 mL of water and 50 mL of brine, dried over magnesium sulfate, and concentrated under reduced pressure. The residue was purified on silica; eluting with 0 to 5% of acetone in dichloromethane to afford 0.225 g of 7-((3R,4R)-4-methoxytetrahydrofuran-3-yl)-2-(methylthio)-7H-pyrrolo[2,3-d]pyrimidine-6-carbonitrile. MS (method A) m/z 291 [M+1]+; t=2.05 min.

### Step 5: Preparation of 7-((3R,4R)-4-methoxytetrahydrofuran-3-yl)-2-(methylsulfonyl)-7H-pyrrolo[2,3-d]pyrimidine-6-carbonitrile

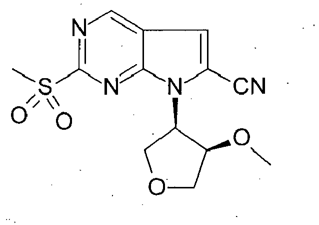

435 mg of 3-chloroperbenzoic acid (77% purity; 2.5 eq.) was added to a solution of 225 mg (1 eq.) of 7-((3R,4R)-4-methoxytetrahydrofuran-3-yl)-2-(methylthio)-7H-pyrrolo[2,3-d]pyrimidine-6-carbonitrile (in 10 mL of dichloromethane at 0°C. The mixture was stirred for 1 hour at room temperature then quenched with 10 mL of dichloromethane and 10 mL of a 10% aqueous sodium thiosulfate solution. The organic layer was washed successively with 10 mL of an aqueous saturated solution of sodium hydrogenocarbonate solution, 10 mL of brine then dried over magnesium sulfate, and concentrated under reduced pressure to afford 233 mg of 7-((3R,4R)-4-methoxytetrahydrofuran-3-yl)-2-(methylsulfonyl)-7H-pyrrolo[2,3-d]pyrimidine-6-carbonitrile. MS (method A) m/z 323 [M+1]+; t=1.15 min.

### Step 6: Preparation of 7-((3R,4R)-4-methoxytetrahydrofuran-3-yl)-2-((1-(oxetan-3-yl)-3-(((R)-1,1,1-trifluoropropan-2-yl)oxy)-1H-pyrazol-4-yl)amino)-7H-pyrrolo[2,3-d]pyrimidine-6-carbonitrile and 7-((3R,4R)-4-methoxytetrahydrofuran-3-yl)-2-((1-(oxetan-3-yl)-3-(((S)-1,1,1-trifluoropropan-2-yl)oxy)-1H-pyrazol-4-yl)amino)-7H-pyrrolo[2,3-d]pyrimidine-6-carbonitrile (mixture)

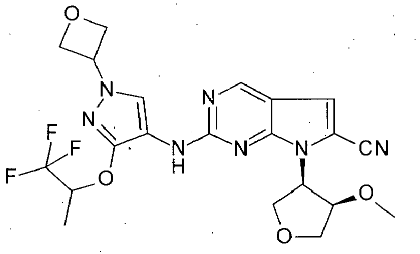

Generally following the procedure described in Step 5 of Example 1, 7-((3R,4R)-4-methoxytetrahydrofuran-3-yl)-2-(methylsulfonyl)-7H-pyrrolo[2,3-d]pyrimidine-6-carbonitrile (100 mg) and racemic N-[1-(oxetan-3-yl)-3-[(2,2,2-trifluoro-1-methyl-ethoxy]pyrazol-4-yl]formamide (intermediate 23) gave a mixture of 7-((3R,4R)-4-methoxytetrahydrofuran-3-yl)-2-((1-(oxetan-3-yl)-3-(((R)-1,1,1-trifluoropropan-2-yl)oxy)-1H-pyrazol-4-yl)amino)-7H-pyrrolo[2,3-d]pyrimidine-6-carbonitrile and 7-((3R,4R)-4-methoxytetrahydrofuran-3-yl)-2-((1-(oxetan-3-yl)-3-(((S)-1,1,1-trifluoropropan-2-yl)oxy)-1H-pyrazol-4-yl)amino)-7H-pyrrolo[2,3-d]pyrimidine-6-carbonitrile (Referred herein as 7-((3R,4R)-4-Methoxytetrahydrofuran-3-yl)-2-((1-(oxetan-3-yl)-3-((1,1,1-trifluoropropan-2-yl)oxy)-1H-pyrazol-4-yl)amino)-7H-pyrrolo[2,3-d]pyrimidine-6-carbonitrile) (120 mg)-Example 54.
1H NMR (400 MHz, DMSO-d6) δ ppm 1.48 (dd, J=7, 2 Hz, 3 H), 2.98 (s, 3 H), 3.85 - 4.08 (m, 2 H), 4.10 - 4,26 (m, 2 H), 4.49 (m, 1 H), 4.89 (d, J=7 Hz, 4 H), 5.25 - 5.52 (m, 3 H), 7.47 (s, 1 H), 8.12 (br s, 1 H), 8.81 (s, 1 H), 8.84 (br s, 1 H). MS (method B) m/z 494 [M+1]+; t=1.62 min.

### Example 55: 2-((4-(2-Hydroxypropan-2-yl)-2-(oxetan-3-yloxy)phenyl)amino)-7-((3R,4R)-4-methoxytetrahydrofuran-3-yl)-7H-pyrrolo[2,3-d]pyrimidine-6-carbonitrile

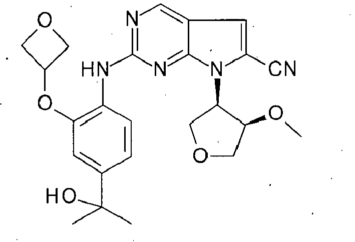

### Step 1: Preparation of methyl 4-((6-cyano-7-((3R,4R)-4-methoxytetrahydrofuran-3-yl)-7H-pyrrolo[2,3-d]pyrimidin-2-yl)amino)-3-(oxetan-3-yloxy)benzoate

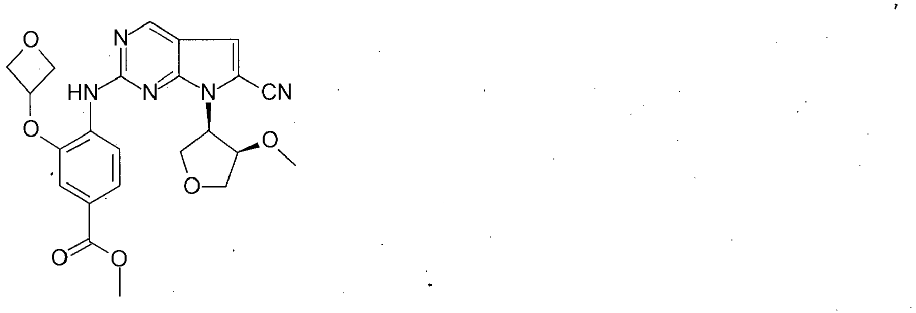

Generally following the procedure described in Step 5 of Example 1, 7-((3R,4R)-4-methoxytetrahydrofuran-3-yl)-2-(methylsulfonyl)-7H-pyrrolo[2,3-d]pyrimidine-6-carbonitrile (step 5 of Example 54) (70 mg) and methyl 4-formamido-3-(oxetan-3-yloxy)benzoate (66 mg) (Intermediate 35) gave methyl 4-((6-cyano-7-((3R,4R)-4-methoxytetrahydrofuran-3-yl)-7H-pyrrolo[2,3-d]pyrimidin-2-yl)amino)-3-(oxetan-3-yloxy)benzoate (97 mg). MS (method A) m/z 466 [M+1]+; t=2.34 min.

### Step 2: Preparation of 2-((4-(2-hydroxypropan-2-yl)-2-(oxetan-3-yloxy)phenyl)amino)-7-((3R,4R)-4-methoxytetrahydrofuran-3-yl)-7H-pyrrolo[2,3-d]pyrimidine-6-carbonitrile - Example 55

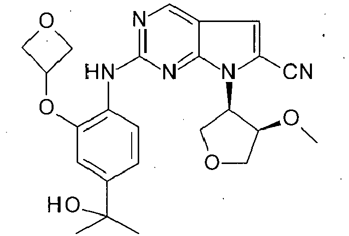

695 µL (10 eq.) of methyl magnesium bromide 3M in tetrahydrofuran was added to a solution of 97 mg (1 eq.) of methyl 4-((6-cyano-7-((3R,4R)-4-methoxyt etrahydrofuran-3-yl)-7H-pyrrolo[2,3-d]pyrimidin-2-yl)amino)-3-(oxetan-3-yloxy)benzoate in 7 mL of tetrahydrofuran at 0°C. The reaction mixture was stirred for 1 hour at 0°C then the reaction mixture was quenched with a saturated aqueous solution of ammonium chloride and extracted with ethyl acetate. The organic layer was washed with 50 mL of water and 50 mL of brine, dried over magnesium sulfate, and concentrated under reduced pressure. The residue was purified on silica, eluting with a mixture of heptane/AcOEt/7N methanolic ammonia solution (80/18/2) to afford 42 mg of 2-((4-(2-hydroxypropan-2-yl)-2-(oxetan-3-yloxy)phenyl)amino)-7-((3R,4R)-4-methoxytetrahydrofuran-3-yl)-7H-pyrrolo[2,3-d]pyrimidine-6-carbonitrile.
1H NMR (400 MHz, DMSO-d6) δ ppm: 1.42 (s, 6 H), 2.97 (s, 3 H), 3.96 (dd, J=10, 4 Hz, 1 H), 4.06 (dd, J=10, 5 Hz, 1 H), 4.19 (m, 1 H), 4.25 (t, J=9 Hz, 1 H), 4.48 (dd, J=9, 7 Hz, 1 H), 4.62 (dd, J=7, 5 Hz, 2 H), 4.92 (t, J=7 Hz, 2 H), 4.98 (s, 1 H), 5.34 (quin, J=6 Hz, 1 H), 5.53 (q, J=7 Hz, 1 H), 6.73 (d, J=2 Hz, 1 H), 7.07 (dd, J=8, 2 Hz, 1 H), 7.52 (s, 1 H), 8.17 (d, J=8 Hz, 1 H), 8.42 (s, 1 H), 8.88 (s, 1 H). MS (method B) m/z 466 [M+1]+; t=1.44 min.

### Example 56 and Example 57: 2-((1-((R)-1-Cyanoethyl)-3-isopropoxy-1H-pyrazol-4-yl)amino)-7-((3R,4R)-4-methoxytetrahydrofuran-3-yl)-7H-pyrrolo[2,3-d]pyrimidine-6-carbonitrile and 2-((1-((S)-1-Cyanoethyl)-3-isopropoxy-1H-pyrazol-4-yl)amino)-7-((3R,4R)-4-methoxytetrahydrofuran-3-yl)-7H-pyrrolo[2,3-d]pyrimidine-6-carbonitrile

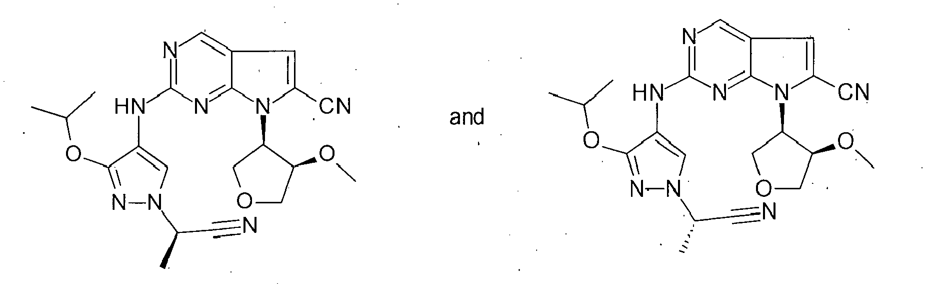

Following the procedure described in Example 47, 7-((3R,4R)-4-methoxytetrahydrofuran-3-yl)-2-(methylsulfonyl)-7H-pyrrolo[2,3-d]pyrimidine-6-carbonitrile (120 mg) (Step 5 of Example 54) and a racemic mixture of (R)-N-(1-(1-cyanoethyl)-3-isopropoxy-1H-pyrazol-4-yl)formamide and (S)-N-(1-(1-cyanoethyl)-3-isopropoxy-1H-pyrazol-4-yl)formamide (racemic mixture) (intermediate 39) gave an isomeric mixture of 2-((1-((R)-1-cyanoethyl)-3-isopropoxy-1H-pyrazol-4-yl)amino)-7-((3R,4R)-4-methoxytetrahydrofuran-3-yl)-7H-pyrrolo[2,3-d]pyrimidine-6-carbonitrile and 2-((1-((S)-1-cyanoethyl)-3-isopropoxy-1H-pyrazol-4-yl)amino)-7-((3R,4R)-4-methoxytetrahydrofuran-3-yl)-7H-pyrrolo[2,3-d]pyrimidine-6-carbonitrile (Referred herein as 2-((1-(1-cyanoethyl)-3-isopropoxy-1H-pyrazol-4-yl)amino)-7-((3R,4R)-4-methoxytetrahydrofuran-3-yl)-7H-pyrrolo[2,3-d]pyrimidine-6-carbonitrile) (95 mg).

Chiral separation performed on 89 mg of the isomeric mixture gave 32 mg of the first eluting isomer and 31 mg of the second eluting isomer (conditions: column ChiralpaK AY, 20 µm, 230X10 mm; liquid phase: Heptane 80%/EtOH 20%/TEA 0.1%; flow rate: 400 mL/min).

Peak 1 (1^{st} isomer): 1H NMR (400 MHz, DMSO-d6) δ ppm: 1.31 (d, J=6 Hz, 6 H), 1.78 (d, J=7 Hz, 3 H), 2.99 (s, 3 H), 3.97 (m, 1 H), 4.05 (m, 1 H), 4.18 (m, 1 H), 4.24 (t, J=9 Hz, 1 H), 4.55 (br dd, J=9, 8 Hz, 1 H), 4.81 (spt, J=6 Hz, 1 H), 5.47 (q, J=7 Hz, 1 H), 5.63 (q, J=7 Hz, 1 H), 7.48 (s, 1 H), 8.16 (br s, 1 H), 8.70 (br s, 1 H), 8.83 (s, 1 H). MS (method B) m/z 437 [M+1]+; t=1.57 min - Example 56 (absolute configuration unknown)

Peak 2 (2^{nd} isomer): 1H NMR (400 MHz, DMSO-d6) δ ppm: 1.31 (d, J=6 Hz, 6 H), 1.78 (d, J=7 Hz, 3 H), 2.99 (s, 3 H), 3.96 (m, 1 H), 4.04 (m, 1 H), 4.15 - 4.29 (m, 2 H), 4.55 (br t, J=8 Hz, 1 H), 4.81 (spt, J=6 Hz, 1 H), 5.47 (q, J=8 Hz, 1 H), 5.62 (q, J=7 Hz, 1 H), 7.48 (s, 1 H), 8.15 (br s, 1 H), 8.71 (br s, 1 H), 8.83 (s, 1 H). MS (method B) m/z 437 [M+1]+; t=1.57 min - Example 57 (absolute configuration unknown)

Generally following the procedure described in Example 54 to 57, reacting 7-((3R,4R)-4-methoxytetrahydrofuran-3-yl)-2-(methylsulfonyl)-7H-pyrrolo[2,3-d]pyrimidine-6-carbonitrile with appropriate formamides gave Example 58 to 69 in table II.

**Table II**

| Example | Name | Formamide | NMR | LC/MS MH+; t, Method |
|---|---|---|---|---|
| 58 | 2-((1-(1-Cyanoethyl)-3 -cyclopropoxy-1H-pyr azol-4-yl)amino)-7-((3 R,4R)-4-methoxytetrah ydrofuran-3-yl)-7H-pyr rolo[2,3-d]pyrimidine-6-carbonitrile | Intermediate 40 | 0.61 - 0.78 (m, 4 H), 1.79 (d, J=7 Hz, 3 H), 2.99 (d, J=1 Hz, 3 H), 3.90 - 4.07 (m, 2 H), 4.10 - 4.29 (m, 3 H), 4.55 (br t, J=7 Hz, 1 H), 5.45 (q, J=7 Hz, 1 H), 5.66 (qd, J=7, 2 Hz, 1 H), 7.47 (s, 1 H), 8.18 (br s, 1 H), 8.81 (s, 1 H), 8.85 (br s, 1 H) | 435; 3.01 C |
| 59 | 2-((1-(2-Cyanopropan-2-yl)-3-cyclopropoxy-1H-pyrazol-4-yl)amino) -7-((3R,4R)-4-methoxy tetrahydrofuran-3-yl)-7H-pyrrolo[2,3-d]pyrimi dine-6-carbonitrile | Intermediate 30 | 0.60 - 0.78 (m, 4 H), 1.96 (s, 6 H), 3.01 (s, 3 H), 3.89 - 4.04 (m, 2 H), 4.11 - 4.29 (m, 3 H), 4.64 (br s, 1 H), 5.36 (q, J=7 Hz, 1 H), 7.47 (s, 1 H), 8.21 (br s, 1 H), 8.82 (s, 1 H), 8.86 (br s, 1 H) | 449; 1.56 B |
| 60 | 2-((1-(2-Cyanopropan-2-yl)-3-isopropoxy-1H-pyrazol-4-yl)amino)-7-((3R,4R)-4-methoxytet rahydrofuran-3-yl)-7H-pyrrolo[2,3-d]pyrimidine-6-carbonitrile | Intermediate 31 | 1.31 (d, J=6 Hz, 6 H), 1.94 (s, 6 H), 3.01 (s, 3 H), 3.87 - 4.08 (m, 2 H), 4.14 - 4.30 (m, 2 H), 4.65 (br s, 1 H), 4.82 (spt, J=6 Hz, 1 H), 5.37 (q, J=7 Hz, 1 H), 7.47 (s, 1 H), 8.19 (br s, 1 H), 8.71 (br s, 1 H), 8.82 (s, 1 H) | 451; 1.66 B |
| 61 | 2-((3-Cyclopropoxy-1-isopropyl-1H-pyrazol-4 -yl)amino)-7-((3R,4R)-4-methoxytetrahydrofu ran-3-yl)-7H-pyrrolo[2, 3-d]pyrimidine-6-carbonitrile | Intermediate 41 | 0.56 - 0.72 (m, 4 H), 1.40 (d, J=7 Hz, 6 H), 3.00 (s, 3 H), 3.94 (dd, J=10, 5 Hz, 1 H), 4.02 (dd, J=10, 5 Hz, 1 H), 4.08(m, 1 H), 4.18 (q, J=7 Hz, 1 H), 4.24 (t, J=9 Hz, 1 H), 4.31 (dt, J=13, 7 Hz, 1 H), 4.56 (m, 1 H), 5.34 (m, 1 H), 7.44 (s, 1 H), 7.96 (br s, 1 H), 8.61 (br s, 1 H), 8.77 (s, 1 H) | 424; 1.50 B |
| 62 | 2-((3-Isopropoxy-1-me thyl-1H-pyrazol-4-yl)a mino)-7-((3R,4R)-4-me thoxytetrahydrofuran-3-yl)-7H-pyrrolo[2,3-d] pyrimidine-6-carbonitrile | Intermediate 38 - | 1.26 (d, J=6 Hz, 6 H), 2.97 (s, 3 H), 3.69 (s, 3 H), 3.96 (dd, J=10, 5 Hz, 1 H), 4.04 (dd, J=10, 5 Hz, 1 H), 4.13 - 4.27 (m, 2 H), 4.47 (br t, J=8 Hz, 1 H), 4.71 (spt, J=6 Hz, 1 H), 5.47 (q, J=7 Hz, 1 H), 7.45 (s, 1 H), 7.87 (br s, 1 H), 8.47 (br s, 1 H), 8.77 (s, 1 H) | 398; 1:39 B |
| 63 | 2-((3-Isopropoxy-1-(oxetan-3-yl)-1H-pyrazol -4-yl)amino)-7-((3R,4R )-4-methoxytetrahydrofuran-3-yl)-7H-pyrrolo[ 2,3-d]pyrimidine-6-carbonitrile | Intermediate 12 | 1.31 (d, J=6 Hz, 6 H), 2.98 (s, 3 H), 3.95 (dd, J=10, 5 Hz, 1 H), 4.03 (dd, J=10, 5 Hz, 1 H), 4.17 (ddd, J=5, 4, 3 Hz, 1 H), 4.22 (t, J=9 Hz, 1 H), 4.52 (br t, J=6 Hz, 1 H), 4.75 - 5.02 (m, 5 H), 5.30 - 5.53 (m, 2 H), 7.46 (s, 1 H), 8.09 (br s, 1 H), 8.57 (br s, 1 H), 8.79 (s, 1 H) | 440; 1.43 B |
| 64 | 2-((3-Cyclopropoxy-1-(oxetan-3-yl)-1H-pyrazol-4-yl)amino)-7-((3R, 4R)-4-methoxytetrahydrofuran-3-yl)-7H-pyrrolo[2,3-d]pyrimidine-6-carbonitrile | Intermediate 11 | 0.60 - 0.76 (m, 4 H), 2.98 (s, 3 H), 3.89 - 4.08 (m, 2 H), 4.16 (m, 2 H), 4.22 (t, J=9 Hz, 1 H), 4.53 (br J=6 Hz, 1 H), 4.84 - 4.95 (m, 4 H), 5.31 - 5.52 (m, 2 H), 7.46 (s, 1 H), 8.14 (br s, 1 H), 8.72 (br s, 1 H), 8.79 (s, 1 H) | t, 438; 1.33 B |
| 65 | 2-((3-(3,3-Difluorocyclobutoxy)-1-methyl-1H-pyrazol-4-yl)amino)-7-((3R,4R)-4-methoxytetrahydrofuran-3-yl)-7H-pyrrolo[2,3-d]pyrimidine-6-carbonitrile | Intermediate 42 . | 2.64 - 2.81 (m, 2 H), 2.97 (s, 3 H), 2.99 - 3.14 (m, 2 H), 3.70 (s, 3 H), 3.95 (dd, J=10, 5 Hz, 1 H), 4.04(m, 1 H), 4.12 - 4.25 (m, 2 H), 4.48 (br t, J=8 Hz, 1 H), 4.83 (m, 1 H), 5.46 (q, J=7 Hz, 1 H), 7.45 (s, 1 H), 7.92 (br s, 1 H), 8.65 - 8.89 (m, 2 H) | 446; 1.46 B |
| 66 | 2-((3-((R)-sec-Butoxy)-1-methyl-1H-pyrazol-4-yl)amino)-7-((3R,4R)-4-methoxytetrahydrofuran-3-yl)-7H-pyrrolo[2,3-d]pyrimidine-6-carbonitrile | Intermediate 47 | 0.87 (t, J=7 Hz, 3 H), 1.23 (d, J=6 Hz, 3 H), 1.46 - 1.73 (m, 2 H), 2.97 (s, 3 H), 3.68 (s, 3 H), 3.95 (dd, J=10, 5 Hz, 1 H), 4.04 (dd, J=10, 5 Hz, 1 H), 4.13 - 4.26 (m, 2 H), 4.41 - 4.58 (m, 2 H), 5.47 (q, J=7 Hz; 1 H), 7.44 (s, 1 H), 7.89 (br s, 1 H), 8.41 (br s, 1 H), 8.77 (s, 1 H) | 412; 1.17 D |
| 67 | 2-((3-((S)-sec-Butoxy)-1-methyl-1H-pyrazol-4-yl)amino)-7-((3R,4R)-4-methoxytetrahydrofuran-3-yl)-7H-pyrrolo[ 2,3-d]pyrimidine-6-carbonitrile | Intermediate 46 | 0.88 (t, J=7 Hz, 3 H), 1.23 (d, J=6 Hz, 3 H), 1.48 - 1.72 (m, 2 H), 2.97 (s, 3 H), 3.68 (s, 3 H), 3.95 (dd, J=10, 5 Hz, 1 H), 4.05 (dd, J=10, 5 Hz, 1 H), 4.17 (td, J=5, 3 Hz, 1 H), 4.22 (dd, J=9, 9 Hz, 1 H), 4.42 - 4.57 (m, 2 H), 5.47 (q, J=7 Hz, 1 H), 7.44 (s, 1 H), 7.86 (br s, 1 H), 8.40 (br s, 1 H), 8.77 (s, 1 H) | 412; 1.17 D |
| 68 | 2-((2,2-Dimethyl-3,4-dihydro-2H-pyrano[3,2-b]pyridin-8-yl)amino)-7-((3R,4R)-4-methoxytetrahydrofuran-3-yl)-7H-pyrrolo[2,3-d]pyrimidine-6-carbonitrile | Intermediate 48 | 1.39 (d, J=2 Hz, 6 H), 1.94 (t, J=7 Hz, 2 H), 2.84 (t, J=7 Hz, 2 H), 2.97 (s, 3 H), 3.99 (dd, J=10, 5 Hz, 1 H), 4.08 (dd, J=10, 5 Hz, 1 H), 4.20 - 4.32 (m, 2 H), 4.53 (dd, J=10, 7 Hz, 1 H), 5.64 (q, J=7 Hz, 1 H), 7.59 (s, 1 H), 8.01 (d, J=5 Hz, 1 H), 8.17 (s, 1 H), 8.36 (d, J=5 Hz, 1 H), 9.00 (s, 1 H) | 421; 0.69 D |
| 69 | 7-((3R,4R)-4-Methoxytetrahydrofuran-3-yl)-2-(((R)-2-methyl-3,4-dih ydro-2H-[1,4]dioxepino[2,3-b]pyridin-9-yl)amino)-7H-pyrrolo[2,3-d]pyrimidine-6-carbonitrile | Intermediate 51 | 1.45 (d, J=6 Hz, 3 H), 1.99(m, 1 H), 2.22 (m, 1 H), 2.98 (s, 3 H), 4.00 (m, 1 H), 4.05 - 4.16 (m, 2 H), 4.20 - 4.33 (m, 3 H), 4.41 (ddd, J=12, 8, 4 Hz, 1 H), 4.52 (dd, J=10, 7 Hz, 1 H), 5.64 (q, J=7Hz, 1 H), 7.62 (s, 1 H), 7.82 (d, J=6 Hz, 1 H), 8.28 (d, J=6 Hz, 1 H), 8.38 (s, 1 H), 9.02 (s, 1 H | 423; 1.14 B |

### Example 70: 7-((3R,4S)-4-Methoxytetrahydrofuran-3-yl)-2-((1-methyl-3-(oxetan-3-yloxy)-1H-pyrazol-4-yl)amino)-7H-pyrrolo[2,3-d]pyrimidine-6-carbonitrile and 7-((3S,4R)-4-methoxytetrahydrofuran-3-yl)-2-((1-methyl-3-(oxetan-3-yloxy)-1H-pyrazol-4-yl)amino)-7H-pyrrolo[2,3-d]pyrimidine-6-carbonitrile (racemic mixture)

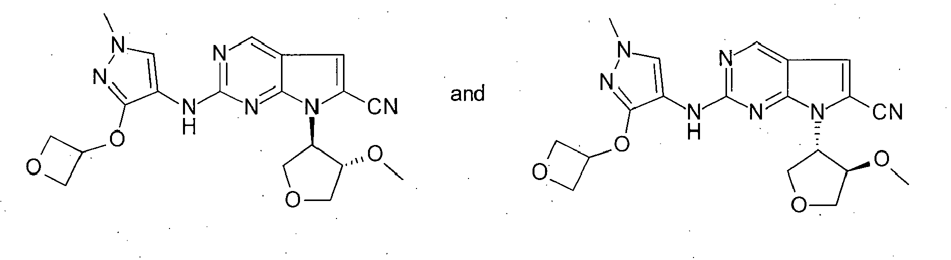

### Step 1: Preparation of methyl ((3S,4R)-4-methoxytetrahydrofuran-3-yl)glycinate and methyl ((3R,4S)-4-methoxytetrahydrofuran-3-yl)glycinate (racemic mixture)

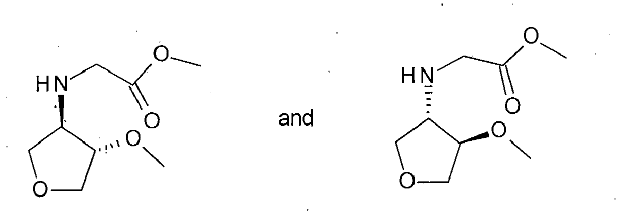

4 mL (1 eq.) of methyl 2-bromoacetate in solution in 10 mL of tetrahydrofuran was added slowly to a cooled solution of 5 g (1 eq.) of 4-methoxytetrahydrofuran-3-amine (commercially available) and 6 mL of triethylamine (1 eq.) in 30 mL of tetrahydrofuran at 0°C. After stirring for 5 hours at room temperature and completion of the reaction, the mixture was filtered. The filtrate was concentrated under vacuum and purified by column chromatography (eluent: dichloromethane 100% then dichloromethane/methanol (95/5) to afford 7 g of a racemic mixture of methyl ((3S,4R)-4-methoxytetrahydrofuran-3-yl)glycinate and methyl ((3R,4S)-4-methoxytetrahydrofuran-3-yl)glycinate.

### Step 2: Preparation of methyl N-(5-formyl-2-(methylthio)pyrimidin-4-yl)-N-((3S,4R)-4-methoxytetrahydrofuran-3-yl)glycinate and methyl N-(5-formyl-2-(methylthio)pyrimidin-4-yl)-N-((3R,4S)-4-methoxytetrahydrofuran-3-yl)glycinate (racemic mixture)

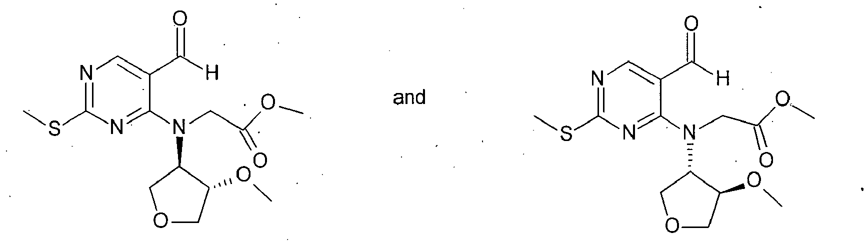

1.6 mL of triethylamine (1.7 eq.) was added to a solution of 1.25 g of 4-chloro-2-(methylthio)pyrimidine-5-carbaldehyde (1 eq.) and 1.4 g of a racemic mixture of methyl ((3S,4R)-4-methoxytetrahydrofuran-3-yl)glycinate and methyl ((3R,4S)-4-methoxytetrahydrofuran-3-yl)glycinate (1.1 eq) in 15 mL of tetrahydrofuran. The mixture was stirred at room temperature for 60 hours, diluted with 100 mL of a 50/50 mixture of ethyl acetate/diethyl ether and poured into water. The organic layer was separated, washed twice with water. The organic layer was dried over magnesium sulfate and concentrated under reduced pressure. The residue was purified on silica, eluting with a mixture of heptane/ethylacetate (100/0 to 70/30) to afford a racemic mixture of methyl N-(5-formyl-2-(methylthio)pyrimidin-4-yl)-N-((3S,4R)-4-methoxytetrahydrofuran-3-yl)glycinate and methyl N-(5-formyl-2-(methylthio)pyrimidin-4-yl)-N-((3R,4S)-4-methoxytetrahydrofuran-3-yl)glycinate. MS (method A) m/z 342 [M+1]+; t=1.83 min.

### Step 3: Preparation of 7-((3S,4R)-4-methoxytetrahydrofuran-3-yl)-2-(methylthio)-7H-pyrrolo[2,3-d]pyrimidine-6-carboxylic acid and 7-((3R,4S)-4-methoxytetrahydrofuran-3-yl)-2-(methylthio)-7H-pyrrolo[2,3-d]pyrimidine-6-carboxylic acid (racemic mixture)

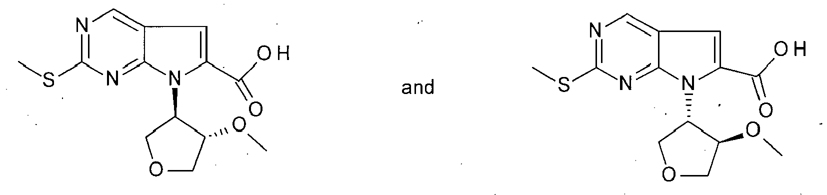

2.65 mL (5 eq.) of 1,8-diazabicyclo[5.4.0]undec7-ene was added dropwise to a solution of 1.16 g of a racemic mixture of methyl N-(5-formyl-2-(methylthio)pyrimidin-4-yl)-N-((3S,4R)-4-methoxytetrahydrofuran-3-yl)glycinate and methyl N-(5-formyl-2-(methylthio)pyrimidin-4-yl)-N-((3R,4S)-4-methoxytetrahydrofuran-3-yl)glycinate (1 eq.) in 15 mL of acetonitrile. The reaction mixture was heated at 80°C for 1 hour. After cooling down to room temperature the reaction mixture was taken into 100 mL of ethyl acetate and washed with a 1N HCl aqueous solution. The organic layer was dried over magnesium sulfate and concentrated under reduced pressure. The residue was dissolved in 32 mL of tetrahydrofurane and 415 mg of lithium hydroxide (5 eq.) in 8.6 mL of water were added. The reaction mixture was stirred at room temperature for 1 hour. A 2N aqueous solution of hydrochloric acid was added (to reach pH 7) and then 100 mL of ethyl acetate was added. The organic layer was washed with 50 mL of water and 50 mL of brine, dried over magnesium sulfate, and concentrated under reduced pressure to afford 1.13 g of a racemic mixture of 7-((3S,4R)-4-methoxytetrahydrofuran-3-yl)-2-(methylthio)-7H-pyrrolo[2,3-d]pyrimidine-6-carboxylic acid and 7-((3R,4S)-4-methoxytetrahydrofuran-3-yl)-2-(methylthio)-7H-pyrrolo[2,3-d]pyrimidine-6-carboxylic acid. MS (method A) m/z 310 [M+ 1]+; t= 2.18 min.

### Step 4: Preparation of 7-((3S,4R)-4-methoxytetrahydrofuran-3-yl)-2-(methylthio)-7H-pyrrolo[2,3-d]pyrimidine-6-carboxamide and 7-((3R,4S)-4-methoxytetrahydrofuran-3-yl)-2-(methylthio)-7H-pyrrolo[2,3-d]pyrimidine-6-carboxamide (racemic mixture)

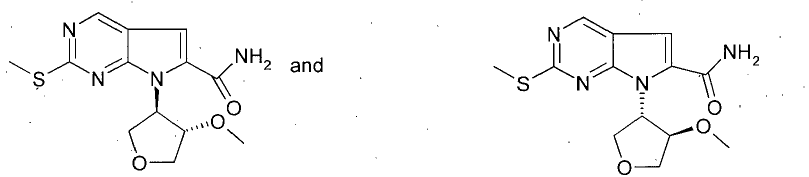

0.74 g (1.25 eq.) of di(1H-imidazol-1-yl)methanone (CDI) was added to a racemic mixture of 1.13 g of 7-((3S,4R)-4-methoxytetrahydrofuran-3-yl)-2-(methylthio)-7H-pyrrolo[2,3-d]pyrimidine-6-carboxylic acid and 7-((3R,4S)-4-methoxytetrahydrofuran-3-yl)-2-(methylthio)-7H-pyrrolo[2,3-d]pyrimidine-6-carboxylic acid (1 eq.) in 11 mL of dimethylformamide. The mixture was stirred for 1 hour at room temperature and 2.4 mL of a 28% ammonium hydroxide solution (5 eq.) was added. The reaction mixture was stirred at room temperature for 2.5 hours, diluted with 100 mL of ethyl acetate, and washed with water. The organic layer was washed with 50 mL of water and 50 mL of brine, dried over magnesium sulfate, and concentrated under reduced pressure to afford 0.53 g of a racemic mixture of 7-((3S,4R)-4-methoxytetrahydrofuran-3-yl)-2-(methylthio)-7H-pyrrolo[2,3-d]pyrimidine-6-carboxamide and 7-((3R,4S)-4-methoxytetrahydrofuran-3-yl)-2-(methylthio)-7H-pyrrolo[2,3-d]pyrimidine-6-carboxamide. MS (method A) m/z 309 [M+ 1]+; t= 1.81 min.

### Step 5: Preparation of 7-((3S,4R)-4-methoxytetrahydrofuran-3-yl)-2-(methylthio)-7H-pyrrolo[2,3-d]pyrimidine-6-carbonitrile and 7-((3R,4S)-4-methoxytetrahydrofuran-3-yl)-2-(methylthio)-7H-pyrrolo[2,3-d]pyrimidine-6-carbonitrile (racemic mixture)

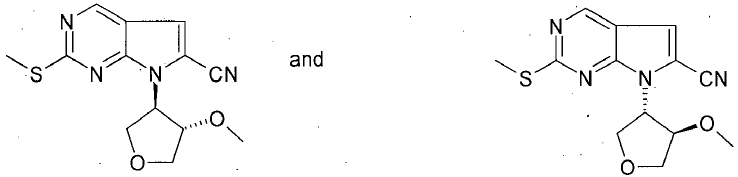

0.87 mL (3.6 eq.) of trifluoroacetic anhydride was added to a solution of 0.53 g of a racemic mixture of 7-((3S,4R)-4-methoxytetrahydrofuran-3-yl)-2-(methylthio)-7H-pyrrolo[2,3-d]pyrimidine-6-carboxamide and 7-((3R,4S)-4-methoxytetrahydrofuran-3-yl)-2-(methylthio)-7H-pyrrolo[2,3-d]pyrimidine-6-carboxamide (1 eq.), 1.1 mL (4.5 eq.) of triethylamine in 8 mL of anhydrous tetrahydrofuran at 0°C under argon. The mixture was stirred for 1 hour at room temperature, poured into water and extracted with twice with 50 mL of ethyl acetate. The combined organic layers were washed twice with 50 mL of water then dried over magnesium sulfate and concentrated under reduced pressure. The residue was purified on silica, eluting with a mixture of heptane/ethylacetate (100/0 to 50/50) to afford 344 mg of a racemic mixture of 7-((3S,4R)-4-methoxytetrahydrofuran-3-yl)-2-(methylthio)-7H-pyrrolo[2,3-d]pyrimidine-6-carbonitrile and 7-((3R,4S)-4-methoxytetrahydrofuran-3-yl)-2-(methylthio)-7H-pyrrolo[2,3-d]pyrimidine-6-carbonitrile. MS (method A) m/z 291 [M+1]+; t=2.07 min.

### Step 6: Preparation of 7-((3S,4R)-4-methoxytetrahydrofuran-3-yl)-2-(methylsulfonyl)-7H-pyrrolo[2,3-d]pyrimidine-6-carbonitrile and 7-((3R,4S)-4-methoxytetrahydrofuran-3-yl)-2-(methylsulfonyl)-7H-pyrrolo[2,3-d]pyrimidine-6-carbonitrile (racemic mixture)

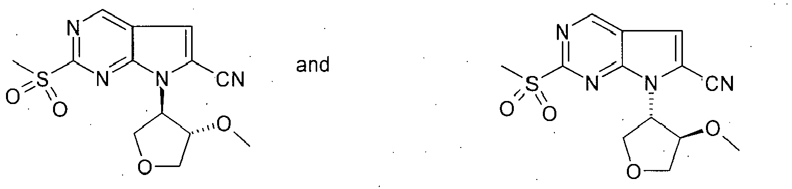

797 mg of 3-chloroperbenzoic acid (77% purity; 2.5 eq.) was added to a solution of 344 mg (1 eq.) of a racemic mixture of 7-((3S,4R)-4-methoxytetrahydrofuran-3-yl)-2-(methylthio)-7H-pyrrolo[2,3-d]pyrimidine-6-carbonitrile and 7-((3R,4S)-4-methoxytetrahydrofuran-3-yl)-2-(methylthio)-7H-pyrrolo[2,3-d]pyrimidine-6-carbonitrile in 6 mL of dichloromethane at 0°C. The mixture was stirred for 1 hour at room temperature then quenched with 10 mL of dichloromethane and 10 mL of a 10% aqueous sodium thiosulfate solution. The organic layer was washed successively with 10 mL of an aqueous saturated solution of sodium hydrogenocarbonate, 10 mL of brine, then dried over magnesium sulfate, and concentrated under reduced pressure to afford 363 mg of a racemic mixture of 7-((3S,4R)-4-methoxytetrahydrofuran-3-yl)-2-(methylsulfonyl)-7H-pyrrolo[2,3-d]pyrimidine-6-carbonitrile and 7-((3R,4S)-4-methoxytetrahydrofuran-3-yl)-2-(methylsulfonyl)-7H-pyrrolo[2,3-d]pyrimidine-6-carbonitrile. MS (method A) m/z 323 [M+1]+; t=1.49 min.

### Step7: Preparation of 7-((3R,4S)-4-methoxytetrahydrofuran-3-yl)-2-((1-methyl-3-(oxetan-3-yloxy)-1H-pyrazol-4-yl)amino)-7H-pyrrolo[2,3-d]pyrimidine-6-carbonitrile and 7-((3S,4R)-4-methoxytetrahydrofuran-3-yl)-2-((1-methyl-3-(oxetan-3-yloxy)-1H-pyrazol-4-yl)amino)-7H-pyrrolo[2,3-d]pyrimidine-6-carbonitrile (racemic mixture) - Example 70

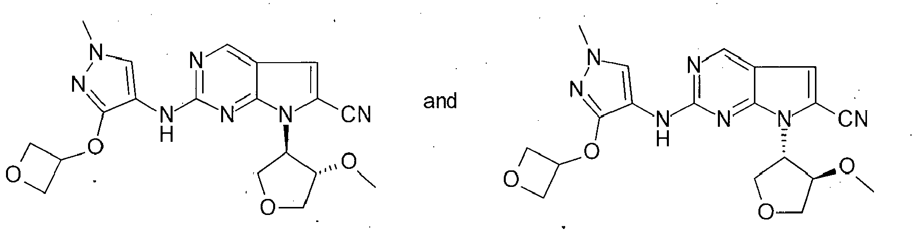

Following the procedure described in Example 47, a racemic mixture of 7-((3S,4R)-4-methoxytetrahydrofuran-3-yl)-2-(methylsulfonyl)-7H-pyrrolo[2,3-d]pyrimidine-6-carbonitrile and 7-((3R,4S)-4-methoxytetrahydrofuran-3-yl)-2-(methylsulfonyl)-7H-pyrrolo[2,3-d]pyrimidine-6-carbonitrile (62 mg) and N-(1-methyl-3-(oxetan-3-yloxy)-1H-pyrazol-4-yl)formamide (intermediate 4) gave a racemic mixture of 7-((3R,4S)-4-methoxytetrahydrofuran-3-yl)-2-((1-methyl-3-(oxetan-3-yloxy)-1H-pyrazol-4-yl)amino)-7H-pyrrolo[2,3-d]pyrimidine-6-carbonitrile and 7-((3S,4R)-4-methoxytetrahydrofuran-3-yl)-2-((1-methyl-3-(oxetan-3-yloxy)-1H-pyrazol-4-yl)amino)-7H-pyrrolo[2,3-d]pyrimidine-6-carbonitrile (Referred herein as 7-((trans)-4-methoxytetrahydrofuran-3-yl)-2-((1-methyl-3-(oxetan-3-yloxy)-1H-pyrazol-4-yl)amino)-7H-pyrrolo[2,3-d]pyrimidine-6-carbonitrile)― Example 70
1H NMR (400 MHz, DMSO-d6) δ ppm: 3.22 (s, 3 H), 3.66 (s, 3 H), 3.78 - 4.00 (m, 2 H), 4.03 (dd, J=9, 7 Hz, 1 H), 4.27 (t, J=9 Hz, 1 H), 4.43 (br s, 1 H), 4.50 - 4.57 (m, 2 H), 4.75 - 4.83 (m, 2 H), 4.86 (br s, 1 H), 5.30 (quin, J=6 Hz, 1 H), 7.49 (s, 1 H), 7.81 (br s, 1 H), 8.83 (s, 1 H), 8.87 (br s, 1 H). MS (method B) m/z 412 [M+1]+; t=1.28 min.

Example 71: 2-((4-(2-Hydroxypropan-2-yl)-2-(oxetan-3-yloxy)phenyl)amino)-7-((3S,4R)-4-methoxytetrahydrofuran-3-yl)-7H-pyrrolo[2,3-d]pyrimidine-6-carbonitrile and 2-((4-(2-hydroxypropan-2-yl)-2-(oxetan-3-yloxy)phenyl)amino)-7-((3R,4S)-4-methoxytetrahydrofuran-3-yl)-7H-pyrrolo[2,3-d]pyrimidine-6-carbonitrile (racemic mixture)

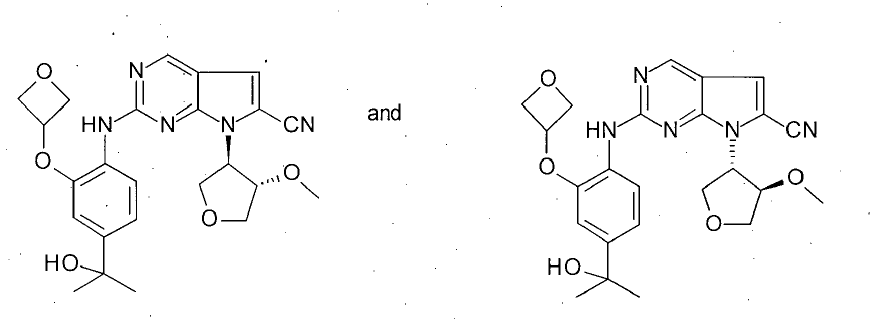

### Step 1: Preparation of methyl 4-((6-cyano-7-((3S,4R)-4-methoxytetrahydrofuran-3-yl)-7H-pyrrolo[2,3-d]pyrimidin-2-yl)amino)-3-(oxetan-3-yloxy)benzoate and methyl 4-((6-cyano-7-((3R,4S)-4-methoxytetrahydrofuran-3-yl)-7H-pyrrolo[2,3-d]pyrimidin-2-yl)amino)-3-(oxetan-3-yloxy)benzoate (racemic mixture)

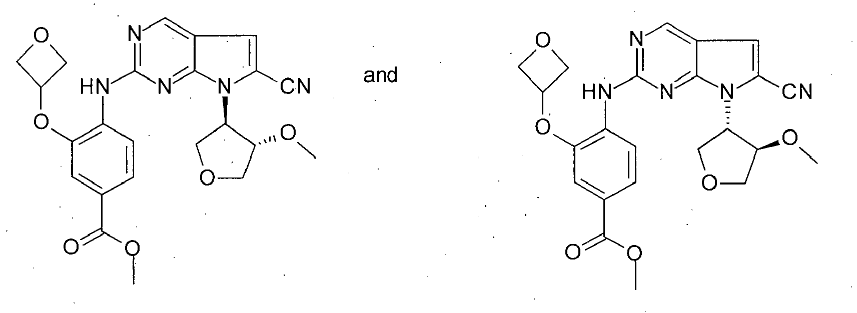

Generally following the procedure described in Step 5 of Example 1, a racemic mixture of 7-((3S,4R)-4-methoxytetrahydrofuran-3-yl)-2-(methylsulfonyl)-7H-pyrrolo[2,3-d]pyrimidine-6-carbonitrile and 7-((3R,4S)-4-methoxytetrahydrofuran-3-yl)-2-(methylsulfonyl)-7H-pyrrolo[2,3-d]pyrimidine-6-carbonitrile (100 mg) (Step 6 of Example 70) and methyl 4-formamido-3-(oxetan-3-yloxy)benzoate (86 mg) (Intermediate 35) gave a racemic mixture of methyl 4-((6-cyano-7-((3S,4R)-4-methoxytetrahydrofuran-3-yl)-7H-pyrrolo[2,3-d]pyrimidin-2-yl)amino)-3-(oxetan-3-yloxy)benzoate and methyl 4-((6-cyano-7-((3R,4S)-4-methoxytetrahydrofuran-3-yl)-7H-pyrrolo[2,3-d]pyrimidin-2-yl)amino)-3-(oxetan-3-yloxy)benzoate (58 mg). MS (method A) m/z 466 [M+1]+; t=2.39 min

### Step 2: Preparation of 2-((4-(2-Hydroxypropan-2-yl)-2-(oxetan-3-yloxy)phenyl)amino)-7-((3S,4R)-4-methoxytetrahydrofuran-3-yl)-7H-pyrrolo[2,3-d]pyrimidine-6-carbonitrile and 2-((4-(2-hydroxypropan-2-yl)-2-(oxetan-3-yloxy)phenyl)amino)-7-((3R,4S)-4-methoxytetrahydrofuran-3-yl)-7H-pyrrolo[2,3-d]pyrimidine-6-carbonitrile (racemic mixture)-Example 71

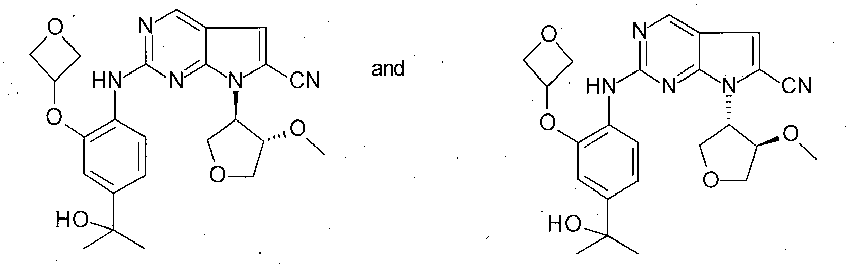

415 µL (10 eq.) of methyl magnesium bromide 3M in tetrahydrofuran was added to a solution of 58 mg (1 eq.) of a racemic mixture of methyl 4-((6-cyano-7-((3S,4R)-4-methoxytetrahydrofuran-3-yl)-7H-pyrrolo[2,3-d]pyrimidin-2-yl)amino)-3-(oxetan-3-yloxy)benzoate and methyl 4-((6-cyano-7-((3R,4S)-4-methoxytetrahydrofuran-3-yl)-7H-pyrrolo[2,3-d]pyrimidin-2-yl)amino)-3-(oxetan-3-yloxy)benzoate in 4 mL of tetrahydrofuran at 0°C. The reaction mixture was stirred for 30 minutes at 0°C then the reaction mixture was quenched with a saturated aqueous solution of ammonium chloride and extracted with ethyl acetate. The organic layer was washed with 50 mL of water and 50 mL of brine, dried over magnesium sulfate, and concentrated under reduced pressure. The residue was purified on silica, eluting with a mixture of heptane/AcOEt /7N methanolic ammonia solution (80/18/2) to afford 31 mg of racemic mixture of 2-((4-(2-hydroxypropan-2-yl)-2-(oxetan-3-yloxy)phenyl)amino)-7-((3S,4R)-4-methoxytetrahydrofuran-3-yl)-7H-pyrrolo[2,3-d]pyrimidine-6-carbonitrile and 2-((4-(2-hydroxypropan-2-yl)-2-(oxetan-3-yloxy)phenyl)amino)-7-((3R,4S)-4-methoxytetrahydrofuran-3-yl)-7H-pyrrolo[2,3-d]pyrimidine-6-carbonitrile (Referred herein as 2-((4-(2-hydroxypropan-2-yl)-2-(oxetan-3-yloxy)phenyl)amino)-7-((trans)-4-methoxytetrahydrofuran-3-yl)-7H-pyrrolo[2,3-d]pyrimidine-6-carbonitrile)-Example 71
1H NMR (400 MHz, DMSO-d6) δ ppm: 1.42 (s, 6 H), 3.23 (s, 3 H), 3.82 (dd, J=10, 3 Hz, 1 H), 3.97 - 4.10 (m, 2 H), 4.28 (t, J=9 Hz, 1 H), 4.49 (dt, J=6, 3 Hz, 1 H), 4.59 (dd, J=7, 5 Hz, 2 H), 4.86 - 4.98 (m, 3 H), 5.00 (s, 1 H), 5.31 (quin, J=6 Hz, 1 H), 6.74 (d, J=2 Hz, 1 H), 7.06 (dd, J=8, 2 Hz, 1 H), 7.55 (s, 1 H), 7.95 (d, J=8 Hz, 1 H), 8.61 (s, 1 H), 8.91 (s, 1 H). MS (method B) m/z 466 [M+1]+; t=1.45 min.

Example 72 and Example 73: 7-((3S,4R)-4-Ethyltetrahydrofuran-3-yl)-2-((3-isopropoxy-1-methyl-1H-pyrazol-4-yl)amino)-7H-pyrrolo[2,3-d]pyrimidine-6-carbonitrile and 7-((3R,4S)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile (racemic *cis* mixture) 7-((3S,4S)-4-Ethyltetrahydrofuran-3-yl)-2-((3-isopropoxy-1-methyl-1H-pyrazol-4-yl)amino)-7H-pyrrolo[2,3-d]pyrimidine-6-carbonitrile and 7-((3R,4R)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile -(racemic *trans* mixture)

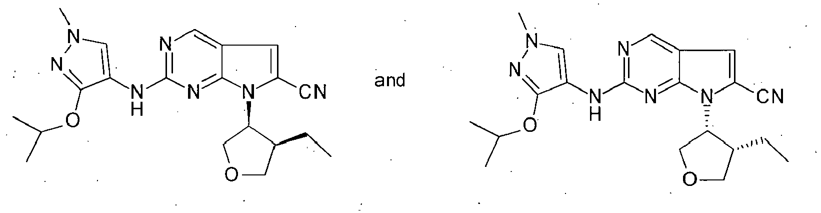

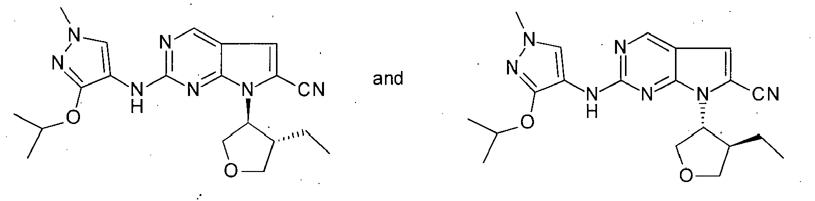

### Step 1: Preparation of 4-ethyltetrahydrofuran-3-ol (racemic cis and trans isomers)

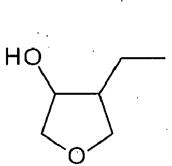

502 mg (1.1 eq.) of sodium borohydride was added portionwise to a solution of 1.38 g (1 eq.) of racemic 4-ethyldihydrofuran-3(2H)-one (commercially available) in a mixture of 15 mL of anhydrous THF and 30 mL of absolute ethanol at 0°C. The reaction mixture was stirred for 1 hour at room temperature then cooled back to 0°C and quenched by addition of 20 mL of a 1N aqueous hydrogen chloride solution. The mixture was poured onto 100 mL of ethyl acetate and 100 mL of brine under stirring and the organic layer was concentrated under vacuum. The residue was dissolved in dichloromethane, dried over sodium sulfate, filtered and concentrated under vacuum. The residue was purified on silica gel, eluting with a gradient of 0 to 60% of ethyl acetate in cyclohexane, to afford 898 mg of a unseparable mixture of racemic *cis* and racemic *trans* isomers of 4-ethyltetrahydrofuran-3-ol in the ratio 1/2 (1H NMR) and as a colorless oil.
1H NMR (400 MHz, CDCl3) δ in ppm:
*Cis* isomer: 1.01 (t, J=7.5 Hz, 3 H), 1.30 (m, 1 H), 1.61 (m, 1 H), 1.90 (broad, 1 H), 2.10 (m, 1 H), 3.54 (dd, J=8 and 10.2 Hz, 1 H), 3.94 (dd, J=9.8 and 4 Hz, 1 H), 3.99 (t, J=8.4 Hz, 1 H), 4.12 (m, 1 H), 4.31 (t, J=4.2 Hz, 1 H).
*Trans* isomer: 0.98 (t, J=7.5 Hz, 3 H), 1.34 (m, 1 H), 1.48 (m, 1 H), 1.90 (broad, 1 H), 2.03 (m, 1 H), 3.47 (dd, J=8.4 and 5 Hz, 1 H), 3.72 (dd, J=10 and 2.3 Hz, 1 H), 3.87 (m, 2 H), 4.15 (m, 1 H).

### Step 2: Preparation of methyl 7-(4-ethyltetrahydrofuran-3-yl)-2-(methylthio)-7H-pyrrolo[2,3-d]pyrimidine-6-carboxylate (racemic cis and trans isomers)

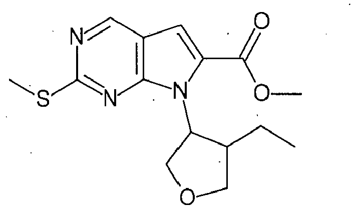

3.05 g (3 eq.) of triphenylphosphine and 1.14 mL (1.5 eq.) of diisopropylazodicarboxylate (DIAD) were added to a solution of 865 mg (1 eq.) of methyl 2-(methylthio)-7H-pyrrolo[2,3-d]pyrimidine-6-carboxylate and 675 mg (1.5 eq.) of a mixture of racemic *cis* and racemic *trans* isomers of 4-ethyltetrahydrofuran-3-ol in 40 mL of anhydrous tetrahydrofuran under argon. The reaction mixture was stirred at 60°C for 2 hours then poured onto 100 mL of ethyl acetate and 50 mL of water. The aqueous layer was extracted with 20 mL of ethyl acetate and the combined organic layers dried over sodium sulfate and concentrated under vacuum. The residue was purified on silica gel, eluting with a gradient of 0 to 30% of ethyl acetate in cyclohexane, to afford 1.23 g of a unseparable mixture of racemic *cis* and racemic *trans* isomers of methyl 7-(4-ethyltetrahydrofuran-3-yl)-2-(methylthio)-7H-pyrrolo[2,3-d]pyrimidine-6-carboxylate in the ratio 36/64 (LC/MS). MS (method F): Cis isomer: m/z 322 [M+1]+; t = 1.96 min ; *Trans* isomer: m/z 322 [M+1]+; t=1.90 min.

### Step 3: Preparation of 7-(4-ethyltetrahydrofuran-3-yl)-2-(methylthio)-7H-pyrrolo[2,3-d]pyrimidine-6-carboxamide (racemic cis and trans isomers)

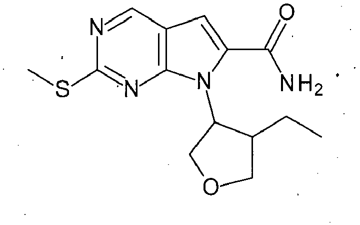

A solution of 1.23 g (1 eq.) of a mixture of racemic *cis* and racemic *trans* isomers of methyl 7-(4-ethyltetrahydrofuran-3-yl)-2-(methylthio)-7H-pyrrolo[2,3-d]pyrimidine-6-carboxylate in 70 mL of a 7N methanolic ammonia solution (130 eq.) in a closed vessel was heated under stirring at 100°C for 4 days. The reaction mixture was cooled, concentrated under vacuum and purified on silica gel, eluting with a gradient of 0 to 70% of ethyl acetate in cyclohexane, to afford 563 mg of a unseparable mixture of racemic cis and racemic *trans* isomers of methyl 7-(4-ethyltetrahydrofuran-3-yl)-2-(methylthio)-7H-pyrrolo[2,3-d]pyrimidine-6-carboxamide in the ratio 33/67 (LC/MS). MS (method F): *Cis* isomer: m/z 307 [M+1]+; t=1.41 min ; *Trans* isomer: m/z 307 [M+1]+; t=1.36 min.

### Step 4: Preparation of 7-(4-ethyltetrahydrofuran-3-yl)-2-(methylthio)-7H-pyrrolo[2,3-d]pyrimidine-6-carbonitrile (racemic cis and trans isomers)

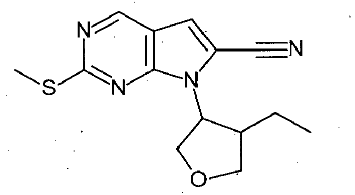

1.16 ml (4.5 eq.) of triethylamine and 920 µL (3.6 eq.) of trifluoroacetic anhydride were added successively dropwise to a cooled (0°C) solution of 563 mg (1 eq.) of a mixture of racemic *cis* and racemic *trans* isomers of methyl 7-(4-ethyltetrahydrofuran-3-yl)-2-(methylthio)-7H-pyrrolo[2,3-d]pyrimidine-6-carboxamide in 17 mL of anhydrous tetrahydrofuran under argon. The reaction mixture was allowed to stir for 1 hour at room temperature, and then poured onto 20 mL of ethyl acetate and 20 mL of a 10% aqueous sodium carbonate solution with vigorous stirring for 15 min. The organic layer was dried over sodium sulfate and concentrated under vacuum. The residue was purified on silica gel, eluting with a gradient of 0 to 20% of ethyl acetate in cyclohexane, to afford 597 mg of a unseparable mixture of racemic *cis* and racemic *trans* isomers of methyl 7-(4-ethyltetrahydrofuran-3-yl)-2-(methylthio)-7H-pyrrolo[2,3-d]pyrimidine-6-carbonitrile. MS (method G): Cis isomer: m/z 289 [M+1]+; t=2.18 min; *Trans* isomer: m/z 289 [M+1]+; t=2.10 min.

### Step 5: Preparation of 7-((3S,4R)-4-ethyltetrahydrofuran-3-yl)-2-(methylsulfonyl)-7H-pyrrolo[2,3-d]pyrimidine-6-carbonitrile and 7-((3R,4S)-4-ethyltetrahydrofuran-3-yl)-2-(methylsulfonyl)-7H-pyrrolo[2,3-d]pyrimidine-6-carbonitrile (racemic cis) and 7-((3S,4S)-4-ethyltetrahydrofuran-3-yl)-2-(methylsulfonyl)-7H-pyrrolo[2,3-d]pyrimidine-6-carbonitrile and 7-((3R,4R)-4-ethyltetrahydrofuran-3-yl)-2-(methylsulfonyl)-7H-pyrrolo[2,3-d]pyrimidine-6-carbonitrile (racemic trans)

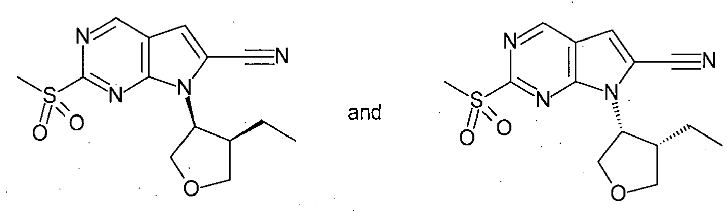

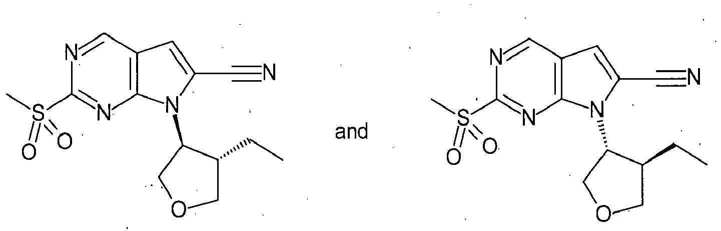

1.05g (2.05 eq.) of m-chloroperbenzoic acid (70% purity) was added by portions to a cooled (0°C) solution of 597 mg (1 eq.) of a mixture of racemic *cis* and racemic *trans* isomers of methyl 7-(4-ethyltetrahydrofuran-3-yl)-2-(methylthio)-7H-pyrrolo[2,3-d]pyrimidine-6-carbonitrile in 20 mL of dichloromethane. The reaction mixture was allowed to stir for 15 minutes at 0°C and then 1 hour at room temperature then was diluted with 30 mL of dichloromethane. The organic layer was washed successively with 10 mL of a 2N aqueous solution of sodium hydrogenosulfite and twice with 20 ml of a 10% aqueous sodium carbonate solution then dried over sodium sulfate and concentrated under vacuum. The residue was purified on silica gel, eluting with a gradient of 0 to 70% of ethyl acetate in cyclohexane, to afford successively 97 mg of a first mixture and 281 mg of a second mixture.

The first mixture was characterized by NMR as the racemic *cis* mixture of 7-((3S,4R)-4-ethyltetrahydrofuran-3-yl)-2-(methylsulfonyl)-7H-pyrrolo[2,3-d]pyrimidine-6-carbonitrile and 7-((3R,4S)-4-ethyltetrahydrofuran-3-yl)-2-(methylsulfonyl)-7H-pyrrolo[2,3-d]pyrimidine-6-carbonitrile. MS (method F) m/z 321 [M+1]+; t=1.37 min

The second mixture was characterized by NMR as the racemic *trans* mixture of 7-((3S,4S)-4-ethyltetrahydrofuran-3-yl)-2-(methylsulfonyl)-7H-pyrrolo[2,3-d]pyrimidine-6-carbonitrile and 7-((3R,4R)-4-ethyltetrahydrofuran-3-yl)-2-(methylsulfonyl)-7H-pyrrolo[2,3-d]pyrimidine-6-carbonitrile. MS (method F) m/z 321 [M+1]+; t=1.34 min.

### Step 6: Preparation of 7-((3S,4R)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile and 7-((3R,4S)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile - racemic cis Example 72

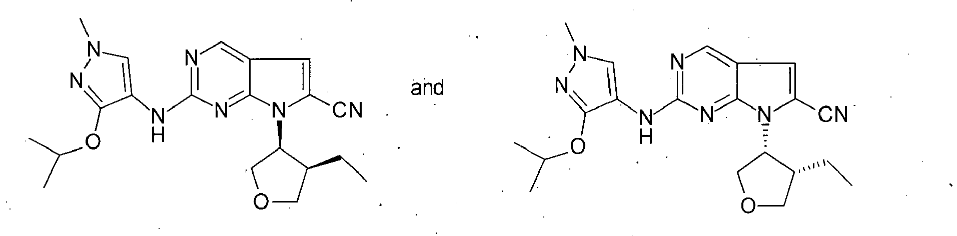

Following the procedure described in Example 47, the racemic *cis* mixture of 7-((3S,4R)-4-ethyltetrahydrofuran-3-yl)-2-(methylsulfonyl)-7H-pyrrolo[2,3-d]pyrimidine-6-carbonitrile and 7-((3R,4S)-4-ethyltetrahydrofuran-3-yl)-2-(methylsulfonyl)-7H-pyrrolo[2,3-d]pyrimidine-6-carbonitrile (150 mg) and N-(3-isopropoxy-1-methyl-1H-pyrazol-4-yl)formamide (90 mg) (intermediate 38) gave 145 mg of a racemic mixture of 7-((3S,4R)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile and 7-((3R,4S)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile (Referred herein as 7-((cis)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile) as a yellow solid- Example 72
1H NMR (500 MHz, DMSO-d6) δ ppm: 0.65 - 0.78 (m, 3 H), 0.85 (m, 1 H), 0.97 (m, 1 H), 1.26 (dd, J=6, 4 Hz, 6 H), 2.47 (m hidden, 1 H), 3.67 (s, 3 H), 3.82 (m, 1 H), 4.01 (br t, J=8 Hz, 1 H), 4.32 (dd, J=10, 8 Hz, 1 H), 4.53 (m, 1 H), 4.71 (spt, J=6 Hz, 1 H), 5.25 (m, 1 H), 7.47 (s, 1 H), 7.99 (br s, 1 H), 8.48 (br s, 1 H), 8.80 (s, 1 H). MS (method D) m/z 396 [M+1]+; t=1.28 min.

### Step 7: Preparation of 7-((3S,4S)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile and 7-((3R,4R)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile ― racemic trans Example 73

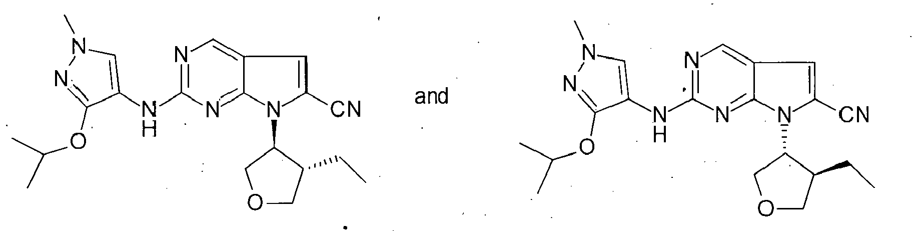

Following the procedure described in Example 47, the racemic *trans* mixture of 7-((3S,4S)-4-ethyltetrahydrofuran-3-yl)-2-(methylsulfonyl)-7H-pyrrolo[2,3-d]pyrimidine-6-carbonitrile and 7-((3R,4R)-4-ethyltetrahydrofuran-3-yl)-2-(methylsulfonyl)-7H-pyrrolo[2,3-d]pyrimidine-6-carbonitrile (97 mg) and N-(3-isopropoxy-1-methyl-1H-pyrazol-4-yl)formamide (58 mg) (intermediate 38) gave 79 mg of a racemic mixture of 7-((3S,4S)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile and 7-((3R,4R)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile (Referred herein as 7-((trans)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile).
1H NMR (500 MHz, DMSO-d6) δ ppm: 0.79 (t, J=7 Hz, 3 H), 1.27 (dd, J=6, 1 Hz, 6 H), 1.44 - 1.58 (m, 2 H), 2.77 (sxt, J=7 Hz, 1 H), 3.54 (t, J=8 Hz, 1 H), 3.67 (s, 3 H), 4.07 - 4.28 (m, 3 H), 4.71 (quin, J=6 Hz, 1 H), 4.88 (m, 1 H), 7.46 (s, 1 H), 8.01 (br s, 1 H), 8.56 (br s, 1 H), 8.80 (s, 1 H). MS (method D) m/z 396 [M+1]+; t=1.30 min.

### Example 74: 2-((1-(2-Cyanopropan-2-yl)-3-isopropoxy-1H-pyrazol-4-yl)amino)-7-(oxetan-3-yl)-7H-pyrrolo[2,3-d]pyrimidine-6-carbonitrile

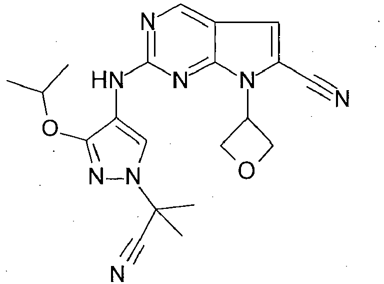

### Step 1: Preparation of methyl oxetan-3-ylglycinate

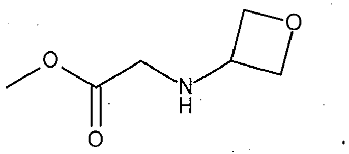

6.28 g (1 eq.) of methyl 2-bromoacetate in solution in 30 mL of tetrahydrofuran was added to a solution of 3 g (1 eq.) of 3-oxetanamine and 28 mL of triethylamine (5 eq.) in 120 mL of tetrahydrofuran and 60 mL of dichloromethane at 0°C. The reaction mixture was stirred at room temperature for 17 days. The reaction mixture was filtered with dichloromethane washes, concentrated under reduced pressure, and then taken into 100 mL of dichloromethane and 10 mL of brine. The aqueous layer was separated and extracted twice with 10 mL of dichloromethane. The combined organic layers were dried over MgSO₄ and concentrated under reduced pressure to afford 5.3 g of methyl oxetan-3-ylglycinate which was used in the next step without further purification.

### Step 2: Preparation of methyl N-(5-formyl-2-(methylthio)pyrimidin-4-yl)-N-(oxetan-3-yl)glycinate

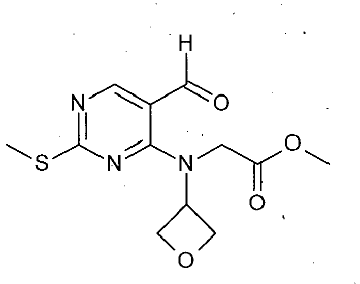

6.7 mL of triethylamine (1.7 eq.) was added to a solution of 5.34 g of 4-chloro-2-(methylthio)pyrimidine-5-carbaldehyde (1 eq.) in 100 mL of tetrahydrofuran and 20 mL of dichloromethane at 0°C. 5.34 g of methyl oxetan-3-ylglycinate (1 eq) was added dropwise. The mixture was stirred at room temperature for 1 week. The reaction mixture was diluted with 100 mL of ethyl acetate and 50 mL of a saturated aqueous NH₄Cl solution. The organic layer was separated and the aqueous layer extracted twice with 50 mL of ethyl acetate. The combined organic layers were washed twice with 50 mL of brine, dried over magnesium sulfate, and concentrated under reduced pressure. The residue was purified on silica gel, eluting with a gradient of 5 to 30% of acetone in dichloromethane, to afford 1.48 g of methyl N-(5-formyl-2-(methylthio)pyrimidin-4-yl)-N-(oxetan-3-yl)glycinate. MS (method A) m/z 298 [M+1]+; t=1.64 min.

### Step 3: Preparation of methyl 2-(methylthio)-7-(oxetan-3-yl)-7H-pyrrolo[2,3-d]pyrimidine-6-carboxylate

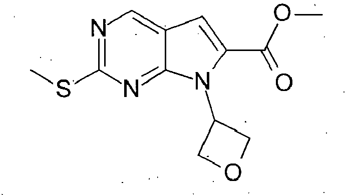

500 µL (5 eq.) of 1,8-diazabicyclo[5.4.0]undec7-ene was added dropwise to a solution of 191 mg of methyl N-(5-formyl-2-(methylthio)pyrimidin-4-yl)-N-(oxetan-3-yl)glycinate (1 eq.) in 8 mL of acetonitrile. The reaction mixture was refluxed for 2.5 hours. After cooling down to room temperature, the reaction mixture was diluted with 10 mL of ethyl acetate and quenched with 3.5 mL of a 1N HCl aqueous solution followed by 3 mL of aqueous NaHCO₃ saturated solution and 5 mL of a 10% aqueous solution of citric acid. The organic layer was separated and the aqueous layer extracted twice with 10 mL of ethyl acetate. The combined organic layers were washed with 10 mL of brine, dried over magnesium sulfate, filtered and concentrated under reduced pressure to afford 180 mg of methyl 2-(methylthio)-7-(oxetan-3-yl)-7H-pyrrolo[2,3-d]pyrimidine-6-carboxylate. MS (method A) m/z 280 [M+1]+; t=2.06 min.

### Step 4: Preparation of 2-(methylthio)-7-(oxetan-3-yl)-7H-pyrrolo[2,3-d]pyrimidine-6-carboxylic acid

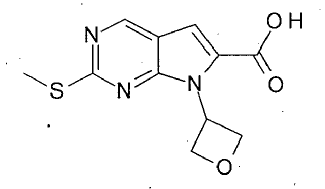

0.61 g (5 eq.) of lithium hydroxide in 10 mL of water was added to a solution of 1.41 g of methyl 2-(methylthio)-7-(oxetan-3-yl)-7H-pyrrolo[2,3-d]pyrimidine-6-carboxylate (1 eq.) in 40 mL of tetrahydrofuran. The mixture was stirred for 3.5 hours at room temperature, cooled to 0°C, and 0.85 mL of a 30% aqueous ammonium hydroxide solution was added. The reaction mixture was stirred at room temperature for 1.5 hours then concentrated under reduced pressure. 18 mL of a 2N HCl aqueous solution and 20 mL of ethyl acetate were added. The precipitate was filtered, washed with water, ethyl acetate and pentane then dried under vacuum to afford 0.98 g of 2-(methylthio)-7-(oxetan-3-yl)-7H-pyrrolo[2,3-d]pyrimidine-6-carboxylic acid. MS (method A) m/z 266 [M+1]+; t=1.71 min.

### Step 5: Preparation of 2-(methylthio)-7-(oxetan-3-yl)-7H-pyrrolo[2,3-d]pyrimidine-6-carboxamide

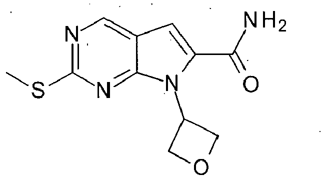

1.06 g (1.5 eq.) of di(1H-imidazol-1-yl)methanone (CDI) was added to a solution of 1.15 g of methyl 2-(methylthio)-7-(oxetan-3-yl)-7H-pyrrolo[2,3-d]pyrimidine-6-carboxylate (1 eq.) in 11 mL of dimethylformamide. The mixture was stirred for 45 minutes at room temperature, cooled to 0°C, and 0.85 mL of a 30% ammonium hydroxide solution was added. The reaction mixture was stirred at room temperature for 1.5 hours, diluted with 11 mL of ethyl acetate, and 55 mL of water. After stirring for 20 minutes, the resulting precipitate was filtered, washed twice with ethyl acetate and once with pentane then dried under vacuum to afford 0.88 g of 2-(methylthio)-7-(oxetan-3-yl)-7H-pyrrolo[2,3-d]pyrimidine-6-carboxamide. MS (method A) m/z 264 [M+1]+; t=1.46 min.

### Step 6: Preparation of 2-(methylthio)-7-(oxetan-3-yl)-7H-pyrrolo[2,3-d]pyrimidine-6-carbonitrile

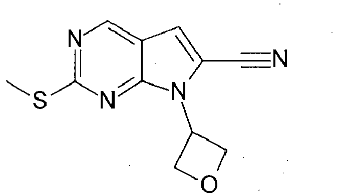

2.1 mL (4.5 eq.) of triethylamine was added to a solution of 0.88 g of 2-(methylthio)-7-(oxetan-3-yl)-7H-pyrrolo[2,3-d]pyrimidine-6-carboxamide (1 eq.) in 20 mL of anhydrous tetrahydofuran at 0°C under argon, followed by 1.68 mL (3.6 eq.) of trifluoroacetic anhydride. The mixture was stirred for 2.5 hours from 0°C to room temperature and quenched with 20 mL of dichloromethane and 20 mL of aqueous saturated NaHCO₃ solution under stirring. The organic layer was separated. The aqueous layer was extracted twice with 20 mL of dichloromethane. The combined organic extracts were washed with 20 mL of brine, dried over magnesium sulfate, and concentrated under reduced pressure. The residue was purified on silica, eluting with 10-50% of ethyl acetate in heptane. The concentrated fractions of interest were triturated with diisopropyl ether, filtered, washed with diisopropyl ether and pentane, and then dried under vacuum to afford 0.76 g of 2-(methylthio)-7-(oxetan-3-yl)-7H-pyrrolo[2,3-d]pyrimidine-6-carbonitrile. MS (method A) m/z 247 [M+1]+; t=1.88 min.

### Step 7: Preparation of 2-(methylsulfonyl)-7-(oxetan-3-yl)-7H-pyrrolo[2,3-d]pyrimidine-6-carbonitrile

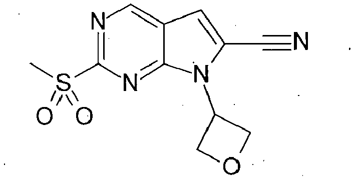

1.72 g of 3-chloroperbenzoic acid (77% purity; 2.5 eq.) was added to a solution of 0.75 g (1 eq.) of 2-(methylthio)-7-(oxetan-3-yl)-7H-pyrrolo[2,3-d]pyrimidine-6-carbonitrile in 40 mL of dichloromethane at 0°C. The mixture was stirred for 5 minutes at 0°C and 5 hours at room temperature. 0.34 g (0.5 eq.) of 3-chloroperbenzoic acid was added and the mixture was stirred for 3 hours, 0.34 g (0.5 eq.) of 3-chloroperbenzoic acid was added, and the mixture was stirred for 45 minutes. It was then quenched with 20 mL of dichloromethane and 20 mL of an aqueous saturated solution of sodium thiosulfate. The organic layer was separated and 10 mL of water, 10 mL of brine and 10 mL of dichloromethane were added. The precipitate was filtered, washed twice with dichloromethane and dried under vacuum to give 0.29 g of 2-(methylsulfonyl)-7-(oxetan-3-yl)-7H-pyrrolo[2,3-d]pyrimidine-6-carbonitrile. The mother liquors were extracted twice with 20 mL of dichloromethane. The combined organic layers were washed twice with 20 mL of aqueous saturated hydrogenocarbonate solution, twice with 20 mL of brine then dried over magnesium sulfate and concentrated under reduced pressure to give 0.57 g of 2-(methylsulfonyl)-7-(oxetan-3-yl)-7H-pyrrolo[2,3-d]pyrimidine-6-carbonitrile The combined solids yielded 0.86 g of 2-(methylsulfonyl)-7-(oxetan-3-yl)-7H-pyrrolo[2,3-d]pyrimidine-6-carbonitrile. MS (method A) m/z 279 [M+1]+; t=1.21 min.

### Step 8: Preparation of 2-((1-(2-cyanopropan-2-yl)-3-isopropoxy-1H-pyrazol-4-yl)amino)-7-(oxetan-3-yl)-7H-pyrrolo[2,3-d]pyrimidine-6-carbonitrile-Example 74

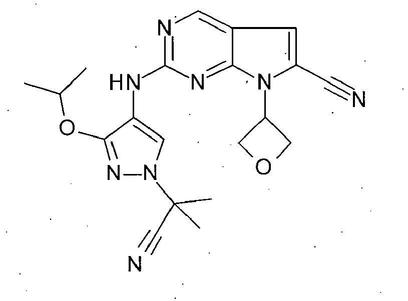

Generally following the procedure described in Step 5 of Example 1, 2-(methylsulfonyl)-7-(oxetan-3-yl)-7H-pyrrolo[2,3-d]pyrimidine-6-carbonitrile (60 mg) and N-[1-(1-cyano-1-methyl-ethyl)-3-isopropoxy-pyrazol-4-yl]formamide (51 mg) (Intermediate 31) gave 2-((1-(2-cyanopropan-2-yl)-3-isopropoxy-1H-pyrazol-4-yl)amino)-7-(oxetan-3-yl)-7H-pyrrolo[2,3-d]pyrimidine-6-carbonitrile (120 mg)- Example 74
¹H NMR (400 MHz, DMSO-d6) δ in ppm: 1.34 (d, J=6 Hz, 6 H), 1.91 (s, 6 H), 4.83 (spt, J=6 Hz, 1 H), 5.01 (t, J=8 Hz, 2 H), 5.44 (br t, J=7 Hz, 2 H), 5.64 (m, 1 H), 7.48 (s, 1 H), 8.43 (br s, 1 H), 8.83 (br s, 1 H), 8.88 (s, 1 H). MS (method C) m/z 407 [M+1]+; t= 3.43 min.

### Example 75: 2-((3-Isopropoxy-1-methyl-1H-pyrazol-4-yl)amino)-7-(oxetan-3-yl)-7H-pyrrolo[2,3-d]pyrimidine-6-carbonitrile

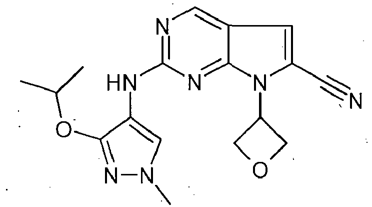

Following the procedure described in Example 47, 2-(methylsulfonyl)-7-(oxetan-3-yl)-7H-pyrrolo[2,3-d]pyrimidine-6-carbonitrile (Example 74, step 7) (174 mg) and N-(3-isopropoxy-1-methyl-1H-pyrazol-4-yl)formamide (115 mg) (Intermediate 38) gave 2-((3-isopropoxy-1-methyl-1H-pyrazol-4-yl)amino)-7-(oxetan-3-yl)-7H-pyrrolo[2,3-d]pyrimidine-6-carbonitrile (149 mg)- Example 75
1H NMR (400 MHz, DMSO-d6) δ ppm: 1.28 (d, J=6 Hz, 6 H), 3.66 (s, 3 H), 4.72 (spt, J=6 Hz, 1 H), 5.00 (t, J=7 Hz, 2 H), 5.36 (t, J=7 Hz, 2 H), 5.76 (quin, J=8 Hz, 1 H), 7.47 (s, 1 H), 8.17 (br s, 1 H), 8.62 (br s, 1 H), 8.82 (s, 1 H). MS (method B) m/z 354 [M+1]+; t= 1.39 min.

### Example 76 and Example 77: 7-[(2S,3R)-2-Methyloxetan-3-yl]-2-[[1-methyl-3-(oxetan-3-yloxy)pyrazol-4-yl]amino]pyrrolo[2,3-d]pyrimidine-6-carbonitrile and 7-[(2R,3S)-2-methyloxetan-3-yl]-2-[[1-methyl-3-(oxetan-3-yloxy)pyrazol-4-yl]amino]pyrrolo[2,3-d]pyrimidine-6-carbonitrile

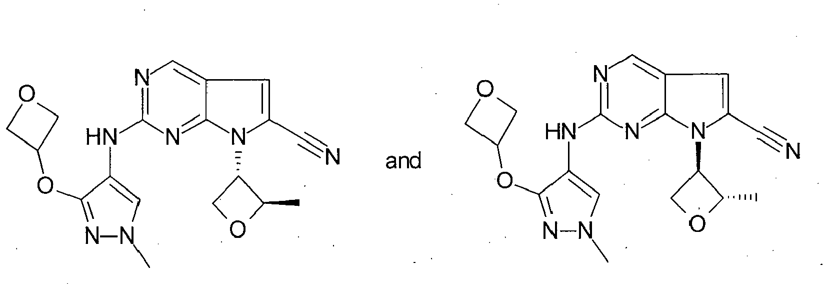

### Step 1: Preparation of methyl (2-methyloxetan-3-yl)glycinate (racemic)

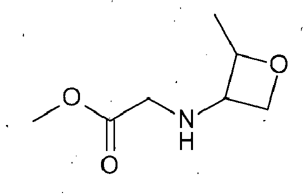

1.06 mL (1 eq.) of methyl 2-bromoacetate was slowly added to a solution of 1 g (1 eq.) of 2-methyloxetan-3-amine and 2.43 mL of triethylamine (1.5 eq.) in 30 mL of tetrahydrofuran and 10 mL of dichloromethane at 0°C. The reaction mixture was stirred at room temperature overnight. The reaction mixture was diluted with dichloromethane and quenched with water. The organic layer was separated, dried over magnesium sulfate, and concentrated under reduced pressure to afford 1.92 g of racemic methyl (2-methyloxetan-3-yl)glycinate which was used in the next step without further purification.

### Step 2: Preparation of methyl N-(5-formyl-2-(methylthio)pyrimidin-4-yl)-N-(2-methyloxetan-3-yl)glycinate (racemic)

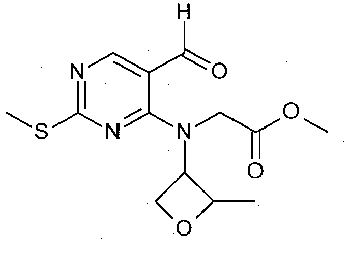

2.6 mL of triethylamine (1.7 eq.) was added to a solution of 2 g of 4-chloro-2-(methylthio)pyrimidine-5-carbaldehyde (1 eq.) in 80 mL of tetrahydrofuran and 18 mL of dichloromethane at 0°C. 1.86 g of racemic methyl (2-methyloxetan-3-yl)glycinate (1.1 eq) was added dropwise. The mixture was stirred at room temperature for 6 days. The reaction mixture was diluted with ethyl acetate and water. The organic layer was separated and the aqueous layer extracted twice with 50 mL of ethyl acetate. The combined organic layers were washed twice with 50 mL of brine, dried over magnesium sulfate, and concentrated under reduced pressure. The residue was purified on silica gel, eluting with 20% of ethyl acetate in heptane, to afford 1.5 g of racemic methyl N-(5-formyl-2-(methylthio)pyrimidin-4-yl)-N-(2-methyloxetan-3-yl)glycinate. MS (method B) m/z 312 [M+1]+; t=1.24 min.

### Step 3: Preparation of methyl 7-(2-methyloxetan-3-yl)-2-(methylthio)-7H-pyrrolo[2,3-d]pyrimidine-6-carboxylate (racemic)

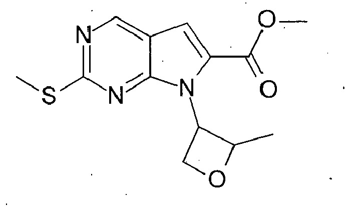

3.75 mL (5 eq.) of 1,8-diazabicyclo[5.4.0]undec7-ene was dropwise added to a solution of 1.5 g of racemic methyl N-(5-formyl-2-(methylthio)pyrimidin-4-yl)-N-(2-methyloxetan-3-yl)glycinate (1 eq.) in 20 mL of acetonitrile. The reaction mixture was refluxed for 30 minutes. After cooling down to room temperature the reaction mixture was diluted with ethyl acetate and quenched with a 1N HCl aqueous solution. The organic layer was dried over magnesium sulfate, filtered, and concentrated under reduced pressure. The residue was purified on silica gel, eluting with 20% of ethyl acetate in heptane, to afford 0.93 g of racemic methyl 7-(2-methyloxetan-3-yl)-2-(methylthio)-7H-pyrrolo[2,3-d]pyrimidine-6-carboxylate. MS (method B) m/z 294 [M+1]+; t=1.69 min.

### Step 4: Preparation of 7-(2-methyloxetan-3-yl)-2-(methylthio)-7H-pyrrolo[2,3-d]pyrimidine-6-carboxylic acid (racemic)

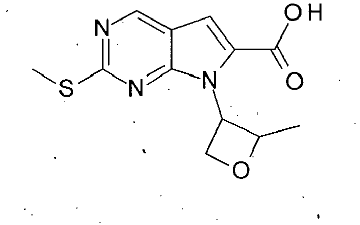

0.38 g (5 eq.) of lithium hydroxide in 8 mL of water was added to a solution of 0.93 g of racemic methyl 7-(2-methyloxetan-3-yl)-2-(methylthio)-7H-pyrrolo[2,3-d]pyrimidine-6-carboxylate (1 eq.) in 30 mL of tetrahydrofuran. The mixture was stirred for 1 hour at room temperature then acidified with addition of a 2N HCl aqueous solution. The mixture was extracted with ethyl acetate, dried with magnesium sulfate, filtered and concentrated under reduced pressure to afford 0.81 g of racemic 7-(2-methyloxetan-3-yl)-2-(methylthio)-7H-pyrrolo[2,3-d]pyrimidine-6-carboxylic acid. MS (method B) m/z 280 [M+1]+; t=1.31 min.

### Step 5: Preparation of 7-(2-methyloxetan-3-yl)-2-(methylthio)-7H-pyrrolo[2,3-d]pyrimidine-6-carboxamide (racemic)

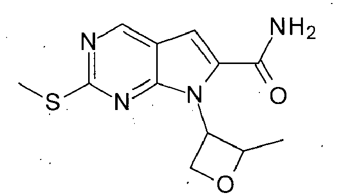

0.74 g (1.5 eq.) of di(1H-imidazol-1-yl)methanone (CDI) was added to a solution of 1.15 g of racemic 7-(2-methyloxetan-3-yl)-2-(methylthio)-7H-pyrrolo[2,3-d]pyrimidine-6-carboxylic acid (1 eq.) in 10 mL of dimethylformamide. The mixture was stirred for 30 minutes at room temperature, cooled to 0°C and 1.88 mL of a 30% aqueous ammonium hydroxide solution was added. The reaction mixture was stirred at room temperature for 30 minutes, poured onto 20 mL of water, and extracted with ethyl acetate. The organic extracts were dried over magnesium sulfate, filtered, and concentrated under reduced pressure. The residue was triturated in diisopropyl ether, the solid filtered and dried under vacuum to afford 0.83 g of racemic 7-(2-methyloxetan-3-yl)-2-(methylthio)-7H-pyrrolo[2,3-d]pyrimidine-6-carboxamide. The crude material was taken into the next step without further purification.

### Step 6: Preparation of 7-(2-methyloxetan-3-yl)-2-(methylthio)-7H-pyrrolo[2,3-d]pyrimidine-6-carbonitrile (racemic)

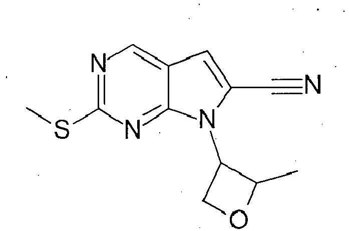

1.89 mL (4.5 eq.) of triethylamine was added to a solution of 0.83 g of racemic 7-(2-methyloxetan-3-yl)-2-(methylthio)-7H-pyrrolo[2,3-d]pyrimidine-6-carboxamide (1 eq.) in 25 mL of anhydrous tetrahydofuran at 0°C under argon, followed by 1.49 mL (3.6 eq.) of trifluoroacetic anhydride. The mixture was stirred for 15 minutes allowing the temperature to warm up to room temperature then diluted with ethyl acetate and quenched with an aqueous saturated sodium hydrogenocarbonate solution. The organic layer was separated. The aqueous layer was extracted twice with ethyl acetate. The combined organic extracts were combined, dried over magnesium sulfate, and concentrated under reduced pressure. The residue was purified on silica, eluting with 20% of ethyl acetate in heptane. The concentrated fractions of interest were triturated with diisopropyl ether, filtered, washed with diisopropyl ether and pentane then dried under vacuum to afford 0.62 g of racemic 7-(2-methyloxetan-3-yl)-2-(methylthio)-7H-pyrrolo[2,3-d]pyrimidine-6-carbonitrile. MS (method B) m/z 261 [M+1]+; t=1.50 min.

### Step 7: Preparation of 7-(2-methyloxetan-3-yl)-2-(methylsulfonyl)-7H-pyrrolo[2,3-d]pyrimidine-6-carbonitrile (racemic)

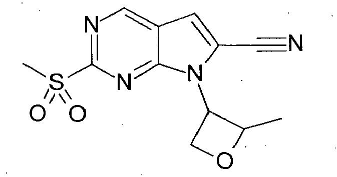

1.64 g of 3-chloroperbenzoic acid (77% purity; 3 eq.) was portionwise added to a solution of 0.62 g (1 eq.) of racemic 7-(2-methyloxetan-3-yl)-2-(methylthio)-7H-pyrrolo[2,3-d]pyrimidine-6-carbonitrile in 20 mL of dichloromethane at 0°C. The mixture was stirred for 1 hour allowing the temperature to warm up to room temperature then quenched with 50 mL of aqueous 10% solution of sodium thiosulfate. The organic layer was separated and washed with 50 mL of aqueous 10% solution of sodium thiosulfate, twice with 50 mL of aqueous saturated sodium hydrogenocarbonate solution, and then dried over magnesium sulfate and concentrated under reduced pressure to give 0.6 g of racemic 7-(2-methyloxetan-3-yl)-2-(methylsulfonyl)-7H-pyrrolo[2,3-d]pyrimidine-6-carbonitrile. The crude material was taken into the next step without further purification.

### Step 8: Preparation of 7-[(2S,3R)-2-methyloxetan-3-yl]-2-[[1-methyl-3-(oxetan-3-yloxy)pyrazol-4-yl]amino]pyrrolo[2,3-d]pyrimidine-6-carbonitrile and 7-[(2R,3S)-2-methyloxetan-3-yl]-2-[[1-methyl-3-(oxetan-3-yloxy)pyrazol-4-yl]amino]pyrrolo[2,3-d]pyrimidine-6-carbonitrile

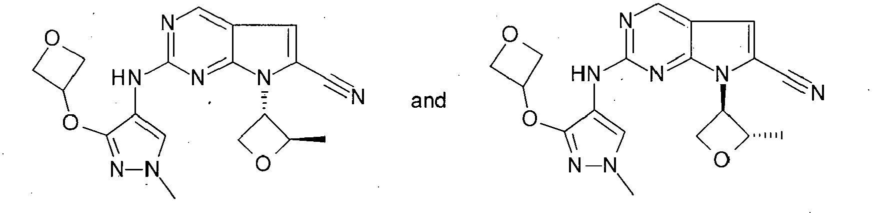

Generally following the procedure described in Step 5 of Example 1, a racemic mixture of 7-(2-methyloxetan-3-yl)-2-(methylsulfonyl)-7H-pyrrolo[2,3-d]pyrimidine-6-carbonitrile (300 mg) and N-(1-methyl-3-(oxetan-3-yloxy)-1H-pyrazol-4-yl)formamide (212 mg) (Intermediate 4) gave a racemic mixture of 7-[(2S,3R)-2-methyloxetan-3-yl]-2-[[1-methyl-3-(oxetan-3-yloxy)pyrazol-4-yl]amino]pyrrolo[2,3-d]pyrimidine-6-carbonitrile and 7-[(2R,3S)-2-methyloxetan-3-yl]-2-[[1-methyl-3-(oxetan-3-yloxy)pyrazol-4-yl]amino]pyrrolo[2,3-d]pyrimidine-6-carbonitrile (Referred herein as 7-[(trans)-2-methyloxetan-3-yl]-2-[[1-methyl-3-(oxetan-3-yloxy)pyrazol-4-yl]amino]pyrrolo[2,3-d]pyrimidine-6-carbonitrile) (275 mg).

Chiral separation performed on 267 mg of the isomeric mixture gave 117 mg of the first eluting isomer and 117 mg of the second eluting isomer (conditions: column Chiralpak AD, 20 µm, 350x76 mm; liquid phase: heptane 50/ethyl alcohol 50/triethylamine 0.1% for 19 minutes then heptane 40/ethyl alcohol 60/triethylamine 0.1%; flow rate: 400 mL/min).

Peak 1 (1^{st} isomer): ¹H NMR (400 MHz, DMSO-d6) δ in ppm: 1.45 (d, J=6 Hz, 3 H), 3.64 (s, 3 H), 4.57 (br t, J=6 Hz, 2 H), 4.75 - 4.86 (m, 3 H), 5.12 - 5.27 (m, 2 H), 5.32 (quin, J=6 Hz, 1 H), 5.56 (quin, J=6 Hz, 1 H), 7.48 (s, 1 H), 8.01 (br s, 1 H), 8.83 (s, 1 H), 8.95 (br s, 1 H). MS (method B) m/z 382 [M+1]+; t=1.43 min - Example 76 (absolute configuration unknown)

Peak 2 (2^{nd} isomer): ¹H NMR (400 MHz, DMSO-d6) δ in ppm: 1.45 (d, J=6 Hz, 3 H), 3.64 (s, 3 H), 4.57 (t, J=6 Hz, 2 H), 4.75 - 4.85 (m, 3 H), 5.12 - 5.26 (m, 2 H), 5.32 (quin, J=6 Hz, 1 H), 5.56 (quin, J=6 Hz, 1 H), 7.48 (s, 1 H), 8.02 (br s, 1 H), 8.83 (s, 1 H), 8.96 (br s, 1 H). MS (method B) m/z 382 [M+1]+; t=1.40 min - Example 77 (absolute configuration unknown)

### Example 78 and Example 79: 7-[(2R,3S)-2-Methyloxetan-3-yl]-2-[(1-methyl-3-propan-2-yloxypyrazol-4-yl)amino]pyrrolo[2,3-d]pyrimidine-6-carbonitrile and 7-[(2S,3R)-2-methyloxetan-3-yl]-2-[(1-methyl-3-propan-2-yloxypyrazol-4-yl)amino]pyrrolo[2,3-d]pyrimidine-6-carbonitrile.

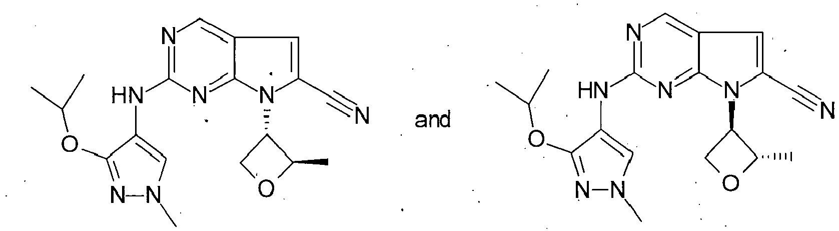

Following the procedure described in Example 47, a racemic mixture of 7-(2-methyloxetan-3-yl)-2-(methylsulfonyl)-7H-pyrrolo[2,3-d]pyrimidine-6-carbonitrile (300 mg) (Example 76, step 7) (300 mg) and N-(3-isopropoxy-1-methyl-1H-pyrazol-4-yl)formamide (197 mg) (Intermediate 38) gave a racemic mixture of 7-[(2R,3S)-2-methyloxetan-3-yl]-2-[(1-methyl-3-propan-2-yloxypyrazol-4-yl)amino]pyrrolo[2,3-d]pyrimidine-6-carbonitrile and 7-[(2S,3R)-2-methyloxetan-3-yl]-2-[(1-methyl-3-propan-2-yloxypyrazol-4-yl)amino]pyrrolo[2,3-d]pyrimidine-6-carbonitrile (Referred herein as 7-[(trans)-2-methyloxetan-3-yl]-2-[(1-methyl-3-propan-2-yloxypyrazol-4-yl)amino]pyrrolo[2,3-d]pyrimidine-6-carbonitrile) (260 mg).

Chiral separation performed on 250 mg of the isomeric mixture gave 115 mg of the first eluting isomer and 112 mg of the second eluting isomer (conditions: column Chiralpak IC, 20 µm, 350×76 mm; liquid phase: heptane 50/ethyl alcohol 50; flow rate: 400 mL/min).

Peak 1 (1^{st} isomer): 1H NMR (400 MHz, DMSO-d6) δ ppm: 1.27 (d, J=6 Hz, 6 H), 1.45 (d, J=6 Hz, 3 H), 3.66 (s, 3 H), 4.72 (spt, J=6 Hz, 1 H), 4.80 (t, J=7 Hz, 1 H), 5.11 - 5.32 (m, 2 H), 5.56 (quin, J=6 Hz, 1 H), 7.48 (s, 1 H), 8.00 (br s, 1 H), 8.61 (br s, 1 H), 8.81 (s, 1 H). MS (method B) m/z 368 [M+1]+; t=1.58 min - Example 78 (absolute configuration unknown)

Peak 2 (2^{nd} isomer): 1H NMR (400 MHz, DMSO-d6) δ ppm: 1.27 (d, J=6 Hz, 6 H), 1.45 (d, J=6 Hz, 3 H), 3.66 (s, 3 H), 4.72 (spt, J=6 Hz, 1 H), 4.80 (t, J=7 Hz, 1 H), 5.12 - 5.32 (m, 2 H), 5.56 (quin, J=6 Hz, 1 H), 7.48 (s, 1 H), 8.00 (br s, 1 H), 8.61 (br s, 1 H), 8.81 (s, 1 H) ). MS (method B) m/z 368 [M+1]+; t=1.66 min ― Example 79 (absolute configuration unknown)

### Example 80: 2-((1-Methyl-3-(oxetan-3-yloxy)-1H-pyrazol-4-yl)amino)-7-(tetrahydro-2H-pyran-4-yl)-7H-pyrrolo[2,3-d]pyrimidine-6-carbonitrile

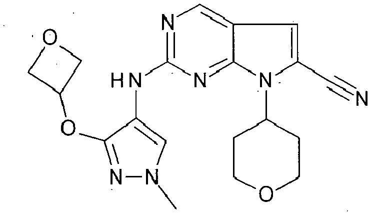

### Step 1: Preparation of 2-(methylsulfonyl)-7-(tetrahydro-2H-pyran-4-yl)-7H-pyrrolo[2,3-d]pyrimidine-6-carbonitrile

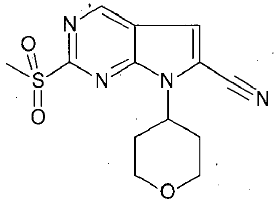

198 mg of 2-(methylsulfonyl)-7-(tetrahydro-2H-pyran-4-yl)-7H-pyrrolo[2,3-d]pyrimidine-6-carbonitrile were prepared according to the procedure described in step 1 and 2, Example 52, starting with 2-(methylthio)-7H-pyrrolo[2,3-d]pyrimidine-6-carbonitrile (intermediate 43) (400 mg) and tetrahydro-2H-pyran-4-ol instead of tetrahydrofuran-3-ol). MS (method A) m/z 307 [M+1]+; t=1.6 min.

### Step 2: Preparation of 2-((1-methyl-3-(oxetan-3-yloxy)-1H-pyrazol-4-yl)amino)-7-(tetrahydro-2H-pyran-4-yl)-7H-pyrrolo[2,3-d]pyrimidine-6-carbonitrile

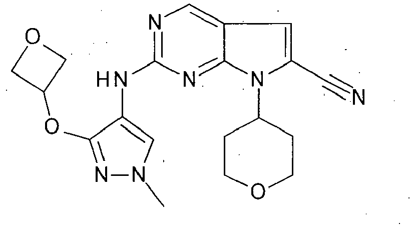

Following the general procedure in step 5 of Example 1, 2-(methylsulfonyl)-7-(tetrahydro-2H-pyran-4-yl)-7H-pyrrolo[2,3-d]pyrimidine-6-carbonitrile (194 mg) and N-[1-methyl-3-(oxetan-3-yloxy)pyrazol-4-yl]formamide (intermediate 4) gave 2-((1-methyl-3-(oxetan-3-yloxy)-1H-pyrazol-4-yl)amino)-7-(tetrahydro-2H-pyran-4-yl)-7H-pyrrolo[2,3-]pyrimidine-6-carbonitrile (83 mg).

1H NMR (400 MHz, DMSO-d6) δ ppm: 1.83 (dd, J=12, 3 Hz, 2 H), 2.59 (m, 2 H), 3.49 (t, J=11 Hz, 2 H), 3.66 (s, 3 H), 4.04 (dd, J=12, 4 Hz, 2 H), 4.56 (dd, J=8, 5 Hz, 2 H), 4.69 (m, 1 H), 4.81 (t, J=7 Hz, 2 H), 5.31 (m, 1 H), 7.45 (s, 1 H), 7.82 (br s, 1 H), 8.81 (s, 1 H), 8.90 (br s, 1 H). MS (method B) m/z 396 [M+1]+; t=2.58 min.

### Example 81 and Example 82: (R)-2-((1-Methyl-3-(oxetan-3-yloxy)-1H-pyrazol-4-yl)amino)-7-(tetrahydro-2H-pyran-3-yl)-7H-pyrrolo[2,3-d]pyrimidine-6-carbonitrile and (S)-2-((1-methyl-3-(oxetan-3-yloxy)-1H-pyrazol-4-yl)amino)-7-(tetrahydro-2H-pyran-3-yl)-7H-pyrrolo[2,3-d]pyrimidine-6-carbonitrile

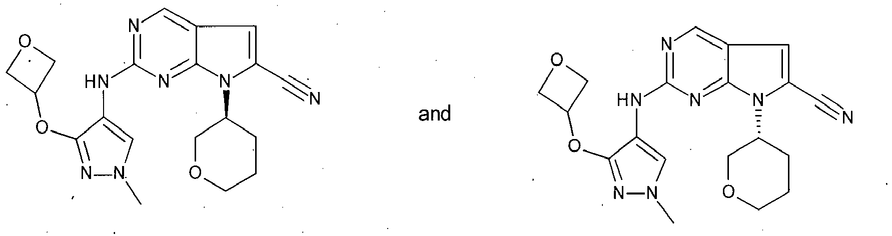

### Step 1: Preparation of methyl 2-(methylthio)-7-(tetrahydro-2H-pyran-3-yl)-7H-pyrrolo[2,3-d]pyrimidine-6-carboxylate (racemic mixture)

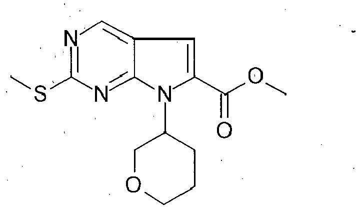

3.17 g (3 eq.) of triphenylphosphine, 2,47 g (3 eq.) of racemic tetrahydro-2H-pyran-3-ol and 2.45g (3 eq.) of diisopropyl azodicarboxylate (DIAD) were added to a solution of 900 mg (1 eq.) of methyl 2-(methylthio)-7H-pyrrolo[2,3-d]pyrimidine-6-carboxylate (intermediate 1) in 50 mL of anhydrous tetrahydrofuran under argon. The mixture was stirred at 50°C for 3 hours and at room temperature for 15 hours, and then another 3 eq: of triphenylphosphine, 3 eq. of tetrahydro-2H-pyran-3-ol and 3 eq. of DIAD were added and the mixture was stirred at 50°C for another 1 hour 30 minutes then concentrated under reduced pressure. The residue was purified on silica, eluting with 0-70% of ethyl acetate in cyclohexane to afford 900 mg of racemic methyl 2-(methylthio)-7-(tetrahydro-2H-pyran-3-yl)-7H-pyrrolo[2,3-d]pyrimidine-6-carboxylate. MS (method F) m/z 308 [M+ 1]+; t=1.86 min.

### Step 2: Preparation of 2-methylsulfanyl-7-tetrahydropyran-3-yl-pyrrolo[2,3-d]pyrimidine-6-carboxylic acid (racemic mixture)

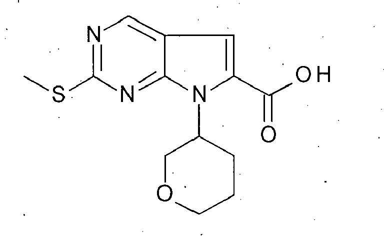

2.93 mL (4 eq.) of a 4N aqueous sodium hydroxide solution was added to a solution of 900 mg (1 eq.) of racemic methyl 2-(methylthio)-7-(tetrahydro-2H-pyran-3-yl)-7H-pyrrolo[2,3-d]pyrimidine-6-carboxylate in 10 mL of methanol and 5 mL of tetrahydrofuran. The mixture was stirred at room temperature for 1 hour 30 minutes then concentrated under reduced pressure. The residue was dissolved in 20 mL of diethyl ether and 20 mL of water and extracted. The aqueous layer was acidified to pH=1 with HCl 2N then extracted with 300 mL of ethyl acetate. The organic layer was dried over sodium sulfate then concentrated under vacuum to afford 756 mg of racemic 2-methylsulfanyl-7-tetrahydropyran-3-yl-pyrrolo[2,3-d]pyrimidine-6-carboxylic acid. MS (method F) m/z 294 [M+1]+; t=1.53 min.

### Step 3: Preparation of 2-methylsulfonyl-7-tetrahydropyran-3-yl-pyrrolo[2,3-d]pyrimidine-6-carbonitrile (racemic mixture)

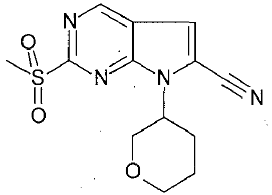

603 mg of racemic 2-methylsulfonyl-7-tetrahydropyran-3-yl-pyrrolo[2,3-d]pyrimidine-6-carbonitrile were prepared according to the procedure described in steps 2,3 and 4 of Example 1, starting with 756 mg of racemic 2-methylsulfanyl-7-tetrahydropyran-3-yl-pyrrolo[2,3-d]pyrimidine-6-carboxylic acid . MS (method F) m/z 307 [M+1]+; t=1.27 min.

### Step 4: Preparation of (R)-2-((1-methyl-3-(oxetan-3-yloxy)-1H-pyrazol-4-yl)amino)-7-(tetrahydro-2H-pyran-3-yl)-7H-pyrrolo[2,3-d]pyrimidine-6-carbonitrile and (S)-2-((1-methyl-3-(oxetan-3-yloxy)-1H-pyrazol-4-yl)amino)-7-(tetrahydro-2H-pyran-3-yl)-7H-pyrrolo[2,3-d]pyrimidine-6-carbonitrile

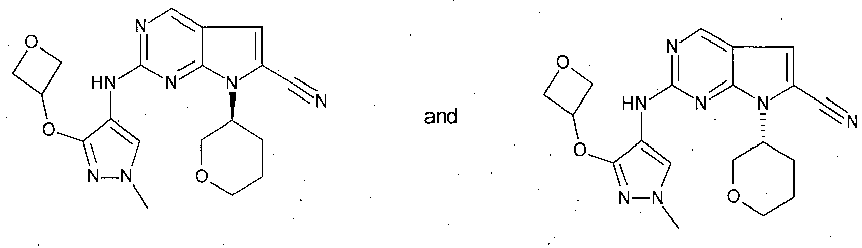

Generally following the procedure described in Step 5 of Example 1, racemic 2-methylsulfonyl-7-tetrahydropyran-3-yl-pyrrolo[2,3-d]pyrimidine-6-carbonitrile (340 mg) and N-[1-methyl-3-(oxetan-3-yloxy)pyrazol-4-yl]formamide (intermediate 4) gave a racemic mixture of (R)-2-((1-methyl-3-(oxetan-3-yloxy)-1H-pyrazol-4-yl)amino)-7-(tetrahydro-2H-pyran-3-yl)-7H-pyrrolo[2,3-d]pyrimidine-6-carbonitrile and (S)-2-((1-methyl-3-(oxetan-3-yloxy)-1H-pyrazol-4-yl)amino)-7-(tetrahydro-2H-pyran-3-yl)-7H-pyrrolo[2,3-d]pyrimidine-6-carbonitrile (216 mg) (Referred herein as 2-((1-methyl-3-(oxetan-3-yloxy)-1H-pyrazol-4-yl)amino)-7-(tetrahydro-2H-pyran-3-yl)-7H-pyrrolo[2,3-d]pyrimidine-6-carbonitrile).

Chiral separation performed on 2.16 mg of the isomeric mixture gave 95 mg of the first eluting isomer and 94 mg of the second eluting isomer (conditions: column Chiralpak AY, 20 µm, 230×100 mm; liquid phase: heptane 40/ethyl alcohol 60 to heptane 20/ethyl alcohol 80/triethylamine 0.1%; flow rate: 400 mL/min).

Peak 1 (1^{st} isomer): 1H NMR (400 MHz, DMSO-d6) δ ppm: 1.77 (m, 2 H), 2.03 (d, J=12 Hz, 1 H), 2.59 (m, 1 H), 3.31 (m, 1 H), 3.67 (s, 3 H), 3.90 (m, 2 H), 4.03 (t, J=11 Hz, 1 H), 4.47 (m, 1 H), 4.54 (t, J=6 Hz, 2 H), 4.80 (t, J=8 Hz, 2 H), 5.31 (quin, J=6 Hz, 1 H), 7.44 (s, 1 H), 7.75 (s, 1 H), 8.79 (s, 1 H), 8.80 (br s, 1 H)). MS (method B) m/z 396 [M+1]+; t=1.47 min - Example 81 (absolute configuration unknown)

Peak 2 (2^{nd} isomer): 1H NMR (400 MHz, DMSO-d6) δ ppm 1.69 - 1.85 (m, 2 H), 2.03 (d, J=12 Hz, 1 H), 2.59 (m, 1 H), 3.32 (m, 1 H), 3.67 (s, 3 H), 3.90 (m, 2 H), 4.03 (t, J=11 Hz, 1 H), 4.47 (m, 1 H), 4.54 (t, J=6 Hz, 2 H), 4.80 (t, J=7 Hz, 2 H), 5.32 (m, 1 H), 7.44 (s, 1 H), 7.75 (s, 1 H), 8.79 (m, 2 H). MS (method B) m/z 396 [M+1]+; t= 1.47 min - Example 82 (absolute configuration unknown)

### Example 83 and Example 84: 2-((1-Methyl-3-(oxetan-3-yloxy)-1H-pyrazol-4-yl)amino)-7-((3R,4S)-3-methyltetrahydro-2H-pyran-4-yl)-7H-pyrrolo[2,3-d]pyrimidine-6-carbonitrile and 2-((1-methyl-3-(oxetan-3-yloxy)-1H-pyrazol-4-yl)amino)-7-((3S,4R)-3-methyltetrahydro-2H-pyran-4-yl)-7H-pyrrolo[2,3-d]pyrimidine-6-carbonitrile

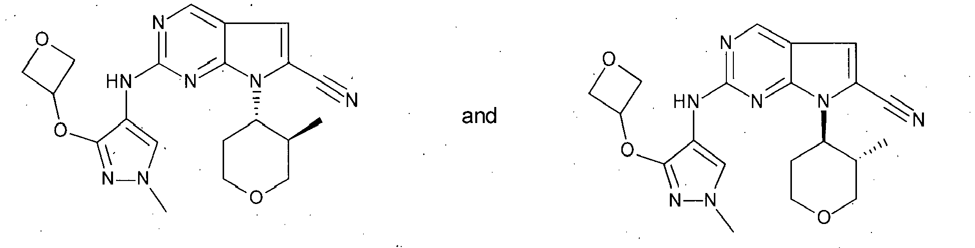

### Step 1: Preparation of 3-methyltetrahydro-2H-pyran-4-yl acetate (racemic trans) and 3-methyltetrahydro-2H-pyran-4-yl acetate (racemic cis)

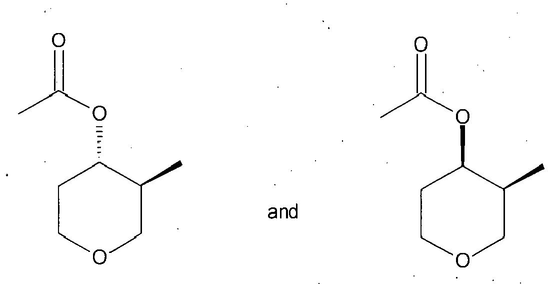

5.44 g (1.25 eq.) of triethylamine, 5.49 g (1.25 eq.) of acetic anhydride and 526 mg (0.1 eq.) of N,N-dimethyl-4-aminopyridine (DMAP) were added to a solution of 5g (1 eq.) of a commercially available mixture of trans and cis 3-methyltetrahydro-2H-pyran-4-ol in their racemic form (4 stereoisomers) in 75 mL of dichloromethane. The mixture was stirred at room temperature for 2 hours then carefully concentrated to the half at 40°C under reduced pressure of 300 mbars. The resulting solution was diluted with 100 mL of pentane and purified on silica, eluting with 0-100% of diethyl ether in pentane to afford successively 3.45 g of 3-methyltetrahydro-2H-pyran-4-yl acetate (racemic *trans)* and 2.15 g of 3-methyltetrahydro-2H-pyran-4-yl acetate (racemic *cis).* 3-methyltetrahydro-2H-pyran-4-yl acetate (racemic *trans):*
¹H NMR (400 MHz, CDCl₃) δ in ppm: 0.85 (d, J=6.7 Hz, 3 H); 1.61 (m, 1 H); 1.83 (m, 1 H); 1.96 (m, 1 H); 2.07 (s, 3 H); 3.10 (dd, J=10.6 Hz and J=11.9 Hz, 1 H); 3.46 (m, 1 H); 3.87 (dd, J=4.7 Hz and J=11.8 Hz, 1 H); 3.95 (m, 1 H); 4.58 (td, J=4.6 Hz and J=10.1 Hz, 1 H).

3-methyltetrahydro-2H-pyran-4-yl acetate (racemic *cis):*
¹H NMR (400 MHz, CDCl₃) δ in ppm: 0.86 (d, J=6.9 Hz, 3. H); 1.80 (m, 2 H); 2.00 (m, 1 H); 2.08 (s, 3 H); 3.47 (dd, J=8.9 Hz and J=12 Hz, 1 H); 3.60 (dd, J=4.2 Hz and J=11.2 Hz, 1 H); 3.68 (m, 2 H); 5.04 (q, J=4.5 Hz, 1 H).

### Step 2: Preparation of 3-methyltetrahydropyran-4-ol (racemic cis)

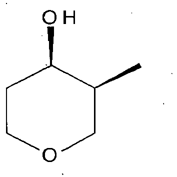

2.15 g (1 eq.) of 3-methyltetrahydro-2H-pyran-4-yl acetate (racemic *cis)* was added to 14.5 mL (1.07 eq.) of a pre-cooled (-5°C) 1N solution of sodium methoxide in methanol. The mixture was stirred at 0°C for 1 hour 30 minutes then quenched with 7.3 mL (1.07 eq.) of a 2N hydrogen chloride solution in diethyl ether. After addition of another 30 mL of diethyl ether, the suspension was filtered over a pad of decalite and the filter cake rinsed twice with 15 mL of diethyl ether. The filtrate was concentrated at 40°C under reduced pressure of 120 mbars and the residue was purified on silica, eluting with 0-100% of diethyl ether in pentane to afford (after concentration at 40°C under reduced pressure of 120 mbars) 1.17g of 3-methyltetrahydropyran-4-ol (racemic *cis*).
¹H NMR (400 MHz, CDCl₃) δ in ppm: 0.91 (d, J=7 Hz, 3 H); 1.56 (br s, 1 H); 1.70 (m, 1 H); 1.80 (m, 1 H); 1.90 (m, 1 H); 3.56 (m, 3 H); 3.80 (ddd, J=11.8 Hz, J=9.6 Hz and J=3.3 Hz, 1 H); 3.93 (m, 1 H).

### Step 3: Preparation of methyl 2-methylsulfanyl-7-[(trans)-3-methyltetrahydropyran-4-yl]pyrrolo[2,3-d]pyrimidine-6-carboxylate (racemic trans)

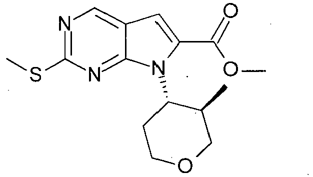

1.75 g (2 eq.) of triphenylphosphine, 568 mg (1.5 eq.) of 3-methyltetrahydropyran-4-ol (racemic *cis)* and 1.32 g (2 eq.) of diisopropyl azodicarboxylate (DIAD) were added to a solution of 750 mg (1 eq.) of methyl 2-(methylthio)-7H-pyrrolo[2,3-d]pyrimidine-6-carboxylate (intermediate 1) in 40 mL of anhydrous tetrahydrofuran under argon. The mixture was stirred at room temperature for 5 hours then another 1 eq. of triphenylphosphine, 1 eq. of 3-methyltetrahydropyran-4-ol (racemic cis) and 1 eq. of DIAD were added and the mixture was stirred at 50°C for 2 hours then concentrated under reduced pressure. The residue was purified on silica, eluting with 0-50% of ethyl acetate in cyclohexane to afford .870 g of methyl 2-methylsulfanyl-7-[(trans)-3-methyltetrahydropyran-4-yl]pyrrolo[2,3-d]pyrimidine-6-carboxylate (racemic *trans).* MS (method E) m/z 322 [M+1]+; t=1.45 min.

### Step 4: Preparation of 2-methylsulfonyl-7-[3-methyltetrahydropyran-4-yl]pyrrolo[2,3-d]pyrimidine-6-carbonitrile (racemic trans)

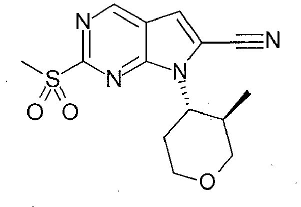

Following step 2 and 3 of Example 81, 1.03 g of methyl 2-methylsulfanyl-7-[3-methyltetrahydropyran-4-yl]pyrrolo[2,3-d]pyrimidine-6-carboxylate (racemic *trans)* gave 700 mg of 2-methylsulfonyl-7-[3-methyltetrahydropyran-4-yl]pyrrolo[2,3-d]pyrimidine-6-carbonitrile (racemic *trans).* MS (method F) m/z 321 [M+1]+; t=1.34 min.

### Step 5: Preparation of 2-((1-methyl-3-(oxetan-3-yloxy)-1H-pyrazol-4-yl)amino)-7-((3R,4S)-3-methyltetrahydro-2H-pyran-4-yl)-7H-pyrrolo[2,3-d]pyrimidine-6-carbonitrile and 2-((1-methyl-3-(oxetan-3-yloxy)-1H-pyrazol-4-yl)amino)-7-((3S,4R)-3-methyltetrahydro-2H-pyran-4-yl)-7H-pyrrolo[2,3-d]pyrimidine-6-carbonitrile

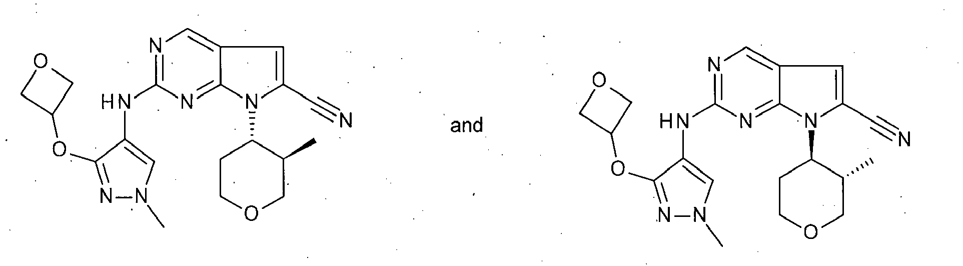

Generally following the procedure described in Step 5 of Example 1, 2-methylsulfonyl-7-[3-methyltetrahydropyran-4-yl]pyrrolo[2,3-d]pyrimidine-6-carbonitrile (racemic *trans)* (350 mg) and N-[1-methyl-3-(oxetan-3-yloxy)pyrazol-4-yl]formamide (226 mg) (intermediate 4) gave 303 mg of a racemic mixture of 2-((1-methyl-3-(oxetan-3-yloxy)-1H-pyrazol-4-yl)amino)-7-((3R,4S)-3-methyltetrahydro-2H-pyran-4-yl)-7H-pyrrolo[2,3-d]pyrimidine-6-carbonitrile and 2-((1-methyl-3-(oxetan-3-yloxy)-1H-pyrazol-4-yl)amino)-7-((3S,4R)-3-methyltetrahydro-2H-pyran-4-yl)-7H-pyrrolo[2,3-d]pyrimidine-6-carbonitrile (305 mg) (Referred herein as 2-((1-methyl-3-(oxetan-3-yloxy)-1H-pyrazol-4-yl)amino)-7-((trans)-3-methyltetrahydro-2H-pyran-4-yl)-7H-pyrrolo[2,3-d]pyrimidine-6-carbonitrile).

Chiral separation performed on 303 mg of the isomeric mixture gave 142 mg of the first eluting isomer and 138 mg of the second eluting isomer (conditions: column Chiralpak IC, 20 µm, 350×76.5 mm; liquid phase: heptane 20/ethyl alcohol 80; flow rate: 400 mL/min).

Peak 1 (1^{st} isomer): 1H NMR (400 MHz, DMSO-d6) δ ppm: 0.55 (d, J=7 Hz, 3 H), 1.84 (dd, J=13, 4 Hz, 1 H), 2.47 (m hidden, 1 H), 2.73 (m, 1 H), 3.13 (t, J=12 Hz, 1 H), 3.51 (t, J=11 Hz, 1 H), 3.66 (s, 3 H), 3.96 (dd, J=11, 4 Hz, 1 H), 4.04 (dd, J=11, 4 Hz, 1 H), 4.25 (br s, 1 H), 4.56 (t, J=6 Hz, 2 H), 4.81 (t, J=7 Hz, 2 H), 5.31 (quin, J=6 Hz, 1 H), 7.47 (s, 1 H), 7.80 (br s, 1 H), 8.82 (s, 1 H), 8.87 (br s, 1 H). MS (method B) m/z 410 [M+1]+; t=1.43 min-Example 83 (absolute configuration unknown)

Peak 2 (2^{nd} isomer): 1H NMR (400 MHz, DMSO-d6) δ ppm: 0.55 (d, J=7 Hz, 3 H), 1.84 (d, J=12 Hz, 1 H), 2.43 (m hidden, 1 H), 2.70 (br s, 1 H), 3.13 (t, J=11 Hz, 1 H), 3.51 (t, J=11 Hz, 1 H), 3.66 (s, 3 H), 3.96 (dd, J=11, 4 Hz, 1 H), 4.04 (dd, J=12, 4 Hz, 1 H), 4.25 (br s, 1 H), 4.56 (t, J=6 Hz, 2 H), 4.81 (t, J=7 Hz, 2 H), 5.31 (quin, J=6 Hz, 1 H), 7.47 (s, 1 H), 7.80 (br s, 1 H), 8.82 (s, 1 H), 8.86 (br s, 1 H). MS (method B) m/z 410 [M+1]+; t=1.43 min - Example 84 (absolute configuration unknown)

### Example 85 and Example 86: 2-((1-Methyl-3-(oxetan-3-yloxy)-1H-pyrazol-4-yl)amino)-7-((3R,4R)-3-methyltetrahydro-2H-pyran-4-yl)-7H-pyrrolo[2,3-d]pyrimidine-6-carbonitrile and 2-((1-methyl-3-(oxetan-3-yloxy)-1H-pyrazol-4-yl)amino)-7-((3S,4S)-3-methyltetrahydro-2H-pyran-4-yl)-7H-pyrrolo[2,3-d]pyrimidine-6-carbonitrile

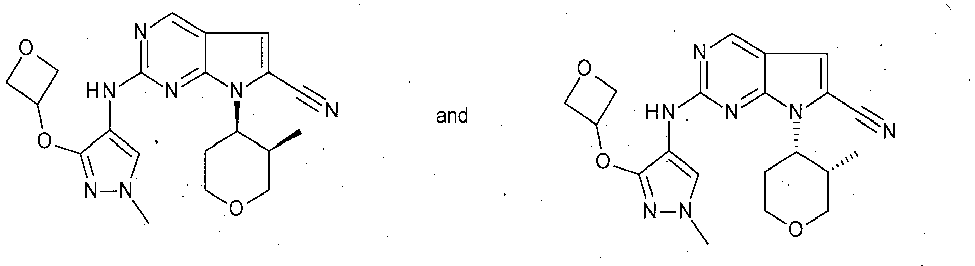

### Step 1: Preparation of 3-methyltetrahydropyran-4-ol (racemic trans)

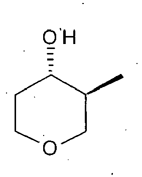

3.45 g (1 eq.) of 3-methyltetrahydro-2H-pyran-4-yl acetate (racemic *trans)* was added to 23.5 mL (1.07 eq.) of a pre-cooled (-5°C) 1N solution of sodium methoxide in methanol. The mixture was stirred at 0°C for 1 hour 30 minutes then quenched with 11.8 mL (1.07 eq.) of a 2N hydrogen chloride solution in diethyl ether. After addition of another 50 mL of diethyl ether, the suspension was filtered over a pad of decalite and the filter cake rinsed twice with 20 mL of diethyl ether. The filtrate was concentrated at 40°C under reduced pressure of 120 mbars and the residue was purified on silica, eluting with 0-100% of diethyl ether in pentane to afford (after concentration at 40°C under reduced pressure of 120 mbars) 1.78g of 3-methyltetrahydropyran-4-ol (racemic *trans*)*.*
¹H NMR (400 MHz, CDCl₃) δ in ppm: 0.92 (d, J=6.6 Hz, 3 H); 1.58 (m, 2 H); 1.84 (br s, 1 H); 1.88 (m, 1 H); 2.98 (t, J=11.1 Hz, 1 H); 3.31 (td, J=10 Hz and J=4.7 Hz, 1 H); 3.40 (td, J=11.5 Hz and J=2.3 Hz, 1 H); 3.82 (dd, J=4.4 Hz and J=11.6 Hz, 1 H); 3.96 (m, 1 H).

### Step 2: Preparation of methyl 2-methylsulfanyl-7-[(cis)-3-methyltetrahydropyran-4-yl]pyrrolo[2,3-d]pyrimidine-6-carboxylate (racemic cis)

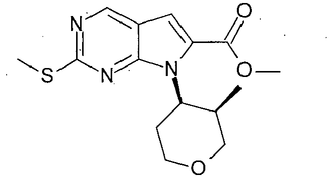

1.75 g (2 eq.) of triphenylphosphine, 568 mg (1.5 eq.) of 3-methyltetrahydropyran-4-ol (racemic *trans*) and 1.32 g (2 eq.) of diisopropyl azodicarboxylate (DIAD) were added to a solution of 750 mg (1 eq.) of methyl 2-(methylthio)-7H-pyrrolo[2,3-d]pyrimidine-6-carboxylate (intermediate 1) in 40 mL of anhydrous tetrahydrofuran under argon. The mixture was stirred at room temperature for 5 hour then another 1 eq. of triphenylphosphine, 1 eq. of racemic (trans)-3-methyltetrahydropyran-4-ol and 1 eq. of DIAD were added and the mixture was stirred at 50°C for 2 hours then concentrated under reduced pressure. The residue was purified on silica, eluting with 0-70% of ethyl acetate in cyclohexane to afford 610 mg of methyl 2-methylsulfanyl-7-[3-methyltetrahydropyran-4-yl]pyrrolo[2,3-d]pyrimidine-6-carboxylate (racemic *cis*). MS (method F) m/z 322 [M+1]+; t=1.88 min.

### Step 3: Preparation of 2-methylsulfanyl-7-[3-methyltetrahydropyran-4-yl]pyrrolo[2,3-d]pyrimidine-6-carboxamide (racemic cis)

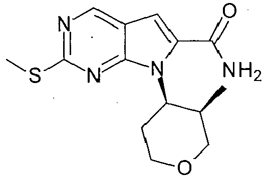

610 mg (1 eq.) of methyl 2-methylsulfanyl-7-[3-methyltetrahydropyran-4-yl]pyrrolo[2,3-d]pyrimidine-6-carboxylate (racemic *cis*) in 50 mL of a 7N solution of ammoniac in methanol in a sealed tube were stirred 3 days at 80°C. The mixture was then concentrated under reduced pressure to afford 503 mg of 2-methylsulfanyl-7-[3-methyltetrahydropyran-4-yl]pyrrolo[2,3-d]pyrimidine-6-carboxamide (racemic *cis*). MS (method E) m/z 307 [M+1]+; t=1.07 min.

### Step 4: Preparation of 2-methylsulfonyl-7-[3-methyltetrahydropyran-4-yl]pyrrolo[2,3-d]pyrimidine-6-carbonitrile (racemic cis)

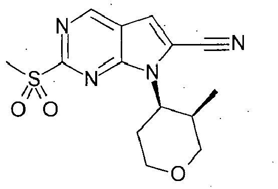

473 mg of 2-methylsulfonyl-7-[3-methyltetrahydropyran-4-yl]pyrrolo[2,3-d]pyrimidine-6-carbonitrile (racemic *cis*) was prepared according to the procedure described in steps 3 and 4 of Example 1, starting with 503 mg of 2-methylsulfanyl-7-[3-methyltetrahydropyran-4-yl]pyrrolo[2,3-d]pyrimidine-6-carboxamide (racemic *cis*). MS (method F) m/z 321 [M+1]+; t=1.29 min.

### Step 5: Preparation of 2-((1-methyl-3-(oxetan-3-yloxy)-1H-pyrazol-4-yl)amino)-7-((3R,4R)-3-methyltetrahydro-2H-pyran-4-yl)-7H-pyrrolo[2,3-d]pyrimidine-6-carbonitrile and 2-((1-methyl-3-(oxetan-3-yloxy)-1H-pyrazol-4-yl)amino)-7-((3S,4S)-3-methyltetrahydro-2H-pyran-4-yl)-7H-pyrrolo[2,3-d]pyrimidine-6-carbonitrile

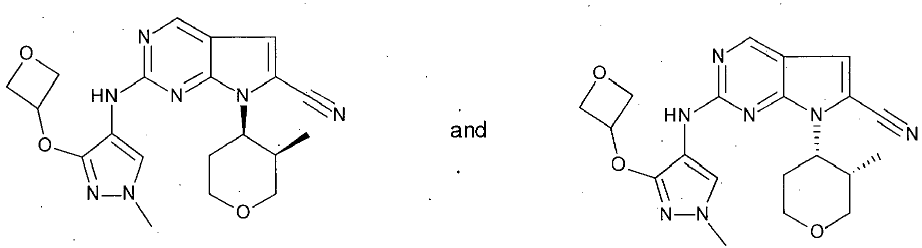

Generally following the procedure described in Step 5 of Example 1, racemic 2-methylsulfonyl-7-[(cis)-3-methyltetrahydropyran-4-yl]pyrrolo[2,3-d]pyrimidine-6-carbonitrile (250 mg) and N-[1-methyl-3-(oxetan-3-yloxy)pyrazol-4-yl]formamide (intermediate 4) gave a racemic mixture of 2-((1-methyl-3-(oxetan-3-yloxy)-1H-pyrazol-4-yl)amino)-7-((3R,4R)-3-methyltetrahydro-2H-pyran-4-yl)-7H-pyrrolo[2,3-d]pyrimidine-6-carbonitrile and 2-((1-methyl-3-(oxetan-3-yloxy)-1H-pyrazol-4-yl)amino)-7-((3S,4S)-3-methyltetrahydro-2H-pyran-4-yl)-7H-pyrrolo[2,3-d]pyrimidine-6-carbonitrile (168 mg) (Referred herein as ((1-methyl-3-(oxetan-3-yloxy)-1H-pyrazol-4-yl)amino )-7-((cis)-3-methyltetrahydro-2H-pyran-4-yl)-7H-pyrrolo[2,3-d]pyrimidine-6-carbonitrile). Chiral separation performed on 162 mg of the isomeric mixture gave 42 mg of the first eluting isomer and 46 mg of the second eluting isomer (conditions: column Chiralpak IF, 5 µm, 250×30 mm; liquid phase: heptane 35/ethyl alcohol 65; flow rate: 45 mL/min).

Peak 1 (1^{st} isomer): 1H NMR (400 MHz, DMSO-d6) δ ppm: 0.92 (d, J=7 Hz, 3 H), 1.82 (d, J=11 Hz, 1 H), 2.18 (s, 1 H), 3.15 (m, 1 H), 3.52 (t, J=11 Hz, 1 H), 3.63 (m, 1 H), 3.67 (s, 3 H), 3.74 (m, 1 H), 4.06 (dd, J=11, 4 Hz, 1 H), 4.55 (t, J=6 Hz, 2 H), 4.80 (t, J=7 Hz, 2 H), 4.97 (dt, J=13, 4 Hz, 1 H), 5.30 (quin, J=6 Hz, 1 H), 7.46 (s, 1 H), 7.76 (s, 1 H), 8.80 (br s, 1 H), 8.80 (s, 1 H). MS (method B) m/z 410 [M+1]+; t=1.45 min - Example 85 (absolute configuration unknown)

Peak 2 (2^{nd} isomer): 1H NMR (400 MHz, DMSO-d6) δ ppm: 0.92 (d, J=7 Hz, 3 H), 1.82 (d, J=11 Hz, 1 H), 2.19 (m, 1 H), 3.17 (m, 1 H), 3.51 (t, J=11 Hz, 1 H), 3.63 (m, 1 H), 3.67 (s, 3 H), 3.74 (m, 1 H), 4.06 (dd, J=11, 5 Hz, 1 H), 4.55 (t, J=6 Hz, 2 H), 4.80 (t, J=7 Hz, 2 H), 4.97 (dt, J=13, 4 Hz, 1 H), 5.30 (quin, J=6 Hz, 1 H), 7.46 (s, 1 H), 7.76 (s, 1 H), 8.80 (br s, 1 H), 8.80 (s, 1 H). MS (method B) m/z 410 [M+1]; t=1.44 min ― Example 86 (absolute configuration unknown)

### Example 87 and 88: 7-[(1S)-2-Methoxy-1-methyl-ethyl]-2-[[1-methyl-3-[(2S,3R)-2-methyloxetan-3-yl]oxy-pyrazol-4-yl]amino]pyrrolo[2,3-d]pyrimidine-6-carbonitrile and 7-[(1S)-2-methoxy-1-methyl-ethyl]-2-[[1-methyl-3-[(2R,3S)-2-methyloxetan-3-yl]oxy-pyrazol-4-yl]amino]pyrrolo[2,3-d]pyrimidine-6-carbonitrile

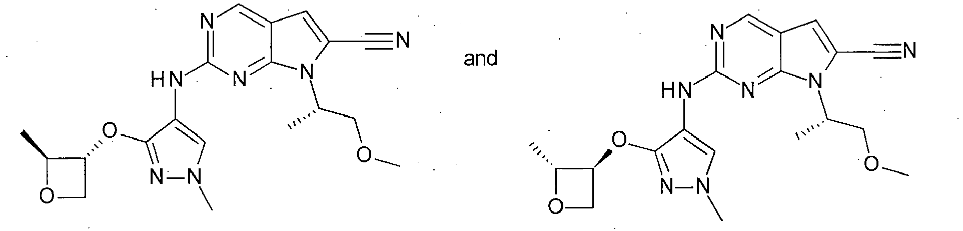

### Step 1: Preparation of methyl (S)-N-(5-formyl-2-(methylthio)pyrimidin-4-yl)-N-(1-methoxypropan-2-yl)glycinate

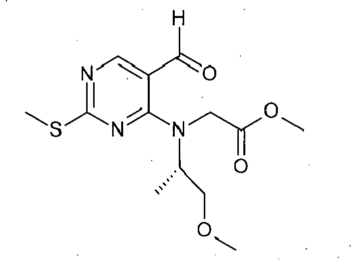

3.8 mL of triethylamine (1.7 eq.) was added to a solution of 3 g of 4-chloro-2-(methylthio)pyrimidine-5-carbaldehyde (1 eq.) in 100 mL of tetrahydrofuran and 20 mL of dichloromethane at 0°C. 3.2 g of methyl (S)-(1-methoxypropan-2-yl)glycinate (1.25 eq) was added dropwise. The mixture was stirred at room temperature for 3 days, diluted with ethyl acetate and water. The organic layer was separated and the aqueous layer extracted twice with 50 mL of ethyl acetate. The combined organic layers were washed twice with 50 mL of brine, dried over magnesium sulfate, and concentrated under reduced pressure. The residue was purified on silica gel, eluting with 50% of ethyl acetate in heptane, to afford 4.9 g of methyl (S)-N-(5-formyl-2-(methylthio)pyrimidin-4-yl)-N-(1-methoxypropan-2-yl)glycinate. MS (method A) m/z 314 [M+1]+; t=1.95 min.

### Step 2: Preparation of methyl (S)-7-(1-methoxypropan-2-yl)-2-(methylthio)-7H-pyrrolo[2,3-d]pyrimidine-6-carboxylate

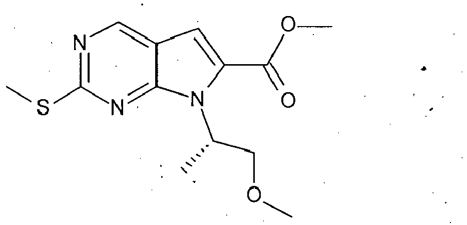

55.68 mL (5 eq.) of 1,8-diazabicyclo[5.4.0]undec7-ene was dropwise added to a solution of 28 g of methyl (S)-N-(5-formyl-2-(methylthio)pyrimidin-4-yl)-N-(1-methoxypropan-2-yl)glycinate (1 eq.) in 350 mL of acetonitrile. The reaction mixture was refluxed for 1 hour. After cooling down to room temperature, the reaction mixture was diluted with ethyl acetate and quenched with a 1N HCl aqueous solution. The organic layer was dried over magnesium sulfate, filtered, and concentrated under reduced pressure. The residue was purified on silica gel, eluting with 20% of ethyl acetate in heptane, to afford 15 g of methyl (S)-7-(1-methoxypropan-2-yl)-2-(methylthio)-7H-pyrrolo[2,3-d]pyrimidine-6-carboxylate. MS (method A) m/z 296 [M+1]+; t=2.11 min.

### Step 3: Preparation of (S)-7-(1-methoxypropan-2-yl)-2-(methylthio)-7H-pyrrolo[2,3-d]pyrimidine-6-carboxamide

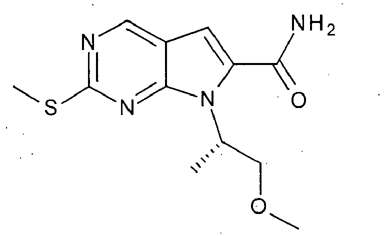

A solution of 15 g of methyl (S)-7-(1-methoxypropan-2-yl)-2-(methylthio)-7H-pyrrolo[2,3-d]pyrimidine-6-carboxylate (1 eq.) in 400 mL of 7N NH₃ in methanol (in a sealed tube) was stirred for 3 days at room temperature then concentrated under reduced pressure to afford 12.5 g of (S)-7-(1-methoxypropan-2-yl)-2-(methylthio)-7H-pyrrolo[2,3-d]pyrimidine-6-carboxamide. MS (method A) m/z 281 [M+1]+; t=1.77 min.

### Step 4: Preparation of (S)-7-(1-methoxypropan-2-yl)-2-(methylthio)-7H-pyrrolo[2,3-d]pyrimidine-6-carbonitrile

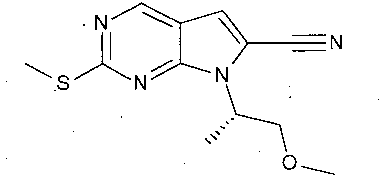

28.2 mL (4.5 eq.) of triethylamine was added to a solution of 12.5 g of (S)-7-(1-methoxypropan-2-yl)-2-(methylthio)-7H-pyrrolo[2,3-d]pyrimidine-6-carboxamide (1 eq.) in 300 mL of anhydrous tetrahydofuran at 0°C under argon, followed by 22.33 mL (3.6 eq.) of trifluoroacetic anhydride. The mixture was stirred for 45 minutes allowing the temperature to warm up to room temperature, and then diluted with ethyl acetate and quenched with an aqueous saturated sodium hydrogenocarbonate solution. The organic layer was separated. The aqueous layer was extracted twice with ethyl acetate. The combined organic extracts were dried over magnesium sulfate and concentrated under reduced pressure to afford 16 g of (S)-7-(1-methoxypropan-2-yl)-2-(methylthio)-7H-pyrrolo[2,3-d]pyrimidine-6-carbonitrile. MS (method A) m/z 263 [M+1]+; t=2.33 min.

### Step 5: Preparation of (S)-7-(1-methoxypropan-2-yl)-2-(methylsulfonyl)-7H-pyrrolo[2,3-d]pyrimidine-6-carbonitrile

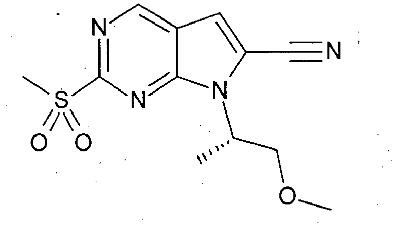

23.68 g of 3-chloroperbenzoic acid (77% purity; 3 eq.) was portionwise added to a solution of 16 g (1 eq.) (S)-7-(1-methoxypropan-2-yl)-2-(methylthio)-7H-pyrrolo[2,3-d]pyrimidine-6-carbonitrile in 300 mL of dichloromethane at 0°C. The mixture was stirred for 3 hours allowing the temperature to warm up to room temperature then quenched with 150 mL of aqueous 10% solution of sodium thiosulfate. The organic layer was separated, washed with 150 mL of aqueous 10% solution of sodium thiosulfate twice with 150 mL of aqueous saturated hydrogenocarbonate solution, then dried over magnesium sulfate, and concentrated under reduced pressure. The crude material was triturated with diisopropyl ether and the solid filtered to afford 10 g of (S)-7-(1-methoxypropan-2-yl)-2-(methylsulfonyl)-7H-pyrrolo[2,3-d]pyrimidine-6-carbonitrile. MS (method A) m/z 295 [M+1]+; t=1.68 min.

### Step 6: Preparation of 7-[(1S)-2-methoxy-1-methyl-ethyl]-2-[[1-methyl-3-[(2S, 3R)-2-methyloxetan-3-yl]oxy-pyrazol-4-yl]amino]pyrrolo[2,3-d]pyrimidine-6-carbonitrile and 7-[(1S)-2-methoxy-1-methyl-ethyl]-2-[[1-methyl-3-[(2R,3S)-2-methyloxetan-3-yl]oxy-pyrazol-4-yl]amino]pyrrolo[2,3-d]pyrimidine-6-carbonitrile

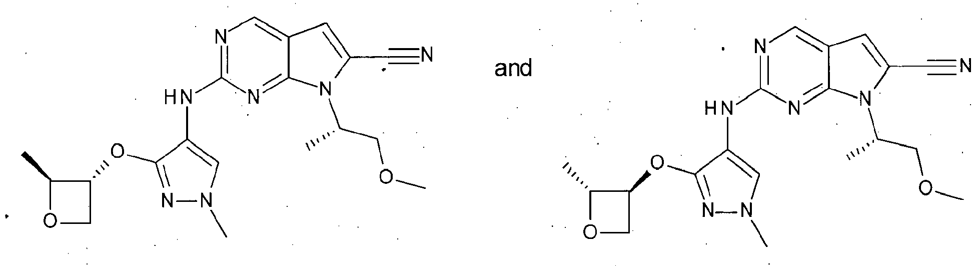

Following the general procedure in step 5 of Example 1, (S)-7-(1-methoxypropan-2-yl)-2-(methylsulfonyl)-7H-pyrrolo[2,3-d]pyrimidine-6-carbonitrile (120 mg) and a racemic mixture of N-(1-methyl-3-(((2R,3S)-2-methyloxetan-3-yl)oxy)-1H-pyrazol-4-yl)formamide and N-(1-methyl-3-(((2S,3R)-2-methyloxetan-3-yl)oxy)-1H-pyrazol-4-yl)formamide (91 mg) (Intermediate 19) gave an isomeric mixture of 7-[(1S)-2-methoxy-1-methyl-ethyl]-2-[[1-methyl-3-[(2S,3R)-2-methyloxetan-3-yl]oxy-pyrazol-4-yl]amino]pyrrolo[2,3-d]pyrimidine-6-carbonitrile and 7-[(1S)-2-methoxy-1-methyl-ethyl]-2-[[1-methyl-3-[(2R,3S)-2-methyloxetan-3-yl]oxy-pyrazol-4-yl]amino]pyrrolo[2,3-d]pyrimidine-6-carbonitrile (119mg) (Referred herein as 7-[(1S)-2-methoxy-1-methylethyl]-2-[[1-methyl-3-[(trans)-2-methyloxetan-3-yl]oxy-pyrazol-4-yl]amino]pyrrolo[2,3-d]pyrimidine-6-carbonitrile).

Chiral separation performed on 110 mg of the isomeric mixture gave 50 mg of the first eluting isomer and 60 mg of the second eluting isomer (conditions: column Chiralpak AY, 20 µm, 250x4.6 mm; liquid phase: methanol 15/ethyl alcohol 85 to methanol 40/ethyl alcohol 60; flow rate: 200 mL/min).

Peak 1 (1^{st} isomer): 1H NMR (400 MHz, DMSO-d6) δ ppm: 1.41 (d, J=6 Hz, 3 H), 1.55 (d, J=7 Hz, 3 H), 3.19 (s, 3 H), 3.64 (dd, J=11, 5 Hz, 1 H), 3.68 (s, 3 H), 3.98 (t, J=10 Hz, 1 H), 4.38 (dd, J=7, 6 Hz, 1 H), 4.65 (t, J=7 Hz, 1 H), 4.77 (quin, J=6 Hz, 1 H), 4.84 - 4.98 (m, 2 H), 7.42 (s, 1 H), 7.72 (s, 1 H), 8.74 (br s, 1 H), 8.79 (s, 1 H). MS (method C) m/z 398 [M+1]+; t=2.82 min ― Example 87 (absolute configuration unknown)

Peak 2 (2^{nd} isomer): 1H NMR (400 MHz, DMSO-d6) δ ppm: 1.40 (d, J=6 Hz, 3 H), 1.54 (d, J=7 Hz, 3 H), 3.19 (s, 3 H), 3.64 (dd, J=10, 5 Hz, 1 H), 3.67 (s, 3 H), 3.97 (t, J=10 Hz, 1 H), 4.37 (dd, J=7, 6 Hz, 1 H), 4.64 (t, J=7 Hz, 1 H), 4.77 (quin, J=6 Hz, 1 H), , 4.88 (q, J=5 Hz, 1 H),4.89 (m, 1 H), 7.41 (s, 1 H), 7.72 (s, 1 H), 8.71 (br s, 1 H), 8.78 (s, 1 H). MS (method B) m/z 398 [M+1]+; t=1.47 min Example 88 (absolute configuration unknown)

### Example 89 and 90: 7-[(1S)-2-methoxy-1-methyl-ethyl]-2-[[3-[(2S,3R)-2-methyloxetan-3-yl]oxy-1-(methyl-d3)pyrazol-4-yl]amino]pyrrolo[2,3-d]pyrimidine-6-carbonitrile and 7-[(1S)-2-methoxy-1-methyl-ethyl]-2-[[3-[(2R,3S)-2-methyloxetan-3-yl]oxy-1-(methyl-d3)pyrazol-4-yl]amino]pyrrolo[2,3-d]pyrimidine-6-carbonitrile

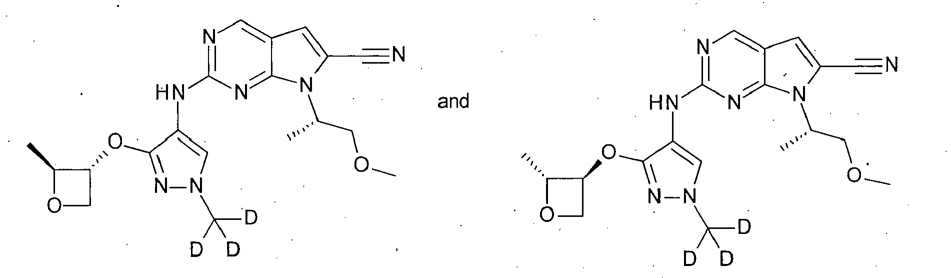

Following the general procedure in step 5 of Example 1,(S)-7-(1-methoxypropan-2-yl)-2-(methylsulfonyl)-7H-pyrrolo[2,3-d]pyrimidine-6-carbonitrile (200 mg) ) (step 5 of Example 87) and a racemic mixture of N-(1-(methyl-d3)-3-(((2R,3S)-2-methyloxetan-3-yl)oxy)-1H-pyrazol-4-yl)formamide and N-(1-(methyl-d3)-3-(((2S,3R)-2-methyloxetan-3-yl)oxy)-1H-pyrazol-4-yl)formamide (160 mg) (Intermediate 21) gave an isomeric mixture of 7-[(1S)-2-methoxy-1-methyl-ethyl]-2-[[3-[(2S,3R)-2-methyloxetan-3-yl]oxy-1-(methyl-d3)pyrazol-4-yl]amino]pyrrolo[2,3-d]pyrimidine-6-carbonitrile and 7-[(1S)-2-methoxy-1-methyl-ethyl]-2-[[3-[(2R,3S)-2-methyloxetan-3-yl]oxy-1-(methyl-d3)pyrazol-4-yl]amino]pyrrolo[2,3-d]pyrimidine-6-carbonitrile (219mg) (Referred herein as 7-[(1S)-2-methoxy-1-methyl-ethyl]-2-[[3-[(trans)-2-methyloxetan-3-yl]oxy-1-(methyl-d3)pyrazol-4-yl]amino]pyrrolo[2,3-d]pyrimidine-6-carbonitrile).

Chiral separation performed on 212 mg of the isomeric mixture gave 98 mg of the first eluting isomer and 95 mg of the second eluting isomer (conditions: column Chiralpak AY, 20 µm, 250×4.6 mm; liquid phase: methanol 15/ethyl alcohol 85 to methanol 40/ethyl alcohol 60; flow rate: 200 mL/min).

Peak 1 (1^{st} isomer): 1H NMR (400 MHz, DMSO-d6) δ ppm: 1.40 (d, J=6 Hz, 3 H), 1.54 (d, J=7 Hz, 3 H), 3.18 (s, 3 H), 3.64 (dd, J=10, 5 Hz, 1 H), 3.97 (t, J=10 Hz, 1 H), 4.38 (dd, J=7, 6 Hz, 1 H), 4.64 (t, J=7 Hz, 1 H), 4.76 (quin, J=6 Hz, 1 H), 4.83 - 4.96 (m, 2 H), 7.41 (s, 1 H), 7.72 (s, 1 H), 8.73 (br s, 1 H), 8.78 (s, 1 H). MS (method C) m/z 401 [M+1]+; t=2.81min - Example 89 (absolute configuration unknown)

Peak 2 (2^{nd} isomer): 1H NMR (400 MHz, DMSO-d6) δ ppm: 1.41 (d, J=6 Hz, 3 H), 1.54 (d, J=7 Hz, 3 H), 3.19 (s, 3 H), 3.64 (dd, J=10, 5 Hz, 1 H), 3.97 (t, J=10 Hz, 1 H), 4.37 (dd, J=7, 6 Hz, 1 H), 4.64 (t, J=7 Hz, 1 H), 4.77 (quin, J=6 Hz, 1 H), 4.88 (q, J=7 Hz, 1 H), 4.89 (m, 1 H), 7.42 (s, 1 H), 7.72 (s, 1 H), 8.77 (br s, 1 H), 8.78 (s, 1 H). MS (method C) m/z 401 [M+1]+; t=2.81 min - Example 90 (absolute configuration unknown)

Generally following the procedure described in Example 88, reacting (S)-7-(1-methoxypropoan-2-yl)-2-(methylsulfonyl)-7H-pyrrolo[2,3-d]pyrimidine-6-carbonitrile with appropriate formamides gave Example 91 to 93 in table III.

**Table III**

| Compound/Example | Name | Formamide | NMR | LC/MS MH+; t, Method |
|---|---|---|---|---|
| 91 | 7-[(2S)-1-Methoxypropan-2-yl]-2-[[3-(oxetan-3-yloxy)-1-(methyl-d3)pyrazol-4-yl]amino]pyrrolo[2,3-d]pyrimidine-6-carbonitrile | Intermediate 26 | 1.54 (d, J=7 Hz, 3 H), 3.18 (s, 3 H), 3.64 (dd, J=10, 5 Hz, 1 H), 3.97 (t, J=10 Hz, 1 H), 4.55 (ddd, J=7, 5, 2 Hz, 2 H), 4.80 (td, J=7, 2 Hz, 2 H), 4.91 (m, 1 H), 5.31 (quin, J=6 Hz, 1 H), 7.42 (s, 1 H), 7.72 (s, 1 H), 8.70 (br s, 1 H), 8.78 (s, 1 H) | 387; 2.62 C |
| 92 | 2-[[3-(Cyclopropoxy)-1-(methoxymethyl) pyrazol-4-yl]amino]-7-[(1S)-2-methoxy-1-methylethyl]pyrrolo[2,3-d]pyrimidine-6-carbonitrile | Intermediate 15 | 0.62 - 0.70 (m, 4 H), 1.54 (d, J=7 Hz, 3 H), 3.18 (s, 3 H), 3.25 (s, 3 H), 3.63 (dd, J=10, 5 Hz, 1 H), 3.98 (t, J=10 Hz, 1 H), 4.09 (tt, J=6, 3 Hz; 1 H), 4.88 (m, 1 H), 5.24 (s, 2 H), 7.41 (s, 1 H), 7.96 (s, 1 H), 8.70 (br s, 1 H), 8.79 (s, 1 H) | 398; 1.55 B |
| 93 | 7-[(1S)-2-Methoxy-1-methyl-ethyl]-2-[[1-(methylsulfonylmethyl)-3-(oxetan-3-yloxy)pyrazol-4-yl]amino]pyrrolo[2,3-d]pyrimidine-6-carbonitrile | Intermediate 13 | 1.56 (d, J=7 Hz, 3 H), 2.98 (s, 3 H), 3.18 (s, 3 H), 3.63 (dd, J=11, 5 Hz, 1 H), 4.02 (t, J=10 Hz, 1 H), 4.60 (ddd, J=7, 5, 1 Hz, 2 H), 4.84 (t, J=7 Hz, 2 H), 4.94 (m, 1 H), 5.40 (quin, J=6 Hz, 1 H), 5.55 (s, 2 H), 7.44 (s, 1 H), 8.02 (s, 1 H), 8.84 (s, 1 H), 9.01 (br s, 1 H) | 462; 1.33 B |

### Example 94: 2-((1-Methyl-3-(oxetan-3-yloxy)-1H-pyrazol-4-yl-5-d)amino)-7-((3R,4R)-4-methyltetrahydrofuran-3-yl)-7H-pyrrolo[2,3-d]pyrimidine-6-carbonitrile

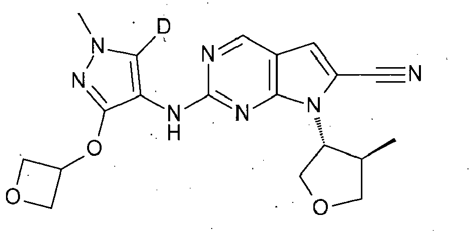

99 mg (1 eq.) of 2-[[1-methyl-3-(oxetan-3-yloxy)pyrazol-4-yl]amino]-7-[(3R,4R)-4-methyltetrahydrofuran-3-yl]pyrrolo[2,3-d]pyrimidine-6-carbonitrile (Example 8) was dissolved in 4 mL of anhydrous 1.4-dioxane in a microwave vial, 5-10 mol% of Ruthenium complex B* (The synthesis of the Ruthenium catalyst B - (Ru(O₂CAd)₂(p-cymene) and the reaction conditions are based on ChemCatChem 2019, 11, 1-6) was added. A homogeneous orange solution was obtained. 2 mL of 99.9% deuterium oxide was added at room temperature and the reaction mixture heated for 4 hours under µW irradiation at 100 °C. Crude reaction mixture was evaporated down to dryness, dissolved in 1 mL of dichloromethane and purified on silica, eluting with 50/50 to 10/90 of heptane/ethyl acetate. 90 mg of 2-((1-methyl-3-(oxetan-3-yloxy)-1H-pyrazol-4-yl-5-d)amino)-7-((3R,4R)-4-methyltetrahydrofuran-3-yl)-7H-pyrrolo[2,3-d]pyrimidine-6-carbonitrile was obtained by dissolving the obtained gum in 50/50 heptane/ethyl acetate and subsequently with 1% NH₃ in methanol.
1H NMR (400 MHz, DMSO-d6) δ ppm: 1.05 (d, J=7 Hz, 3 H), 2.93 (m, 1 H), 3.47 (t, J=9 Hz, 1 H), 3.65 (s, 3 H), 4.09 - 4.26 (m, 2 H), 4.29 (m, 1 H), 4.53 - 4.62 (m, 2 H), 4.76 (t, J=6 Hz, 1 H), 4.81 (t, J=7 Hz, 2 H), 5.31 (quin, J=6 Hz, 1 H), 7.46 (s, 1 H), 8.82 (s, 1 H), 8.90 (br s, 1 H). MS (method C) m/z 397 [M+1]+; t=2.7 min - Example 94

### PHARMACOLOGY

The in vitro potency of compounds in inhibiting LRRK2 kinase may be determined by the procedures detailed below.

LRRK2-G2019S Kinase activity assay:
The measurements of the activities were made using a LRRK2-G2019S(human) Kinase activity assay as described hereunder. The potency of compounds was evaluated *in vitro* using the Lanthascreen^{®} technology (ThermoFisher Scientific).

The biological assay requires the DYKDDDDK-tagged full-length LRRK2 G2019S protein, the fluorescent peptide substrate named LRRKtide or ERM (RLGRDKYKTLRQIRQ supplied by PolyPeptide Group), the ATP substrate, and different concentrations of test compounds. In a typical experiment, the reaction assay buffer contains 50mM Tris pH8.2, 1mM EGTA, 2.5mM MgCl2, 0.05% Triton X100, 0.15% BSA and 1mM DTT. The detection of the assay is performed using 1nM Terbium labeled anti-phospho-LRRKtide (ThermoFisher Scientific) diluted in a TR-FRET buffer (ThermoFisher Scientific).

The experimental procedure was performed with Greiner small volume^{®} 384-well. The assay is started by a pre-incubation step of full-length LRRK2 G2019S mutant protein with reaction assay buffer and a dilution series of test compound for at least 30 minutes at room temperature. The working solution of compounds was prepared in a separate Greiner microplate.

The kinase reaction was initiated by addition of the ATP/LRRKtide substrate mix diluted in the reaction assay buffer. The reaction mix was incubated for at least 10 minutes at room temperature (initial velocity conditions). Reaction was stopped by addition of Terbium labeled anti-phospho-LRRKtide diluted into the detection buffer supplemented with EDTA (ThermoFisher Scientific). Plates are incubated for at least 30 minutes at room temperature and read on an Envision^{™} multimode plate reader (PerkinElmer) with LanthaScreen^{™}. The TR-FRET 520:495 nm emission ratio was calculated and used to determine the IC50 value from a dose response curve fit to the 4-parameter logistic equation. The activity with respect to LRRK2 kinase in this test is given by the concentration which inhibits 50% of the LRRK2 activity (or IC50) in nM.

LRRK2 WT assay (assay A):
The kinase activity of LRRK2(human) WT was evaluated according to same method described in LRRK2-G2019 kinase activity assay as described above, except DYDDDDK tagged full-length LRRK2 wild type is used instead of DYKDDDDK-tagged full-length LRRK2 G2019S, and 500uM ATP is used in WT assay instead of 1.1mM ATP.

LRRK2 WT assay (assay B):
LRRK2(human) is incubated with 50 mM HEPES pH 8.0, 0.2 mM EDTA, 0.01% Brij-35, 1% (w/v) BSA, 5 mM DTT, 250 Mm RLGRDKYKTLRQIRQ, 10 mM Magnesium acetate and [8-33P]-ATP (specific activity and concentration as required).

The reaction is initiated by the addition of the Mg/ATP mix. After incubation for 40 minutes at room temperature, the reaction is stopped by the addition of phosphoric acid to a concentration of 0.5%. 10 µL of the reaction is then spotted onto a P30 filtermat and washed four times for 4 minutes in 0.425% phosphoric acid and once in methanol prior to drying and scintillation counting.

### Biologic data

The table (Table IV) below shows the IC50 values obtained as described above for the exemplified compounds.

**Table IV:**

| Compound / Example | Name | LRRK2/G2019S IC50 (nM) | LRRK2 WT IC50 (nM) |
|---|---|---|---|
| 1 | 2-[[3-(1-acetylazetidin-3-yl)oxy-1-methyl-pyrazol-4-yl]amino]-7-[(3R,4R)-4-methyltetrahydrofuran-3-yl]pyrrolo[2,3-d]pyrimidine-6-carbonitrile | 5 | 6 |
| 2 | 2-[[1-methyl-3-[(3S,4R)-4-methyltetrahydrofuran-3-yl]oxy-1H-pyrazol-4-yl]amino]-7-[(3R,4R)-4-methyltetrahydrofuran-3-yl]pyrrolo[2,3-d]pyrimidine-6-carbonitrile or 2-[[1-methyl-3-[(3R,4S)-4-methyltetrahydrofuran-3-yl]oxy-1H-pyrazol-4-yl]amino]-7-[(3R,4R)-4-methyltetrahydrofuran-3-yl]pyrrolo[2,3-d]pyrimidine-6-carbonitrile (1^{st} isomer) | 5 | 5 |
| 3 | 2-[[1-methyl-3-[(3S,4R)-4-methyltetrahydrofuran-3-yl]oxy-1H-pyrazol-4-yl]amino]-7-[(3R,4R)-4-methyltetrahydrofuran-3-yl]pyrrolo[2,3-d]pyrimidine-6-carbonitrile or 2-[[1-methyl-3-[(3R,4S)-4-methyltetrahydrofuran-3-yl]oxy-1H-pyrazol-4-yl]amino]-7-[(3R,4R)-4-methyltetrahydrofuran-3-yl]pyrrolo[2,3-d]pyrimidine-6-carbonitrile (2^{nd} isomer) | 4 | 5 |
| 4 | 2-[[1-[(1R)-2,2-difluoro-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile or 2-[[1-[(1S)-2,2-difluoro-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile (1^{st} isomer) | 1 | 1 |
| 5 | 2-[[1-[(1R)-2,2-difluoro-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile or 2-[[1-[(1S)-2,2-difluoro-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile (2^{nd} isomer) | 5 | 4 |
| 6 | 2-((1-methyl-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile or 2-((1-methyl-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile (1^{st} isomer) | 4 | 5 |
| 7 | 2-((1-methyl-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile or 2-((1-methyl-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile (2^{nd} isomer) | 2 | 2 |
| 8 | 2-[[1-methyl-3-(oxetan-3-yloxy)pyrazol-4-yl]amino]-7-[(3R,4R)-4-methyltetrahydrofuran-3-yl]pyrrolo[2,3-d]pyrimidine-6-carbonitrile | 4 | 6 |
| 9 | 2-[[1-[2-hydroxy-1-methyl-ethyl]-3-(oxetan-3-yloxy)pyrazol-4-yl]amino]-7-[(3R,4R)-4-methyltetrahydrofuran-3-yl]pyrrolo[2,3-d]pyrimidine-6-carbonitrile | 5 | 5 |
| 10 | 7-[(3R,4R)-4-methyltetrahydrofuran-3-yl]-2-[[1-(oxetan-3-yl)-3-[(1R)-2,2,2-trifluoro-1-methyl-ethoxy]pyrazol-4-yl]amino]pyrrolo[2,3-d]pyrimidine-6-carbonitrile or 7-[(3R,4R)-4-methyltetrahydrofuran-3-yl]-2-[[1-(oxetan-3-yl)-3-[(1S)-2,2,2-trifluoro-1-methyl-ethoxy]pyrazol-4-yl]amino]pyrrolo[2,3-d]pyrimidine-6-carbonitrile (1^{st} isomer) | 1 | 1 |
| 11 | 7-[(3R,4R)-4-methyltetrahydrofuran-3-yl]-2-[[1-(oxetan-3-yl)-3-[(1R)-2,2,2-trifluoro-1-methyl-ethoxy]pyrazol-4-yl]amino]pyrrolo[2,3-d]pyrimidine-6-carbonitrile or 7-[(3R,4R)-4-methyltetrahydrofuran-3-yl]-2-[[1-(oxetan-3-yl)-3-[(1S)-2,2,2-trifluoro-1-methyl-ethoxy]pyrazol-4-yl]amino]pyrrolo[2,3-d]pyrimidine-6-carbonitrile (2^{nd} isomer) | 19 | 27 |
| 12 | 2-[[3-[(3S,4S)-4-methoxytetrahydrofuran-3-yl]oxy-1-methyl-pyrazol-4-yl]amino]-7-[(3R,4R)-4-methyltetrahydrofuran-3-yl]pyrrolo[2,3-d]pyrimidine-6-carbonitrile or 2-[[3-[(3R,4R)-4-methoxytetrahydrofu ran-3-yl]oxy-1-methyl-pyrazol-4-yl]amino]-7-[(3R,4R)-4-methyltetrahydrofuran-3-yl]pyrrolo[2,3-d]pyrimidine-6-carbonitrile (1^{st} isomer) | 6 | 8 |
| 13 | 2-[[3-[(3S,4S)-4-methoxytetrahydrofuran-3-yl]oxy-1-methyl-pyrazol-4-yl]amino]-7-[(3R,4R)-4-methyltetrahydrofuran-3-yl]pyrrolo[2,3-d]pyrimidine-6-carbonitrile or 2-[[3-[(3R,4R)-4-methoxytetrahydrofuran-3-yl]oxy-1-methyl-pyrazol-4-yl]amino]-7-[(3R,4R)-4-methyltetrahydrofuran-3-yl]pyrrolo[2,3-d]pyrimidine-6-carbonitrile (2^{nd} isomer) | 9 | 13 |
| 14 | 2-[[1-(methylsulfonylmethyl)-3-(oxetan-3-yloxy)pyrazol-4-yl]amino]-7-[(3R,4R)-4-methyltetrahydrofuran-3-yl]pyrrolo[2,3-d]pyrimidine-6-carbonitrile | 2 | 2 |
| 15 | 2-((1-(methyl-d3)-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile or 2-((1-(methyl-d3)-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile (1^{st} isomer) | 7 | 7 |
| 16 | 2-((1-(methyl-d3)-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile or 2-((1-(methyl-d3)-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile (2^{nd} isomer) | 3 | 4 |
| 17 | 2-((3-(((R)-1-acetyl-2,2-dimethylazetidin-3-yl)oxy)-1-methyl-1H-pyrazol-4-yl)amino)-7-((3R,4R)-4-methyltetrahydrofuran-3-yl)-7H-pyrrolo[2,3-d]pyrimidine-6-carbonitrile or 2-((3-(((S)-1-acetyl-2,2-dimethylazetidin-3-yl)oxy)-1-methyl-1H-pyrazol-4-yl)amino)-7-((3R,4R)-4-methyltetrahydrofuran-3-yl)-7H-pyrrolo[2,3-d]pyrimidine-6-carbonitrile (1^{st} isomer) | 3 | 3 |
| 18 | 2-((3-(((R)-1-acetyl-2,2-dimethylazetidin-3-yl)oxy)-1-methyl-1H-pyrazol-4-yl)amino)-7-((3R,4R)-4-methyltetrahydrofuran-3-yl)-7H-pyrrolo[2,3-d]pyrimidine-6-carbonitrile or 2-((3-(((S)-1-acetyl-2,2-dimethylazetidin-3-yl)oxy)-1-methyl-1H-pyrazol-4-yl)amino)-7-((3R,4R)-4-methyltetrahydrofuran-3-yl)-7H-pyrrolo[2,3-d]pyrimidine-6-carbonitrile (2^{nd} isomer) | 2 | 2 |
| 19 | 2-((1-(methyl-d3)-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile | 5 | 5 |
| 20 | 2-((4-(2-hydroxypropan-2-yl)-2-(oxetan-3-yloxy)phenyl)amino)-7-((3R,4R)-4-methyltetrahydrofuran-3-yl)-7H-pyrrolo[2,3-d]pyrimidine-6-carbonitrile | 3 | 3 |
| 21 | 2-((1-methyl-3-(((R)-1,1,1-trifluoropropan-2-yl)oxy)-1H-pyrazol-4-yl)amino)-7-((3R,4R)-4-methyltetrahydrofuran-3-yl)-7H-pyrrolo[2,3-d]pyrimidine-6-carbonitrile or 2-((1-methyl-3-(((S)-1,1,1-trifluoropropan-2-yl)oxy)-1H-pyrazol-4-yl)amino)-7-((3R,4R)-4-methyltetrahydrofuran-3-yl)-7H-pyrrolo[2,3-d]pyrimidine-6-carbonitrile (1^{st} isomer) | 1 | 1 |
| 22 | 2-((1-methyl-3-(((R)-1,1,1-trifluoropropan-2-yl)oxy)-1H-pyrazol-4-yl)amino)-7-((3R,4R)-4-methyltetrahydrofuran-3-yl)-7H-pyrrolo[2,3-d]pyrimidine-6-carbonitrile or 2-((1-methyl-3-(((S)-1,1,1-trifluoropropan-2-yl)oxy)-1H-pyrazol-4-yl)amino)-7-((3R,4R)-4-methyltetrahydrofuran-3-yl)-7H-pyrrolo[2,3-d]pyrimidine-6-carbonitrile (2^{nd} isomer) | 25 | 30 |
| 23 | 2-((1-(methyl-d3)-3-(((2R,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile or 2-((1-(methyl-d3)-3-(((2S,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile (1^{st} isomer) | 8 | 10 |
| 24 | 2-((1-(methyl-d3)-3-(((2R,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile or 2-((1-(methyl-d3)-3-(((2S,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile (2^{nd} isomer) | 4 | 5 |
| 25 | 2-((3-isopropoxy-1-(2,2,2-trifluoroethyl)-1H-pyrazol-4-yl)amino)-7-((3R,4R)-4-methyltetrahydrofuran-3-yl)-7H-pyrrolo[2,3-d]pyrimidine-6-carbonitri le | 1 | 1 |
| 26 | 2-((3-isopropoxy-1-methyl-1H-pyrazol-4-yl)amino)-7-((3R,4R)-4-methyltetrahydrofuran-3-yl)-7H-pyrrolo[2,3-d]pyrimidine-6-carbonitrile | 1 | 1 |
| 27 | 2-((1-(2-cyanopropan-2-yl)-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile | 1 | 1 |
| 28 | 2-((3-cyclopropoxy-1-(methyl-d3)-1H-pyrazol-4-yl)amino)-7-((3R,4R)-4-methyltetrahydrofuran-3-yl)-7H-pyrrolo[2,3-d]pyrimidine-6-carbonitrile | 3 | 3 |
| 29 | 2-((1(2-cyanopropan-2-yl)-3-isopropoxy-1H-pyrazol-4-yl)amino)-7-((3R,4R)-4-methyltetrahydrofuran-3-yl)-7H-pyrrolo[2,3-d]pyrimidine-6-carbonitrile | 1 | 1 |
| 30 | 2-((3-cyclopropoxy-1-(oxetan-3-yl)-1H-pyrazol-4-yl)amino)-7-((3R,4R)-4-methyltetrahydrofuran-3-yl)-7H-pyrrolo[2,3-d]pyrimidine-6-carbonitrile | 3 | 5 |
| 31 | 2-((3-isopropoxy-1-(oxetan-3-yl)-1H-pyrazol-4-yl)amino)-7-((3R,4R)-4-methyltetrahydrofuran-3-yl)-7H-pyrrolo[2,3-d]pyrimidine-6-carbonitrile | 2 | 2 |
| 32 | 2-((3-(2-hydroxy-2-methylpropoxy)-1-methyl-1H-pyrazol-4-yl)amino)-7-((3R,4R)-4-methyltetrahydrofuran-3-yl)-7H-pyrrolo[2,3-d]pyrimidine-6-carbonitrile | 7 | 7 |
| 33 | 2-((1-(methoxymethyl)-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile | 9 | 14 |
| 34 | 2-((1-(methylsulfonyl)-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile | 5 | 7 |
| 35 | 2-((3-cyclopropoxy-1-(methoxymethyl)-1H-pyrazol-4-yl)amino)-7-((3R,4R)-4-methyltetrahydrofuran-3-yl)-7H-pyrrolo[2,3-d]pyrimidine-6-carbonitrile | 6 | 4 |
| 36 | 2-((3-cyclopropoxy-1-((methylsulfonyl)methyl)-1H-pyrazol-4-yl)amino)-7-((3R,4R)-4-methyltetrahydrofuran-3-yl)-7H-pyrrolo[2,3-d]pyrimidine-6-carbonitrile | 1 | 1 |
| 37 | 2-((1-cyclopropyl-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile | 6 | 8 |
| 38 | 2-((1-ethyl-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile | 5 | 6 |
| 39 | 2-[[1-(methoxymethyl)-3-[(trans)-2-methyloxetan-3-yl]oxy-pyrazol-4-yl]amino]-7-[(3R,4R)-4-methyltetrahydrofuran-3-yl]pyrrolo[2,3-d]pyrimdine-6-carbonitrile | 8 | 10 |
| 40 | 2-[[1-methyl-3-[(2-oxaspiro[3.3]heptan-7-yl)oxy]pyrazol-4-yl]amino]-7-[(3R,4R)-4-methyltetrahydrofuran-3-yl]pyrrolo[2,3-d]pyrimidine-6-carbonitrile | 1 | 1 |
| 41 | 2-((2-cyclopropoxy-4-((dimethylamino)methyl)phenyl)amino)-7-((3R,4R)-4-methyltetrahydrofu ran-3-yl)-7H-pyrrolo[2,3-d]pyrimidine-6-carbonitrile | 3 | 4 |
| 42 | 2-((3-isopropoxy-1-(methyl-d3)-1H-pyrazol-4-yl)amino)-7-((3R,4R)-4-methyltetrahydrofuran-3-yl)-7H-pyrrolo[2,3-d]pyrimidine-6-carbonitrile | 2 | 1 |
| 43 | 2-((3-cyclopropoxy-1-(1,1-difluoropropan-2-yl)-1H-pyrazol-4-yl)amino)-7-((3R,4R)-4-methyltetrahydrofuran-3-yl)-7H-pyrrolo[2,3-d]pyrimidine-6-carbonitrile | 1 | 2 |
| 44 | 2-((1-methyl-3-(oxetan-3-yloxy)-1H-pyrazol-4-yl)amino)-7-((3S,4S)-4-methyltetrahydrofuran-3-yl)-7H-pyrrolo[2,3-d]pyrimidine-6-carbonitrile | 191 | 196 |
| 45 | 2-((1-methyl-3-(oxetan-3-yloxy)-1H-pyrazol-4-yl)amino)-7-((3S,4R)-4-methyltetrahydrofuran-3-yl)-7H-pyrrolo[2,3-d]pyrimidine-6-carbonitrile or 2-((1-methyl-3-(oxetan-3-yloxy)-1H-pyrazol-4-yl)amino)-7-((3R,4S)-4-methyltetrahydrofuran-3-yl)-7H-pyrrolo[2,3-d]pyrimidine-6-carbonitrile (1^{st} isomer) | 35 | 46 |
| 46 | 2-((1-methyl-3-(oxetan-3-yloxy)-1H-pyrazol-4-yl)amino)-7-((3S,4R)-4-methyltetrahydrofuran-3-yl)-7H-pyrrolo[2,3-d]pyrimidine-6-carbonitrile or 2-((1-methyl-3-(oxetan-3-yloxy)-1H-pyrazol-4-yl)amino)-7-((3R,4S)-4-methyltetrahydrofuran-3-yl)-7H-pyrrolo[2,3-d]pyrimidine-6-carbonitrile (2^{nd} isomer) | 413 | 489 |
| 47 | 2-((3-isopropoxy-1-methyl-1H-pyrazol-4-yl)amino)-7-((cis)-4-methyltetrahydrofuran-3-yl)-7H-pyrrolo[2,3-d]pyrimidine-6-carbonitrile | 31 | 36 |
| 48 | 2-((1-((R)-1-Cyanoethyl)-3-isopropoxy-1H-pyrazol-4-yl)amino)-7-((3S,4R)-4-methyltetrahydrofuran-3-yl)-7H-pyrrolo[2,3-d]pyrimidine-6-carbonitrile or 2-((1-((S)-1-cyanoethyl)-3-isopropoxy-1H-pyrazol-4-yl)amino)-7-((3S,4R)-4-methyltetrahydrofuran-3-yl)-7H-pyrrolo[2,3-d]pyrimidine-6-carbonitrile or 2-((1-((R)-1-cyanoethyl)-3-isopropoxy-1H-pyrazol-4-yl)amino)-7-((3R,4S)-4-methyltetrahydrofuran-3-yl)-7H-pyrrolo[2,3-d]pyrimidine-6-carbonitrile or 2-((1-((S)-1-cyanoethyl)-3-isopropoxy-1H-pyrazol-4-yl)amino)-7-((3R,4S)-4-methyltetrahydrofuran-3-yl)-7H-pyrrolo[2,3-d]pyrimidine-6-carbonitrile (1^{st} isomer of 1^{st} mixture) | NT | 256* |
| 49 | 2-((1-((R)-1-Cyanoethyl)-3-isopropoxy-1H-pyrazol-4-yl)amino)-7-((3S,4R)-4-methyltetrahydrofuran-3-yl)-7H-pyrrolo[2,3-d]pyrimidine-6-carbonitrile or 2-((1-((S)-1-cyanoethyl)-3-isopropoxy-1H-pyrazol-4-yl)amino)-7-((3S,4R)-4-methyltetrahydrofuran-3-yl)-7H-pyrrolo[2,3-d]pyrimidine-6-carbonitrile or 2-((1-((R)-1-cyanoethyl)-3-isopropoxy-1H-pyrazol-4-yl)amino)-7-((3R,4S)-4-methyltetrahydrofuran-3-yl)-7H-pyrrolo[2,3-d]pyrimidine-6-carbonitrile or 2-((1-((S)-1-cyanoethyl)-3-isopropoxy-1H-pyrazol-4-yl)amino)-7-((3R,4S)-4-methyltetrahydrofuran-3-yl)-7H-pyrrolo[2,3-d]pyrimidine-6-carbonitrile (2^{nd} isomer of 1^{st} mixture) | NT | 274 * |
| 50 | 2-((1-((R)-1-Cyanoethyl)-3-isopropoxy-1H-pyrazol-4-yl)amino)-7-((3S,4R)-4-methyltetrahydrofuran-3-yl)-7H-pyrrolo[2,3-d]pyrimidine-6-carbonitrile or 2-((1-((S)-1-cyanoethyl)-3-isopropoxy-1H-pyrazol-4-yl)amino)-7-((3S,4R)-4-methyltetrahydrofuran-3-yl)-7H-pyrrolo[2,3-d]pyrimidine-6-carbonitrile or 2-((1-((R)-1-cyanoethyl)-3-isopropoxy-1H-pyrazol-4-yl)amino)-7-((3R,4S)-4-methyltetrahydrofuran-3-yl)-7H-pyrrolo[2,3-d]pyrimidine-6-carbonitrile or 2-((1-((S)-1-cyanoethyl)-3-isopropoxy-1H-pyrazol-4-yl)amino)-7-((3R,4S)-4-methyltetrahydrofuran-3-yl)-7H-pyrrolo[2,3-d]pyrimidine-6-carbonitrile (1^{st} isomer of 2^{nd} mixture) | NT | 7* |
| 51 | 2-((1-((R)-1-Cyanoethyl)-3-isopropoxy-1H-pyrazol-4-yl)amino)-7-((3S,4R)-4-methyltetrahydrofuran-3-yl)-7H-pyrrolo[2,3-d]pyrimidine-6-carbonitrile or 2-((1-((S)-1-cyanoethyl)-3-isopropoxy-1H-pyrazol-4-yl)amino)-7-((3S,4R)-4-methyltetrahydrofuran-3-yl)-7H-pyrrolo[2,3-d]pyrimidine-6-carbonitrile or 2-((1-((R)-1-cyanoethyl)-3-isopropoxy-1H-pyrazol-4-yl)amino)-7-((3R,4S)-4-methyltetrahydrofuran-3-yl)-7H-pyrrolo[2,3-d]pyrimidine-6-carbonitrile or 2-((1-((S)-1-cyanoethyl)-3-isopropoxy-1H-pyrazol-4-yl)amino)-7-((3R,4S)-4-methyltetrahydrofuran-3-yl)-7H-pyrrolo[2,3-d]pyrimidine-6-carbonitrile (2^{nd} isomer of 2^{nd} mixture) | NT | 5* |
| 52 | (R)-2-((1-methyl-3-(oxetan-3-yloxy)-1H-pyrazol-4-yl)amino)-7-(tetrahydrofuran-3-yl)-7H-pyrrolo[2,3-d]pyrimidine-6-carbonitrile or ((S)-2-((1-methyl-3-(oxetan-3-yloxy)-1H-pyrazol-4-yl)amino)-7-(tetrahydrofuran-3-yl)-7H-pyrrolo[2,3-d]pyrimidine-6-carbonitrile (1^{st} isomer) | NT | 26 * |
| 53 | (R)-2-((1-methyl-3-(oxetan-3-yloxy)-1H-pyrazol-4-yl)amino)-7-(tetrahyd rofu ran-3-yl)-7H-pyrrolo[2,3-d]pyrimidine-6-carbonitrile or ((S)-2-((1-methyl-3-(oxetan-3-yloxy)-1H-pyrazol-4-yl)amino)-7-(tetrahydrofuran-3-yl)-7H-pyrrolo[2,3-d]pyrimidine-6-carbonitrile (2^{nd} isomer) | NT | 266 * |
| 54 | 7-((3R,4R)-4-Methoxytetrahydrofuran-3-yl)-2-((1-(oxetan-3-yl)-3-((1,1,1-trifluoropropan-2-yl)oxy)-1H-pyrazol-4-yl)amino)-7H-pyrrolo[2,3-d]pyrimidine-6-carbonitrile | 27 | 61 |
| 55 | 2-((4-(2-hydroxypropan-2-yl)-2-(oxetan-3-yloxy)phenyl)amino)-7-((3R,4R)-4-methoxytetrahydrofuran-3-yl)-7H-pyrrolo[2,3-d]pyrimidine-6-carbonitrile | 18 | 41 |
| 56 | 2-((1-((S)-1-cyanoethyl)-3-isopropoxy-1H-pyrazol-4-yl)amino)-7-((3R,4R)-4-methoxytetrahydrofuran-3-yl)-7H-pyrrolo[2,3-d]pyrimidine-6-carbonitrile or 2-((1-((R)-1-cyanoethyl)-3-isopropoxy-1H-pyrazol-4-yl)amino)-7-((3R,4R)-4-methoxytetrahydrofuran-3-yl)-7H-pyrrolo[2,3-d]pyrimidine-6-carbonitrile (1^{st} isomer) | 4 | 7 |
| 57 | 2-((1-((S)-1-cyanoethyl)-3-isopropoxy-1H-pyrazol-4-yl)amino)-7-((3R,4R)-4-methoxytetrahydrofuran-3-yl)-7H-pyrrolo[2,3-d]pyrimidine-6-carbonitrile or 2-((1-((R)-1-cyanoethyl)-3-isopropoxy-1H-pyrazol-4-yl)amino)-7-((3R,4R)-4-methoxytetrahydrofuran-3-yl)-7H-pyrrolo[2,3-d]pyrimidine-6-carbonitrile (2^{nd} isomer) | NT | 27 * |
| 58 | 2-((1-(1-cyanoethyl)-3-cyclopropoxy-1H-pyrazol-4-yl)amino)-7-((3R,4R)-4-methoxytetrahydrofuran-3-yl)-7H-pyrrolo[2,3-d]pyrimidine-6-carbonitrile | NT | 15 * |
| 59 | 2-((1-(2-cyanopropan-2-yl)-3-cyclopropoxy-1H-pyrazol-4-yl)amino)-7-((3R,4R)-4-methoxytetrahyd rofuran-3-yl)-7H-pyrrolo[2,3-d]pyrimid ine-6-carbonitrile | NT | 31 * |
| 60 | 2-((1-(2-cyanopropan-2-yl)-3-isopropoxy-1H-pyrazol-4-yl)amino)-7-((3R,4R)-4-methoxytetrahydrofuran-3-yl)-7H-pyrrolo[2,3-d]pyrimidine-6-carbonitrile | NT | 42 * |
| 61 | 2-((3-cyclopropoxy-1-isopropyl-1H-pyrazol-4-yl)amino)-7-((3R,4R)-4-methoxytetrahydrofuran-3-yl)-7H-pyrrolo[2,3-d]pyrimidine-6-carbonitrile | NT | 133 * |
| 62 | 2-((3-isopropoxy-1-methyl-1H-pyrazol-4-yl)amino)-7-((3R,4R)-4-methoxytetrahydrofuran-3-yl)-7H-pyrrolo[2,3-d]pyrimidine-6-carbonitrile | NT | 114 * |
| 63 | 2-((3-isopropoxy-1-(oxetan-3-yl)-1H-pyrazol-4-yl)amino)-7-((3R,4R)-4-methoxytetrahydrofuran-3-yl)-7H-pyrrolo[2,3-d]pyrimidine-6-carbonitrile | NT | 201 * |
| 64 | 2-((3-cyclopropoxy-1-(oxetan-3-yl)-1H-pyrazol-4-yl)amino)-7-((3R,4R)-4-methoxytetrahydrofuran-3-yl)-7H-pyrrolo[2,3-d]pyrimidine-6-carbonitrile | NT | 135 * |
| 65 | 2-((3-(3,3-difluorocyclobutoxy)-1-methyl-1H-pyrazol-4-yl)amino)-7-((3R,4R)-4-methoxytetrahydrofuran-3-yl)-7H-pyrrolo[2,3-d]pyrimidine-6-carbonitrile | NT | 187 * |
| 66 | 2-((3-((R)-sec-butoxy)-1-methyl-1H-pyrazol-4-yl)amino)-7-((3R,4R)-4-methoxytetrahydrofuran-3-yl)-7H-pyrrolo[2,3-d]pyrimidine-6-carbonitrile | NT | 119 * |
| 67 | 2-((3-((S)-sec-butoxy)-1-methyl-1H-pyrazol-4-yl)amino)-7-((3R,4R)-4-methoxytetrahydrofuran-3-yl)-7H-pyrrolo[2,3-d]pyrimidine-6-carbonitrile | NT | 297 * |
| 68 | 2-((2,2-dimethyl-3,4-dihydro-2H-pyrano[3,2-b]pyridin-8-yl)amino)-7-((3R,4R)-4-methoxytetrahydrofuran-3-yl)-7H-pyrrolo[2,3-d]pyrimidine-6-carbonitrile | NT | > 1000 * |
| 69 | 7-((3R,4R)-4-methoxytetrahydrofuran-3-yl)-2-(((R)-2-methyl-3,4-dihydro-2H-[1,4]dioxepino[2,3-b]pyridin-9-yl)amino)-7H-pyrrolo[2,3-d]pyrimidine-6-carbonitrile | NT | 597 * |
| 70 | 7-((trans)-4-methoxytetrahydrofuran-3-yl)-2-((1-methyl-3-(oxetan-3-yloxy)-1H-pyrazol-4-yl)amino)-7H-pyrrolo[2,3-d]pyrimidine-6-carbonitrile | 160 | 270 |
| 71 | 2-((4-(2-hydroxypropan-2-yl)-2-(oxetan-3-yloxy)phenyl)amino)-7-((trans)-4-methoxytetrahydrofuran-3-yl)-7H-pyrrolo[2,3-d]pyrimidine-6-carbonitrile | 69 | 109 |
| 72 | 7-((cis)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile | NT | 83 * |
| 73 | 7-((trans)-4-ethyltetrahydrofuran-3-yl)-2-((3-isopropoxy-1-methyl-1H-pyrazol-4-yl)amino)-7H-pyrrolo[2,3-d]pyrimidine-6-carbonitrile | NT | 11 * |
| 74 | 2-((1-(2-cyanopropan-2-yl)-3-isopropoxy-1H-pyrazol-4-yl)amino)-7-(oxetan-3-yl)-7H-pyrrolo[2,3-d]pyrimidine-6-carbonitrile | 14 | 20 |
| 75 | 2-((3-isopropoxy-1-methyl-1H-pyrazol-4-yl)amino)-7-(oxetan-3-yl)-7H-pyrrolo[2,3-d]pyrimidine-6-carbonitrile | NT | 44 * |
| 76 | 7-[(2S,3R)-2-methyloxetan-3-yl]-2-[[1-methyl-3-(oxetan-3-yloxy)pyrazol-4-yl]amino]pyrrolo[2,3-d]pyrimidine-6-carbonitrile or 7-[(2R,3S)-2-methyloxetan-3-yl]-2-[[1-methyl-3-(oxetan-3-yloxy)pyrazol-4-yl]amino]pyrrolo[2,3-d]pyrimidine-6-carbonitrile (1^{st} isomer) | 200 | 241 |
| 77 | 7-[(2S,3R)-2-methyloxetan-3-yl]-2-[[1-methyl-3-(oxetan-3-yloxy)pyrazol-4-yl]amino]pyrrolo[2,3-d]pyrimidine-6-carbonitrile or 7-[(2R,3S)-2-methyloxetan-3-yl]-2-[[1-methyl-3-(oxetan-3-yloxy)pyrazol-4-yl]amino]pyrrolo[2,3-d]pyrimidine-6-carbonitrile (2^{nd} isomer) | 21 | 34 |
| 78 | 7-[(2R,3S)-2-methyloxetan-3-yl]-2-[(1-methyl-3-propan-2-yloxypyrazol-4-yl)amino]pyrrolo[2,3-d]pyrimidine-6-carbonitrile or 7-[(2S,3R)-2-methyloxetan-3-yl]-2-[(1-methyl-3-propan-2-yloxypyrazol-4-yl)amino]pyrrolo[2,3-d]pyrimidine-6-carbonitrile (1^{st} isomer) | 64 | 79 |
| 79 | 7-[(2R,3S)-2-methyloxetan-3-yl]-2-[(1-methyl-3-propan-2-yloxypyrazol-4-yl)amino]pyrrolo[2,3-d]pyrimidine-6-carbonitrile or 7-[(2S,3R)-2-methyloxetan-3-yl]-2-[(1-methyl-3-propan-2-yloxypyrazol-4-yl)amino]pyrrolo[2,3-d]pyrimidine-6-carbonitrile (2^{nd} isomer) | 6 | 9 |
| 80 | 2-((1-methyl-3-(oxetan-3-yloxy)-1H-pyrazol-4-yl)amino)-7-(tetrahydro-2H-pyran-4-yl)-7H-pyrrolo[2,3-d]pyrimidine-6-carbonitrile | NT | 20 * |
| 81 | (R)-2-((1-Methyl-3-(oxetan-3-yloxy)-1H-pyrazol-4-yl)amino)-7-(tetrahydro-2H-pyran-3-yl)-7H-pyrrolo[2,3-d]pyrimidine-6-carbonitrile or (S)-2-((1-methyl-3-(oxetan-3-yloxy)-1H-pyrazol-4-yl)amino)-7-(tetrahydro-2H-pyran-3-yl)-7H-pyrrolo[2,3-d]pyrimidine-6-carbonitrile (1^{st} isomer) | NT | 183 * |
| 82 | (R)-2-((1-Methyl-3-(oxetan-3-yloxy)-1H-pyrazol-4-yl)amino)-7-(tetrahydro-2H-pyran-3-yl)-7H-pyrrolo[2,3-d]pyrimidine-6-carbonitrile or (S)-2-((1-methyl-3-(oxetan-3-yloxy)-1H-pyrazol-4-yl)amino)-7 -(tetrahydro-2 H-pyran-3-yl)-7H-pyrrolo[2, 3-d]pyrimidine-6-carbonitrile (2^{nd} isomer) | NT | 104 * |
| 83 | 2-((1-methyl-3-(oxetan-3-yloxy)-1H-pyrazol-4-yl)amino)-7-((3R,4S)-3-methyltetrahydro-2H-pyran-4-yl)-7H-pyrrolo[2,3-d]pyrimidine-6-carbonitrile or 2-((1-methyl-3-(oxetan-3-yloxy)-1H-pyrazol-4-yl)amino)-7-((3S,4R)-3-methyltetrahydro-2H-pyran-4-yl)-7H-pyrrolo[2,3-d]pyrimidine-6-carbonitrile (1isomer) | NT | 134 * |
| 84 | 2-((1-methyl-3-(oxetan-3-yloxy)-1H-pyrazol-4-yl)amino)-7-((3R,4S)-3-methyltetrahydro-2H-pyran-4-yl)-7H-pyrrolo[2,3-d]pyrimidine-6-carbonitrile or 2-((1-methyl-3-(oxetan-3-yloxy)-1H-pyrazol-4-yl)amino)-7-((3S,4R)-3-methyltetrahydro-2H-pyran-4-yl)-7H-pyrrolo[2,3-d]pyrimidine-6-carbonitrile (2^{nd} isomer) | NT | 7 * |
| 85 | 2-((1-methyl-3-(oxetan-3-yloxy)-1H-pyrazol-4-yl)amino)-7-((3R,4R)-3-methyltetrahydro-2H-pyran-4-yl)-7H-pyrrolo[2,3-d]pyrimidine-6-carbonitrile or 2-((1-methyl-3-(oxetan-3-yloxy)-1H-pyrazol-4-yl)amino)-7-((3S,4S)-3-methyltetrahydro-2H-pyran-4-yl)-7H-pyrrolo[2,3-d]pyrimidine-6-carbonitrile (1^{st} isomer) | NT | 41 * |
| 86 | 2-((1-methyl-3-(oxetan-3-yloxy)-1H-pyrazol-4-yl)amino)-7-((3R,4R)-3-methyltetrahydro-2H-pyran-4-yl)-7H-pyrrolo[2,3-d]pyrimidine-6-carbonitrile or 2-((1-methyl-3-(oxetan-3-yloxy)-1H-pyrazol-4-yl)amino)-7-((3S,4S)-3-methyltetrahydro-2H-pyran-4-yl)-7H-pyrrolo[2,3-d]pyrimidine-6-carbonitrile (2^{nd} isomer) | NT | 435 (* |
| 87 | 7-[(1S)-2-methoxy-1-methyl-ethyl]-2-[[1-methyl-3-[(2S, 3R)-2-methyloxetan-3-yl]oxy-pyrazol-4-yl]amino]pyrrolo[2,3-d]pyrimidine-6-carbonitri le or 7-[(1S)-2-methoxy-1-methyl-ethyl]-2-[[1-methyl-3-[(2R, 3S)-2-methyloxetan-3-yl]oxy-pyrazol-4-yl]amino]pyrrolo[2,3-d]pyrimidine-6-carbonitrile (1^{st} isomer) | 5 | 8 |
| 88 | 7-[(1S)-2-methoxy-1-methyl-ethyl]-2-[[1-methyl-3-[(2S, 3R)-2-methyloxetan-3-yl]oxy-pyrazol-4-yl]amino]pyrrolo[2,3-d]pyrimidine-6-carbonitrile or 7-[(1S)-2-methoxy-1-methyl-ethyl]-2-[[1-methyl-3-[(2R, 3S)-2-methyloxetan-3-yl]oxy-pyrazol-4-yl]amino]pyrrolo[2,3-d]pyrimidine-6-carbonitrile (2^{nd} isomer) | 12 | 20 |
| 89 | 7-[(1S)-2-methoxy-1-methyl-ethyl]-2-[[3-[(2S,3R)-2-methyloxetan-3-yl]oxy-1-(methyl-d3)pyrazol-4-yl]amino]pyrrolo[2,3-d]pyrimidine-6-carbonitrile or 7-[(1S)-2-methoxy-1-methyl-ethyl]-2-[[3-[(2R,3S)-2-methyloxetan-3-yl]oxy-1-(methyl-d3)pyrazol-4-yl]amino]pyrrolo[2,3-d]pyrimidine-6-carbonitrile (1^{st} isomer) | 5 | 9 |
| 90 | 7-[(1S)-2-methoxy-1-methyl-ethyl]-2-[[3-[(2S,3R)-2-methyloxetan-3-yl]oxy-1-(methyl-d3)pyrazol-4-yl]amino]pyrrolo[2,3-d]pyrimidine-6-carbonitrile or 7-[(1S)-2-methoxy-1-methyl-ethyl]-2-[[3-[(2R,3S)-2-methyloxetan-3-yl]oxy-1-(methyl-d3)pyrazol-4-yl]amino]pyrrolo[2,3-d]pyrimidine-6-carbonitrile (2^{nd} isomer) | 8 | 16 |
| 91 | 7-[(2S)-1-methoxypropan-2-yl]-2-[[3-(oxetan-3-yloxy)-1-(methyl-d3)pyrazol-4-yl]amino]pyrrolo[2,3-d]pyrimidine-6-carbonitrile | 7 | 13 |
| 92 | 2-[[3-(cyclopropoxy)-1-(methoxymethyl)pyrazol-4-yl]amino]-7-[(1S)-2-methoxy-1-methyl-ethyl]pyrrolo[2,3-d]pyrimidine-6-carbonitrile | 4 | 6 |
| 93 | 7-[(1S)-2-methoxy-1-methyl-ethyl]-2-[[1-(methylsulfonylmethyl)-3-(oxetan-3-yloxy)pyrazol-4-yl]amino]pyrrolo[2,3-d]pyrimidine-6-carbonitrile | 4 | 8 |
| 94 | 2-((1-methyl-3-(oxetan-3-yloxy)-1H-pyrazol-4-yl-5-d)amino)-7-((3R,4R)-4-methyltetrahydrofuran-3-yl)-7H-pyrrolo[2,3-d]pyrimidine-6-carbonitrile | NT | 7 * |

| | | | |
|---|---|---|---|
| "*" data obtained with assay B. "NT" not tested. | | | |

The compounds of formula I and pharmaceutically acceptable salts thereof may be used for the preparation of a medicament for treating a neurodegenerative disease selected from the group consisting of Parkinson's disease, multiple sclerosis, Lewy body dementia, HIV-induced dementia, amyotrophic lateral sclerosis, Pick disease, progressive supranuclear palsy, and frontotemporal dementia.

According to one of its aspects, the disclosure relates to a compound of formula (I), or a pharmaceutically acceptable salt thereof, for treating a disease or disorder related to LRRK2 kinase.

According to one of its aspects, the disclosure, relates to a compound of formula (I), or a pharmaceutically acceptable salt thereof, for treating a neurodegenerative disease selected from the group consisting of Parkinson's disease, multiple sclerosis, Lewy body dementia, HIV-induced dementia, amyotrophic lateral sclerosis, Pick disease, progressive supranuclear palsy, and frontotemporal dementia.

According to another of its aspects, the present disclosure relates to pharmaceutical compositions comprising a compound of formula (I), or a pharmaceutically acceptable salt thereof, and, optionally one or more pharmaceutically acceptable excipients. In some aspects, a pharmaceutical composition contains an effective dose of at least one compound of formula (I) or a pharmaceutically acceptable salt thereof, and also optionally at least one pharmaceutically acceptable excipient.

In some aspects, the pharmaceutical compositions for oral or sublingual administration, the compound of formula (I) above, or pharmaceutically acceptable salt thereof, may be administered in unit administration form, as a mixture with one or more pharmaceutically acceptable excipients, to patients for the treatment of the above disorders or diseases.

By way of example, a unit administration form of a compound according to the disclosure in tablet form may comprise the following components:

| | |
|---|---|
| Compound disclosed herein | 50.0 mg |
| Mannitol | 223.75 mg |
| Croscarmellose sodium | 6.0 mg |
| Corn starch | 15.0 mg |
| Hydroxypropylmethylcellulose | 2.25 mg |
| Magnesium stearate | 3.0 mg |

Via the oral route, the dose of active principle administered per day may range from 0.1 to 20 mg/kg, in one or more dosage intakes.

According to the usual practice, the dosage that is appropriate to each patient is determined by the practitioner according to the mode of administration and the weight and response of the said patient.

According to another of its aspects, the present disclosure also relates to a method for treating a neurodegenerative disease selected from the group consisting of Parkinson's disease, multiple sclerosis, Lewy body dementia, HIV-induced dementia, amyotrophic lateral sclerosis, Pick disease, progressive supranuclear palsy, and frontotemporal dementia, comprising administering to a patient in need thereof an effective dose of a compound according of formula (I) or a pharmaceutically acceptable salt thereof.

## Claims

1. A compound of Formula (I) 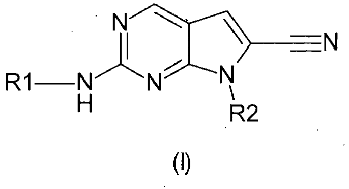 wherein:
R1 is selected from the group consisting of an aryl group, an ortho-fused bicyclic heteroaryl group and a heteroaryl group, wherein said ortho-fused bicyclic heteroaryl group is unsubstituted or substituted with one or more -(C₁-C₃)-alkyl group; and wherein said aryl and heteroaryl groups are unsubstituted or substituted with one or more substituents independently selected from the group consisting of
a) a deuterium atom,
b) a fluorine atom,
c) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
d) a cycloalkyl group which is unsubstituted or substituted with one or more fluorine atom or lower alkyl group,
e) a heterocycloalkyl group which is unsubstituted or substituted with one or more substituents independently selected from an alkyl group, an alkyloxyl group, and an alkylcarbonyl group,
f) an alkyloxyl group which is unsubstituted or substituted with one or more substituents independently selected from the group consisting of a hydroxy group and a fluorine atom,
g) an -O-cycloalkyl group which is unsubstituted or substituted with one or more fluorine atom or lower alkyl group,
h) an -O-heterocycloalkyl group which is unsubstituted or substituted with one or more substituents independently selected from the group consisting of an alkyl group, an alkyloxyl group, and an alkylcarbonyl group,
i) an -O-spirocycle group,
j) an alkylsulfonylalkyl group, and
k) an alkylsulfonyl group; and
R2 is selected from the group consisting an alkyloxylalkyl group and a heterocycloalkyl group, wherein said heterocycloalkyl group represented by R2 is attached via a carbon atom and is unsubstituted or substituted with an alkyl group, an alkyloxyl group, or one or more fluorine;
or a pharmaceutically acceptable salt thereof.

2. The compound of claim 1, wherein
R1 is selected from the group consisting of a phenyl group and a heteroaryl group, wherein said phenyl and heteroaryl groups are unsubstituted or substituted with one or more substituents independently selected from the group consisting of
a) a fluorine atom,
b) an alkyl group which is unsubstituted or substituted with one or more substituents independently selected from the group consisting of a hydroxy group, a fluorine atom, a deuterium atom, a cyano group, an.alkyloxyl group, an alkylamino group, and a dialkylamino group,
c) a cycloalkyl group which is unsubstituted or substituted with one or more fluorine atom or lower alkyl group,
d) a heterocycloalkyl group which is unsubstituted or substituted with one or more substituents independently selected from an alkyl group, an alkyloxyl group, and an alkylcarbonyl group,
e) an alkyloxyl group which is unsubstituted or substituted with one or more substituents independently selected from the group consisting of a hydroxy group and a fluorine atom,
f) an -O-cycloalkyl group which is unsubstituted or substituted with one or more fluorine atom or lower alkyl group,
g) an -O-heterocycloalkyl group which is unsubstituted or substituted with one or more substituents independently selected from the group consisting of an alkyl group, an alkyloxyl group, and an alkylcarbonyl group,
h) an -O-spirocycle group,
i) an alkylsulfonylalkyl group, and
j) an alkylsulfonyl group;
or a pharmaceutically acceptable salt thereof.

3. The compound of claim 1, having formula (Ia) 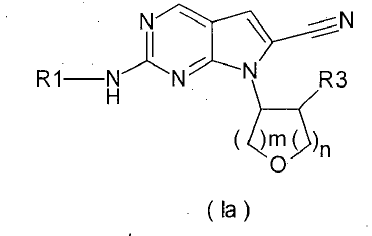 wherein:
R3 is selected from the group consisting a hydrogen atom, a -(C₁-C₃)-alkyl group and a -(C₁-C₃)-alkyloxyl group;
m represents 1, 2 or 3; and
n represents 0 or 1;
or a pharmaceutically acceptable salt thereof.

4. The compound of claim 1 or claim 2, having formula (Ia) 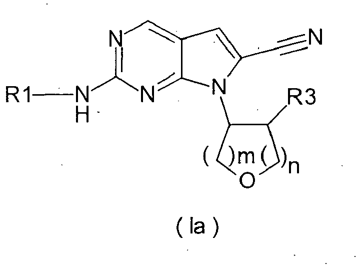 wherein
R1 is selected from the group consisting of a phenyl group and a heteroaryl group, wherein said phenyl and heteroaryl groups are unsubstituted or substituted with one or more substituents independently selected from the group consisting of
a) a fluorine atom,
b) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
c) a cycloalkyl group which is unsubstituted or substituted with one or more fluorine atom or lower alkyl group,
d) a heterocycloalkyl group which is unsubstituted or substituted with one or more substituents independently selected from an alkyl group, an alkyloxyl group, and an alkylcarbonyl group,
e) an alkyloxyl group which is unsubstituted or substituted with one or more substituents independently selected from the group consisting of a hydroxy group and a fluorine atom,
f) an -O-cycloalkyl group which is unsubstituted or substituted with one or more fluorine atom or lower alkyl group,
g) an -O-heterocycloalkyl group which is unsubstituted or substituted with one or more substituents independently selected from the group consisting of an alkyl group, an alkyloxyl group, and an alkylcarbonyl group,
h) an -O-spirocycle group,
i) an alkylsulfonylalkyl group, and
j) an alkylsulfonyl group;
m represents 1 or 2; and
n represents 0 or 1;
or a pharmaceutically acceptable salt thereof.

5. The compound of any one of claims 1 to 3, having formula (lb) 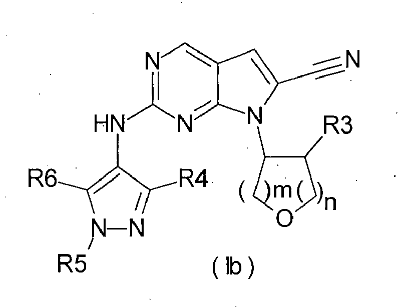 wherein:
R3 is selected from the group consisting a hydrogen atom, a -(C₁-C₃)-alkyl group and a -(C₁-C₃)-alkyloxyl group;
R4 is selected from the group consisting of
a) an alkyloxyl group which is unsubstituted or substituted with one or more substituents independently selected from the group consisting of a hydroxy group and a fluorine atom,
b) an -O-cycloalkyl group which is unsubstituted or substituted with one or more fluorine atom or lower alkyl group,
c) an -O-heterocycloalkyl group which is unsubstituted or substituted with one or more substituents independently selected from the group consisting of an alkyl group, an alkyloxyl group, and an alkylcarbonyl group, and
d) an -O-spirocycle group;
R5 is selected from the group consisting of
a) a hydrogen atom,
b) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
c) a cycloalkyl group which is unsubstituted or substituted with one or more fluorine atom or lower alkyl group,
d) a heterocycloalkyl group which is unsubstituted or substituted with one or more substituents independently selected from an alkyl group, an alkyloxyl group, and an alkylcarbonyl group,
e) an alkylsulfonylalkyl group, and
f) an alkylsulfonyl group;
R6 is selected from the group consisting a hydrogen atom and a deuterium atom;
m represents 1, 2 or 3; and
n represents 0 or 1;
or a pharmaceutically acceptable salt thereof.

6. The compound of claim 5, wherein
R3 is selected from the group consisting a -(C₁-C₃)-alkyl group and a -(C₁-C₃)-alkyloxyl group;
R6 is a hydrogen atom;
m represents 1 or 2; and
n represents 0 or 1;
or a pharmaceutically acceptable salt thereof.

7. The compound of claim 5, wherein
R3 is selected from the group consisting a -(C₁-C₃)-alkyl group and a -(C₁-C₃)-alkyloxyl group;
R4 is selected from the group consisting of
a) an alkyloxyl group which is unsubstituted or substituted with one or more substituents independently selected from the group consisting of a hydroxy group and a fluorine atom,
b) an -O-cycloalkyl group which is unsubstituted or substituted with one or more fluorine atom or lower alkyl group, and
c) an -O-heterocycloalkyl group which is unsubstituted or substituted with one or more substituents independently selected from the group consisting of an alkyl group, an alkyloxyl group, and an alkylcarbonyl group;
R5 is selected from the group consisting of
a) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
b) a cycloalkyl group which is unsubstituted or substituted with one or more fluorine atom or lower alkyl group, and
c) a heterocycloalkyl group which is unsubstituted or substituted with one or more substituents independently selected from an alkyl group, an alkyloxyl group, and an alkylcarbonyl group ;
R6 is a hydrogen atom;
m represents 1; and
n represents 1;
or a pharmaceutically acceptable salt thereof.

8. The compound of claim 5, wherein
R3 is a -(C₁-C₃)-alkyl group;
R4 is selected from the group consisting of
a) an -O-cycloalkyl group which is unsubstituted or substituted with one or more fluorine atom or lower alkyl group, and
b) an -O-heterocycloalkyl group which is unsubstituted or substituted with one or more substituents independently selected from the group consisting of an alkyl group, an alkyloxyl group, and an alkylcarbonyl group,
R5 is an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group;
R6 is a hydrogen atom;
m represents 1; and
n represents 1;
or a pharmaceutically acceptable salt thereof.

9. The compound of claim 1, having formula (Ic) 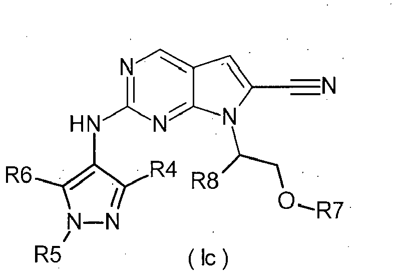 wherein:
R4 is selected from the group consisting of
a) an alkyloxyl group which is unsubstituted or substituted with one or more substituents independently selected from the group consisting of a hydroxy group and a fluorine atom,
b) an -O-cycloalkyl group which is unsubstituted or substituted with one or more fluorine atom or lower alkyl group,
c) an -O-heterocycloalkyl group which is unsubstituted or substituted with one or more substituents independently selected from the group consisting of an alkyl group, an alkyloxyl group, and an alkylcarbonyl group, and
d) an -O-spirocycle group;
R5 is selected from the group consisting of
a) a hydrogen atom,
b) an alkyl group which is unsubstituted or substituted with one or more substituents independently selected from the group consisting of a hydroxy group, a fluorine atom, a deuterium atom, a cyano group, an alkyloxyl group, an alkylamino group, and a dialkylamino group,
c) a cycloalkyl group which is unsubstituted or substituted with one or more fluorine atom or lower alkyl group,
d) a heterocycloalkyl group which is unsubstituted or substituted with one or more substituents independently selected from an alkyl group, an alkyloxyl group, and an alkylcarbonyl group,
e) an alkylsulfonylalkyl group, and
f) an alkylsulfonyl group;
R6 is selected from the group consisting a hydrogen atom and a deuterium atom;
R7 is a -(C₁-C₃)-alkyl group; and
R8 is a -(C₁-C₃)-alkyl group;
or a pharmaceutically acceptable salt thereof.

10. A compound selected from the group consisting of:
2-((1-methyl-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-methyl-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-[[1-methyl-3-(oxetan-3-yloxy)pyrazol-4-yl]amino]-7-[(3R,4R)-4-methyltetrahydrofuran-3-yl]pyrrolo[2,3-d]pyrimidine-6-carbonitrile;
2-((1-(methyl-d3)-3-(((2R,3S)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-(methyl-d3)-3-(((2S,3R)-2-methyloxetan-3-yl)oxy)-1H-pyrazol-4-yl)amino)-7-((3R,4R)-4-methyltetrahydrofuran-3-yl)-7H-pyrrolo[2,3-d]pyrimidine-6-carbonitrile;
2-((1-(methyl-d3)-3-(oxetan-3-yloxy)-1H-pyrazol-4-yl)amino)-7-((3R,4R)-4-methyltetrahydrofuran-3-yl)-7H-pyrrolo[2,3-d]pyrimidine-6-carbonitrile;
2-((3-isopropoxy-1-methyl-1H-pyrazol-4-yl)amino)-7-((3R,4R)-4-methyltetrahydrofuran-3-yl)-7H-pyrrolo[2,3-d]pyrimidine-6-carbonitrile;
2-((1-methyl-3-(oxetan-3-yloxy)-1H-pyrazol-4-yl)amino)-7-(tetrahydro-2H-pyran-4-yl)-7H-pyrrolo[2,3-d]pyrimidine-6-carbonitrile;
2-((1-methyl-3-(oxetan-3-yloxy)-1H-pyrazol-4-yl)amino)-7-((3R,4S)-3-methyltetrahydro-2H-pyran-4-yl)-7H-pyrrolo[2,3-d]pyrimidine-6-carbonitrile;
2-((1-methyl-3-(oxetan-3-yloxy)-1H-pyrazol-4-yl)amino)-7-((3S,4R)-3-methyltetrahydro-2H-pyran-4-yl)-7H-pyrrolo[2,3-d]pyrimidine-6-carbonitrile;
7-[(1S)-2-methoxy-1-methyl-ethyl]-2-[[1-methyl-3-[(2S,3R)-2-methyloxetan-3-yl]oxy-pyrazol-4-yl]amino]pyrrolo[2,3-d]pyrimidine-6-carbonitrile;
7-[(1S)-2-methoxy-1-methyl-ethyl]-2-[[1-methyl-3-[(2R,3S)-2-methyloxetan-3-yl]oxy-pyrazol-4-yl]amino]pyrrolo[2,3-d]pyrimidine-6-carbonitrile;
7-[(1S)-2-methoxy-1methyl-ethyl]-2-[[3-[(2S,3R)-2-methyloxetan-3-yl]oxy-1-(methyl-d3)pyrazol-4-yl]amino]pyrrolo[2,3-d]pyrimidine-6-carbonitrile;
7-[(1S)-2-methoxy-1-methyl-ethyl]-2-[[3-[(2R,3S)-2-methyloxetan-3-yl]oxy-1-(methyl-d3)pyrazol-4-yl]amino]pyrrolo[2,3-d]pyrimidine-6-carbonitrile; and
2-[[3-(cyclopropoxy)-1-(methoxymethyl)pyrazol-4-yl]amino]-7-[(1S)-2-methoxy-1-methyl-ethyl]pyrrolo[2,3-d]pyrimidine-6-carbonitrile;
or a pharmaceutically acceptable salt thereof.

11. A process for preparing a compound according to claim 1, comprising reacting a compound of formula (11X) with a compound of formula (15X): 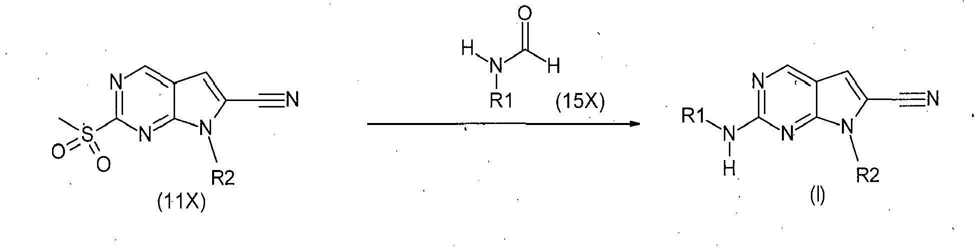 wherein R1 and R2 are as defined for a compound of formula (I) in claim 1.

12. A pharmaceutical composition comprising a compound of any one of claims 1 to 10 or a pharmaceutically acceptable salt thereof, and a pharmaceutically acceptable excipient.

13. A method for treating a neurodegenerative disease, said method comprising administering to a patient in need of said treatment a pharmaceutically effective amount of the compound of any one of claims 1 to 10.

14. The method of claim 13, wherein the neurodegenerative disease is selected from the group consisting of Parkinson's disease, multiple sclerosis, HIV-induced dementia, amyotrophic lateral sclerosis, Lewy body dementia, Pick disease, progressive supranuclear palsy, and frontotemporal dementia.

15. The method of claim 13, wherein the neurodegenerative disease is Parkinson's disease.

16. A medicament, **characterized in that** it comprises a compound of formula (I) according to any one of claims 1 to 10, or a pharmaceutically acceptable salt thereof.

17. A compound of formula (I) according to any one of claims 1 to 10, or a pharmaceutically acceptable salt thereof, for use in the treatment of a neurodegenerative disease.

18. A compound of formula (I) according to claim 17, or a pharmaceutically acceptable salt thereof, wherein the neurodegenerative disease is selected from the group consisting of Parkinson's disease, multiple sclerosis, HIV-induced dementia, amyotrophic lateral sclerosis, Lewy body dementia, Pick disease, progressive supranuclear palsy, and frontotemporal dementia.

19. A compound of formula (I) according to claim 17, or a pharmaceutically acceptable salt thereof, wherein the neurodegenerative disease is Parkinson' s disease.
